# EUROPEAN PATENT SPECIFICATION

(11) **EP 3 075 843 B1**
(45) Date of publication and mention of the grant of the patent: **03.07.2019**
(21) Application number: 14865818.0
(22) Date of filing: 28.11.2014
(51) Int. Cl.: C12N 1/21, C12N 15/09, C12P 5/00

(54) **METHOD FOR PRODUCING ISOPRENE MONOMER**
VERFAHREN ZUR HERSTELLUNG VON ISOPREN-MONOMER
PROCÉDÉ DE PRODUCTION DE MONOMÈRE ISOPRÈNE

(30) Priority: 28.11.2013 JP 2013246350
(43) Date of publication of application: 05.10.2016
(73) Proprietor: Ajinomoto Co., Inc., Tokyo 104-8315 (JP)
(72) Inventor: TAJIMA, Yoshinori, Kawasaki-shi Kanagawa 210-8681 (JP); OONUKI, Akiko, Kawasaki-shi Kanagawa 210-8681 (JP); RACHI, Hiroaki, Kawasaki-shi Kanagawa 210-8681 (JP)
(74) Representative: Grünecker Patent- und Rechtsanwälte PartG mbB
(86) International application number: PCT/JP2014/081645
(87) International publication number: WO 2015/080273

(56) References cited:
- WO-A1-2012/088462
- WO-A1-2012/088462
- WO-A1-2013/179722
- WO-A2-2013/166211
- JP-A- 2011 505 841
- JP-A- 2012 516 677
- JP-A- 2013 216 850
- JP-A- 2013 216 850
- MARY C. WILDERMUTH ET AL.: 'Biochemical Characterization of Stromal and Thylakoid-Bound Isoforms of Isoprene Synthase in Willow Leaves' PLANT PHYSIOLOGY vol. 116, 1998, pages 1111 - 1123, XP055346242

### SEQUENCE LISTING

<110> Ajinomoto Co., Inc.
<120> METHOD OF PRODUCING ISOPRENE MONOMER
<130> PAMA-261022
<150> JP2013-246350
   <151> 2013-11-28
<160> 184
<170> PatentIn version 3.5
<210> 1
   <211> 2414
   <212> DNA
   <213> Artificial Sequence
<220>
   <223> Genomic fragment containing lambda attL (Lambda phage attachment site-Left) - Tet (tetracycline-resistance marker) - lambda attR (Lambda phage attachment site-Right) -Ptac (tac promoter)
<400> 1
   tgaagcctgc ttttttatac taagttggca ttataaaaaa gcattgctta tcaatttgtt      60
   gcaacgaaca ggtcactatc agtcaaaata aaatcattat ttgatttcga attccccgga     120
   tccgtcgacc tgcagggaaa aaggttatgc tgcttttaag acccactttc acatttaagt     180
   tgtttttcta atccgcatat gatcaattca aggccgaata agaaggctgg ctctgcacct     240
   tggtgatcaa ataattcgat agcttgtcgt aataatggcg gcatactatc agtagtaggt     300
   gtttcccttt cttctttagc gacttgatgc tcttgatctt ccaatacgca acctaaagta     360
   aaatgcccca cagcgctgag tgcatataat gcattctcta gtgaaaaacc ttgttggcat     420
   aaaaaggcta attgattttc gagagtttca tactgttttt ctgtaggccg tgtacctaaa     480
   tgtacttttg ctccatcgcg atgacttagt aaagcacatc taaaactttt agcgttatta     540
   cgtaaaaaat cttgccagct ttccccttct aaagggcaaa agtgagtatg gtgcctatct     600
   aacatctcaa tggctaaggc gtcgagcaaa gcccgcttat tttttacatg ccaatacaat     660
   gtaggctgct ctacacctag cttctgggcg agtttacggg ttgttaaacc ttcgattccg     720
   acctcattaa gcagctctaa tgcgctgtta atcactttac ttttatctaa tctagacatc     780
   attaattcct aatttttgtt gacactctat cattgataga gttattttac cactccctat     840
   cagtgataga gaaaagtgaa atgaatagtt cgacaaagat cgcattggta attacgttac     900
   tcgatgccat ggggattggc cttatcatgc cagtcttgcc aacgttatta cgtgaattta     960
   ttgcttcgga agatatcgct aaccactttg gcgtattgct tgcactttat gcgttaatgc    1020
   aggttatctt tgctccttgg cttggaaaaa tgtctgaccg atttggtcgg cgcccagtgc    1080
   tgttgttgtc attaataggc gcatcgctgg attacttatt gctggctttt tcaagtgcgc    1140
   tttggatgct gtatttaggc cgtttgcttt cagggatcac aggagctact ggggctgtcg   1200
   cggcatcggt cattgccgat accacctcag cttctcaacg cgtgaagtgg ttcggttggt   1260
   taggggcaag ttttgggctt ggtttaatag cggggcctat tattggtggt tttgcaggag   1320
   agatttcacc gcatagtccc ttttttatcg ctgcgttgct aaatattgtc actttccttg   1380
   tggttatgtt ttggttccgt gaaaccaaaa atacacgtga taatacagat accgaagtag   1440
   gggttgagac gcaatcgaat tcggtataca tcactttatt taaaacgatg cccattttgt   1500
   tgattattta tttttcagcg caattgatag gccaaattcc cgcaacggtg tgggtgctat   1560
   ttaccgaaaa tcgttttgga tggaatagca tgatggttgg cttttcatta gcgggtcttg   1620
   gtcttttaca ctcagtattc caagcctttg tggcaggaag aatagccact aaatggggcg   1680
   aaaaaacggc agtactgctc gaatttattg cagatagtag tgcatttgcc tttttagcgt   1740
   ttatatctga aggttggtta gatttccctg ttttaatttt attggctggt ggtgggatcg   1800
   ctttacctgc attacaggga gtgatgtcta tccaaacaaa gagtcatgag caaggtgctt   1860
   tacagggatt attggtgagc cttaccaatg caaccggtgt tattggccca ttactgttta   1920
   ctgttattta taatcattca ctaccaattt gggatggctg gatttggatt attggtttag   1980
   cgttttactg tattattatc ctgctatcga tgaccttcat gttaacccct caagctcagg   2040
   ggagtaaaca ggagacaagt gcttagttat ttcgtcacca aatgatgtta ttccgcgaaa   2100
   tataatgacc ctcttgataa cccaagaggg cattttttac gataaagaag atttagcttc   2160
   tgcagtctgt tacaggtcac taataccatc taagtagttg attcatagtg actgcatatg   2220
   ttgtgtttta cagtattatg tagtctgttt tttatgcaaa atctaattta atatattgat   2280
   atttatatca ttttacgttt ctcgttcagc ttttttatac taacttgagc gagatctccc   2340
   tgttgacaat taatcatcgg ctctataatg tgtggaatcg tgagcggata acaatttcac   2400
   acaaggagac tgcc                                                     2414
<210> 2
   <211> 89
   <212> DNA
   <213> Artificial Sequence
<220>
   <223> Primer 1 for replacing a promoter region of ppa gene
<400> 2
   ttgtaatcgc tttcatctca ctatgaaaaa tgcggctacg gttatggatt ttcctgctct    60
   tgaagcctgc ttttttatac taagttggc                                       89
<210> 3
   <211> 89
   <212> DNA
   <213> Artificial Sequence
<220>
   <223> Primer 2 for replacing a promoter region of ppa gene
<400> 3
   aataacaacg tagatgtctt ccggcagatc tttacccgca gggacgttga gtaagctcat 60
   agctgtttcc tgtgtgaaat tgttatccg 89
<210> 4
   <211> 24
   <212> DNA
   <213> Artificial Sequence
<220>
   <223> Primer 1 for confirming replacement of a promoter region of ppa gene with tac promoter
<400> 4
   acagattgcg ttacatcaca cttc 24
<210> 5
   <211> 24
   <212> DNA
   <213> Artificial Sequence
<220>
   <223> Primer 2 for confirming replacement of a promoter region of ppa gene with tac promoter
<400> 5
   gtatttgatc ggatctgcgt tagc 24
<210> 6
   <211> 1785
   <212> DNA
   <213> Mucuna bracteata
<220>
   <221> CDS
   <222> (1)..(1785)
<400> 6
   atg gca acc aac cct tca tgc tta tct act cca ttt ttg tcc tcc aca      48
   Met Ala Thr Asn Pro Ser Cys Leu Ser Thr Pro Phe Leu Ser Ser Thr
   1               5                   10                  15
   cca gca cta agt act aga ttt cca tta agt gag aac ttc aca caa aaa      96
   Pro Ala Leu Ser Thr Arg Phe Pro Leu Ser Glu Asn Phe Thr Gln Lys
               20                  25                  30
   aca tct ctt gtc aat ccc aaa cct tgg cca ctt att tct gca gtc agc     144
   Thr Ser Leu Val Asn Pro Lys Pro Trp Pro Leu Ile Ser Ala Val Ser
           35                  40                  45
   tct caa ttt agc caa ata gca gaa gat aat agt cgt cgt tca gct aat     192
   Ser Gln Phe Ser Gln Ile Ala Glu Asp Asn Ser Arg Arg Ser Ala Asn
       50                  55                  60
   tac cac cca aac ctc tgg gat ttt gaa ttt ctg cag tct ctc gaa aat     240
   Tyr His Pro Asn Leu Trp Asp Phe Glu Phe Leu Gln Ser Leu Glu Asn
   65                  70                  75                  80
   gac tct aag atg gaa aag ctg gaa gag aaa gca aca aag ttg gag gag     288
   Asp Ser Lys Met Glu Lys Leu Glu Glu Lys Ala Thr Lys Leu Glu Glu
                   85                  90                  95
   gaa gtg cga aac atg atg aac gaa gca aag aca gaa gca cta agc tta     336
   Glu Val Arg Asn Met Met Asn Glu Ala Lys Thr Glu Ala Leu Ser Leu
               100                 105                 110
   ttg gaa ttg ata gac gac gtc cag cgt ctg gga ttg acc tac aag ttt     384
   Leu Glu Leu Ile Asp Asp Val Gln Arg Leu Gly Leu Thr Tyr Lys Phe
           115                 120                 125
   gag aag gac ata atc aaa gcc ctt gag aag att gtt cca ttg gat gag     432
   Glu Lys Asp Ile Ile Lys Ala Leu Glu Lys Ile Val Pro Leu Asp Glu
       130                 135                 140
   agt ggg ctg cat gtt act tct ctc agc ttc cgt ata ctt aga caa cat     480
   Ser Gly Leu His Val Thr Ser Leu Ser Phe Arg Ile Leu Arg Gln His
   145                 150                 155                 160
   ggc ttt gag gtt tcc caa gat gtg ttt aag aga ttt aag gac aag gag     528
   Gly Phe Glu Val Ser Gln Asp Val Phe Lys Arg Phe Lys Asp Lys Glu
                   165                 170                 175
   gga ggt ttt tgt gct gaa ctt aaa gac gat gtt caa ggg ttg cta agt     576
   Gly Gly Phe Cys Ala Glu Leu Lys Asp Asp Val Gln Gly Leu Leu Ser
               180                 185                 190
   cta tat gaa gca tcc tat ctt ggt ttt gag gga gaa agt ctc tta gac     624
   Leu Tyr Glu Ala Ser Tyr Leu Gly Phe Glu Gly Glu Ser Leu Leu Asp
           195                 200                 205
   gag gca agg gca ttt tca ata aca cat ctc aag aac aac cta aac aaa     672
   Glu Ala Arg Ala Phe Ser Ile Thr His Leu Lys Asn Asn Leu Asn Lys
       210                 215                 220
   gga ata aac acc aaa gta gcc caa caa gtt agc cat gca ctg gaa ctt     720
   Gly Ile Asn Thr Lys Val Ala Gln Gln Val Ser His Ala Leu Glu Leu
   225                 230                 235                 240
   cct tat cat cga aga ctg cat aga ctg gaa gca cga tgg ctc ctt gac     768
   Pro Tyr His Arg Arg Leu His Arg Leu Glu Ala Arg Trp Leu Leu Asp
                   245                 250                 255
   aaa tat gaa cca aag gaa ccc cac cat cat tta cta cac gag ctt gca     816
   Lys Tyr Glu Pro Lys Glu Pro His His His Leu Leu His Glu Leu Ala
               260                 265                 270
   aag ttg gat ttc aat ttg gtc caa tca ttg tac cag aaa gag ttg cga     864
   Lys Leu Asp Phe Asn Leu Val Gln Ser Leu Tyr Gln Lys Glu Leu Arg
           275                 280                 285
   gaa ttg tca ctg tgg tgg agg gag att ggg ctc aca agc aag ttg gac     912
   Glu Leu Ser Leu Trp Trp Arg Glu Ile Gly Leu Thr Ser Lys Leu Asp
       290                 295                 300
   ttt gtt cga gac aga tta atg gaa gtg tac ttt tgg gcg ctg gga atg     960
   Phe Val Arg Asp Arg Leu Met Glu Val Tyr Phe Trp Ala Leu Gly Met
   305                 310                 315                 320
   gca cct gat cct caa ttt agt gaa tgt cgt aaa gtc gtc act aaa atg    1008
   Ala Pro Asp Pro Gln Phe Ser Glu Cys Arg Lys Val Val Thr Lys Met
                   325                 330                 335
   ttt ggg cta gtt act atc atc gat gat gta tat gac gtt tac ggt act    1056
   Phe Gly Leu Val Thr Ile Ile Asp Asp Val Tyr Asp Val Tyr Gly Thr
               340                 345                 350
   ttg gac gag cta caa ctc ttc acc gat gct gtt gag aga tgg gac gtg    1104
   Leu Asp Glu Leu Gln Leu Phe Thr Asp Ala Val Glu Arg Trp Asp Val
           355                 360                 365
   aat gcg ata aat aca ctt cca gac tat atg aaa ttg tgc tat tta gcc    1152
   Asn Ala Ile Asn Thr Leu Pro Asp Tyr Met Lys Leu Cys Tyr Leu Ala
       370                 375                 380
   ctt tat aac acc gtc aat gac aca gct tat agc atc ctt aaa gaa aag    1200
   Leu Tyr Asn Thr Val Asn Asp Thr Ala Tyr Ser Ile Leu Lys Glu Lys
   385                 390                 395                 400
   gga cat aac aac att tct tat ttg aca aaa tct tgg tgt gag ttg tgc    1248
   Gly His Asn Asn Ile Ser Tyr Leu Thr Lys Ser Trp Cys Glu Leu Cys
                   405                 410                 415
   aaa gca ttc ctc caa gaa gca aaa tgg tca aac aac aaa atc att cca    1296
   Lys Ala Phe Leu Gln Glu Ala Lys Trp Ser Asn Asn Lys Ile Ile Pro
               420                 425                 430
   gca ttc aac aag tac cta gac aat gca tcg gtg tcc tcc tct ggt gtg    1344
   Ala Phe Asn Lys Tyr Leu Asp Asn Ala Ser Val Ser Ser Ser Gly Val
           435                 440                 445
   gct ttg ctt gct cct tcc tac ttc tta gtg tgc caa gaa caa gac att    1392
   Ala Leu Leu Ala Pro Ser Tyr Phe Leu Val Cys Gln Glu Gln Asp Ile
       450                 455                 460
   tca gac caa gct ctt cat tcc tta act aat ttc cat ggc ctt gtg cgt    1440
   Ser Asp Gln Ala Leu His Ser Leu Thr Asn Phe His Gly Leu Val Arg
   465                 470                 475                 480
   tca tca tgc acc att ttt agg ctt tgc aat gat ctg gct acc tca tcg    1488
   Ser Ser Cys Thr Ile Phe Arg Leu Cys Asn Asp Leu Ala Thr Ser Ser
                   485                 490                 495
   gct gag cta gag aga ggt gaa aca aca aat tca atc aca tcg tac atg    1536
   Ala Glu Leu Glu Arg Gly Glu Thr Thr Asn Ser Ile Thr Ser Tyr Met
               500                 505                 510
   cat gag aat gag act tct gag gag caa gca tgt aag gag ttg aga aat    1584
   His Glu Asn Glu Thr Ser Glu Glu Gln Ala Cys Lys Glu Leu Arg Asn
           515                 520                 525
   ttg atc gat gca gag tgg aag aag atg aat gaa gag cga gtt tca aat    1632
   Leu Ile Asp Ala Glu Trp Lys Lys Met Asn Glu Glu Arg Val Ser Asn
       530                 535                 540
   tct aca ctc cca aaa gca ttt agg gaa ata gct att aac atg gct cgg    1680
   Ser Thr Leu Pro Lys Ala Phe Arg Glu Ile Ala Ile Asn Met Ala Arg
   545                 550                 555                 560
   att tcc cat tgc aca tac caa tat gga gac gga ctt gga agg ccc gac    1728
   Ile Ser His Cys Thr Tyr Gln Tyr Gly Asp Gly Leu Gly Arg Pro Asp
                   565                 570                 575
   tac acc aca gag aac agg ata aag ttg cta cta ata gac cct ttt cca    1776
   Tyr Thr Thr Glu Asn Arg Ile Lys Leu Leu Leu Ile Asp Pro Phe Pro
               580                 585                 590
   att aat tag                                                        1785
   Ile Asn
<210> 7
   <211> 594
   <212> PRT
   <213> Mucuna bracteata
<400> 7
   Met Ala Thr Asn Pro Ser Cys Leu Ser Thr Pro Phe Leu Ser Ser Thr
   1               5                   10                  15
   Pro Ala Leu Ser Thr Arg Phe Pro Leu Ser Glu Asn Phe Thr Gln Lys
               20                  25                  30
   Thr Ser Leu Val Asn Pro Lys Pro Trp Pro Leu Ile Ser Ala Val Ser
           35                  40                  45
   Ser Gln Phe Ser Gln Ile Ala Glu Asp Asn Ser Arg Arg Ser Ala Asn
       50                  55                  60
   Tyr His Pro Asn Leu Trp Asp Phe Glu Phe Leu Gln Ser Leu Glu Asn
   65                  70                  75                  80
   Asp Ser Lys Met Glu Lys Leu Glu Glu Lys Ala Thr Lys Leu Glu Glu
                   85                  90                  95
   Glu Val Arg Asn Met Met Asn Glu Ala Lys Thr Glu Ala Leu Ser Leu
               100                 105                 110
   Leu Glu Leu Ile Asp Asp Val Gln Arg Leu Gly Leu Thr Tyr Lys Phe
           115                 120                 125
   Glu Lys Asp Ile Ile Lys Ala Leu Glu Lys Ile Val Pro Leu Asp Glu
       130                 135                 140
   Ser Gly Leu His Val Thr Ser Leu Ser Phe Arg Ile Leu Arg Gln His
   145                 150                 155                 160
   Gly Phe Glu Val Ser Gln Asp Val Phe Lys Arg Phe Lys Asp Lys Glu
                   165                 170                 175
   Gly Gly Phe Cys Ala Glu Leu Lys Asp Asp Val Gln Gly Leu Leu Ser
               180                 185                 190
   Leu Tyr Glu Ala Ser Tyr Leu Gly Phe Glu Gly Glu Ser Leu Leu Asp
           195                 200                 205
   Glu Ala Arg Ala Phe Ser Ile Thr His Leu Lys Asn Asn Leu Asn Lys
       210                 215                 220
   Gly Ile Asn Thr Lys Val Ala Gln Gln Val Ser His Ala Leu Glu Leu
   225                 230                 235                 240
   Pro Tyr His Arg Arg Leu His Arg Leu Glu Ala Arg Trp Leu Leu Asp
                   245                 250                 255
   Lys Tyr Glu Pro Lys Glu Pro His His His Leu Leu His Glu Leu Ala
               260                 265                 270
   Lys Leu Asp Phe Asn Leu Val Gln Ser Leu Tyr Gln Lys Glu Leu Arg
           275                 280                 285
   Glu Leu Ser Leu Trp Trp Arg Glu Ile Gly Leu Thr Ser Lys Leu Asp
       290                 295                 300
   Phe Val Arg Asp Arg Leu Met Glu Val Tyr Phe Trp Ala Leu Gly Met
   305                 310                 315                 320
   Ala Pro Asp Pro Gln Phe Ser Glu Cys Arg Lys Val Val Thr Lys Met
                   325                 330                 335
   Phe Gly Leu Val Thr Ile Ile Asp Asp Val Tyr Asp Val Tyr Gly Thr
               340                 345                 350
   Leu Asp Glu Leu Gln Leu Phe Thr Asp Ala Val Glu Arg Trp Asp Val
           355                 360                 365
   Asn Ala Ile Asn Thr Leu Pro Asp Tyr Met Lys Leu Cys Tyr Leu Ala
       370                 375                 380
   Leu Tyr Asn Thr Val Asn Asp Thr Ala Tyr Ser Ile Leu Lys Glu Lys
   385                 390                 395                 400
   Gly His Asn Asn Ile Ser Tyr Leu Thr Lys Ser Trp Cys Glu Leu Cys
                   405                 410                 415
   Lys Ala Phe Leu Gln Glu Ala Lys Trp Ser Asn Asn Lys Ile Ile Pro
               420                 425                 430
   Ala Phe Asn Lys Tyr Leu Asp Asn Ala Ser Val Ser Ser Ser Gly Val
           435                 440                 445
   Ala Leu Leu Ala Pro Ser Tyr Phe Leu Val Cys Gln Glu Gln Asp Ile
       450                 455                 460
   Ser Asp Gln Ala Leu His Ser Leu Thr Asn Phe His Gly Leu Val Arg
   465                 470                 475                 480
   Ser Ser Cys Thr Ile Phe Arg Leu Cys Asn Asp Leu Ala Thr Ser Ser
                   485                 490                 495
   Ala Glu Leu Glu Arg Gly Glu Thr Thr Asn Ser Ile Thr Ser Tyr Met
               500                 505                 510
   His Glu Asn Glu Thr Ser Glu Glu Gln Ala Cys Lys Glu Leu Arg Asn
           515                 520                 525
   Leu Ile Asp Ala Glu Trp Lys Lys Met Asn Glu Glu Arg Val Ser Asn
       530                 535                 540
   Ser Thr Leu Pro Lys Ala Phe Arg Glu Ile Ala Ile Asn Met Ala Arg
   545                 550                 555                 560
   Ile Ser His Cys Thr Tyr Gln Tyr Gly Asp Gly Leu Gly Arg Pro Asp
                   565                 570                 575
   Tyr Thr Thr Glu Asn Arg Ile Lys Leu Leu Leu Ile Asp Pro Phe Pro
               580                 585                 590
   Ile Asn
<210> 8
   <211> 608
   <212> PRT
   <213> Pueraria montana
<400> 8
   Met Ala Thr Asn Leu Leu Cys Leu Ser Asn Lys Leu Ser Ser Pro Thr
   1               5                   10                  15
   Pro Thr Pro Ser Thr Arg Phe Pro Gln Ser Lys Asn Phe Ile Thr Gln
               20                  25                  30
   Lys Thr Ser Leu Ala Asn Pro Lys Pro Trp Arg Val Ile Cys Ala Thr
           35                  40                  45
   Ser Ser Gln Phe Thr Gln Ile Thr Glu His Asn Ser Arg Arg Ser Ala
       50                  55                  60
   Asn Tyr Gln Pro Asn Leu Trp Asn Phe Glu Phe Leu Gln Ser Leu Glu
   65                  70                  75                  80
   Asn Asp Leu Lys Val Glu Lys Leu Glu Glu Lys Ala Thr Lys Leu Glu
                   85                  90                  95
   Glu Glu Val Arg Cys Met Ile Asn Arg Val Asp Thr Gln Pro Leu Ser
               100                 105                 110
   Leu Leu Glu Leu Ile Asp Asp Val Gln Arg Leu Gly Leu Thr Tyr Lys
           115                 120                 125
   Phe Glu Lys Asp Ile Ile Lys Ala Leu Glu Asn Ile Val Leu Leu Asp
       130                 135                 140
   Glu Asn Lys Lys Asn Lys Ser Asp Leu His Ala Thr Ala Leu Ser Phe
   145                 150                 155                 160
   Arg Leu Leu Arg Gln His Gly Phe Glu Val Ser Gln Asp Val Phe Glu
                   165                 170                 175
   Arg Phe Lys Asp Lys Glu Gly Gly Phe Ser Gly Glu Leu Lys Gly Asp
               180                 185                 190
   Val Gln Gly Leu Leu Ser Leu Tyr Glu Ala Ser Tyr Leu Gly Phe Glu
           195                 200                 205
   Gly Glu Asn Leu Leu Glu Glu Ala Arg Thr Phe Ser Ile Thr His Leu
       210                 215                 220
   Lys Asn Asn Leu Lys Glu Gly Ile Asn Thr Lys Val Ala Glu Gln Val
   225                 230                 235                 240
   Ser His Ala Leu Glu Leu Pro Tyr His Gln Arg Leu His Arg Leu Glu
                   245                 250                 255
   Ala Arg Trp Phe Leu Asp Lys Tyr Glu Pro Lys Glu Pro His His Gln
               260                 265                 270
   Leu Leu Leu Glu Leu Ala Lys Leu Asp Phe Asn Met Val Gln Thr Leu
           275                 280                 285
   His Gln Lys Glu Leu Gln Asp Leu Ser Arg Trp Trp Thr Glu Met Gly
       290                 295                 300
   Leu Ala Ser Lys Leu Asp Phe Val Arg Asp Arg Leu Met Glu Val Tyr
   305                 310                 315                 320
   Phe Trp Ala Leu Gly Met Ala Pro Asp Pro Gln Phe Gly Glu Cys Arg
                   325                 330                 335
   Lys Ala Val Thr Lys Met Phe Gly Leu Val Thr Ile Ile Asp Asp Val
               340                 345                 350
   Tyr Asp Val Tyr Gly Thr Leu Asp Glu Leu Gln Leu Phe Thr Asp Ala
           355                 360                 365
   Val Glu Arg Trp Asp Val Asn Ala Ile Asn Thr Leu Pro Asp Tyr Met
       370                 375                 380
   Lys Leu Cys Phe Leu Ala Leu Tyr Asn Thr Val Asn Asp Thr Ser Tyr
   385                 390                 395                 400
   Ser Ile Leu Lys Glu Lys Gly His Asn Asn Leu Ser Tyr Leu Thr Lys
                   405                 410                 415
   Ser Trp Arg Glu Leu Cys Lys Ala Phe Leu Gln Glu Ala Lys Trp Ser
               420                 425                 430
   Asn Asn Lys Ile Ile Pro Ala Phe Ser Lys Tyr Leu Glu Asn Ala Ser
           435                 440                 445
   Val Ser Ser Ser Gly Val Ala Leu Leu Ala Pro Ser Tyr Phe Ser Val
       450                 455                 460
   Cys Gln Gln Gln Glu Asp Ile Ser Asp His Ala Leu Arg Ser Leu Thr
   465                 470                 475                 480
   Asp Phe His Gly Leu Val Arg Ser Ser Cys Val Ile Phe Arg Leu Cys
                   485                 490                 495
   Asn Asp Leu Ala Thr Ser Ala Ala Glu Leu Glu Arg Gly Glu Thr Thr
               500                 505                 510
   Asn Ser Ile Ile Ser Tyr Met His Glu Asn Asp Gly Thr Ser Glu Glu
           515                 520                 525
   Gln Ala Arg Glu Glu Leu Arg Lys Leu Ile Asp Ala Glu Trp Lys Lys
       530                 535                 540
   Met Asn Arg Glu Arg Val Ser Asp Ser Thr Leu Leu Pro Lys Ala Phe
   545                 550                 555                 560
   Met Glu Ile Ala Val Asn Met Ala Arg Val Ser His Cys Thr Tyr Gln
                   565                 570                 575
   Tyr Gly Asp Gly Leu Gly Arg Pro Asp Tyr Ala Thr Glu Asn Arg Ile
               580                 585                 590
   Lys Leu Leu Leu Ile Asp Pro Phe Pro Ile Asn Gln Leu Met Tyr Val
           595                 600                 605
<210> 9
   <211> 1827
   <212> DNA
   <213> Pueraria montana
<400> 9
   atggcaacca accttttatg cttgtctaat aaattatcgt cccccacacc aacaccaagt      60
   actagatttc cacaaagtaa gaacttcatc acacaaaaaa catctcttgc caatcccaaa      120
   ccttggcgag ttatttgtgc tacgagctct caatttaccc aaataacaga acataatagt      180
   cggcgttcag ctaattacca gccaaacctc tggaattttg aatttctgca gtctctggaa      240
   aatgacctta aggtggaaaa actagaagag aaggcaacaa agctagagga ggaggtacga      300
   tgcatgatca acagagtaga cacacaacca ttaagcttac tagaattgat cgacgatgtc      360
   cagcgtctag gattgaccta caagtttgag aaggacataa tcaaagccct tgagaatatt      420
   gttttgctgg atgagaataa gaaaaataaa agtgacctcc atgctactgc tctcagcttc      480
   cgtttactta gacaacatgg ctttgaggtt tcccaagatg tgtttgagag atttaaggac      540
   aaggagggag gtttcagtgg tgaacttaaa ggtgatgtgc aagggttgct gagtctatat      600
   gaagcatcct atcttggctt tgagggagaa aatctcttgg aggaggcaag gacattttca      660
   ataacacatc tcaagaacaa cctaaaagaa ggaataaaca ccaaagtggc agaacaagtt      720
   agtcatgcac tggaacttcc ctatcatcaa agattgcata gactagaagc acgatggttc      780
   cttgacaaat atgaaccaaa ggaaccccac catcagttac tactcgagct tgcaaagcta      840
   gatttcaata tggtgcaaac attgcaccag aaagaactgc aagacctgtc aaggtggtgg      900
   acggagatgg ggctagcaag caagctagac tttgtccgag acagattaat ggaagtgtat      960
   ttttgggcgt tgggaatggc acctgatcct caattcggtg aatgtcgtaa agctgtcact     1020
   aaaatgtttg gattggtcac catcatcgat gatgtatatg acgtttatgg tactttggat     1080
   gagctacaac tcttcactga tgctgttgag agatgggacg tgaatgccat aaacacactt     1140
   ccagactaca tgaagttgtg cttcctagca ctttataaca ccgtcaatga cacgtcttat     1200
   agcatcctta aagaaaaagg acacaacaac ctttcctatt tgacaaaatc ttggcgtgag     1260
   ttatgcaaag cattccttca agaagcaaaa tggtcgaaca acaaaatcat tccagcattt     1320
   agcaagtacc tggaaaatgc atcggtgtcc tcctccggtg tggctttgct tgctccttcc     1380
   tacttctcag tgtgccaaca acaagaagat atctcagacc atgctcttcg ttctttaact     1440
   gatttccatg gccttgtgcg ctcctcatgc gtcattttca gactctgcaa tgatttggct     1500
   acctcagcgg ctgagctaga gaggggtgag acgacaaatt caataatatc ttatatgcat     1560
   gagaatgacg gcacttctga agagcaagca cgtgaggagt tgagaaaatt gatcgatgca     1620
   gagtggaaga agatgaaccg agagcgagtt tcagattcta cactactccc aaaagctttt     1680
   atggaaatag ctgttaacat ggctcgagtt tcgcattgca cataccaata tggagacgga     1740
   cttggaaggc cagactacgc cacagagaat agaatcaagt tgctacttat agaccccttt     1800
   ccaatcaatc aactaatgta cgtgtaa                                          1827
<210> 10
   <211> 1695
   <212> DNA
   <213> Artificial Sequence
<220>
   <223> Polynucleotide having modified codons, which encodes isoprene synthase derived from Pueraria montana (IspSK gene)
<400> 10
   atgtgtgcga cctcttctca atttactcag attaccgagc ataattcccg tcgttccgca      60
   aactatcagc caaacctgtg gaatttcgaa ttcctgcaat ccctggagaa cgacctgaaa     120
   gtggaaaagc tggaggagaa agcgaccaaa ctggaggaag aagttcgctg catgatcaac     180
   cgtgtagaca cccagccgct gtccctgctg gagctgatcg acgatgtgca gcgcctgggt     240
   ctgacctaca aatttgaaaa agacatcatt aaagccctgg aaaacatcgt actgctggac     300
   gaaaacaaaa agaacaaatc tgacctgcac gcaaccgctc tgtctttccg tctgctgcgt     360
   cagcacggtt tcgaggtttc tcaggatgtt tttgagcgtt tcaaggataa agaaggtggt     420
   ttcagcggtg aactgaaagg tgacgtccaa ggcctgctga gcctgtatga agcgtcttac     480
   ctgggtttcg agggtgagaa cctgctggag gaggcgcgta ccttttccat cacccacctg     540
   aagaacaacc tgaaagaagg cattaatacc aaggttgcag aacaagtgag ccacgccctg     600
   gaactgccat atcaccagcg tctgcaccgt ctggaggcac gttggttcct ggataaatac     660
   gaaccgaaag aaccgcatca ccagctgctg ctggagctgg cgaagctgga ttttaacatg     720
   gtacagaccc tgcaccagaa agagctgcaa gatctgtccc gctggtggac cgagatgggc     780
   ctggctagca aactggattt tgtacgcgac cgcctgatgg aagtttattt ctgggcactg     840
   ggtatggcgc cagacccgca gtttggtgaa tgtcgcaaag ctgttactaa aatgtttggt     900
   ctggtgacga tcatcgatga cgtgtatgac gtttatggca ctctggacga actgcaactg     960
   ttcaccgatg ctgtagagcg ctgggacgtt aacgctatta acaccctgcc ggactatatg   1020
   aaactgtgtt tcctggcact gtacaacacc gttaacgaca cgtcctattc tattctgaaa   1080
   gagaaaggtc ataacaacct gtcctatctg acgaaaagct ggcgtgaact gtgcaaagcc   1140
   tttctgcaag aggcgaaatg gtccaacaac aaaattatcc cggctttctc caagtacctg   1200
   gaaaacgcca gcgtttcctc ctccggtgta gcgctgctgg cgccgtctta cttttccgta   1260
   tgccagcagc aggaagacat ctccgaccac gcgctgcgtt ccctgaccga cttccatggt   1320
   ctggtgcgtt ctagctgcgt tatcttccgc ctgtgcaacg atctggccac ctctgcggcg   1380
   gagctggaac gtggcgagac taccaattct atcattagct acatgcacga aaacgatggt   1440
   accagcgagg aacaggcccg cgaagaactg cgtaaactga tcgacgccga atggaaaaag   1500
   atgaatcgtg aacgcgttag cgactccacc ctgctgccta aagcgttcat ggaaatcgca   1560
   gttaacatgg cacgtgtttc ccactgcacc taccagtatg gcgatggtct gggtcgccca   1620
   gactacgcga ctgaaaaccg catcaaactg ctgctgattg accctttccc gattaaccag   1680
   ctgatgtatg tctaa                                                    1695
<210> 11
   <211> 595
   <212> PRT
   <213> Populus alba x Populus tremula
<400> 11
   Met Ala Thr Glu Leu Leu Cys Leu His Arg Pro Ile Ser Leu Thr His
   1               5                   10                  15
   Lys Leu Phe Arg Asn Pro Leu Pro Lys Val Ile Gln Ala Thr Pro Leu
               20                  25                  30
   Thr Leu Lys Leu Arg Cys Ser Val Ser Thr Glu Asn Val Ser Phe Thr
           35                  40                  45
   Glu Thr Glu Thr Glu Ala Arg Arg Ser Ala Asn Tyr Glu Pro Asn Ser
       50                  55                  60
   Trp Asp Tyr Asp Phe Leu Leu Ser Ser Asp Thr Asp Glu Ser Ile Glu
   65                  70                  75                  80
   Val Tyr Lys Asp Lys Ala Lys Lys Leu Glu Ala Glu Val Arg Arg Glu
                   85                  90                  95
   Ile Asn Asn Glu Lys Ala Glu Phe Leu Thr Leu Leu Glu Leu Ile Asp
               100                 105                 110
   Asn Val Gln Arg Leu Gly Leu Gly Tyr Arg Phe Glu Ser Asp Ile Arg
               115                 120                 125
   Arg Ala Leu Asp Arg Phe Val Ser Ser Gly Gly Phe Asp Gly Val Thr
       130                 135                 140
   Lys Thr Ser Leu His Ala Thr Ala Leu Ser Phe Arg Leu Leu Arg Gln
   145                 150                 155                 160
   His Gly Phe Glu Val Ser Gln Glu Ala Phe Ser Gly Phe Lys Asp Gln
                   165                 170                 175
   Asn Gly Asn Phe Leu Glu Asn Leu Lys Glu Asp Thr Lys Ala Ile Leu
               180                 185                 190
   Ser Leu Tyr Glu Ala Ser Phe Leu Ala Leu Glu Gly Glu Asn Ile Leu
           195                 200                 205
   Asp Glu Ala Arg Val Phe Ala Ile Ser His Leu Lys Glu Leu Ser Glu
       210                 215                 220
   Glu Lys Ile Gly Lys Glu Leu Ala Glu Gln Val Asn His Ala Leu Glu
   225                 230                 235                 240
   Leu Pro Leu His Arg Arg Thr Gln Arg Leu Glu Ala Val Trp Ser Ile
                   245                 250                 255
   Glu Ala Tyr Arg Lys Lys Glu Asp Ala Asn Gln Val Leu Leu Glu Leu
               260                 265                 270
   Ala Ile Leu Asp Tyr Asn Met Ile Gln Ser Val Tyr Gln Arg Asp Leu
           275                 280                 285
   Arg Glu Thr Ser Arg Trp Trp Arg Arg Val Gly Leu Ala Thr Lys Leu
       290                 295                 300
   His Phe Ala Lys Asp Arg Leu Ile Glu Ser Phe Tyr Trp Ala Val Gly
   305                 310                 315                 320
   Val Ala Phe Glu Pro Gln Tyr Ser Asp Cys Arg Asn Ser Val Ala Lys
                   325                 330                 335
   Met Phe Ser Phe Val Thr Ile Ile Asp Asp Ile Tyr Asp Val Tyr Gly
               340                 345                 350
   Thr Leu Asp Glu Leu Glu Leu Phe Thr Asp Ala Val Glu Arg Trp Asp
           355                 360                 365
   Val Asn Ala Ile Asn Asp Leu Pro Asp Tyr Met Lys Leu Cys Phe Leu
       370                 375                 380
   Ala Leu Tyr Asn Thr Ile Asn Glu Ile Ala Tyr Asp Asn Leu Lys Asp
   385                 390                 395                 400
   Lys Gly Glu Asn Ile Leu Pro Tyr Leu Thr Lys Ala Trp Ala Asp Leu
                   405                 410                 415
   Cys Asn Ala Phe Leu Gln Glu Ala Lys Trp Leu Tyr Asn Lys Ser Thr
               420                 425                 430
   Pro Thr Phe Asp Asp Tyr Phe Gly Asn Ala Trp Lys Ser Ser Ser Gly
           435                 440                 445
   Pro Leu Gln Leu Ile Phe Ala Tyr Phe Ala Val Val Gln Asn Ile Lys
       450                 455                 460
   Lys Glu Glu Ile Glu Asn Leu Gln Lys Tyr His Asp Ile Ile Ser Arg
   465                 470                 475                 480
   Pro Ser His Ile Phe Arg Leu Cys Asn Asp Leu Ala Ser Ala Ser Ala
                   485                 490                 495
   Glu Ile Ala Arg Gly Glu Thr Ala Asn Ser Val Ser Cys Tyr Met Arg
               500                 505                 510
   Thr Lys Gly Ile Ser Glu Glu Leu Ala Thr Glu Ser Val Met Asn Leu
           515                 520                 525
   Ile Asp Glu Thr Cys Lys Lys Met Asn Lys Glu Lys Leu Gly Gly Ser
       530                 535                 540
   Leu Phe Ala Lys Pro Phe Val Glu Thr Ala Ile Asn Leu Ala Arg Gln
   545                 550                 555                 560
   Ser His Cys Thr Tyr His Asn Gly Asp Ala His Thr Ser Pro Asp Glu
                   565                 570                 575
   Leu Thr Arg Lys Arg Val Leu Ser Val Ile Thr Glu Pro Ile Leu Pro
               580                 585                 590
   Phe Glu Arg
           595
<210> 12
   <211> 1788
   <212> DNA
   <213> Populus alba x Populus tremula
<400> 12
   atggcaactg aattattgtg cttgcaccgt ccaatctcac tgacacacaa actgttcaga      60
   aatcccttac ctaaagtcat ccaggccact cccttaactt tgaaactcag atgttctgta     120
   agcacagaaa acgtcagctt cacagaaaca gaaacagaag ccagacggtc tgccaattat     180
   gaaccaaata gctgggatta tgattttttg ctgtcttcag acactgacga atcgattgaa     240
   gtatacaaag acaaggccaa aaagctggag gctgaggtga gaagagagat taacaatgaa     300
   aaggcagagt ttttgactct gcttgaactg atagataatg tccaaaggtt aggattgggt     360
   taccggttcg agagtgacat aaggagagcc ctcgacagat ttgtttcttc aggaggattt     420
   gatggtgtta caaaaactag ccttcatgct actgctctta gcttcaggct tctcagacag     480
   catggctttg aggtctctca agaagcgttc agtggattca aggatcaaaa tggcaatttc     540
   ttggaaaacc ttaaggagga caccaaggca atactaagcc tatatgaagc ttcatttctt     600
   gcattagaag gagaaaatat cttggatgag gccagggtgt ttgcaatatc acatctaaaa     660
   gagctcagcg aagaaaagat tggaaaagag ctggccgaac aggtgaatca tgcattggag     720
   cttccattgc atcgcaggac gcaaagacta gaagctgttt ggagtattga agcataccgt     780
   aaaaaggaag atgcaaatca agtactgcta gaacttgcta tattggacta caacatgatt     840
   caatcagtat accaaagaga tcttcgcgag acatcaaggt ggtggaggcg agtgggtctt     900
   gcaacaaagt tgcattttgc taaagacagg ttaattgaaa gcttttactg ggcagttgga     960
   gttgcgttcg aacctcaata cagtgattgc cgtaattcag tagcaaaaat gttttcattt    1020
   gtaacaatca ttgatgatat ctatgatgtt tatggtactc tggatgagct ggagctattt    1080
   acagatgctg ttgagagatg ggatgttaac gccatcaatg atcttccgga ttatatgaag    1140
   ctctgcttcc tagctctcta caacactatc aatgagatag cttatgacaa tctgaaggac    1200
   aagggggaaa acattcttcc atacctaaca aaagcgtggg cagatttatg caatgcattc    1260
   ctacaagaag caaaatggct gtacaataag tccacaccaa catttgatga ctatttcgga    1320
   aatgcatgga aatcatcctc agggcctctt caactaattt ttgcctactt tgccgtggtt    1380
   caaaacatca agaaagagga aattgaaaac ttacaaaagt atcatgatat catcagtagg    1440
   ccttcccaca tctttcgtct ttgcaacgac ctggcttcag catcggctga gatagcgaga    1500
   ggtgaaactg cgaattccgt atcctgctac atgcgtacaa aaggcatttc tgaggaactt    1560
   gctactgaat ccgtaatgaa tttgatcgac gaaacctgta aaaagatgaa caaagaaaag    1620
   cttggtggct ctttgtttgc aaaacctttt gtcgaaacag ctattaacct tgcacggcaa    1680
   tcccattgca cttatcataa cggagatgcg catacttcac cagacgagct aactaggaaa    1740
   cgtgtcctgt cagtaatcac agagcctatt ctaccctttg agagataa                 1788
<210> 13
   <211> 1680
   <212> DNA
   <213> Artificial Sequence
<220>
   <223> Polynucleotide having modified codons, which encodes isoprene synthase derived from Populus alba x Populus tremula (IspSP gene)
<400> 13
   atgtgctctg tttctaccga gaacgtttcc ttcactgaga cggaaaccga ggcacgtcgt     60
   agcgcgaact acgagccgaa tagctgggac tacgatttcc tgctgtcttc cgatactgac    120
   gaatctattg aggtgtacaa agacaaagca aagaaactgg aggctgaagt gcgccgcgaa    180
   attaacaacg agaaagctga attcctgact ctgctggagc tgatcgataa cgtacagcgc    240
   ctgggtctgg gttaccgctt cgaatctgat atccgtcgcg cactggatcg tttcgtaagc    300
   agcggcggtt tcgatggcgt gaccaaaacg agcctgcacg ctaccgcgct gtccttccgt    360
   ctgctgcgtc agcacggctt cgaagtttct caggaagcat tctccggttt caaagatcaa    420
   aacggtaact tcctggaaaa cctgaaagaa gacactaagg cgatcctgag cctgtatgag    480
   gcaagctttc tggccctgga gggtgagaac atcctggatg aggcgcgcgt attcgccatc    540
   tcccatctga aagagctgtc tgaagagaaa atcggtaagg aactggcaga gcaggttaat    600
   cacgcactgg aactgccgct gcatcgtcgt acccagcgtc tggaggcggt ttggtccatc    660
   gaagcgtacc gcaaaaagga ggatgctaac caggttctgc tggaactggc catcctggac    720
   tacaacatga tccagtccgt ttaccagcgt gatctgcgtg aaacctcccg ttggtggcgc    780
   cgtgtgggcc tggcgaccaa actgcacttc gctaaggacc gcctgattga gtctttttac    840
   tgggcagtcg gcgttgcgtt cgaacctcag tattctgact gccgtaacag cgttgcgaaa    900
   atgttcagct tcgttactat tatcgacgac atctacgacg tttacggtac tctggacgag    960
   ctggaactgt ttaccgacgc tgtcgaacgt tgggatgtta acgccatcaa cgatctgcct   1020
   gactacatga aactgtgctt cctggcactg tataacacga tcaacgaaat tgcatacgac   1080
   aacctgaaag acaaaggtga aaacatcctg ccgtacctga ctaaagcgtg ggcggatctg   1140
   tgtaacgctt ttctgcaaga agcgaaatgg ctgtataaca aatccactcc gacctttgac   1200
   gattatttcg gcaatgcctg gaaatccagc tctggcccgc tgcaactgat cttcgcttat   1260
   tttgcggttg tccaaaacat caaaaaggag gaaattgaaa acctgcaaaa ataccacgat   1320
   atcattagcc gtccttctca tatctttcgc ctgtgcaacg acctggcaag cgcgtccgca   1380
   gagatcgcac gtggcgaaac cgctaactct gtttcctgct acatgcgcac caagggcatt   1440
   tccgaagagc tggcaaccga gagcgtaatg aatctgatcg acgaaacctg taagaaaatg   1500
   aacaaagaaa aactgggtgg ctccctgttc gctaaaccgt tcgtagagac tgctattaac   1560
   ctggcacgtc agagccactg cacctaccac aatggtgacg cacatactag cccggatgaa   1620
   ctgactcgta aacgtgtact gtctgttatc accgaaccga ttctgccgtt cgaacgttaa   1680
<210> 14
   <211> 1785
   <212> DNA
   <213> Artificial Sequence
<220>
   <223> Polynucleotide which encodes isoprene synthase derived from Mucuna bracteata and which is fused with chloroplast-localization signal (IspSM(L) gene)
<400> 14
   atggctacca acccgtcctg tctgtcaacc ccgttcctgt cttcaacccc ggctctgtcc 60
   acccgcttcc cgctgtccga aaacttcacc cagaaaacga gcctggttaa cccgaaaccg 120
   tggccgctga tttctgcggt cagctctcag tttagtcaaa tcgcggaaga taattctcgt 180
   cgcagtgcca actatcatcc gaatctgtgg gattttgaat tcctgcagtc gctggaaaac 240
   gacagcaaaa tggaaaaact ggaagaaaaa gcgaccaaac tggaagaaga agtgcgtaac 300
   atgatgaatg aagcgaaaac ggaagccctg tctctgctgg aactgattga tgacgttcaa 360
   cgcctgggtc tgacctacaa attcgaaaaa gatatcatca aagccctgga aaaaattgtc 420
   ccgctggacg aatcaggtct gcacgtgacc tccctgtcat ttcgtatcct gcgccagcat 480
   ggcttcgaag tttcgcaaga tgtctttaaa cgtttcaaag acaaagaagg cggtttctgc 540
   gcagaactga aagatgacgt gcagggtctg ctgtctctgt atgaagctag ttacctgggt 600
   tttgaaggcg aatccctgct ggatgaagcg cgcgccttct caattaccca cctgaaaaac 660
   aatctgaaca aaggcatcaa tacgaaagtg gcacagcaag ttagtcatgc tctggaactg 720
   ccgtatcacc gtcgcctgca tcgtctggaa gcccgctggc tgctggataa atacgaaccg 780
   aaagaaccgc atcaccatct gctgcacgaa ctggcaaaac tggactttaa tctggttcag 840
   tcgctgtatc aaaaagaact gcgtgaactg agcctgtggt ggcgcgaaat tggtctgacc 900
   tctaaactgg attttgtgcg tgaccgcctg atggaagttt acttctgggc actgggcatg 960
   gctccggatc cgcagtttag cgaatgccgt aaagtggtta ccaaaatgtt cggtctggtg 1020
   acgattatcg atgacgtcta tgatgtgtac ggcaccctgg acgaactgca actgttcacg 1080
   gatgcagtcg aacgctggga cgtgaacgct atcaataccc tgccggatta tatgaaactg 1140
   tgttatctgg cactgtacaa caccgttaat gacacggctt acagcatcct gaaagaaaaa 1200
   ggtcataaca acatctccta cctgaccaaa tcatggtgcg aactgtgtaa agcgtttctg 1260
   caggaagcca aatggtctaa caataaaatt atcccggcgt tcaacaaata tctggataat 1320
   gccagtgtta gttcctcagg cgtcgcactg ctggctccgt cctactttct ggtctgtcag 1380
   gaacaagata tttcggacca ggcactgcac agcctgacca actttcatgg tctggttcgt 1440
   tcgagctgca ccatcttccg cctgtgtaat gatctggcga cgtctagtgc cgaactggaa 1500
   cgtggcgaaa ccacgaactc cattacctca tatatgcacg aaaatgaaac gagtgaagaa 1560
   caggcgtgca aagaactgcg taacctgatc gatgccgaat ggaagaaaat gaacgaagaa 1620
   cgcgtgtcga atagcaccct gccgaaagcc tttcgtgaaa ttgcaatcaa tatggctcgc 1680
   atttcccatt gtacgtatca gtacggcgat ggtctgggcc gcccggacta cacgaccgaa 1740
   aaccgcatta aactgctgct gattgacccg ttcccgatta actga 1785
<210> 15
   <211> 1656
   <212> DNA
   <213> Artificial Sequence
<220>
   <223> Polynucleotide without chloroplast-localization signal, which encodes isoprene synthase derived from Mucuna bracteata (IspSM gene)
<400> 15
   atgtccgccg tttcaagcca gttctctcaa atcgccgaag acaatagccg tcgctcagca      60
   aattatcatc cgaatctgtg ggactttgaa tttctgcagt ctctggaaaa cgatagtaaa     120
   atggaaaaac tggaagaaaa agccaccaaa ctggaagaag aagtgcgtaa catgatgaat     180
   gaagcgaaaa cggaagccct gagcctgctg gaactgattg atgacgtcca acgcctgggt     240
   ctgacctaca aattcgaaaa agatatcatc aaagcactgg aaaaaattgt cccgctggac     300
   gaatcaggtc tgcacgtgac gtctctgagt tttcgtatcc tgcgccagca tggcttcgaa     360
   gtttcgcaag atgtctttaa acgtttcaaa gacaaagaag gcggtttctg cgcggaactg     420
   aaagatgacg tgcagggtct gctgtccctg tatgaagcct catacctggg ttttgaaggc     480
   gaatccctgc tggatgaagc gcgcgccttc tcaattaccc acctgaaaaa caatctgaac     540
   aaaggcatca atacgaaagt ggcacagcaa gttagccatg ctctggaact gccgtatcac     600
   cgtcgcctgc atcgtctgga agcacgctgg ctgctggata aatacgaacc gaaagaaccg     660
   catcaccatc tgctgcacga actggcgaaa ctggacttta atctggttca gtcgctgtat     720
   caaaaagaac tgcgtgaact gagcctgtgg tggcgcgaaa ttggtctgac ctctaaactg     780
   gattttgtgc gtgaccgcct gatggaagtt tacttctggg cactgggcat ggctccggat     840
   ccgcagttta gcgaatgccg taaagtggtt accaaaatgt tcggtctggt cacgattatc     900
   gatgacgtct atgatgtgta cggcaccctg gacgaactgc aactgttcac ggatgcggtc     960
   gaacgctggg acgtgaacgc catcaatacc ctgccggatt atatgaaact gtgttatctg    1020
   gcgctgtaca acaccgttaa tgacacggcc tatagcatcc tgaaagaaaa aggtcataac    1080
   aacatctcgt acctgaccaa aagctggtgc gaactgtgta aagcgtttct gcaggaagcc    1140
   aaatggtcta acaacaaaat catcccggca ttcaacaaat acctggataa tgctagtgtt 1200
   agctctagtg gcgtcgcact gctggctccg tcctactttc tggtgtgtca ggaacaagat 1260
   atttctgacc aggcgctgca cagtctgacc aactttcatg gtctggttcg ttcctcatgc 1320
   accatcttcc gcctgtgtaa tgatctggcg acgtcgagcg ccgaactgga acgtggcgaa 1380
   accacgaact cgattaccag ctatatgcac gaaaatgaaa cgagtgaaga acaggcatgc 1440
   aaagaactgc gtaacctgat cgatgctgaa tggaagaaaa tgaacgaaga acgcgtgtcc 1500
   aattcaaccc tgccgaaagc ctttcgtgaa attgcaatca atatggctcg catttcccat 1560
   tgtacgtatc agtacggcga tggtctgggc cgcccggact acacgaccga aaaccgtatt 1620
   aaactgctgc tgattgaccc gttcccgatt aactaa 1656
<210> 16
   <211> 399
   <212> DNA
   <213> Artificial Sequence
<220>
   <223> Ptac-Ttrp
<400> 16
   ggtaccagat ctccctgttg acaattaatc atcggctcta taatgtgtgg aatcgtgagc 60
   ggataacaat ttcacacaag gagactcccg ggagccgcca gttccgctgg cggcatttta 120
   actttcttta atgaagccgg aaaaatccta aattcattta atatttatct ttttaccgtt 180
   tcgcttaccc cggtcgaacg tcaacttacg tcatttttcc gcccaacagt aatataatca 240
   aacaaattaa tcccgcaaca taacaccagt aaaatcaata attttctcta agtcacttat 300
   tcctcaggta attgttaata tatccagaat gttcctcaaa atatattttc cctctatctt 360
   ctcgttgcgc ttaatttgac taattctcat tagggatcc 399
<210> 17
   <211> 3383
   <212> DNA
   <213> Artificial Sequence
<220>
   <223> Expression plasmid pSTV28-Ptac-Ttrp
<400> 17
   cgtatggcaa tgaaagacgg tgagctggtg atatgggata gtgttcaccc ttgttacacc     60
   gttttccatg agcaaactga aacgttttca tcgctctgga gtgaatacca cgacgatttc    120
   cggcagtttc tacacatata ttcgcaagat gtggcgtgtt acggtgaaaa cctggcctat    180
   ttccctaaag ggtttattga gaatatgttt ttcgtctcag ccaatccctg ggtgagtttc    240
   accagttttg atttaaacgt ggccaatatg gacaacttct tcgcccccgt tttcaccatg    300
   ggcaaatatt atacgcaagg cgacaaggtg ctgatgccgc tggcgattca ggttcatcat    360
   gccgtttgtg atggcttcca tgtcggcaga atgcttaatg aattacaaca gtactgcgat 420
   gagtggcagg gcggggcgta atttttttaa ggcagttatt ggtgccctta aacgcctggt 480
   gctacgcctg aataagtgat aataagcgga tgaatggcag aaattcgaaa gcaaattcga 540
   cccggtcgtc ggttcagggc agggtcgtta aatagccgct tatgtctatt gctggtttac 600
   cggtttattg actaccggaa gcagtgtgac cgtgtgcttc tcaaatgcct gaggccagtt 660
   tgctcaggct ctccccgtgg aggtaataat tgacgatatg atcatttatt ctgcctccca 720
   gagcctgata aaaacggtta gcgcttcgtt aatacagatg taggtgttcc acagggtagc 780
   cagcagcatc ctgcgatgca gatccggaac ataatggtgc agggcgcttg tttcggcgtg 840
   ggtatggtgg caggccccgt ggccggggga ctgttgggcg ctgccggcac ctgtcctacg 900
   agttgcatga taaagaagac agtcataagt gcggcgacga tagtcatgcc ccgcgcccac 960
   cggaaggagc taccggacag cggtgcggac tgttgtaact cagaataaga aatgaggccg 1020
   ctcatggcgt tccaatacgc aaaccgcctc tccccgcgcg ttggccgatt cattaatgca 1080
   gctggcacga caggtttccc gactggaaag cgggcagtga gcgcaacgca attaatgtga 1140
   gttagctcac tcattaggca ccccaggctt tacactttat gcttccggct cgtatgttgt 1200
   gtggaattgt gagcggataa caatttcaca caggaaacag ctatgaccat gattacgaat 1260
   tcgagctcgg taccagatct ccctgttgac aattaatcat cggctctata atgtgtggaa 1320
   tcgtgagcgg ataacaattt cacacaagga gactcccggg agccgccagt tccgctggcg 1380
   gcattttaac tttctttaat gaagccggaa aaatcctaaa ttcatttaat atttatcttt 1440
   ttaccgtttc gcttaccccg gtcgaacgtc aacttacgtc atttttccgc ccaacagtaa 1500
   tataatcaaa caaattaatc ccgcaacata acaccagtaa aatcaataat tttctctaag 1560
   tcacttattc ctcaggtaat tgttaatata tccagaatgt tcctcaaaat atattttccc 1620
   tctatcttct cgttgcgctt aatttgacta attctcatta gggatcctct agagtcgacc 1680
   tgcaggcatg caagcttggc actggccgtc gttttacaac gtcgtgactg ggaaaaccct 1740
   ggcgttaccc aacttaatcg ccttgcagca catccccctt tcgccagctg gcgtaatagc 1800
   gaagaggccc gcaccgatcg cccttcccaa cagttgcgca gcctgaatgg cgaatgagct 1860
   tatcgatgat aagctgtcaa acatgagaat tacaacttat atcgtatggg gctgacttca 1920
   ggtgctacat ttgaagagat aaattgcact gaaatctaga aatattttat ctgattaata 1980
   agatgatctt cttgagatcg ttttggtctg cgcgtaatct cttgctctga aaacgaaaaa 2040
   accgccttgc agggcggttt ttcgaaggtt ctctgagcta ccaactcttt gaaccgaggt 2100
   aactggcttg gaggagcgca gtcaccaaaa cttgtccttt cagtttagcc ttaaccggcg 2160
   catgacttca agactaactc ctctaaatca attaccagtg gctgctgcca gtggtgcttt 2220
   tgcatgtctt tccgggttgg actcaagacg atagttaccg gataaggcgc agcggtcgga 2280
   ctgaacgggg ggttcgtgca tacagtccag cttggagcga actgcctacc cggaactgag 2340
   tgtcaggcgt ggaatgagac aaacgcggcc ataacagcgg aatgacaccg gtaaaccgaa 2400
   aggcaggaac aggagagcgc acgagggagc cgccagggga aacgcctggt atctttatag 2460
   tcctgtcggg tttcgccacc actgatttga gcgtcagatt tcgtgatgct tgtcaggggg 2520
   gcggagccta tggaaaaacg gctttgccgc ggccctctca cttccctgtt aagtatcttc 2580
   ctggcatctt ccaggaaatc tccgccccgt tcgtaagcca tttccgctcg ccgcagtcga 2640
   acgaccgagc gtagcgagtc agtgagcgag gaagcggaat atatcctgta tcacatattc 2700
   tgctgacgca ccggtgcagc cttttttctc ctgccacatg aagcacttca ctgacaccct 2760
   catcagtgcc aacatagtaa gccagtatac actccgctag cgctgatgtc cggcggtgct 2820
   tttgccgtta cgcaccaccc cgtcagtagc tgaacaggag ggacagctga tagaaacaga 2880
   agccactgga gcacctcaaa aacaccatca tacactaaat cagtaagttg gcagcatcac 2940
   ccgacgcact ttgcgccgaa taaatacctg tgacggaaga tcacttcgca gaataaataa 3000
   atcctggtgt ccctgttgat accgggaagc cctgggccaa cttttggcga aaatgagacg 3060
   ttgatcggca cgtaagaggt tccaactttc accataatga aataagatca ctaccgggcg 3120
   tattttttga gttatcgaga ttttcaggag ctaaggaagc taaaatggag aaaaaaatca 3180
   ctggatatac caccgttgat atatcccaat ggcatcgtaa agaacatttt gaggcatttc 3240
   agtcagttgc tcaatgtacc tataaccaga ccgttcagct ggatattacg gcctttttaa 3300
   agaccgtaaa gaaaaataag cacaagtttt atccggcctt tattcacatt cttgcccgcc 3360
   tgatgaatgc tcatccggaa ttt 3383
<210> 18
   <211> 79
   <212> DNA
   <213> Artificial Sequence
<220>
   <223> Primer for amplifying IspSK gene (Ptac-IspS(K)F)
<400> 18
   gataacaatt tcacacaata attttgttta actttaagaa ggagatataa tgtgtgcgac 60
   ctcttctcaa tttactcag 79
<210> 19
   <211> 42
   <212> DNA
   <213> Artificial Sequence
<220>
   <223> Primer for amplifying IspSK gene (IspS(K)R-MCSR)
<400> 19
   acggccagtg aattcttaga catacatcag ctggttaatc gg 42
<210> 20
   <211> 79
   <212> DNA
   <213> Artificial Sequence
<220>
   <223> Primer for amplifying IspSP gene (Ptac-IspS(P)F)
<400> 20
   gataacaatt tcacacaata attttgttta actttaagaa ggagatataa tgtgctctgt 60
   ttctaccgag aacgtttcc 79
<210> 21
   <211> 43
   <212> DNA
   <213> Artificial Sequence
<220>
   <223> Primer for amplifying IspSP gene (IspS(P)R-MCSR)
<400> 21
   acggccagtg aattcttaac gttcgaacgg cagaatcggt tcg 43
<210> 22
   <211> 69
   <212> DNA
   <213> Artificial Sequence
<220>
   <223> Primer for amplifying IspSM gene (Ptac-IspS(K)F)
<400> 22
   gataacaatt tcacacaata attttgttta actttaagaa ggagatataa tgtccgccgt 60
   ttcaagcca 69
<210> 23
   <211> 35
   <212> DNA
   <213> Artificial Sequence
<220>
   <223> Primer for amplifying IspSM gene
<400> 23
   acggccagtg aattcttagt taatcgggaa cgggt 35
<210> 24
   <211> 79
   <212> DNA
   <213> Artificial Sequence
<220>
   <223> Primer for amplifying IspSM(L) gene
<400> 24
   gataacaatt tcacacaata attttgttta actttaagaa ggagatataa tggctaccaa 60
   cccgtcctgt ctgtcaacc 79
<210> 25
   <211> 35
   <212> DNA
   <213> Artificial Sequence
<220>
   <223> Primer for amplifying IspSM(L) gene
<400> 25
   acggccagtg aattctcagt taatcgggaa cgggt 35
<210> 26
   <211> 29
   <212> DNA
   <213> Artificial Sequence
<220>
   <223> Primer for amplifying IpSTV28-Ptac-Ttrp construct (pSTV28-F)
<400> 26
   gtgtgaaatt gttatccgct cacaattcc 29
<210> 27
   <211> 27
   <212> DNA
   <213> Artificial Sequence
<220>
   <223> Primer for amplifying pSTV28-Ptac-Ttrp construct (pSTV28-R)
<400> 27
   gaattcactg gccgtcgttt tacaacg 27
<210> 28
   <211> 41
   <212> DNA
   <213> Artificial Sequence
<220>
   <223> Primer for amplifying dxs gene (dxs-F)
<400> 28
   caggaaacag ctatgagttt tgatattgcc aaatacccga c 41
<210> 29
   <211> 28
   <212> DNA
   <213> Artificial Sequence
<220>
   <223> Primer for amplifying dxs gene (dxs-R)
<400> 29
   gctgccactc ctgctatact cgtcatac 28
<210> 30
   <211> 30
   <212> DNA
   <213> Artificial Sequence
<220>
   <223> Primer for amplifying dxs gene (pMW219-F)
<400> 30
   catagctgtt tcctgtgtga aattgttatc 30
<210> 31
   <211> 40
   <212> DNA
   <213> Artificial Sequence
<220>
   <223> Primer for amplifying dxs gene (pMW219-R)
<400> 31
   agcaggagtg gcagcgaatt cgagctcggt acccggggat 40
<210> 32
   <211> 40
   <212> DNA
   <213> Artificial Sequence
<220>
   <223> Primer for amplifying ERG12 gene (MVK-IFS_5742-33-1)
<400> 32
   acacaaggag actcccatgt cattaccgtt cttaacttct 40
<210> 33
   <211> 46
   <212> DNA
   <213> Artificial Sequence
<220>
   <223> Primer for amplifying ERG12 gene (MVK-IFA_5742-33-2)
<400> 33
   ggaactggcg gctcccgggt tattatgaag tccatggtaa attcgt 46
<210> 34
   <211> 38
   <212> DNA
   <213> Artificial Sequence
<220>
   <223> Primer for amplifying ERG8 gene (PMK-IFS_5742-33-3)
<400> 34
   acacaaggag actcccatgt cagagttgag agccttca 38
<210> 35
   <211> 46
   <212> DNA
   <213> Artificial Sequence
<220>
   <223> Primer for amplifying ERG8 gene (PMK-IFA_5742-33-4)
<400> 35
   ggaactggcg gctcccgggt tattatttat caagataagt ttccgg 46
<210> 36
   <211> 37
   <212> DNA
   <213> Artificial Sequence
<220>
   <223> Primer for amplifying ERG19 gene (MVD-IFS_5742-33-5)
<400> 36
   acacaaggag actcccatga ccgtttacac agcatcc 37
<210> 37
   <211> 46
   <212> DNA
   <213> Artificial Sequence
<220>
   <223> Primer for amplifying ERG19 gene (MVD-IFA_5742-33-6)
<400> 37
   ggaactggcg gctcccgggt tattattcct ttggtagacc agtctt 46
<210> 38
   <211> 36
   <212> DNA
   <213> Artificial Sequence
<220>
   <223> Primer for amplifying IDI1 gene (yIDI-IFS_5742-33-7)
<400> 38
   acacaaggag actcccatgc cccatggtgc agtatc 36
<210> 39
   <211> 49
   <212> DNA
   <213> Artificial Sequence
<220>
   <223> Primer for amplifying IDI1 gene (yIDI-IFA_5742-33-8)
<400> 39
   ggaactggcg gctcccgggt tattatagca ttctatgaat ttgcctgtc 49
<210> 40
   <211> 5892
   <212> DNA
   <213> Artificial Sequence
<220>
   <223> Nucleotide sequence of prepared plasmid pUC-mvk-pmk
<400> 40
   agcgcccaat acgcaaaccg cctctccccg cgcgttggcc gattcattaa tgcagctggc 60
   acgacaggtt tcccgactgg aaagcgggca gtgagcgcaa cgcaattaat gtgagttagc 120
   tcactcatta ggcaccccag gctttacact ttatgcttcc ggctcgtatg ttgtgtggaa 180
   ttgtgagcgg ataacaattt cacacaggaa acagctatga ccatgattac gaattcgagc 240
   tcggtaccca tgtcattacc gttcttaact tctgcaccgg gaaaggttat tatttttggt 300
   gaacactctg ctgtgtacaa caagcctgcc gtcgctgcta gtgtgtctgc gttgagaacc 360
   tacctgctaa taagcgagtc atctgcacca gatactattg aattggactt cccggacatt 420
   agctttaatc ataagtggtc catcaatgat ttcaatgcca tcaccgagga tcaagtaaac 480
   tcccaaaaat tggccaaggc tcaacaagcc accgatggct tgtctcagga actcgttagt 540
   cttttggatc cgttgttagc tcaactatcc gaatccttcc actaccatgc agcgttttgt 600
   ttcctgtata tgtttgtttg cctatgcccc catgccaaga atattaagtt ttctttaaag 660
   tctactttac ccatcggtgc tgggttgggc tcaagcgcct ctatttctgt atcactggcc 720
   ttagctatgg cctacttggg ggggttaata ggatctaatg acttggaaaa gctgtcagaa 780
   aacgataagc atatagtgaa tcaatgggcc ttcataggtg aaaagtgtat tcacggtacc 840
   ccttcaggaa tagataacgc tgtggccact tatggtaatg ccctgctatt tgaaaaagac 900
   tcacataatg gaacaataaa cacaaacaat tttaagttct tagatgattt cccagccatt 960
   ccaatgatcc taacctatac tagaattcca aggtctacaa aagatcttgt tgctcgcgtt 1020
   cgtgtgttgg tcaccgagaa atttcctgaa gttatgaagc caattctaga tgccatgggt 1080
   gaatgtgccc tacaaggctt agagatcatg actaagttaa gtaaatgtaa aggcaccgat 1140
   gacgaggctg tagaaactaa taatgaactg tatgaacaac tattggaatt gataagaata 1200
   aatcatggac tgcttgtctc aatcggtgtt tctcatcctg gattagaact tattaaaaat 1260
   ctgagcgatg atttgagaat tggctccaca aaacttaccg gtgctggtgg cggcggttgc 1320
   tctttgactt tgttacgaag agacattact caagagcaaa ttgacagctt caaaaagaaa 1380
   ttgcaagatg attttagtta cgagacattt gaaacagact tgggtgggac tggctgctgt 1440
   ttgttaagcg caaaaaattt gaataaagat cttaaaatca aatccctagt attccaatta 1500
   tttgaaaata aaactaccac aaagcaacaa attgacgatc tattattgcc aggaaacacg 1560
   aatttaccat ggacttcata agctaatttg cgataggcct gcacccttaa ggaggaaaaa 1620
   aacatgtcag agttgagagc cttcagtgcc ccagggaaag cgttactagc tggtggatat 1680
   ttagttttag atacaaaata tgaagcattt gtagtcggat tatcggcaag aatgcatgct 1740
   gtagcccatc cttacggttc attgcaaggg tctgataagt ttgaagtgcg tgtgaaaagt 1800
   aaacaattta aagatgggga gtggctgtac catataagtc ctaaaagtgg cttcattcct 1860
   gtttcgatag gcggatctaa gaaccctttc attgaaaaag ttatcgctaa cgtatttagc 1920
   tactttaaac ctaacatgga cgactactgc aatagaaact tgttcgttat tgatattttc 1980
   tctgatgatg cctaccattc tcaggaggat agcgttaccg aacatcgtgg caacagaaga 2040
   ttgagttttc attcgcacag aattgaagaa gttcccaaaa cagggctggg ctcctcggca 2100
   ggtttagtca cagttttaac tacagctttg gcctcctttt ttgtatcgga cctggaaaat 2160
   aatgtagaca aatatagaga agttattcat aatttagcac aagttgctca ttgtcaagct 2220
   cagggtaaaa ttggaagcgg gtttgatgta gcggcggcag catatggatc tatcagatat 2280
   agaagattcc cacccgcatt aatctctaat ttgccagata ttggaagtgc tacttacggc 2340
   agtaaactgg cgcatttggt tgatgaagaa gactggaata ttacgattaa aagtaaccat 2400
   ttaccttcgg gattaacttt atggatgggc gatattaaga atggttcaga aacagtaaaa 2460
   ctggtccaga aggtaaaaaa ttggtatgat tcgcatatgc cagaaagctt gaaaatatat 2520
   acagaactcg atcatgcaaa ttctagattt atggatggac tatctaaact agatcgctta 2580
   cacgagactc atgacgatta cagcgatcag atatttgagt ctcttgagag gaatgactgt 2640
   acctgtcaaa agtatcctga aatcacagaa gttagagatg cagttgccac aattagacgt 2700
   tcctttagaa aaataactaa agaatctggt gccgatatcg aacctcccgt acaaactagc 2760
   ttattggatg attgccagac cttaaaagga gttcttactt gcttaatacc tggtgctggt 2820
   ggttatgacg ccattgcagt gattactaag caagatgttg atcttagggc tcaaaccgct 2880
   aatgacaaaa gattttctaa ggttcaatgg ctggatgtaa ctcaggctga ctggggtgtt 2940
   aggaaagaaa aagatccgga aacttatctt gataaataag gggatcctct agagtcgacc 3000
   tgcaggcatg caagcttggc actggccgtc gttttacaac gtcgtgactg ggaaaaccct 3060
   ggcgttaccc aacttaatcg ccttgcagca catccccctt tcgccagctg gcgtaatagc 3120
   gaagaggccc gcaccgatcg cccttcccaa cagttgcgca gcctgaatgg cgaatggcgc 3180
   ctgatgcggt attttctcct tacgcatctg tgcggtattt cacaccgcat acgtcaaagc 3240
   aaccatagta cgcgccctgt agcggcgcat taagcgcggc gggtgtggtg gttacgcgca 3300
   gcgtgaccgc tacacttgcc agcgccctag cgcccgctcc tttcgctttc ttcccttcct 3360
   ttctcgccac gttcgccggc tttccccgtc aagctctaaa tcgggggctc cctttagggt 3420
   tccgatttag tgctttacgg cacctcgacc ccaaaaaact tgatttgggt gatggttcac 3480
   gtagtgggcc atcgccctga tagacggttt ttcgcccttt gacgttggag tccacgttct 3540
   ttaatagtgg actcttgttc caaactggaa caacactcaa ccctatctcg ggctattctt 3600
   ttgatttata agggattttg ccgatttcgg cctattggtt aaaaaatgag ctgatttaac 3660
   aaaaatttaa cgcgaatttt aacaaaatat taacgtttac aattttatgg tgcactctca 3720
   gtacaatctg ctctgatgcc gcatagttaa gccagccccg acacccgcca acacccgctg 3780
   acgcgccctg acgggcttgt ctgctcccgg catccgctta cagacaagct gtgaccgtct 3840
   ccgggagctg catgtgtcag aggttttcac cgtcatcacc gaaacgcgcg agacgaaagg 3900
   gcctcgtgat acgcctattt ttataggtta atgtcatgat aataatggtt tcttagacgt 3960
   caggtggcac ttttcgggga aatgtgcgcg gaacccctat ttgtttattt ttctaaatac 4020
   attcaaatat gtatccgctc atgagacaat aaccctgata aatgcttcaa taatattgaa 4080
   aaaggaagag tatgagtatt caacatttcc gtgtcgccct tattcccttt tttgcggcat 4140
   tttgccttcc tgtttttgct cacccagaaa cgctggtgaa agtaaaagat gctgaagatc 4200
   agttgggtgc acgagtgggt tacatcgaac tggatctcaa cagcggtaag atccttgaga 4260
   gttttcgccc cgaagaacgt tttccaatga tgagcacttt taaagttctg ctatgtggcg 4320
   cggtattatc ccgtattgac gccgggcaag agcaactcgg tcgccgcata cactattctc 4380
   agaatgactt ggttgagtac tcaccagtca cagaaaagca tcttacggat ggcatgacag 4440
   taagagaatt atgcagtgct gccataacca tgagtgataa cactgcggcc aacttacttc 4500
   tgacaacgat cggaggaccg aaggagctaa ccgctttttt gcacaacatg ggggatcatg 4560
   taactcgcct tgatcgttgg gaaccggagc tgaatgaagc cataccaaac gacgagcgtg 4620
   acaccacgat gcctgtagca atggcaacaa cgttgcgcaa actattaact ggcgaactac 4680
   ttactctagc ttcccggcaa caattaatag actggatgga ggcggataaa gttgcaggac 4740
   cacttctgcg ctcggccctt ccggctggct ggtttattgc tgataaatct ggagccggtg 4800
   agcgtgggtc tcgcggtatc attgcagcac tggggccaga tggtaagccc tcccgtatcg 4860
   tagttatcta cacgacgggg agtcaggcaa ctatggatga acgaaataga cagatcgctg 4920
   agataggtgc ctcactgatt aagcattggt aactgtcaga ccaagtttac tcatatatac 4980
   tttagattga tttaaaactt catttttaat ttaaaaggat ctaggtgaag atcctttttg 5040
   ataatctcat gaccaaaatc ccttaacgtg agttttcgtt ccactgagcg tcagaccccg 5100
   tagaaaagat caaaggatct tcttgagatc ctttttttct gcgcgtaatc tgctgcttgc 5160
   aaacaaaaaa accaccgcta ccagcggtgg tttgtttgcc ggatcaagag ctaccaactc 5220
   tttttccgaa ggtaactggc ttcagcagag cgcagatacc aaatactgtc cttctagtgt 5280
   agccgtagtt aggccaccac ttcaagaact ctgtagcacc gcctacatac ctcgctctgc 5340
   taatcctgtt accagtggct gctgccagtg gcgataagtc gtgtcttacc gggttggact 5400
   caagacgata gttaccggat aaggcgcagc ggtcgggctg aacggggggt tcgtgcacac 5460
   agcccagctt ggagcgaacg acctacaccg aactgagata cctacagcgt gagctatgag 5520
   aaagcgccac gcttcccgaa gggagaaagg cggacaggta tccggtaagc ggcagggtcg 5580
   gaacaggaga gcgcacgagg gagcttccag ggggaaacgc ctggtatctt tatagtcctg 5640
   tcgggtttcg ccacctctga cttgagcgtc gatttttgtg atgctcgtca ggggggcgga 5700
   gcctatggaa aaacgccagc aacgcggcct ttttacggtt cctggccttt tgctggcctt 5760
   ttgctcacat gttctttcct gcgttatccc ctgattctgt ggataaccgt attaccgcct 5820
   ttgagtgagc tgataccgct cgccgcagcc gaacgaccga gcgcagcgag tcagtgagcg 5880
   aggaagcgga ag 5892
<210> 41
   <211> 39
   <212> DNA
   <213> Artificial Sequence
<220>
   <223> Primer for amplifying ERG12 gene (KKS1-6038-2-1)
<400> 41
   tcgagctcgg tacccatgtc attaccgttc ttaacttct 39
<210> 42
   <211> 53
   <212> DNA
   <213> Artificial Sequence
<220>
   <223> Primer for amplifying ERG12 gene (KKA1-6038-2-2)
<400> 42
   ttaagggtgc aggcctatcg caaattagct tatgaagtcc atggtaaatt cgt 53
<210> 43
   <211> 50
   <212> DNA
   <213> Artificial Sequence
<220>
   <223> Primer for amplifying ERG8 gene (KKS2-6083-2-3)
<400> 43
   ggcctgcacc cttaaggagg aaaaaaacat gtcagagttg agagccttca 50
<210> 44
   <211> 39
   <212> DNA
   <213> Artificial Sequence
<220>
   <223> Primer for amplifying ERG8 gene (KKA2-6083-2-4)
<400> 44
   ctctagagga tccccttatt tatcaagata agtttccgg 39
<210> 45
   <211> 6135
   <212> DNA
   <213> Artificial Sequence
<220>
   <223> Nucleotide sequence of prepared plasmid pTWV-dmd-yidi
<400> 45
   gacgaaaggg cctcgtgata cgcctatttt tataggttaa tgtcatgata ataatggttt      60
   cttagacgtc aggtggcact tttcggggaa atgtgcgcgg aacccctatt tgtttatttt     120
   tctaaataca ttcaaatatg tatccgctca tgagacaata accctgataa atgcttcaat     180
   aatattgaaa aaggaagagt atgagtattc aacatttccg tgtcgccctt attccctttt     240
   ttgcggcatt ttgccttcct gtttttgctc acccagaaac gctggtgaaa gtaaaagatg     300
   ctgaagatca gttgggtgca cgagtgggtt acatcgaact ggatctcaac agcggtaaga     360
   tccttgagag ttttcgcccc gaagaacgtt ttccaatgat gagcactttt aaagttctgc     420
   tatgtggcgc ggtattatcc cgtattgacg ccgggcaaga gcaactcggt cgccgcatac     480
   actattctca gaatgacttg gttgagtact caccagtcac agaaaagcat cttacggatg     540
   gcatgacagt aagagaatta tgcagtgctg ccataaccat gagtgataac actgcggcca     600
   acttacttct gacaacgatc ggaggaccga aggagctaac cgcttttttg cacaacatgg     660
   gggatcatgt aactcgcctt gatcgttggg aaccggagct gaatgaagcc ataccaaacg     720
   acgagcgtga caccacgatg cctgtagcaa tggcaacaac gttgcgcaaa ctattaactg     780
   gcgaactact tactctagct tcccggcaac aattaataga ctggatggag gcggataaag     840
   ttgcaggacc acttctgcgc tcggcccttc cggctggctg gtttattgct gataaatctg     900
   gagccggtga gcgtgggtct cgcggtatca ttgcagcact ggggccagat ggtaagccct     960
   cccgtatcgt agttatctac acgacgggga gtcaggcaac tatggatgaa cgaaatagac    1020
   agatcgctga gataggtgcc tcactgatta agcattggta actgtcagac caagtttact    1080
   catatatact ttagattgat ttaaaacttc atttttaatt taaaaggatc taggtgaaga    1140
   tcctttttga taatctcatg accaaaatcc cttaacgtga gttttcgttc cactgagcgt    1200
   cagaccccgt agaaaagatc aaaggatctt cttgagatcc tttttttctg cgcgtaatct    1260
   gctgcttgca aacaaaaaaa ccaccgctac cagcggtggt ttgtttgccg gatcaagagc    1320
   taccaactct ttttccgaag gtaactggct tcagcagagc gcagatacca aatactgtcc    1380
   ttctagtgta gccgtagtta ggccaccact tcaagaactc tgtagcaccg cctacatacc    1440
   tcgctctgct aatcctgtta ccagtggctg ctgccagtgg cgataagtcg tgtcttaccg    1500
   ggttggactc aagacgatag ttaccggata aggcgcagcg gtcgggctga acggggggtt    1560
   cgtgcacaca gcccagcttg gagcgaacga cctacaccga actgagatac ctacagcgtg   1620
   agctatgaga aagcgccacg cttcccgaag ggagaaaggc ggacaggtat ccggtaagcg   1680
   gcagggtcgg aacaggagag cgcacgaggg agcttccagg gggaaacgcc tggtatcttt   1740
   atagtcctgt cgggtttcgc cacctctgac ttgagcgtcg atttttgtga tgctcgtcag   1800
   gggggcggag cctatggaaa aacgccagca acgcggcctt tttacggttc ctggcctttt   1860
   gctggccttt tgctcacatg ttctttcctg cgttatcccc tgattctgtg gataaccgta   1920
   ttaccgcctt tgagtgagct gataccgctc gccgcagccg aacgaccgag cgcagcgagt   1980
   cagtgagcga ggaagcggaa gagcgcctga tgcggtattt tctccttacg catctgtgcg   2040
   gtatttcaca ccgcatatgg tgcactctca gtacaatctg ctctgatgcc gcatagttaa   2100
   gccagtatac actccgctat cgctacgtga ctgggtcatg gctgcgcccc gacacccgcc   2160
   aacacccgct gacgcgccct gacgggcttg tctgctcccg gcatccgctt acagacaagc   2220
   tgtgaccgtc tccgggagct gcatgtgtca gaggttttca ccgtcatcac cgaaacgcgc   2280
   gaggcagctg cggtaaagct catcagcgtg gtcgtgaagc gattcacaga tgtctgcctg   2340
   ttcatccgcg tccagctcgt tgagtttctc cagaagcgtt aatgtctggc ttctgataaa   2400
   gcgggccatg ttaagggcgg ttttttcctg tttggtcact gatgcctccg tgtaaggggg   2460
   atttctgttc atgggggtaa tgataccgat gaaacgagag aggatgctca cgatacgggt   2520
   tactgatgat gaacatgccc ggttactgga acgttgtgag ggtaaacaac tggcggtatg   2580
   gatgcggcgg gaccagagaa aaatcactca gggtcaatgc cagcgcttcg ttaatacaga   2640
   tgtaggtgtt ccacagggta gccagcagca tcctgcgatg cagatccgga acataatggt   2700
   gcagggcgct gacttccgcg tttccagact ttacgaaaca cggaaaccga agaccattca   2760
   tgttgttgct caggtcgcag acgttttgca gcagcagtcg cttcacgttc gctcgcgtat   2820
   cggtgattca ttctgctaac cagtaaggca accccgccag cctagccggg tcctcaacga   2880
   caggagcacg atcatgcgca cccgtggcca ggacccaacg ctgcccgaga tgcgccgcgt   2940
   gcggctgctg gagatggcgg acgcgatgga tatgttctgc caagtaatgt gagttagctc   3000
   actcattagg caccccaggc tttacacttt atgcttccgg ctcgtatgtt gtgtggaatt   3060
   gtgagcggat aacaatttca cacaggaaac agctatgacc atgattacga attcgagctc   3120
   ggtacccatg accgtttaca cagcatccgt taccgcaccc gtcaacatcg caacccttaa   3180
   gtattggggg aaaagggaca cgaagttgaa tctgcccacc aattcgtcca tatcagtgac   3240
   tttatcgcaa gatgacctca gaacgttgac ctctgcggct actgcacctg agtttgaacg   3300
   cgacactttg tggttaaatg gagaaccaca cagcatcgac aatgaaagaa ctcaaaattg   3360
   tctgcgcgac ctacgccaat taagaaagga aatggaatcg aaggacgcct cattgcccac   3420
   attatctcaa tggaaactcc acattgtctc cgaaaataac tttcctacag cagctggttt   3480
   agcttcctcc gctgctggct ttgctgcatt ggtctctgca attgctaagt tataccaatt 3540
   accacagtca acttcagaaa tatctagaat agcaagaaag gggtctggtt cagcttgtag 3600
   atcgttgttt ggcggatacg tggcctggga aatgggaaaa gctgaagatg gtcatgattc 3660
   catggcagta caaatcgcag acagctctga ctggcctcag atgaaagctt gtgtcctagt 3720
   tgtcagcgat attaaaaagg atgtgagttc cactcagggt atgcaattga ccgtggcaac 3780
   ctccgaacta tttaaagaaa gaattgaaca tgtcgtacca aagagatttg aagtcatgcg 3840
   taaagccatt gttgaaaaag atttcgccac ctttgcaaag gaaacaatga tggattccaa 3900
   ctctttccat gccacatgtt tggactcttt ccctccaata ttctacatga atgacacttc 3960
   caagcgtatc atcagttggt gccacaccat taatcagttt tacggagaaa caatcgttgc 4020
   atacacgttt gatgcaggtc caaatgctgt gttgtactac ttagctgaaa atgagtcgaa 4080
   actctttgca tttatctata aattgtttgg ctctgttcct ggatgggaca agaaatttac 4140
   tactgagcag cttgaggctt tcaaccatca atttgaatca tctaacttta ctgcacgtga 4200
   attggatctt gagttgcaaa aggatgttgc cagagtgatt ttaactcaag tcggttcagg 4260
   cccacaagaa acaaacgaat ctttgattga cgcaaagact ggtctaccaa aggaataaga 4320
   tcaattcgct gcatcgccct taggaggtaa aaaaaaatga ctgccgacaa caatagtatg 4380
   ccccatggtg cagtatctag ttacgccaaa ttagtgcaaa accaaacacc tgaagacatt 4440
   ttggaagagt ttcctgaaat tattccatta caacaaagac ctaatacccg atctagtgag 4500
   acgtcaaatg acgaaagcgg agaaacatgt ttttctggtc atgatgagga gcaaattaag 4560
   ttaatgaatg aaaattgtat tgttttggat tgggacgata atgctattgg tgccggtacc 4620
   aagaaagttt gtcatttaat ggaaaatatt gaaaagggtt tactacatcg tgcattctcc 4680
   gtctttattt tcaatgaaca aggtgaatta cttttacaac aaagagccac tgaaaaaata 4740
   actttccctg atctttggac taacacatgc tgctctcatc cactatgtat tgatgacgaa 4800
   ttaggtttga agggtaagct agacgataag attaagggcg ctattactgc ggcggtgaga 4860
   aaactagatc atgaattagg tattccagaa gatgaaacta agacaagggg taagtttcac 4920
   tttttaaaca gaatccatta catggcacca agcaatgaac catggggtga acatgaaatt 4980
   gattacatcc tattttataa gatcaacgct aaagaaaact tgactgtcaa cccaaacgtc 5040
   aatgaagtta gagacttcaa atgggtttca ccaaatgatt tgaaaactat gtttgctgac 5100
   ccaagttaca agtttacgcc ttggtttaag attatttgcg agaattactt attcaactgg 5160
   tgggagcaat tagatgacct ttctgaagtg gaaaatgaca ggcaaattca tagaatgcta 5220
   taaggggatc ctctagagtc gacctgcagg catgcaagct tggcactggc cgtcgtttta 5280
   caacgtcgtg actgggaaaa ccctggcgtt acccaactta atcgccttgc agcacatccc 5340
   cctttcgcca gctggcgtaa tagcgaagag gcccgcaccg atcgcccttc ccaacagttg 5400
   cgcagcctga atggcgaatg gcgcctgatg cggtattttc tccttacgca tctgtgcggt 5460
   atttcacacc gcatacgtca aagcaaccat agtacgcgcc ctgtagcggc gcattaagcg 5520
   cggcgggtgt ggtggttacg cgcagcgtga ccgctacact tgccagcgcc ctagcgcccg 5580
   ctcctttcgc tttcttccct tcctttctcg ccacgttcgc cggctttccc cgtcaagctc 5640
   taaatcgggg gctcccttta gggttccgat ttagtgcttt acggcacctc gaccccaaaa 5700
   aacttgattt gggtgatggt tcacgtagtg ggccatcgcc ctgatagacg gtttttcgcc 5760
   ctttgacgtt ggagtccacg ttctttaata gtggactctt gttccaaact ggaacaacac 5820
   tcaaccctat ctcgggctat tcttttgatt tataagggat tttgccgatt tcggcctatt 5880
   ggttaaaaaa tgagctgatt taacaaaaat ttaacgcgaa ttttaacaaa atattaacgt 5940
   ttacaatttt atggtgcact ctcagtacaa tctgctctga tgccgcatag ttaagccagc 6000
   cccgacaccc gccaacaccc gctgacgcgc cctgacgggc ttgtctgctc ccggcatccg 6060
   cttacagaca agctgtgacc gtctccggga gctgcatgtg tcagaggttt tcaccgtcat 6120
   caccgaaacg cgcga 6135
<210> 46
   <211> 36
   <212> DNA
   <213> Artificial Sequence
<220>
   <223> Primer for amplifying ERG19 gene (DyIS1-6083-2-5)
<400> 46
   tcgagctcgg tacccatgac cgtttacaca gcatcc 36
<210> 47
   <211> 60
   <212> DNA
   <213> Artificial Sequence
<220>
   <223> Primer for amplifying ERG19 gene (DyIA1-6083-2-6)
<400> 47
   tttttttacc tcctaagggc gatgcagcga attgatctta ttcctttggt agaccagtct 60
<210> 48
   <211> 57
   <212> DNA
   <213> Artificial Sequence
<220>
   <223> Primer for amplifying IDI1 gene (DyIS2-6083-2-7)
<400> 48
   taggaggtaa aaaaaaatga ctgccgacaa caatagtatg ccccatggtg cagtatc 57
<210> 49
   <211> 42
   <212> DNA
   <213> Artificial Sequence
<220>
   <223> Primer for amplifying IDI1 gene (DyIA2-6083-2-8)
<400> 49
   ctctagagga tccccttata gcattctatg aatttgcctg tc 42
<210> 50
   <211> 9257
   <212> DNA
   <213> Artificial Sequence
<220>
   <223> Nucleotide sequence of prepared plasmid pTrc-KKDyI(beta))
<400> 50
   gtttgacagc ttatcatcga ctgcacggtg caccaatgct tctggcgtca ggcagccatc      60
   ggaagctgtg gtatggctgt gcaggtcgta aatcactgca taattcgtgt cgctcaaggc     120
   gcactcccgt tctggataat gttttttgcg ccgacatcat aacggttctg gcaaatattc     180
   tgaaatgagc tgttgacaat taatcatccg gctcgtataa tgtgtggaat tgtgagcgga     240
   taacaatttc acacaggaaa cagcgccgct gagaaaaagc gaagcggcac tgctctttaa     300
   caatttatca gacaatctgt gtgggcactc gaccggaatt atcgattaac tttattatta     360
   aaaattaaag aggtatatat taatgtatcg attaaataag gaggaataaa ccatgtcatt     420
   accgttctta acttctgcac cgggaaaggt tattattttt ggtgaacact ctgctgtgta     480
   caacaagcct gccgtcgctg ctagtgtgtc tgcgttgaga acctacctgc taataagcga     540
   gtcatctgca ccagatacta ttgaattgga cttcccggac attagcttta atcataagtg     600
   gtccatcaat gatttcaatg ccatcaccga ggatcaagta aactcccaaa aattggccaa     660
   ggctcaacaa gccaccgatg gcttgtctca ggaactcgtt agtcttttgg atccgttgtt     720
   agctcaacta tccgaatcct tccactacca tgcagcgttt tgtttcctgt atatgtttgt     780
   ttgcctatgc ccccatgcca agaatattaa gttttcttta aagtctactt tacccatcgg    840
   tgctgggttg ggctcaagcg cctctatttc tgtatcactg gccttagcta tggcctactt     900
   gggggggtta ataggatcta atgacttgga aaagctgtca gaaaacgata agcatatagt     960
   gaatcaatgg gccttcatag gtgaaaagtg tattcacggt accccttcag gaatagataa    1020
   cgctgtggcc acttatggta atgccctgct atttgaaaaa gactcacata atggaacaat    1080
   aaacacaaac aattttaagt tcttagatga tttcccagcc attccaatga tcctaaccta    1140
   tactagaatt ccaaggtcta caaaagatct tgttgctcgc gttcgtgtgt tggtcaccga    1200
   gaaatttcct gaagttatga agccaattct agatgccatg ggtgaatgtg ccctacaagg 1260
   cttagagatc atgactaagt taagtaaatg taaaggcacc gatgacgagg ctgtagaaac 1320
   taataatgaa ctgtatgaac aactattgga attgataaga ataaatcatg gactgcttgt 1380
   ctcaatcggt gtttctcatc ctggattaga acttattaaa aatctgagcg atgatttgag 1440
   aattggctcc acaaaactta ccggtgctgg tggcggcggt tgctctttga ctttgttacg 1500
   aagagacatt actcaagagc aaattgacag cttcaaaaag aaattgcaag atgattttag 1560
   ttacgagaca tttgaaacag acttgggtgg gactggctgc tgtttgttaa gcgcaaaaaa 1620
   tttgaataaa gatcttaaaa tcaaatccct agtattccaa ttatttgaaa ataaaactac 1680
   cacaaagcaa caaattgacg atctattatt gccaggaaac acgaatttac catggacttc 1740
   ataagctaat ttgcgatagg cctgcaccct taaggaggaa aaaaacatgt cagagttgag 1800
   agccttcagt gccccaggga aagcgttact agctggtgga tatttagttt tagatacaaa 1860
   atatgaagca tttgtagtcg gattatcggc aagaatgcat gctgtagccc atccttacgg 1920
   ttcattgcaa gggtctgata agtttgaagt gcgtgtgaaa agtaaacaat ttaaagatgg 1980
   ggagtggctg taccatataa gtcctaaaag tggcttcatt cctgtttcga taggcggatc 2040
   taagaaccct ttcattgaaa aagttatcgc taacgtattt agctacttta aacctaacat 2100
   ggacgactac tgcaatagaa acttgttcgt tattgatatt ttctctgatg atgcctacca 2160
   ttctcaggag gatagcgtta ccgaacatcg tggcaacaga agattgagtt ttcattcgca 2220
   cagaattgaa gaagttccca aaacagggct gggctcctcg gcaggtttag tcacagtttt 2280
   aactacagct ttggcctcct tttttgtatc ggacctggaa aataatgtag acaaatatag 2340
   agaagttatt cataatttag cacaagttgc tcattgtcaa gctcagggta aaattggaag 2400
   cgggtttgat gtagcggcgg cagcatatgg atctatcaga tatagaagat tcccacccgc 2460
   attaatctct aatttgccag atattggaag tgctacttac ggcagtaaac tggcgcattt 2520
   ggttgatgaa gaagactgga atattacgat taaaagtaac catttacctt cgggattaac 2580
   tttatggatg ggcgatatta agaatggttc agaaacagta aaactggtcc agaaggtaaa 2640
   aaattggtat gattcgcata tgccagaaag cttgaaaata tatacagaac tcgatcatgc 2700
   aaattctaga tttatggatg gactatctaa actagatcgc ttacacgaga ctcatgacga 2760
   ttacagcgat cagatatttg agtctcttga gaggaatgac tgtacctgtc aaaagtatcc 2820
   tgaaatcaca gaagttagag atgcagttgc cacaattaga cgttccttta gaaaaataac 2880
   taaagaatct ggtgccgata tcgaacctcc cgtacaaact agcttattgg atgattgcca 2940
   gaccttaaaa ggagttctta cttgcttaat acctggtgct ggtggttatg acgccattgc 3000
   agtgattact aagcaagatg ttgatcttag ggctcaaacc gctaatgaca aaagattttc 3060
   taaggttcaa tggctggatg taactcaggc tgactggggt gttaggaaag aaaaagatcc 3120
   ggaaacttat cttgataaat aacttaaggt agctgcatgc agaattcgcc cttaaggagg 3180
   aaaaaaaaat gaccgtttac acagcatccg ttaccgcacc cgtcaacatc gcaaccctta 3240
   agtattgggg gaaaagggac acgaagttga atctgcccac caattcgtcc atatcagtga 3300
   ctttatcgca agatgacctc agaacgttga cctctgcggc tactgcacct gagtttgaac 3360
   gcgacacttt gtggttaaat ggagaaccac acagcatcga caatgaaaga actcaaaatt 3420
   gtctgcgcga cctacgccaa ttaagaaagg aaatggaatc gaaggacgcc tcattgccca 3480
   cattatctca atggaaactc cacattgtct ccgaaaataa ctttcctaca gcagctggtt 3540
   tagcttcctc cgctgctggc tttgctgcat tggtctctgc aattgctaag ttataccaat 3600
   taccacagtc aacttcagaa atatctagaa tagcaagaaa ggggtctggt tcagcttgta 3660
   gatcgttgtt tggcggatac gtggcctggg aaatgggaaa agctgaagat ggtcatgatt 3720
   ccatggcagt acaaatcgca gacagctctg actggcctca gatgaaagct tgtgtcctag 3780
   ttgtcagcga tattaaaaag gatgtgagtt ccactcaggg tatgcaattg accgtggcaa 3840
   cctccgaact atttaaagaa agaattgaac atgtcgtacc aaagagattt gaagtcatgc 3900
   gtaaagccat tgttgaaaaa gatttcgcca cctttgcaaa ggaaacaatg atggattcca 3960
   actctttcca tgccacatgt ttggactctt tccctccaat attctacatg aatgacactt 4020
   ccaagcgtat catcagttgg tgccacacca ttaatcagtt ttacggagaa acaatcgttg 4080
   catacacgtt tgatgcaggt ccaaatgctg tgttgtacta cttagctgaa aatgagtcga 4140
   aactctttgc atttatctat aaattgtttg gctctgttcc tggatgggac aagaaattta 4200
   ctactgagca gcttgaggct ttcaaccatc aatttgaatc atctaacttt actgcacgtg 4260
   aattggatct tgagttgcaa aaggatgttg ccagagtgat tttaactcaa gtcggttcag 4320
   gcccacaaga aacaaacgaa tctttgattg acgcaaagac tggtctacca aaggaataag 4380
   atcaattcgc tgcatcgccc ttaggaggta aaaaaaaatg actgccgaca acaatagtat 4440
   gccccatggt gcagtatcta gttacgccaa attagtgcaa aaccaaacac ctgaagacat 4500
   tttggaagag tttcctgaaa ttattccatt acaacaaaga cctaataccc gatctagtga 4560
   gacgtcaaat gacgaaagcg gagaaacatg tttttctggt catgatgagg agcaaattaa 4620
   gttaatgaat gaaaattgta ttgttttgga ttgggacgat aatgctattg gtgccggtac 4680
   caagaaagtt tgtcatttaa tggaaaatat tgaaaagggt ttactacatc gtgcattctc 4740
   cgtctttatt ttcaatgaac aaggtgaatt acttttacaa caaagagcca ctgaaaaaat 4800
   aactttccct gatctttgga ctaacacatg ctgctctcat ccactatgta ttgatgacga 4860
   attaggtttg aagggtaagc tagacgataa gattaagggc gctattactg cggcggtgag 4920
   aaaactagat catgaattag gtattccaga agatgaaact aagacaaggg gtaagtttca 4980
   ctttttaaac agaatccatt acatggcacc aagcaatgaa ccatggggtg aacatgaaat 5040
   tgattacatc ctattttata agatcaacgc taaagaaaac ttgactgtca acccaaacgt 5100
   caatgaagtt agagacttca aatgggtttc accaaatgat ttgaaaacta tgtttgctga 5160
   cccaagttac aagtttacgc cttggtttaa gattatttgc gagaattact tattcaactg 5220
   gtgggagcaa ttagatgacc tttctgaagt ggaaaatgac aggcaaattc atagaatgct 5280
   ataactgcag ctggtaccat atgggaattc gaagctttct agaacaaaaa ctcatctcag 5340
   aagaggatct gaatagcgcc gtcgaccatc atcatcatca tcattgagtt taaacggtct 5400
   ccagcttggc tgttttggcg gatgagagaa gattttcagc ctgatacaga ttaaatcaga 5460
   acgcagaagc ggtctgataa aacagaattt gcctggcggc agtagcgcgg tggtcccacc 5520
   tgaccccatg ccgaactcag aagtgaaacg ccgtagcgcc gatggtagtg tggggtctcc 5580
   ccatgcgaga gtagggaact gccaggcatc aaataaaacg aaaggctcag tcgaaagact 5640
   gggcctttcg ttttatctgt tgtttgtcgg tgaacgctct cctgagtagg acaaatccgc 5700
   cgggagcgga tttgaacgtt gcgaagcaac ggcccggagg gtggcgggca ggacgcccgc 5760
   cataaactgc caggcatcaa attaagcaga aggccatcct gacggatggc ctttttgcgt 5820
   ttctacaaac tctttttgtt tatttttcta aatacattca aatatgtatc cgctcatgag 5880
   acaataaccc tgataaatgc ttcaataata ttgaaaaagg aagagtatga gtattcaaca 5940
   tttccgtgtc gcccttattc ccttttttgc ggcattttgc cttcctgttt ttgctcaccc 6000
   agaaacgctg gtgaaagtaa aagatgctga agatcagttg ggtgcacgag tgggttacat 6060
   cgaactggat ctcaacagcg gtaagatcct tgagagtttt cgccccgaag aacgttttcc 6120
   aatgatgagc acttttaaag ttctgctatg tggcgcggta ttatcccgtg ttgacgccgg 6180
   gcaagagcaa ctcggtcgcc gcatacacta ttctcagaat gacttggttg agtactcacc 6240
   agtcacagaa aagcatctta cggatggcat gacagtaaga gaattatgca gtgctgccat 6300
   aaccatgagt gataacactg cggccaactt acttctgaca acgatcggag gaccgaagga 6360
   gctaaccgct tttttgcaca acatggggga tcatgtaact cgccttgatc gttgggaacc 6420
   ggagctgaat gaagccatac caaacgacga gcgtgacacc acgatgcctg tagcaatggc 6480
   aacaacgttg cgcaaactat taactggcga actacttact ctagcttccc ggcaacaatt 6540
   aatagactgg atggaggcgg ataaagttgc aggaccactt ctgcgctcgg cccttccggc 6600
   tggctggttt attgctgata aatctggagc cggtgagcgt gggtctcgcg gtatcattgc 6660
   agcactgggg ccagatggta agccctcccg tatcgtagtt atctacacga cggggagtca 6720
   ggcaactatg gatgaacgaa atagacagat cgctgagata ggtgcctcac tgattaagca 6780
   ttggtaactg tcagaccaag tttactcata tatactttag attgatttaa aacttcattt 6840
   ttaatttaaa aggatctagg tgaagatcct ttttgataat ctcatgacca aaatccctta 6900
   acgtgagttt tcgttccact gagcgtcaga ccccgtagaa aagatcaaag gatcttcttg 6960
   agatcctttt tttctgcgcg taatctgctg cttgcaaaca aaaaaaccac cgctaccagc 7020
   ggtggtttgt ttgccggatc aagagctacc aactcttttt ccgaaggtaa ctggcttcag 7080
   cagagcgcag ataccaaata ctgtccttct agtgtagccg tagttaggcc accacttcaa 7140
   gaactctgta gcaccgccta catacctcgc tctgctaatc ctgttaccag tggctgctgc 7200
   cagtggcgat aagtcgtgtc ttaccgggtt ggactcaaga cgatagttac cggataaggc 7260
   gcagcggtcg ggctgaacgg ggggttcgtg cacacagccc agcttggagc gaacgaccta 7320
   caccgaactg agatacctac agcgtgagct atgagaaagc gccacgcttc ccgaagggag 7380
   aaaggcggac aggtatccgg taagcggcag ggtcggaaca ggagagcgca cgagggagct 7440
   tccaggggga aacgcctggt atctttatag tcctgtcggg tttcgccacc tctgacttga 7500
   gcgtcgattt ttgtgatgct cgtcaggggg gcggagccta tggaaaaacg ccagcaacgc 7560
   ggccttttta cggttcctgg ccttttgctg gccttttgct cacatgttct ttcctgcgtt 7620
   atcccctgat tctgtggata accgtattac cgcctttgag tgagctgata ccgctcgccg 7680
   cagccgaacg accgagcgca gcgagtcagt gagcgaggaa gcggaagagc gcctgatgcg 7740
   gtattttctc cttacgcatc tgtgcggtat ttcacaccgc atatggtgca ctctcagtac 7800
   aatctgctct gatgccgcat agttaagcca gtatacactc cgctatcgct acgtgactgg 7860
   gtcatggctg cgccccgaca cccgccaaca cccgctgacg cgccctgacg ggcttgtctg 7920
   ctcccggcat ccgcttacag acaagctgtg accgtctccg ggagctgcat gtgtcagagg 7980
   ttttcaccgt catcaccgaa acgcgcgagg cagcagatca attcgcgcgc gaaggcgaag 8040
   cggcatgcat ttacgttgac accatcgaat ggtgcaaaac ctttcgcggt atggcatgat 8100
   agcgcccgga agagagtcaa ttcagggtgg tgaatgtgaa accagtaacg ttatacgatg 8160
   tcgcagagta tgccggtgtc tcttatcaga ccgtttcccg cgtggtgaac caggccagcc 8220
   acgtttctgc gaaaacgcgg gaaaaagtgg aagcggcgat ggcggagctg aattacattc 8280
   ccaaccgcgt ggcacaacaa ctggcgggca aacagtcgtt gctgattggc gttgccacct 8340
   ccagtctggc cctgcacgcg ccgtcgcaaa ttgtcgcggc gattaaatct cgcgccgatc 8400
   aactgggtgc cagcgtggtg gtgtcgatgg tagaacgaag cggcgtcgaa gcctgtaaag 8460
   cggcggtgca caatcttctc gcgcaacgcg tcagtgggct gatcattaac tatccgctgg 8520
   atgaccagga tgccattgct gtggaagctg cctgcactaa tgttccggcg ttatttcttg 8580
   atgtctctga ccagacaccc atcaacagta ttattttctc ccatgaagac ggtacgcgac 8640
   tgggcgtgga gcatctggtc gcattgggtc accagcaaat cgcgctgtta gcgggcccat 8700
   taagttctgt ctcggcgcgt ctgcgtctgg ctggctggca taaatatctc actcgcaatc 8760
   aaattcagcc gatagcggaa cgggaaggcg actggagtgc catgtccggt tttcaacaaa 8820
   ccatgcaaat gctgaatgag ggcatcgttc ccactgcgat gctggttgcc aacgatcaga 8880
   tggcgctggg cgcaatgcgc gccattaccg agtccgggct gcgcgttggt gcggatatct 8940
   cggtagtggg atacgacgat accgaagaca gctcatgtta tatcccgccg tcaaccacca 9000
   tcaaacagga ttttcgcctg ctggggcaaa ccagcgtgga ccgcttgctg caactctctc 9060
   agggccaggc ggtgaagggc aatcagctgt tgcccgtctc actggtgaaa agaaaaacca 9120
   ccctggcgcc caatacgcaa accgcctctc cccgcgcgtt ggccgattca ttaatgcagc 9180
   tggcacgaca ggtttcccga ctggaaagcg ggcagtgagc gcaacgcaat taatgtgagt 9240
   tagcgcgaat tgatctg 9257
<210> 51
   <211> 36
   <212> DNA
   <213> Artificial Sequence
<220>
   <223> Primer (KKDS2_6038-3-2)
<400> 51
   gaggaataaa ccatgtcatt accgttctta acttct 36
<210> 52
   <211> 55
   <212> DNA
   <213> Artificial Sequence
<220>
   <223> Primer (KKMyIA_6038-2-9)
<400> 52
   aagggcgaat tctgcatgca gctaccttaa gttatttatc aagataagtt tccgg 55
<210> 53
   <211> 51
   <212> DNA
   <213> Artificial Sequence
<220>
   <223> Primer (KMS_6038-6-1)
<400> 53
   gcagaattcg cccttaagga ggaaaaaaaa atgaccgttt acacagcatc c 51
<210> 54
   <211> 47
   <212> DNA
   <213> Artificial Sequence
<220>
   <223> Primer (KDyIA_6038-3-3)
<400> 54
   ccatatggta ccagctgcag ttatagcatt ctatgaattt gcctgtc 47
<210> 55
   <211> 11386
   <212> DNA
   <213> Artificial Sequence
<220>
   <223> Nucleotide sequence of prepared plasmid pMW219-KKDyI-TaspA
<400> 55
   gacagtaaga cgggtaagcc tgttgatgat accgctgcct tactgggtgc attagccagt      60
   ctgaatgacc tgtcacggga taatccgaag tggtcagact ggaaaatcag agggcaggaa     120
   ctgcagaaca gcaaaaagtc agatagcacc acatagcaga cccgccataa aacgccctga     180
   gaagcccgtg acgggctttt cttgtattat gggtagtttc cttgcatgaa tccataaaag     240
   gcgcctgtag tgccatttac ccccattcac tgccagagcc gtgagcgcag cgaactgaat     300
   gtcacgaaaa agacagcgac tcaggtgcct gatggtcgga gacaaaagga atattcagcg     360
   atttgcccga gcttgcgagg gtgctactta agcctttagg gttttaaggt ctgttttgta     420
   gaggagcaaa cagcgtttgc gacatccttt tgtaatactg cggaactgac taaagtagtg     480
   agttatacac agggctggga tctattcttt ttatcttttt ttattctttc tttattctat     540
   aaattataac cacttgaata taaacaaaaa aaacacacaa aggtctagcg gaatttacag     600
   agggtctagc agaatttaca agttttccag caaaggtcta gcagaattta cagataccca     660
   caactcaaag gaaaaggact agtaattatc attgactagc ccatctcaat tggtatagtg     720
   attaaaatca cctagaccaa ttgagatgta tgtctgaatt agttgttttc aaagcaaatg     780
   aactagcgat tagtcgctat gacttaacgg agcatgaaac caagctaatt ttatgctgtg     840
   tggcactact caaccccacg attgaaaacc ctacaaggaa agaacggacg gtatcgttca     900
   cttataacca atacgctcag atgatgaaca tcagtaggga aaatgcttat ggtgtattag     960
   ctaaagcaac cagagagctg atgacgagaa ctgtggaaat caggaatcct ttggttaaag    1020
   gctttgagat tttccagtgg acaaactatg ccaagttctc aagcgaaaaa ttagaattag    1080
   tttttagtga agagatattg ccttatcttt tccagttaaa aaaattcata aaatataatc    1140
   tggaacatgt taagtctttt gaaaacaaat actctatgag gatttatgag tggttattaa    1200
   aagaactaac acaaaagaaa actcacaagg caaatataga gattagcctt gatgaattta    1260
   agttcatgtt aatgcttgaa aataactacc atgagtttaa aaggcttaac caatgggttt    1320
   tgaaaccaat aagtaaagat ttaaacactt acagcaatat gaaattggtg gttgataagc    1380
   gaggccgccc gactgatacg ttgattttcc aagttgaact agatagacaa atggatctcg    1440
   taaccgaact tgagaacaac cagataaaaa tgaatggtga caaaatacca acaaccatta    1500
   catcagattc ctacctacat aacggactaa gaaaaacact acacgatgct ttaactgcaa 1560
   aaattcagct caccagtttt gaggcaaaat ttttgagtga catgcaaagt aagcatgatc 1620
   tcaatggttc gttctcatgg ctcacgcaaa aacaacgaac cacactagag aacatactgg 1680
   ctaaatacgg aaggatctga ggttcttatg gctcttgtat ctatcagtga agcatcaaga 1740
   ctaacaaaca aaagtagaac aactgttcac cgttacatat caaagggaaa actgtccata 1800
   tgcacagatg aaaacggtgt aaaaaagata gatacatcag agcttttacg agtttttggt 1860
   gcattcaaag ctgttcacca tgaacagatc gacaatgtaa cagatgaaca gcatgtaaca 1920
   cctaatagaa caggtgaaac cagtaaaaca aagcaactag aacatgaaat tgaacacctg 1980
   agacaacttg ttacagctca acagtcacac atagacagcc tgaaacaggc gatgctgctt 2040
   atcgaatcaa agctgccgac aacacgggag ccagtgacgc ctcccgtggg gaaaaaatca 2100
   tggcaattct ggaagaaata gcgccattcg ccattcaggc tgcgcaactg ttgggaaggg 2160
   cgatcggtgc gggcctcttc gctattacgc cagctggcga aagggggatg tgctgcaagg 2220
   cgattaagtt gggtaacgcc agggttttcc cagtcacgac gttgtaaaac gacggccagt 2280
   gaattcgagc tcggtaccct gtttttccac tcttcgttca ctttcgccag gtagctggtg 2340
   aagacgaagg aagtcccgga gccatctgcg cggcgtacta cagcaatgtt ttgtgaaggc 2400
   agtttcagac ccggattcag tttggcgatg gcttcatcat cccacttctt gattttgccc 2460
   aggtagatgt cgccgagggt tttaccatcc agcaccagtt cgccagactt cagccctgga 2520
   atgttaaccg ccagcaccac gccgccaatc acggtcggga actggaacag accttcctga 2580
   gccagttttt cgtcagacag cggcgcgtca gaggcaccaa aatcaacggt attagcgata 2640
   atctgtttta cgccaccgga agaaccgata ccctggtagt taactttatt accggtttct 2700
   ttctggtaag tgtcagccca tttggcatac accggcgcag ggaaggttgc acctgcacct 2760
   gtcaggcttg cttctgcaaa cacagagaaa gcactcatcg ataaggtcgc ggcgacaaca 2820
   gttgcgacgg tggtacgcat aactttcata atgtctcctg ggaggattca taaagcattg 2880
   tttgttggct acgagaagca aaataggaca aacaggtgac agttatatgt aaggaatatg 2940
   acagttttat gacagagaga taaagtcttc agtctgattt aaataagcgt tgatattcag 3000
   tcaattacaa acattaataa cgaagagatg acagaaaaat tttcattctg tgacagagaa 3060
   aaagtagccg aagatgacgg tttgtcacat ggagttggca ggatgtttga ttaaaatgaa 3120
   gcctgctttt ttatactaag ttggcattat aaaaaagcat tgcttatcaa tttgttgcaa 3180
   cgaacaggtc actatcagtc aaaataaaat cattatttga tttcgaattc tcatgtttga 3240
   cagcttatca tcgataagct ttaatgcggt agtttatcac agttaaattg ctaacgcagt 3300
   caggcaccgt gtatgaaatc taacaatgcg ctcatcgtca tcctcggcac cgtcaccctg 3360
   gatgctgtag gcataggctt ggttatgccg gtactgccgg gcctcttgcg ggatatcgtc 3420
   cattccgaca gcatcgccag tcactatggc gtgctgctag cgctatatgc gttgatgcaa 3480
   tttctatgcg cacccgttct cggagcactg tccgaccgct ttggccgccg cccagtcctg 3540
   ctcgcttcgc tacttggagc cactatcgac tacgcgatca tggcgaccac acccgtcctg 3600
   tggatctccg gataagtaga cagcctgata agtcgcacga aaaacaggta ttgacaacat 3660
   gaagtaacat gcagtaagat acaaatcgct aggtaacact agcagcgtca accgggcgct 3720
   ctagctagag ccaagctagc ttggccggat ccgagatttt caggagctaa ggaagctaaa 3780
   atggagaaaa aaatcactgg atataccacc gttgatatat cccaatggca tcgtaaagaa 3840
   cattttgagg catttcagtc agttgctcaa tgtacctata accagaccgt tcagctggat 3900
   attacggcct ttttaaagac cgtaaagaaa aataagcaca agttttatcc ggcctttatt 3960
   cacattcttg cccgcctgat gaatgctcat ccggaattcc gtatggcaat gaaagacggt 4020
   gagctggtga tatgggatag tgttcaccct tgttacaccg ttttccatga gcaaactgaa 4080
   acgttttcat cgctctggag tgaataccac gacgatttcc ggcagtttct acacatatat 4140
   tcgcaagatg tggcgtgtta cggtgaaaac ctggcctatt tccctaaagg gtttattgag 4200
   aatatgtttt tcgtctcagc caatccctgg gtgagtttca ccagttttga tttaaacgtg 4260
   gccaatatgg acaacttctt cgcccccgtt ttcaccatgg gcaaatatta tacgcaaggc 4320
   gacaaggtgc tgatgccgct ggcgattcag gttcatcatg ccgtctgtga tggcttccat 4380
   gtcggcagaa tgcttaatga attacaacag tactgcgatg agtggcaggg cggggcgtaa 4440
   tttttttaag gcagttattg gtgcccttaa acgcctggtg ctacgcctga ataagtgata 4500
   ataagcggat gaatggcaga aattcgtcga agcttaacac agaaaaaagc ccgcacctga 4560
   cagtgcgggc tttttttttc gaccactgca gtctgttaca ggtcactaat accatctaag 4620
   tagttgattc atagtgactg catatgttgt gttttacagt attatgtagt ctgtttttta 4680
   tgcaaaatct aatttaatat attgatattt atatcatttt acgtttctcg ttcagctttt 4740
   ttatactaac ttgagcggcc cttgacgatg ccacatcctg agcaaataat tcaaccacta 4800
   attgtgagcg gataacacaa ggaggaaaca gctatgtcat taccgttctt aacttctgca 4860
   ccgggaaagg ttattatttt tggtgaacac tctgctgtgt acaacaagcc tgccgtcgct 4920
   gctagtgtgt ctgcgttgag aacctacctg ctaataagcg agtcatctgc accagatact 4980
   attgaattgg acttcccgga cattagcttt aatcataagt ggtccatcaa tgatttcaat 5040
   gccatcaccg aggatcaagt aaactcccaa aaattggcca aggctcaaca agccaccgat 5100
   ggcttgtctc aggaactcgt tagtcttttg gatccgttgt tagctcaact atccgaatcc 5160
   ttccactacc atgcagcgtt ttgtttcctg tatatgtttg tttgcctatg cccccatgcc 5220
   aagaatatta agttttcctt aaagtctact ttacccatcg gtgctgggtt gggctcaagc 5280
   gcctctattt ctgtatcact ggccttagct atggcctact tgggggggtt aataggatct 5340
   aatgacttgg aaaagctgtc agaaaacgat aagcatatag tgaatcaatg ggccttcata 5400
   ggtgaaaagt gtattcacgg taccccttca ggaatagata acgctgtggc cacttatggt 5460
   aatgccctgc tatttgaaaa agactcacat aatggaacaa taaacacaaa caattttaag 5520
   ttcttagatg atttcccagc cattccaatg atcctaacct atactagaat tccaaggtct 5580
   acaaaagatc ttgttgctcg cgttcgtgtg ttggtcaccg agaaatttcc tgaagttatg 5640
   aagccaattc tagatgccat gggtgaatgt gccctacaag gcttagagat catgactaag 5700
   ttaagtaaat gtaaaggcac cgatgacgag gctgtagaaa ctaataatga actgtatgaa 5760
   caactattgg aattgataag aataaatcat ggactgcttg tctcaatcgg tgtttctcat 5820
   cctggattag aacttattaa aaatctgagc gatgatttga gaattggctc cacaaaactt 5880
   accggtgctg gtggcggcgg ttgctctttg actttgttac gaagagacat tactcaagag 5940
   caaattgaca gcttcaaaaa gaaattgcaa gatgatttta gttacgagac atttgaaaca 6000
   gacttgggtg ggactggctg ctgtttgtta agcgcaaaaa atttgaataa agatcttaaa 6060
   atcaaatccc tagtattcca attatttgaa aataaaacta ccacaaagca acaaattgac 6120
   gatctattat tgccaggaaa cacgaattta ccatggactt cataagctaa tttgcgatag 6180
   gcctgcaccc ttaaggagga aaaaaacatg tcagagttga gagccttcag tgccccaggg 6240
   aaagcgttac tagctggtgg atatttagtt ttagatacaa aatatgaagc atttgtagtc 6300
   ggattatcgg caagaatgca tgctgtagcc catccttacg gttcattgca agggtctgat 6360
   aagtttgaag tgcgtgtgaa aagtaaacaa tttaaagatg gggagtggct gtaccatata 6420
   agtcctaaaa gtggcttcat tcctgtttcg ataggcggat ctaagaaccc tttcattgaa 6480
   aaagttatcg ctaacgtatt tagctacttt aaacctaaca tggacgacta ctgcaataga 6540
   aacttgttcg ttattgatat tttctctgat gatgcctacc attctcagga ggatagcgtt 6600
   accgaacatc gtggcaacag aagattgagt tttcattcgc acagaattga agaagttccc 6660
   aaaacagggc tgggctcctc ggcaggttta gtcacagttt taactacagc tttggcctcc 6720
   ttttttgtat cggacctgga aaataatgta gacaaatata gagaagttat tcataattta 6780
   gcacaagttg ctcattgtca agctcagggt aaaattggaa gcgggtttga tgtagcggcg 6840
   gcagcatatg gatctatcag atatagaaga ttcccacccg cattaatctc taatttgcca 6900
   gatattggaa gtgctactta cggcagtaaa ctggcgcatt tggttgatga agaagactgg 6960
   aatattacga ttaaaagtaa ccatttacct tcgggattaa ctttatggat gggcgatatt 7020
   aagaatggtt cagaaacagt aaaactggtc cagaaggtaa aaaattggta tgattcgcat 7080
   atgccagaaa gcttgaaaat atatacagaa ctcgatcatg caaattctag atttatggat 7140
   ggactatcta aactagatcg cttacacgag actcatgacg attacagcga tcagatattt 7200
   gagtctcttg agaggaatga ctgtacctgt caaaagtatc ctgaaatcac agaagttaga 7260
   gatgcagttg ccacaattag acgttccttt agaaaaataa ctaaagaatc tggtgccgat 7320
   atcgaacctc ccgtacaaac tagcttattg gatgattgcc agaccttaaa aggagttctt 7380
   acttgcttaa tacctggtgc tggtggttat gacgccattg cagtgattac taagcaagat 7440
   gttgatctta gggctcaaac cgctaatgac aaaagatttt ctaaggttca atggctggat 7500
   gtaactcagg ctgactgggg tgttaggaaa gaaaaagatc cggaaactta tcttgataaa 7560
   taacttaagg tagctgcatg cagaattcgc ccttaaggag gaaaaaaaaa tgaccgttta 7620
   cacagcatcc gttaccgcac ccgtcaacat cgcaaccctt aagtattggg ggaaaaggga 7680
   cacgaagttg aatctgccca ccaattcgtc catatcagtg actttatcgc aagatgacct 7740
   cagaacgttg acctctgcgg ctactgcacc tgagtttgaa cgcgacactt tgtggttaaa 7800
   tggagaacca cacagcatcg acaatgaaag aactcaaaat tgtctgcgcg acctacgcca 7860
   attaagaaag gaaatggaat cgaaggacgc ctcattgccc acattatctc aatggaaact 7920
   ccacattgtc tccgaaaata actttcctac agcagctggt ttagcttcct ccgctgctgg 7980
   ctttgctgca ttggtctctg caattgctaa gttataccaa ttaccacagt caacttcaga 8040
   aatatctaga atagcaagaa aggggtctgg ttcagcttgt agatcgttgt ttggcggata 8100
   cgtggcctgg gaaatgggaa aagctgaaga tggtcatgat tccatggcag tacaaatcgc 8160
   agacagctct gactggcctc agatgaaagc ttgtgtccta gttgtcagcg atattaaaaa 8220
   ggatgtgagt tccactcagg gtatgcaatt gaccgtggca acctccgaac tatttaaaga 8280
   aagaattgaa catgtcgtac caaagagatt tgaagtcatg cgtaaagcca ttgttgaaaa 8340
   agatttcgcc acctttgcaa aggaaacaat gatggattcc aactctttcc atgccacatg 8400
   tttggactct ttccctccaa tattctacat gaatgacact tccaagcgta tcatcagttg 8460
   gtgccacacc attaatcagt tttacggaga aacaatcgtt gcatacacgt ttgatgcagg 8520
   tccaaatgct gtgttgtact acttagctga aaatgagtcg aaactctttg catttatcta 8580
   taaattgttt ggctctgttc ctggatggga caagaaattt actactgagc agcttgaggc 8640
   tttcaaccat caatttgaat catctaactt tactgcacgt gaattggatc ttgagttgca 8700
   aaaggatgtt gccagagtga ttttaactca agtcggttca ggcccacaag aaacaaacga 8760
   atctttgatt gacgcaaaga ctggtctacc aaaggaataa gatcaattcg ctgcatcgcc 8820
   cttaggaggt aaaaaaaaat gactgccgac aacaatagta tgccccatgg tgcagtatct 8880
   agttacgcca aattagtgca aaaccaaaca cctgaagaca ttttggaaga gtttcctgaa 8940
   attattccat tacaacaaag acctaatacc cgatctagtg agacgtcaaa tgacgaaagc 9000
   ggagaaacat gtttttctgg tcatgatgag gagcaaatta agttaatgaa tgaaaattgt 9060
   attgttttgg attgggacga taatgctatt ggtgccggta ccaagaaagt ttgtcattta 9120
   atggaaaata ttgaaaaggg tttactacat cgtgcattct ccgtctttat tttcaatgaa 9180
   caaggtgaat tacttttaca acaaagagcc actgaaaaaa taactttccc tgatctttgg 9240
   actaacacat gctgctctca tccactatgt attgatgacg aattaggttt gaagggtaag 9300
   ctagacgata agattaaggg cgctattact gcggcggtga gaaaactaga tcatgaatta 9360
   ggtattccag aagatgaaac taagacaagg ggtaagtttc actttttaaa cagaatccat 9420
   tacatggcac caagcaatga accatggggt gaacatgaaa ttgattacat cctattttat 9480
   aagatcaacg ctaaagaaaa cttgactgtc aacccaaacg tcaatgaagt tagagacttc 9540
   aaatgggttt caccaaatga tttgaaaact atgtttgctg acccaagtta caagtttacg 9600
   ccttggttta agattatttg cgagaattac ttattcaact ggtgggagca attagatgac 9660
   ctttctgaag tggaaaatga caggcaaatt catagaatgc tataacaacg cgtctacaaa 9720
   taaaaaaggc acgtcagatg acgtgccttt tttcttgggg ccggggatcc tctagagtcg 9780
   acctgcaggc atgcaagctt ggcgtaatca tggtcatagc tgtttcctgt gtgaaattgt 9840
   tatccgctca caattccaca caacatacga gccggaagca taaagtgtaa agcctggggt 9900
   gcctaatgag tgagctaact cacattaatt gcgttgcgct cactgcccgc tttccagtcg 9960
   ggaaacctgt cgtgccagct gcattaatga atcggccaac gcgcggggag aggcggtttg 10020
   cgtattgggc gctttctcat agctcacgct gtaggtatct cagttcggtg taggtcgttc 10080
   gctccaagct gggctgtgtg ccgaacccca gagtcccgct cagaagaact cgtcaagaag 10140
   gcgatagaag gcgatgcgct gcgaatcggg agcggcgata ccgtaaagca cgaggaagcg 10200
   gtcagcccat tcgccgccaa gctcttcagc aatatcacgg gtagccaacg ctatgtcctg 10260
   atagcggtcc gccacaccca gccggccaca gtcgatgaat ccagaaaagc ggccattttc 10320
   caccatgata ttcggcaagc aggcatcgcc atgggtcacg acgagatcct cgccgtcggg 10380
   catgcgcgcc ttgagcctgg cgaacagttc ggctggcgcg agcccctgat gctcttcgtc 10440
   cagatcatcc tgatcgacaa gaccggcttc catccgagta cgtgctcgct cgatgcgatg 10500
   tttcgcttgg tggtcgaatg ggcaggtagc cggatcaagc gtatgcagcc gccgcattgc 10560
   atcagccatg atggatactt tctcggcagg agcaaggtga gatgacagga gatcctgccc 10620
   cggcacttcg cccaatagca gccagtccct tcccgcttca gtgccaacgt cgagcacagc 10680
   tgcgcaagga acgcccgtcg tggccagcca cgatagccgc gctgcctcgt cctgcagttc 10740
   attcagggca ccggacaggt cggtcttgac aaaaagaacc gggcgcccct gcgctgacag 10800
   ccggaacacg gcggcatcag agcagccgat tgtctgttgt gcccagtcat agccgaatag 10860
   cctctccacc caagcggccg gagaacctgc gtgcaatcca tcttgttcaa tcatgcgaaa 10920
   cgatcctcat cctgtctctt gatcactacc gcattaaagc atatcgatga taagctgtca 10980
   aacatgagcg ttcttcgggg cgaaaactct caaggatctt accgctgttg agatccagtt  11040
   cgatgtaacc cactcgtgca cccaactgat cttcagcatc ttttactttc accagcgttt  11100
   ctgggtgagc aaaaacagga aggcaaaatg ccgcaaaaaa gggaataagg gcgacacgga  11160
   aatgttgaat actcatactc ttcctttttc aatattattg aagcatttat cagggttatt  11220
   gtctcatgag cggatacata tttgaatgta tttagaaaaa taaacaaata ggggttccgc  11280
   gcacatttcc ccgaaaagtg ccacctgacg tctaagaaac cattattatc atgacattaa  11340
   cctataaaaa taggcgtatc acgaggccct ttcgtcttca agaatt                 11386
<210> 56
   <211> 12232
   <212> DNA
   <213> Artificial Sequence
<220>
   <223> Nucleotide sequence of the prepared plasmid pMW-Tn7-Pgi-KKDyI-TaspA-Tn7
<400> 56
   gacagtaaga cgggtaagcc tgttgatgat accgctgcct tactgggtgc attagccagt     60
   ctgaatgacc tgtcacggga taatccgaag tggtcagact ggaaaatcag agggcaggaa    120
   ctgcagaaca gcaaaaagtc agatagcacc acatagcaga cccgccataa aacgccctga    180
   gaagcccgtg acgggctttt cttgtattat gggtagtttc cttgcatgaa tccataaaag    240
   gcgcctgtag tgccatttac ccccattcac tgccagagcc gtgagcgcag cgaactgaat    300
   gtcacgaaaa agacagcgac tcaggtgcct gatggtcgga gacaaaagga atattcagcg    360
   atttgcccga gcttgcgagg gtgctactta agcctttagg gttttaaggt ctgttttgta    420
   gaggagcaaa cagcgtttgc gacatccttt tgtaatactg cggaactgac taaagtagtg    480
   agttatacac agggctggga tctattcttt ttatcttttt ttattctttc tttattctat    540
   aaattataac cacttgaata taaacaaaaa aaacacacaa aggtctagcg gaatttacag    600
   agggtctagc agaatttaca agttttccag caaaggtcta gcagaattta cagataccca    660
   caactcaaag gaaaaggact agtaattatc attgactagc ccatctcaat tggtatagtg    720
   attaaaatca cctagaccaa ttgagatgta tgtctgaatt agttgttttc aaagcaaatg    780
   aactagcgat tagtcgctat gacttaacgg agcatgaaac caagctaatt ttatgctgtg    840
   tggcactact caaccccacg attgaaaacc ctacaaggaa agaacggacg gtatcgttca    900
   cttataacca atacgctcag atgatgaaca tcagtaggga aaatgcttat ggtgtattag    960
   ctaaagcaac cagagagctg atgacgagaa ctgtggaaat caggaatcct ttggttaaag   1020
   gctttgagat tttccagtgg acaaactatg ccaagttctc aagcgaaaaa ttagaattag   1080
   tttttagtga agagatattg ccttatcttt tccagttaaa aaaattcata aaatataatc   1140
   tggaacatgt taagtctttt gaaaacaaat actctatgag gatttatgag tggttattaa 1200
   aagaactaac acaaaagaaa actcacaagg caaatataga gattagcctt gatgaattta 1260
   agttcatgtt aatgcttgaa aataactacc atgagtttaa aaggcttaac caatgggttt 1320
   tgaaaccaat aagtaaagat ttaaacactt acagcaatat gaaattggtg gttgataagc 1380
   gaggccgccc gactgatacg ttgattttcc aagttgaact agatagacaa atggatctcg 1440
   taaccgaact tgagaacaac cagataaaaa tgaatggtga caaaatacca acaaccatta 1500
   catcagattc ctacctacat aacggactaa gaaaaacact acacgatgct ttaactgcaa 1560
   aaattcagct caccagtttt gaggcaaaat ttttgagtga catgcaaagt aagcatgatc 1620
   tcaatggttc gttctcatgg ctcacgcaaa aacaacgaac cacactagag aacatactgg 1680
   ctaaatacgg aaggatctga ggttcttatg gctcttgtat ctatcagtga agcatcaaga 1740
   ctaacaaaca aaagtagaac aactgttcac cgttacatat caaagggaaa actgtccata 1800
   tgcacagatg aaaacggtgt aaaaaagata gatacatcag agcttttacg agtttttggt 1860
   gcattcaaag ctgttcacca tgaacagatc gacaatgtaa cagatgaaca gcatgtaaca 1920
   cctaatagaa caggtgaaac cagtaaaaca aagcaactag aacatgaaat tgaacacctg 1980
   agacaacttg ttacagctca acagtcacac atagacagcc tgaaacaggc gatgctgctt 2040
   atcgaatcaa agctgccgac aacacgggag ccagtgacgc ctcccgtggg gaaaaaatca 2100
   tggcaattct ggaagaaata gcgccattcg ccattcaggc tgcgcaactg ttgggaaggg 2160
   cgatcggtgc gggcctcttc gctattacgc cagctggcga aagggggatg tgctgcaagg 2220
   cgattaagtt gggtaacgcc agggttttcc cagtcacgac gttgtaaaac gacggccagt 2280
   gaattcgagc tcggtaccct gtttttccac tcttcgttca ctttcgccag gtagctggtg 2340
   aagacgaagg aagtcccgga gccatctgcg cggcgtacta cagcaatgtt ttgtgaaggc 2400
   agtttcagac ccggattcag tttggcgatg gcttcatcat cccacttctt gattttgccc 2460
   aggtagatgt cgccgagggt tttaccatcc agcaccagtt cgccagactt cagccctgga 2520
   atgttaaccg ccagcaccac gccgccaatc acggtcggga actggaacag accttcctga 2580
   gccagttttt cgtcagacag cggcgcgtca gaggcaccaa aatcaacggt attagcgata 2640
   atctgtttta cgccaccgga agaaccgata ccctggtagt taactttatt accggtttct 2700
   ttctggtaag tgtcagccca tttggcatac accggcgcag ggaaggttgc acctgcacct 2760
   gtcaggcttg cttctgcaaa cacagagaaa gcactcatcg ataaggtcgc ggcgacaaca 2820
   gttgcgacgg tggtacgcat aactttcata atgtctcctg ggaggattca taaagcattg 2880
   tttgttggct acgagaagca aaataggaca aacaggtgac agttatatgt aaggaatatg 2940
   acagttttat gacagagaga taaagtcttc agtctgattt aaataagcgt tgatattcag 3000
   tcaattacaa acattaataa cgaagagatg acagaaaaat tttcattctg tgacagagaa 3060
   aaagtagccg aagatgacgg tttgtcacat ggagttggca ggatgtttga ttaaaatgaa 3120
   gcctgctttt ttatactaag ttggcattat aaaaaagcat tgcttatcaa tttgttgcaa 3180
   cgaacaggtc actatcagtc aaaataaaat cattatttga tttcgaattc tcatgtttga 3240
   cagcttatca tcgataagct ttaatgcggt agtttatcac agttaaattg ctaacgcagt 3300
   caggcaccgt gtatgaaatc taacaatgcg ctcatcgtca tcctcggcac cgtcaccctg 3360
   gatgctgtag gcataggctt ggttatgccg gtactgccgg gcctcttgcg ggatatcgtc 3420
   cattccgaca gcatcgccag tcactatggc gtgctgctag cgctatatgc gttgatgcaa 3480
   tttctatgcg cacccgttct cggagcactg tccgaccgct ttggccgccg cccagtcctg 3540
   ctcgcttcgc tacttggagc cactatcgac tacgcgatca tggcgaccac acccgtcctg 3600
   tggatctccg gataagtaga cagcctgata agtcgcacga aaaacaggta ttgacaacat 3660
   gaagtaacat gcagtaagat acaaatcgct aggtaacact agcagcgtca accgggcgct 3720
   ctagctagag ccaagctagc ttggccggat ccgagatttt caggagctaa ggaagctaaa 3780
   atggagaaaa aaatcactgg atataccacc gttgatatat cccaatggca tcgtaaagaa 3840
   cattttgagg catttcagtc agttgctcaa tgtacctata accagaccgt tcagctggat 3900
   attacggcct ttttaaagac cgtaaagaaa aataagcaca agttttatcc ggcctttatt 3960
   cacattcttg cccgcctgat gaatgctcat ccggaattcc gtatggcaat gaaagacggt 4020
   gagctggtga tatgggatag tgttcaccct tgttacaccg ttttccatga gcaaactgaa 4080
   acgttttcat cgctctggag tgaataccac gacgatttcc ggcagtttct acacatatat 4140
   tcgcaagatg tggcgtgtta cggtgaaaac ctggcctatt tccctaaagg gtttattgag 4200
   aatatgtttt tcgtctcagc caatccctgg gtgagtttca ccagttttga tttaaacgtg 4260
   gccaatatgg acaacttctt cgcccccgtt ttcaccatgg gcaaatatta tacgcaaggc 4320
   gacaaggtgc tgatgccgct ggcgattcag gttcatcatg ccgtctgtga tggcttccat 4380
   gtcggcagaa tgcttaatga attacaacag tactgcgatg agtggcaggg cggggcgtaa 4440
   tttttttaag gcagttattg gtgcccttaa acgcctggtg ctacgcctga ataagtgata 4500
   ataagcggat gaatggcaga aattcgtcga agcttaacac agaaaaaagc ccgcacctga 4560
   cagtgcgggc tttttttttc gaccactgca gtctgttaca ggtcactaat accatctaag 4620
   tagttgattc atagtgactg catatgttgt gttttacagt attatgtagt ctgtttttta 4680
   tgcaaaatct aatttaatat attgatattt atatcatttt acgtttctcg ttcagctttt 4740
   ttatactaac ttgagcggcc cttgacgatg ccacatcctg agcaaataat tcaaccacta 4800
   attgtgagcg gataacacaa ggaggaaaca gctatgtcat taccgttctt aacttctgca 4860
   ccgggaaagg ttattatttt tggtgaacac tctgctgtgt acaacaagcc tgccgtcgct 4920
   gctagtgtgt ctgcgttgag aacctacctg ctaataagcg agtcatctgc accagatact 4980
   attgaattgg acttcccgga cattagcttt aatcataagt ggtccatcaa tgatttcaat 5040
   gccatcaccg aggatcaagt aaactcccaa aaattggcca aggctcaaca agccaccgat 5100
   ggcttgtctc aggaactcgt tagtcttttg gatccgttgt tagctcaact atccgaatcc 5160
   ttccactacc atgcagcgtt ttgtttcctg tatatgtttg tttgcctatg cccccatgcc 5220
   aagaatatta agttttcctt aaagtctact ttacccatcg gtgctgggtt gggctcaagc 5280
   gcctctattt ctgtatcact ggccttagct atggcctact tgggggggtt aataggatct 5340
   aatgacttgg aaaagctgtc agaaaacgat aagcatatag tgaatcaatg ggccttcata 5400
   ggtgaaaagt gtattcacgg taccccttca ggaatagata acgctgtggc cacttatggt 5460
   aatgccctgc tatttgaaaa agactcacat aatggaacaa taaacacaaa caattttaag 5520
   ttcttagatg atttcccagc cattccaatg atcctaacct atactagaat tccaaggtct 5580
   acaaaagatc ttgttgctcg cgttcgtgtg ttggtcaccg agaaatttcc tgaagttatg 5640
   aagccaattc tagatgccat gggtgaatgt gccctacaag gcttagagat catgactaag 5700
   ttaagtaaat gtaaaggcac cgatgacgag gctgtagaaa ctaataatga actgtatgaa 5760
   caactattgg aattgataag aataaatcat ggactgcttg tctcaatcgg tgtttctcat 5820
   cctggattag aacttattaa aaatctgagc gatgatttga gaattggctc cacaaaactt 5880
   accggtgctg gtggcggcgg ttgctctttg actttgttac gaagagacat tactcaagag 5940
   caaattgaca gcttcaaaaa gaaattgcaa gatgatttta gttacgagac atttgaaaca 6000
   gacttgggtg ggactggctg ctgtttgtta agcgcaaaaa atttgaataa agatcttaaa 6060
   atcaaatccc tagtattcca attatttgaa aataaaacta ccacaaagca acaaattgac 6120
   gatctattat tgccaggaaa cacgaattta ccatggactt cataagctaa tttgcgatag 6180
   gcctgcaccc ttaaggagga aaaaaacatg tcagagttga gagccttcag tgccccaggg 6240
   aaagcgttac tagctggtgg atatttagtt ttagatacaa aatatgaagc atttgtagtc 6300
   ggattatcgg caagaatgca tgctgtagcc catccttacg gttcattgca agggtctgat 6360
   aagtttgaag tgcgtgtgaa aagtaaacaa tttaaagatg gggagtggct gtaccatata 6420
   agtcctaaaa gtggcttcat tcctgtttcg ataggcggat ctaagaaccc tttcattgaa 6480
   aaagttatcg ctaacgtatt tagctacttt aaacctaaca tggacgacta ctgcaataga 6540
   aacttgttcg ttattgatat tttctctgat gatgcctacc attctcagga ggatagcgtt 6600
   accgaacatc gtggcaacag aagattgagt tttcattcgc acagaattga agaagttccc 6660
   aaaacagggc tgggctcctc ggcaggttta gtcacagttt taactacagc tttggcctcc 6720
   ttttttgtat cggacctgga aaataatgta gacaaatata gagaagttat tcataattta 6780
   gcacaagttg ctcattgtca agctcagggt aaaattggaa gcgggtttga tgtagcggcg 6840
   gcagcatatg gatctatcag atatagaaga ttcccacccg cattaatctc taatttgcca 6900
   gatattggaa gtgctactta cggcagtaaa ctggcgcatt tggttgatga agaagactgg 6960
   aatattacga ttaaaagtaa ccatttacct tcgggattaa ctttatggat gggcgatatt 7020
   aagaatggtt cagaaacagt aaaactggtc cagaaggtaa aaaattggta tgattcgcat 7080
   atgccagaaa gcttgaaaat atatacagaa ctcgatcatg caaattctag atttatggat 7140
   ggactatcta aactagatcg cttacacgag actcatgacg attacagcga tcagatattt 7200
   gagtctcttg agaggaatga ctgtacctgt caaaagtatc ctgaaatcac agaagttaga 7260
   gatgcagttg ccacaattag acgttccttt agaaaaataa ctaaagaatc tggtgccgat 7320
   atcgaacctc ccgtacaaac tagcttattg gatgattgcc agaccttaaa aggagttctt 7380
   acttgcttaa tacctggtgc tggtggttat gacgccattg cagtgattac taagcaagat 7440
   gttgatctta gggctcaaac cgctaatgac aaaagatttt ctaaggttca atggctggat 7500
   gtaactcagg ctgactgggg tgttaggaaa gaaaaagatc cggaaactta tcttgataaa 7560
   taacttaagg tagctgcatg cagaattcgc ccttaaggag gaaaaaaaaa tgaccgttta 7620
   cacagcatcc gttaccgcac ccgtcaacat cgcaaccctt aagtattggg ggaaaaggga 7680
   cacgaagttg aatctgccca ccaattcgtc catatcagtg actttatcgc aagatgacct 7740
   cagaacgttg acctctgcgg ctactgcacc tgagtttgaa cgcgacactt tgtggttaaa 7800
   tggagaacca cacagcatcg acaatgaaag aactcaaaat tgtctgcgcg acctacgcca 7860
   attaagaaag gaaatggaat cgaaggacgc ctcattgccc acattatctc aatggaaact 7920
   ccacattgtc tccgaaaata actttcctac agcagctggt ttagcttcct ccgctgctgg 7980
   ctttgctgca ttggtctctg caattgctaa gttataccaa ttaccacagt caacttcaga 8040
   aatatctaga atagcaagaa aggggtctgg ttcagcttgt agatcgttgt ttggcggata 8100
   cgtggcctgg gaaatgggaa aagctgaaga tggtcatgat tccatggcag tacaaatcgc 8160
   agacagctct gactggcctc agatgaaagc ttgtgtccta gttgtcagcg atattaaaaa 8220
   ggatgtgagt tccactcagg gtatgcaatt gaccgtggca acctccgaac tatttaaaga 8280
   aagaattgaa catgtcgtac caaagagatt tgaagtcatg cgtaaagcca ttgttgaaaa 8340
   agatttcgcc acctttgcaa aggaaacaat gatggattcc aactctttcc atgccacatg 8400
   tttggactct ttccctccaa tattctacat gaatgacact tccaagcgta tcatcagttg 8460
   gtgccacacc attaatcagt tttacggaga aacaatcgtt gcatacacgt ttgatgcagg 8520
   tccaaatgct gtgttgtact acttagctga aaatgagtcg aaactctttg catttatcta 8580
   taaattgttt ggctctgttc ctggatggga caagaaattt actactgagc agcttgaggc 8640
   tttcaaccat caatttgaat catctaactt tactgcacgt gaattggatc ttgagttgca 8700
   aaaggatgtt gccagagtga ttttaactca agtcggttca ggcccacaag aaacaaacga 8760
   atctttgatt gacgcaaaga ctggtctacc aaaggaataa gatcaattcg ctgcatcgcc 8820
   cttaggaggt aaaaaaaaat gactgccgac aacaatagta tgccccatgg tgcagtatct 8880
   agttacgcca aattagtgca aaaccaaaca cctgaagaca ttttggaaga gtttcctgaa 8940
   attattccat tacaacaaag acctaatacc cgatctagtg agacgtcaaa tgacgaaagc 9000
   ggagaaacat gtttttctgg tcatgatgag gagcaaatta agttaatgaa tgaaaattgt 9060
   attgttttgg attgggacga taatgctatt ggtgccggta ccaagaaagt ttgtcattta 9120
   atggaaaata ttgaaaaggg tttactacat cgtgcattct ccgtctttat tttcaatgaa 9180
   caaggtgaat tacttttaca acaaagagcc actgaaaaaa taactttccc tgatctttgg 9240
   actaacacat gctgctctca tccactatgt attgatgacg aattaggttt gaagggtaag 9300
   ctagacgata agattaaggg cgctattact gcggcggtga gaaaactaga tcatgaatta 9360
   ggtattccag aagatgaaac taagacaagg ggtaagtttc actttttaaa cagaatccat 9420
   tacatggcac caagcaatga accatggggt gaacatgaaa ttgattacat cctattttat 9480
   aagatcaacg ctaaagaaaa cttgactgtc aacccaaacg tcaatgaagt tagagacttc 9540
   aaatgggttt caccaaatga tttgaaaact atgtttgctg acccaagtta caagtttacg 9600
   ccttggttta agattatttg cgagaattac ttattcaact ggtgggagca attagatgac 9660
   ctttctgaag tggaaaatga caggcaaatt catagaatgc tataacaacg cgtctacaaa 9720
   taaaaaaggc acgtcagatg acgtgccttt tttcttgggg ccggggatcc tctagagtcg 9780
   aaagaaaaat gccccgctta cgcagggcat ccatttatta ctcaaccgta accgattttg 9840
   ccaggttacg cggctggtca acgtcggtgc ctttgatcag cgcgacatgg taagccagca 9900
   gctgcagcgg aacggtgtag aagatcggtg caatcacctc ttccacatgc ggcatctcga 9960
   tgatgtgcat gttatcgcta cttacaaaac ccgcatcctg atcggcgaag acatacaact 10020
   gaccgccacg cgcgcgaact tcttcaatgt tggatttcag tttttccagc aattcgttgt 10080
   tcggtgcaac aacaataacc ggcatatcgg catcaattag cgccagcgga ccgtgtttca 10140
   gttcgccagc agcgtaggct tcagcgtgaa tgtaagagat ctctttcaac ttcaatgcgc 10200
   cttccagcgc gattgggtac tgatcgccac ggcccaggaa cagcgcgtga tgtttgtcag 10260
   agaaatcttc tgccagcgct tcaatgcgtt tgtcctgaga cagcatctgc tcaatacggc 10320
   tcggcagcgc ctgcagacca tgcacgatgt catgttcaat ggaggcatcc agacctttca 10380
   ggcgagacag cttcgccacc agcatcaaca gcacagttaa ctgagtggtg aatgctttag 10440
   tggatgccac gccgatttct gtacccgcgt tggtcattag cgccagatcg gattcgcgca 10500
   ccagagaaga acccggaacg ttacagattg ccagtgaacc aaggtaaccc agctctttcg 10560
   acagacgcag gccagccagg gtatccgcgg tttcgccaga ctgtgacagg ggatcctcta  10620
   gagtcgacct gcaggcatgc aagcttggcg taatcatggt catagctgtt tcctgtgtga  10680
   aattgttatc cgctcacaat tccacacaac atacgagccg gaagcataaa gtgtaaagcc  10740
   tggggtgcct aatgagtgag ctaactcaca ttaattgcgt tgcgctcact gcccgctttc  10800
   cagtcgggaa acctgtcgtg ccagctgcat taatgaatcg gccaacgcgc ggggagaggc  10860
   ggtttgcgta ttgggcgctt tctcatagct cacgctgtag gtatctcagt tcggtgtagg  10920
   tcgttcgctc caagctgggc tgtgtgccga accccagagt cccgctcaga agaactcgtc  10980
   aagaaggcga tagaaggcga tgcgctgcga atcgggagcg gcgataccgt aaagcacgag  11040
   gaagcggtca gcccattcgc cgccaagctc ttcagcaata tcacgggtag ccaacgctat  11100
   gtcctgatag cggtccgcca cacccagccg gccacagtcg atgaatccag aaaagcggcc  11160
   attttccacc atgatattcg gcaagcaggc atcgccatgg gtcacgacga gatcctcgcc  11220
   gtcgggcatg cgcgccttga gcctggcgaa cagttcggct ggcgcgagcc cctgatgctc  11280
   ttcgtccaga tcatcctgat cgacaagacc ggcttccatc cgagtacgtg ctcgctcgat  11340
   gcgatgtttc gcttggtggt cgaatgggca ggtagccgga tcaagcgtat gcagccgccg  11400
   cattgcatca gccatgatgg atactttctc ggcaggagca aggtgagatg acaggagatc  11460
   ctgccccggc acttcgccca atagcagcca gtcccttccc gcttcagtgc caacgtcgag  11520
   cacagctgcg caaggaacgc ccgtcgtggc cagccacgat agccgcgctg cctcgtcctg  11580
   cagttcattc agggcaccgg acaggtcggt cttgacaaaa agaaccgggc gcccctgcgc  11640
   tgacagccgg aacacggcgg catcagagca gccgattgtc tgttgtgccc agtcatagcc  11700
   gaatagcctc tccacccaag cggccggaga acctgcgtgc aatccatctt gttcaatcat  11760
   gcgaaacgat cctcatcctg tctcttgatc actaccgcat taaagcatat cgatgataag  11820
   ctgtcaaaca tgagcgttct tcggggcgaa aactctcaag gatcttaccg ctgttgagat  11880
   ccagttcgat gtaacccact cgtgcaccca actgatcttc agcatctttt actttcacca  11940
   gcgtttctgg gtgagcaaaa acaggaaggc aaaatgccgc aaaaaaggga ataagggcga  12000
   cacggaaatg ttgaatactc atactcttcc tttttcaata ttattgaagc atttatcagg  12060
   gttattgtct catgagcgga tacatatttg aatgtattta gaaaaataaa caaatagggg  12120
   ttccgcgcac atttccccga aaagtgccac ctgacgtcta agaaaccatt attatcatga  12180
   cattaaccta taaaaatagg cgtatcacga ggccctttcg tcttcaagaa tt          12232
<210> 57
   <211> 9175
   <212> DNA
   <213> Artificial Sequence
<220>
   <223> Nucleotide sequence containing genomic sequence encoding downstream enzymes in the mevalonate pathway
<400> 57
   ttagtacagc ggcttaccgc tactgtcttt aatattggtc ttccacgcag cgcgaacctg 60
   ttcaactaca ctatccggca ggctggcgta atccaggtcg ttcgcctgtt tagccccggt 120
   tttgtacgcc cagtcgaaga atttcagcac ttctgtgcct tgttctggtt tcttctgatc 180
   tttgtggatc agaatgaacg tggtagaggt aataggccat gcatcttcgc ctttctggtt 240
   ggtcagatcc tgagcgaagg ttttgctcca gtctgcacct tttgctgcat tagcgaagtt 300
   ttcttcggtc ggactaaccg gtttaccatc agcggagatc agtttggtgt acgccaggtt 360
   gttctgcttc gcgtaagcat attcaacata accaattgca cccggcagac gctgaacgaa 420
   cgcggcgata ccgtcgttac ctttaccgcc cagaccgatc ggccatttta cggtagagcc 480
   agtaccaacg ttgtttttcc actcttcgtt cactttcgcc aggtagctgg tgaagacgaa 540
   ggaagtcccg gagccatctg cgcggcgtac tacagcaatg ttttgtgaag gcagtttcag 600
   acccggattc agtttggcga tggcttcatc atcccacttc ttgattttgc ccaggtagat 660
   gtcgccgagg gttttaccat ccagcaccag ttcgccagac ttcagccctg gaatgttaac 720
   cgccagcacc acgccgccaa tcacggtcgg gaactggaac agaccttcct gagccagttt 780
   ttcgtcagac agcggcgcgt cagaggcacc aaaatcaacg gtattagcga taatctgttt 840
   tacgccaccg gaagaaccga taccctggta gttaacttta ttaccggttt ctttctggta 900
   agtgtcagcc catttggcat acaccggcgc agggaaggtt gcacctgcac ctgtcaggct 960
   tgcttctgca aacacagaga aagcactcat cgataaggtc gcggcgacaa cagttgcgac 1020
   ggtggtacgc ataactttca taatgtctcc tgggaggatt cataaagcat tgtttgttgg 1080
   ctacgagaag caaaatagga caaacaggtg acagttatat gtaaggaata tgacagtttt 1140
   atgacagaga gataaagtct tcagtctgat ttaaataagc gttgatattc agtcaattac 1200
   aaacattaat aacgaagaga tgacagaaaa attttcattc tgtgacagag aaaaagtagc 1260
   cgaagatgac ggtttgtcac atggagttgg caggatgttt gattaaaatg aagcctgctt 1320
   ttttatacta agttggcatt ataaaaaagc attgcttatc aatttgttgc aacgaacagg 1380
   tcactatcag tcaaaataaa atcattattt gatttcgaat tctcatgttt gacagcttat 1440
   catcgataag ctttaatgcg gtagtttatc acagttaaat tgctaacgca gtcaggcacc 1500
   gtgtatgaaa tctaacaatg cgctcatcgt catcctcggc accgtcaccc tggatgctgt 1560
   aggcataggc ttggttatgc cggtactgcc gggcctcttg cgggatatcg tccattccga 1620
   cagcatcgcc agtcactatg gcgtgctgct agcgctatat gcgttgatgc aatttctatg 1680
   cgcacccgtt ctcggagcac tgtccgaccg ctttggccgc cgcccagtcc tgctcgcttc 1740
   gctacttgga gccactatcg actacgcgat catggcgacc acacccgtcc tgtggatctc 1800
   cggataagta gacagcctga taagtcgcac gaaaaacagg tattgacaac atgaagtaac 1860
   atgcagtaag atacaaatcg ctaggtaaca ctagcagcgt caaccgggcg ctctagctag 1920
   agccaagcta gcttggccgg atccgagatt ttcaggagct aaggaagcta aaatggagaa 1980
   aaaaatcact ggatatacca ccgttgatat atcccaatgg catcgtaaag aacattttga 2040
   ggcatttcag tcagttgctc aatgtaccta taaccagacc gttcagctgg atattacggc 2100
   ctttttaaag accgtaaaga aaaataagca caagttttat ccggccttta ttcacattct 2160
   tgcccgcctg atgaatgctc atccggaatt ccgtatggca atgaaagacg gtgagctggt 2220
   gatatgggat agtgttcacc cttgttacac cgttttccat gagcaaactg aaacgttttc 2280
   atcgctctgg agtgaatacc acgacgattt ccggcagttt ctacacatat attcgcaaga 2340
   tgtggcgtgt tacggtgaaa acctggccta tttccctaaa gggtttattg agaatatgtt 2400
   tttcgtctca gccaatccct gggtgagttt caccagtttt gatttaaacg tggccaatat 2460
   ggacaacttc ttcgcccccg ttttcaccat gggcaaatat tatacgcaag gcgacaaggt 2520
   gctgatgccg ctggcgattc aggttcatca tgccgtctgt gatggcttcc atgtcggcag 2580
   aatgcttaat gaattacaac agtactgcga tgagtggcag ggcggggcgt aattttttta 2640
   aggcagttat tggtgccctt aaacgcctgg tgctacgcct gaataagtga taataagcgg 2700
   atgaatggca gaaattcgtc gaagcttaac acagaaaaaa gcccgcacct gacagtgcgg 2760
   gctttttttt tcgaccactg cagtctgtta caggtcacta ataccatcta agtagttgat 2820
   tcatagtgac tgcatatgtt gtgttttaca gtattatgta gtctgttttt tatgcaaaat 2880
   ctaatttaat atattgatat ttatatcatt ttacgtttct cgttcagctt ttttatacta 2940
   acttgagcgg cccttgacga tgccacatcc tgagcaaata attcaaccac taattgtgag 3000
   cggataacac aaggaggaaa cagctatgtc attaccgttc ttaacttctg caccgggaaa 3060
   ggttattatt tttggtgaac actctgctgt gtacaacaag cctgccgtcg ctgctagtgt 3120
   gtctgcgttg agaacctacc tgctaataag cgagtcatct gcaccagata ctattgaatt 3180
   ggacttcccg gacattagct ttaatcataa gtggtccatc aatgatttca atgccatcac 3240
   cgaggatcaa gtaaactccc aaaaattggc caaggctcaa caagccaccg atggcttgtc 3300
   tcaggaactc gttagtcttt tggatccgtt gttagctcaa ctatccgaat ccttccacta 3360
   ccatgcagcg ttttgtttcc tgtatatgtt tgtttgccta tgcccccatg ccaagaatat 3420
   taagttttcc ttaaagtcta ctttacccat cggtgctggg ttgggctcaa gcgcctctat 3480
   ttctgtatca ctggccttag ctatggccta cttggggggg ttaataggat ctaatgactt 3540
   ggaaaagctg tcagaaaacg ataagcatat agtgaatcaa tgggccttca taggtgaaaa 3600
   gtgtattcac ggtacccctt caggaataga taacgctgtg gccacttatg gtaatgccct 3660
   gctatttgaa aaagactcac ataatggaac aataaacaca aacaatttta agttcttaga 3720
   tgatttccca gccattccaa tgatcctaac ctatactaga attccaaggt ctacaaaaga 3780
   tcttgttgct cgcgttcgtg tgttggtcac cgagaaattt cctgaagtta tgaagccaat 3840
   tctagatgcc atgggtgaat gtgccctaca aggcttagag atcatgacta agttaagtaa 3900
   atgtaaaggc accgatgacg aggctgtaga aactaataat gaactgtatg aacaactatt 3960
   ggaattgata agaataaatc atggactgct tgtctcaatc ggtgtttctc atcctggatt 4020
   agaacttatt aaaaatctga gcgatgattt gagaattggc tccacaaaac ttaccggtgc 4080
   tggtggcggc ggttgctctt tgactttgtt acgaagagac attactcaag agcaaattga 4140
   cagcttcaaa aagaaattgc aagatgattt tagttacgag acatttgaaa cagacttggg 4200
   tgggactggc tgctgtttgt taagcgcaaa aaatttgaat aaagatctta aaatcaaatc 4260
   cctagtattc caattatttg aaaataaaac taccacaaag caacaaattg acgatctatt 4320
   attgccagga aacacgaatt taccatggac ttcataagct aatttgcgat aggcctgcac 4380
   ccttaaggag gaaaaaaaca tgtcagagtt gagagccttc agtgccccag ggaaagcgtt 4440
   actagctggt ggatatttag ttttagatac aaaatatgaa gcatttgtag tcggattatc 4500
   ggcaagaatg catgctgtag cccatcctta cggttcattg caagggtctg ataagtttga 4560
   agtgcgtgtg aaaagtaaac aatttaaaga tggggagtgg ctgtaccata taagtcctaa 4620
   aagtggcttc attcctgttt cgataggcgg atctaagaac cctttcattg aaaaagttat 4680
   cgctaacgta tttagctact ttaaacctaa catggacgac tactgcaata gaaacttgtt 4740
   cgttattgat attttctctg atgatgccta ccattctcag gaggatagcg ttaccgaaca 4800
   tcgtggcaac agaagattga gttttcattc gcacagaatt gaagaagttc ccaaaacagg 4860
   gctgggctcc tcggcaggtt tagtcacagt tttaactaca gctttggcct ccttttttgt 4920
   atcggacctg gaaaataatg tagacaaata tagagaagtt attcataatt tagcacaagt 4980
   tgctcattgt caagctcagg gtaaaattgg aagcgggttt gatgtagcgg cggcagcata 5040
   tggatctatc agatatagaa gattcccacc cgcattaatc tctaatttgc cagatattgg 5100
   aagtgctact tacggcagta aactggcgca tttggttgat gaagaagact ggaatattac 5160
   gattaaaagt aaccatttac cttcgggatt aactttatgg atgggcgata ttaagaatgg 5220
   ttcagaaaca gtaaaactgg tccagaaggt aaaaaattgg tatgattcgc atatgccaga 5280
   aagcttgaaa atatatacag aactcgatca tgcaaattct agatttatgg atggactatc 5340
   taaactagat cgcttacacg agactcatga cgattacagc gatcagatat ttgagtctct 5400
   tgagaggaat gactgtacct gtcaaaagta tcctgaaatc acagaagtta gagatgcagt 5460
   tgccacaatt agacgttcct ttagaaaaat aactaaagaa tctggtgccg atatcgaacc 5520
   tcccgtacaa actagcttat tggatgattg ccagacctta aaaggagttc ttacttgctt 5580
   aatacctggt gctggtggtt atgacgccat tgcagtgatt actaagcaag atgttgatct   5640
   tagggctcaa accgctaatg acaaaagatt ttctaaggtt caatggctgg atgtaactca   5700
   ggctgactgg ggtgttagga aagaaaaaga tccggaaact tatcttgata aataacttaa   5760
   ggtagctgca tgcagaattc gcccttaagg aggaaaaaaa aatgaccgtt tacacagcat   5820
   ccgttaccgc acccgtcaac atcgcaaccc ttaagtattg gggaaaaagg gacacgaagt   5880
   tgaatctgcc caccaattcg tccatatcag tgactttatc gcaagatgac ctcagaacgt   5940
   tgacctctgc ggctactgca cctgagtttg aacgcgacac tttgtggtta aatggagaac   6000
   cacacagcat cgacaatgaa agaactcaaa attgtctgcg cgacctacgc caattaagaa   6060
   aggaaatgga atcgaaggac gcctcattgc ccacattatc tcaatggaaa ctccacattg   6120
   tctccgaaaa taactttcct acagcagctg gtttagcttc ctccgctgct ggctttgctg   6180
   cattggtctc tgcaattgct aagttatacc aattaccaca gtcaacttca gaaatatcta   6240
   gaatagcaag aaaggggtct ggttcagctt gtagatcgtt gtttggcgga tacgtggcct   6300
   gggaaatggg aaaagctgaa gatggtcatg attccatggc agtacaaatc gcagacagct   6360
   ctgactggcc tcagatgaaa gcttgtgtcc tagttgtcag cgatattaaa aaggatgtga   6420
   gttccactca gggtatgcaa ttgaccgtgg caacctccga actatttaaa gaaagaattg   6480
   aacatgtcgt accaaagaga tttgaagtca tgcgtaaagc cattgttgaa aaagatttcg   6540
   ccacctttgc aaaggaaaca atgatggatt ccaactcttt ccatgccaca tgtttggact   6600
   ctttccctcc aatattctac atgaatgaca cttccaagcg tatcatcagt tggtgccaca   6660
   ccattaatca gttttacgga gaaacaatcg ttgcatacac gtttgatgca ggtccaaatg   6720
   ctgtgttgta ctacttagct gaaaatgagt cgaaactctt tgcatttatc tataaattgt   6780
   ttggctctgt tcctggatgg gacaagaaat ttactactga gcagcttgag gctttcaacc   6840
   atcaatttga atcatctaac tttactgcac gtgaattgga tcttgagttg caaaaggatg   6900
   ttgccagagt gattttaact caagtcggtt caggcccaca agaaacaaac gaatctttga   6960
   ttgacgcaaa gactggtcta ccaaaggaat aagatcaatt cgctgcatcg cccttaggag   7020
   gtaaaaaaaa atgactgccg acaacaatag tatgccccat ggtgcagtat ctagttacgc   7080
   caaattagtg caaaaccaaa cacctgaaga cattttggaa gagtttcctg aaattattcc   7140
   attacaacaa agacctaata cccgatctag tgagacgtca aatgacgaaa gcggagaaac   7200
   atgtttttct ggtcatgatg aggagcaaat taagttaatg aatgaaaatt gtattgtttt   7260
   ggattgggac gataatgcta ttggtgccgg taccaagaaa gtttgtcatt taatggaaaa   7320
   tattgaaaag ggtttactac atcgtgcatt ctccgtcttt attttcaatg aacaaggtga   7380
   attactttta caacaaagag ccactgaaaa aataactttc cctgatcttt ggactaacac   7440
   atgctgctct catccactat gtattgatga cgaattaggt ttgaagggta agctagacga 7500
   taagattaag ggcgctatta ctgcggcggt gagaaaacta gatcatgaat taggtattcc 7560
   agaagatgaa actaagacaa ggggtaagtt tcacttttta aacagaatcc attacatggc 7620
   accaagcaat gaaccatggg gtgaacatga aattgattac atcctatttt ataagatcaa 7680
   cgctaaagaa aacttgactg tcaacccaaa cgtcaatgaa gttagagact tcaaatgggt 7740
   ttcaccaaat gatttgaaaa ctatgtttgc tgacccaagt tacaagttta cgccttggtt 7800
   taagattatt tgcgagaatt acttattcaa ctggtgggag caattagatg acctttctga 7860
   agtggaaaat gacaggcaaa ttcatagaat gctataacaa cgcgtctaca aataaaaaag 7920
   gcacgtcaga tgacgtgcct tttttcttgg ggccgaaaaa tgccccgctt acgcagggca 7980
   tccatttatt actcaaccgt aaccgatttt gccaggttac gcggctggtc aacgtcggtg 8040
   cctttgatca gcgcgacatg gtaagccagc agctgcagcg gaacggtgta gaagatcggt 8100
   gcaatcacct cttccacatg cggcatctcg atgatgtgca tgttatcgct acttacaaaa 8160
   cccgcatcct gatcggcgaa gacatacaac tgaccgccac gcgcgcgaac ttcttcaatg 8220
   ttggatttca gtttttccag caattcgttg ttcggtgcaa caacaataac cggcatatcg 8280
   gcatcaatta gcgccagcgg accgtgtttc agttcgccag cagcgtaggc ttcagcgtga 8340
   atgtaagaga tctctttcaa cttcaatgcg ccttccagcg cgattgggta ctgatcgcca 8400
   cggcccagga acagcgcgtg atgtttgtca gagaaatctt ctgccagcgc ttcaatgcgt 8460
   ttgtcctgag acagcatctg ctcaatacgg ctcggcagcg cctgcagacc atgcacgatg 8520
   tcatgttcaa tggaggcatc cagacctttc aggcgagaca gcttcgccac cagcatcaac 8580
   agcacagtta actgagtggt gaatgcttta gtggatgcca cgccgatttc tgtacccgcg 8640
   ttggtcatta gcgccagatc ggattcgcgc accagagaag aacccggaac gttacagatt 8700
   gccagtgaac caaggtaacc cagctctttc gacagacgca ggccagccag ggtatccgcg 8760
   gtttcgccag actgtgacaa ggtgatcatc aggctgttac gacgcacggc agatttgcga 8820
   tagcggaatt cagaggcgat ttcgacgtcg cacggaatac ctgctagcga ttcaaaccag 8880
   tagcgggaaa ccataccgga gttataagaa gtaccacagg cgaggatctg aatatgctca 8940
   accttcgaca gcagttcgtc ggcgttcggt cccagctcgc ttaaatcaac ctgaccgtgg 9000
   ctgatgcgtc cggtaagggt gtttttgatc gcgttcggct gttcgtagat ctctttctgc 9060
   atgtagtgac ggtaaatgcc tttatcgccc gcgtcatatt gcagattgga ttcgatatcc 9120
   tgacgtttta cttccgcgcc agttttatcg aagatgttta ccgaacggcg agtga 9175
<210> 58
   <211> 40
   <212> DNA
   <213> Artificial Sequence
<220>
   <223> Primer (Tn7dS_6038-7-1)
<400> 58
   tcgagctcgg taccctgttt ttccactctt cgttcacttt 40
<210> 59
   <211> 33
   <212> DNA
   <213> Artificial Sequence
<220>
   <223> Primer (Tn7dA_6038-7-2)
<400> 59
   aggcttcatt ttaatcaaac atcctgccaa ctc 33
<210> 60
   <211> 25
   <212> DNA
   <213> Artificial Sequence
<220>
   <223> Primer (Tn7dattLcmS_6038-7-4)
<400> 60
   attaaaatga agcctgcttt tttat 25
<210> 61
   <211> 32
   <212> DNA
   <213> Artificial Sequence
<220>
   <223> Primer (PgiattRcmA_6038-7-5)
<400> 61
   ggcatcgtca agggccgctc aagttagtat aa 32
<210> 62
   <211> 99
   <212> DNA
   <213> Artificial Sequence
<220>
   <223> Primer (gi1.2-MVK-S_6038-7-6
<400> 62
   gcccttgacg atgccacatc ctgagcaaat aattcaacca ctaattgtga gcggataaca 60
   caaggaggaa acagctatgt cattaccgtt cttaacttc 99
<210> 63
   <211> 96
   <212> DNA
   <213> Artificial Sequence
<220>
   <223> Primer (pMW-TaspA-yIDIA_6038-7-7)
<400> 63
   ctctagagga tccccggccc caagaaaaaa ggcacgtcat ctgacgtgcc ttttttattt 60
   gtagacgcgt tgttatagca ttctatgaat ttgcct 96
<210> 64
   <211> 36
   <212> DNA
   <213> Artificial Sequence
<220>
   <223> Primer (Tn7upSv02_6038-24-1)
<400> 64
   atcctctaga gtcgaaagaa aaatgccccg cttacg 36
<210> 65
   <211> 37
   <212> DNA
   <213> Artificial Sequence
<220>
   <223> Primer (Tn7upAv02_6038-24-2)
<400> 65
   atgcctgcag gtcgactgtc acagtctggc gaaaccg 37
<210> 66
   <211> 20
   <212> DNA
   <213> Artificial Sequence
<220>
   <223> Primer (Tn7v02-F_6038-22-5)
<400> 66
   acgaactgct gtcgaaggtt 20
<210> 67
   <211> 20
   <212> DNA
   <213> Artificial Sequence
<220>
   <223> Primer (Tn7v02-R_6038-22-6)
<400> 67
   ggtgtacgcc aggttgttct 20
<210> 68
   <211> 2414
   <212> DNA
   <213> Artificial Sequence
<220>
   <223> Nucleotide sequence of genomic fragment containing attL-Tet-attR-Ptac
<400> 68
   tgaagcctgc ttttttatac taagttggca ttataaaaaa gcattgctta tcaatttgtt      60
   gcaacgaaca ggtcactatc agtcaaaata aaatcattat ttgatttcga attccccgga    120
   tccgtcgacc tgcagggaaa aaggttatgc tgcttttaag acccactttc acatttaagt    180
   tgtttttcta atccgcatat gatcaattca aggccgaata agaaggctgg ctctgcacct    240
   tggtgatcaa ataattcgat agcttgtcgt aataatggcg gcatactatc agtagtaggt    300
   gtttcccttt cttctttagc gacttgatgc tcttgatctt ccaatacgca acctaaagta    360
   aaatgcccca cagcgctgag tgcatataat gcattctcta gtgaaaaacc ttgttggcat    420
   aaaaaggcta attgattttc gagagtttca tactgttttt ctgtaggccg tgtacctaaa    480
   tgtacttttg ctccatcgcg atgacttagt aaagcacatc taaaactttt agcgttatta    540
   cgtaaaaaat cttgccagct ttccccttct aaagggcaaa agtgagtatg gtgcctatct    600
   aacatctcaa tggctaaggc gtcgagcaaa gcccgcttat tttttacatg ccaatacaat    660
   gtaggctgct ctacacctag cttctgggcg agtttacggg ttgttaaacc ttcgattccg    720
   acctcattaa gcagctctaa tgcgctgtta atcactttac ttttatctaa tctagacatc    780
   attaattcct aatttttgtt gacactctat cattgataga gttattttac cactccctat    840
   cagtgataga gaaaagtgaa atgaatagtt cgacaaagat cgcattggta attacgttac    900
   tcgatgccat ggggattggc cttatcatgc cagtcttgcc aacgttatta cgtgaattta    960
   ttgcttcgga agatatcgct aaccactttg gcgtattgct tgcactttat gcgttaatgc   1020
   aggttatctt tgctccttgg cttggaaaaa tgtctgaccg atttggtcgg cgcccagtgc   1080
   tgttgttgtc attaataggc gcatcgctgg attacttatt gctggctttt tcaagtgcgc   1140
   tttggatgct gtatttaggc cgtttgcttt cagggatcac aggagctact ggggctgtcg   1200
   cggcatcggt cattgccgat accacctcag cttctcaacg cgtgaagtgg ttcggttggt   1260
   taggggcaag ttttgggctt ggtttaatag cggggcctat tattggtggt tttgcaggag   1320
   agatttcacc gcatagtccc ttttttatcg ctgcgttgct aaatattgtc actttccttg   1380
   tggttatgtt ttggttccgt gaaaccaaaa atacacgtga taatacagat accgaagtag   1440
   gggttgagac gcaatcgaat tcggtataca tcactttatt taaaacgatg cccattttgt   1500
   tgattattta tttttcagcg caattgatag gccaaattcc cgcaacggtg tgggtgctat   1560
   ttaccgaaaa tcgttttgga tggaatagca tgatggttgg cttttcatta gcgggtcttg   1620
   gtcttttaca ctcagtattc caagcctttg tggcaggaag aatagccact aaatggggcg   1680
   aaaaaacggc agtactgctc gaatttattg cagatagtag tgcatttgcc tttttagcgt   1740
   ttatatctga aggttggtta gatttccctg ttttaatttt attggctggt ggtgggatcg   1800
   ctttacctgc attacaggga gtgatgtcta tccaaacaaa gagtcatgag caaggtgctt 1860
   tacagggatt attggtgagc cttaccaatg caaccggtgt tattggccca ttactgttta 1920
   ctgttattta taatcattca ctaccaattt gggatggctg gatttggatt attggtttag 1980
   cgttttactg tattattatc ctgctatcga tgaccttcat gttaacccct caagctcagg 2040
   ggagtaaaca ggagacaagt gcttagttat ttcgtcacca aatgatgtta ttccgcgaaa 2100
   tataatgacc ctcttgataa cccaagaggg cattttttac gataaagaag atttagcttc 2160
   tgcagtctgt tacaggtcac taataccatc taagtagttg attcatagtg actgcatatg 2220
   ttgtgtttta cagtattatg tagtctgttt tttatgcaaa atctaattta atatattgat 2280
   atttatatca ttttacgttt ctcgttcagc ttttttatac taacttgagc gagatctccc 2340
   tgttgacaat taatcatcgg ctctataatg tgtggaatcg tgagcggata acaatttcac 2400
   acaaggagac tgcc 2414
<210> 69
   <211> 7464
   <212> DNA
   <213> Artificial Sequence
<220>
   <223> Nucleotide sequence containing genomic sequence encoding downstream enzymes in the mevalonate pathway under control of tac promoter
<400> 69
   ttagtacagc ggcttaccgc tactgtcttt aatattggtc ttccacgcag cgcgaacctg 60
   ttcaactaca ctatccggca ggctggcgta atccaggtcg ttcgcctgtt tagccccggt 120
   tttgtacgcc cagtcgaaga atttcagcac ttctgtgcct tgttctggtt tcttctgatc 180
   tttgtggatc agaatgaacg tggtagaggt aataggccat gcatcttcgc ctttctggtt 240
   ggtcagatcc tgagcgaagg ttttgctcca gtctgcacct tttgctgcat tagcgaagtt 300
   ttcttcggtc ggactaaccg gtttaccatc agcggagatc agtttggtgt acgccaggtt 360
   gttctgcttc gcgtaagcat attcaacata accaattgca cccggcagac gctgaacgaa 420
   cgcggcgata ccgtcgttac ctttaccgcc cagaccgatc ggccatttta cggtagagcc 480
   agtaccaacg ttgtttttcc actcttcgtt cactttcgcc aggtagctgg tgaagacgaa 540
   ggaagtcccg gagccatctg cgcggcgtac tacagcaatg ttttgtgaag gcagtttcag 600
   acccggattc agtttggcga tggcttcatc atcccacttc ttgattttgc ccaggtagat 660
   gtcgccgagg gttttaccat ccagcaccag ttcgccagac ttcagccctg gaatgttaac 720
   cgccagcacc acgccgccaa tcacggtcgg gaactggaac agaccttcct gagccagttt 780
   ttcgtcagac agcggcgcgt cagaggcacc aaaatcaacg gtattagcga taatctgttt 840
   tacgccaccg gaagaaccga taccctggta gttaacttta ttaccggttt ctttctggta    900
   agtgtcagcc catttggcat acaccggcgc agggaaggtt gcacctgcac ctgtcaggct    960
   tgcttctgca aacacagaga aagcactcat cgataaggtc gcggcgacaa cagttgcgac   1020
   ggtggtacgc ataactttca taatgtctcc tgggaggatt cataaagcat tgtttgttgg   1080
   ctacgagaag caaaatagga caaacaggtg acagttatat gtaaggaata tgacagtttt   1140
   atgacagaga gataaagtct tcagtctgat ttaaataagc gttgatattc agtcaattac   1200
   tgaagcctgc ttttttatac taacttgagc gagatctccc tgttgacaat taatcatcgg    1260
   ctctataatg tgtggaatcg tgagcggata acaatttcac acaaggagac tgccatgtca   1320
   ttaccgttct taacttctgc accgggaaag gttattattt ttggtgaaca ctctgctgtg   1380
   tacaacaagc ctgccgtcgc tgctagtgtg tctgcgttga gaacctacct gctaataagc   1440
   gagtcatctg caccagatac tattgaattg gacttcccgg acattagctt taatcataag   1500
   tggtccatca atgatttcaa tgccatcacc gaggatcaag taaactccca aaaattggcc   1560
   aaggctcaac aagccaccga tggcttgtct caggaactcg ttagtctttt ggatccgttg   1620
   ttagctcaac tatccgaatc cttccactac catgcagcgt tttgtttcct gtatatgttt   1680
   gtttgcctat gcccccatgc caagaatatt aagttttcct taaagtctac tttacccatc   1740
   ggtgctgggt tgggctcaag cgcctctatt tctgtatcac tggccttagc tatggcctac   1800
   ttgggggggt taataggatc taatgacttg gaaaagctgt cagaaaacga taagcatata   1860
   gtgaatcaat gggccttcat aggtgaaaag tgtattcacg gtaccccttc aggaatagat   1920
   aacgctgtgg ccacttatgg taatgccctg ctatttgaaa aagactcaca taatggaaca   1980
   ataaacacaa acaattttaa gttcttagat gatttcccag ccattccaat gatcctaacc   2040
   tatactagaa ttccaaggtc tacaaaagat cttgttgctc gcgttcgtgt gttggtcacc   2100
   gagaaatttc ctgaagttat gaagccaatt ctagatgcca tgggtgaatg tgccctacaa   2160
   ggcttagaga tcatgactaa gttaagtaaa tgtaaaggca ccgatgacga ggctgtagaa   2220
   actaataatg aactgtatga acaactattg gaattgataa gaataaatca tggactgctt   2280
   gtctcaatcg gtgtttctca tcctggatta gaacttatta aaaatctgag cgatgatttg   2340
   agaattggct ccacaaaact taccggtgct ggtggcggcg gttgctcttt gactttgtta   2400
   cgaagagaca ttactcaaga gcaaattgac agcttcaaaa agaaattgca agatgatttt   2460
   agttacgaga catttgaaac agacttgggt gggactggct gctgtttgtt aagcgcaaaa   2520
   aatttgaata aagatcttaa aatcaaatcc ctagtattcc aattatttga aaataaaact   2580
   accacaaagc aacaaattga cgatctatta ttgccaggaa acacgaattt accatggact   2640
   tcataagcta atttgcgata ggcctgcacc cttaaggagg aaaaaaacat gtcagagttg   2700
   agagccttca gtgccccagg gaaagcgtta ctagctggtg gatatttagt tttagataca   2760
   aaatatgaag catttgtagt cggattatcg gcaagaatgc atgctgtagc ccatccttac 2820
   ggttcattgc aagggtctga taagtttgaa gtgcgtgtga aaagtaaaca atttaaagat 2880
   ggggagtggc tgtaccatat aagtcctaaa agtggcttca ttcctgtttc gataggcgga 2940
   tctaagaacc ctttcattga aaaagttatc gctaacgtat ttagctactt taaacctaac 3000
   atggacgact actgcaatag aaacttgttc gttattgata ttttctctga tgatgcctac 3060
   cattctcagg aggatagcgt taccgaacat cgtggcaaca gaagattgag ttttcattcg 3120
   cacagaattg aagaagttcc caaaacaggg ctgggctcct cggcaggttt agtcacagtt 3180
   ttaactacag ctttggcctc cttttttgta tcggacctgg aaaataatgt agacaaatat 3240
   agagaagtta ttcataattt agcacaagtt gctcattgtc aagctcaggg taaaattgga 3300
   agcgggtttg atgtagcggc ggcagcatat ggatctatca gatatagaag attcccaccc 3360
   gcattaatct ctaatttgcc agatattgga agtgctactt acggcagtaa actggcgcat 3420
   ttggttgatg aagaagactg gaatattacg attaaaagta accatttacc ttcgggatta 3480
   actttatgga tgggcgatat taagaatggt tcagaaacag taaaactggt ccagaaggta 3540
   aaaaattggt atgattcgca tatgccagaa agcttgaaaa tatatacaga actcgatcat 3600
   gcaaattcta gatttatgga tggactatct aaactagatc gcttacacga gactcatgac 3660
   gattacagcg atcagatatt tgagtctctt gagaggaatg actgtacctg tcaaaagtat 3720
   cctgaaatca cagaagttag agatgcagtt gccacaatta gacgttcctt tagaaaaata 3780
   actaaagaat ctggtgccga tatcgaacct cccgtacaaa ctagcttatt ggatgattgc 3840
   cagaccttaa aaggagttct tacttgctta atacctggtg ctggtggtta tgacgccatt 3900
   gcagtgatta ctaagcaaga tgttgatctt agggctcaaa ccgctaatga caaaagattt 3960
   tctaaggttc aatggctgga tgtaactcag gctgactggg gtgttaggaa agaaaaagat 4020
   ccggaaactt atcttgataa ataacttaag gtagctgcat gcagaattcg cccttaagga 4080
   ggaaaaaaaa atgaccgttt acacagcatc cgttaccgca cccgtcaaca tcgcaaccct 4140
   taagtattgg gggaaaaggg acacgaagtt gaatctgccc accaattcgt ccatatcagt 4200
   gactttatcg caagatgacc tcagaacgtt gacctctgcg gctactgcac ctgagtttga 4260
   acgcgacact ttgtggttaa atggagaacc acacagcatc gacaatgaaa gaactcaaaa 4320
   ttgtctgcgc gacctacgcc aattaagaaa ggaaatggaa tcgaaggacg cctcattgcc 4380
   cacattatct caatggaaac tccacattgt ctccgaaaat aactttccta cagcagctgg 4440
   tttagcttcc tccgctgctg gctttgctgc attggtctct gcaattgcta agttatacca 4500
   attaccacag tcaacttcag aaatatctag aatagcaaga aaggggtctg gttcagcttg 4560
   tagatcgttg tttggcggat acgtggcctg ggaaatggga aaagctgaag atggtcatga 4620
   ttccatggca gtacaaatcg cagacagctc tgactggcct cagatgaaag cttgtgtcct 4680
   agttgtcagc gatattaaaa aggatgtgag ttccactcag ggtatgcaat tgaccgtggc 4740
   aacctccgaa ctatttaaag aaagaattga acatgtcgta ccaaagagat ttgaagtcat 4800
   gcgtaaagcc attgttgaaa aagatttcgc cacctttgca aaggaaacaa tgatggattc 4860
   caactctttc catgccacat gtttggactc tttccctcca atattctaca tgaatgacac 4920
   ttccaagcgt atcatcagtt ggtgccacac cattaatcag ttttacggag aaacaatcgt 4980
   tgcatacacg tttgatgcag gtccaaatgc tgtgttgtac tacttagctg aaaatgagtc 5040
   gaaactcttt gcatttatct ataaattgtt tggctctgtt cctggatggg acaagaaatt 5100
   tactactgag cagcttgagg ctttcaacca tcaatttgaa tcatctaact ttactgcacg 5160
   tgaattggat cttgagttgc aaaaggatgt tgccagagtg attttaactc aagtcggttc 5220
   aggcccacaa gaaacaaacg aatctttgat tgacgcaaag actggtctac caaaggaata 5280
   agatcaattc gctgcatcgc ccttaggagg taaaaaaaaa tgactgccga caacaatagt 5340
   atgccccatg gtgcagtatc tagttacgcc aaattagtgc aaaaccaaac acctgaagac 5400
   attttggaag agtttcctga aattattcca ttacaacaaa gacctaatac ccgatctagt 5460
   gagacgtcaa atgacgaaag cggagaaaca tgtttttctg gtcatgatga ggagcaaatt 5520
   aagttaatga atgaaaattg tattgttttg gattgggacg ataatgctat tggtgccggt 5580
   accaagaaag tttgtcattt aatggaaaat attgaaaagg gtttactaca tcgtgcattc 5640
   tccgtcttta ttttcaatga acaaggtgaa ttacttttac aacaaagagc cactgaaaaa 5700
   ataactttcc ctgatctttg gactaacaca tgctgctctc atccactatg tattgatgac 5760
   gaattaggtt tgaagggtaa gctagacgat aagattaagg gcgctattac tgcggcggtg 5820
   agaaaactag atcatgaatt aggtattcca gaagatgaaa ctaagacaag gggtaagttt 5880
   cactttttaa acagaatcca ttacatggca ccaagcaatg aaccatgggg tgaacatgaa 5940
   attgattaca tcctatttta taagatcaac gctaaagaaa acttgactgt caacccaaac 6000
   gtcaatgaag ttagagactt caaatgggtt tcaccaaatg atttgaaaac tatgtttgct 6060
   gacccaagtt acaagtttac gccttggttt aagattattt gcgagaatta cttattcaac 6120
   tggtgggagc aattagatga cctttctgaa gtggaaaatg acaggcaaat tcatagaatg 6180
   ctataacaac gcgtctacaa ataaaaaagg cacgtcagat gacgtgcctt ttttcttggg 6240
   gccgaaaaat gccccgctta cgcagggcat ccatttatta ctcaaccgta accgattttg 6300
   ccaggttacg cggctggtca acgtcggtgc ctttgatcag cgcgacatgg taagccagca 6360
   gctgcagcgg aacggtgtag aagatcggtg caatcacctc ttccacatgc ggcatctcga 6420
   tgatgtgcat gttatcgcta cttacaaaac ccgcatcctg atcggcgaag acatacaact 6480
   gaccgccacg cgcgcgaact tcttcaatgt tggatttcag tttttccagc aattcgttgt 6540
   tcggtgcaac aacaataacc ggcatatcgg catcaattag cgccagcgga ccgtgtttca 6600
   gttcgccagc agcgtaggct tcagcgtgaa tgtaagagat ctctttcaac ttcaatgcgc 6660
   cttccagcgc gattgggtac tgatcgccac ggcccaggaa cagcgcgtga tgtttgtcag 6720
   agaaatcttc tgccagcgct tcaatgcgtt tgtcctgaga cagcatctgc tcaatacggc 6780
   tcggcagcgc ctgcagacca tgcacgatgt catgttcaat ggaggcatcc agacctttca 6840
   ggcgagacag cttcgccacc agcatcaaca gcacagttaa ctgagtggtg aatgctttag 6900
   tggatgccac gccgatttct gtacccgcgt tggtcattag cgccagatcg gattcgcgca 6960
   ccagagaaga acccggaacg ttacagattg ccagtgaacc aaggtaaccc agctctttcg 7020
   acagacgcag gccagccagg gtatccgcgg tttcgccaga ctgtgacaag gtgatcatca 7080
   ggctgttacg acgcacggca gatttgcgat agcggaattc agaggcgatt tcgacgtcgc 7140
   acggaatacc tgctagcgat tcaaaccagt agcgggaaac cataccggag ttataagaag 7200
   taccacaggc gaggatctga atatgctcaa ccttcgacag cagttcgtcg gcgttcggtc 7260
   ccagctcgct taaatcaacc tgaccgtggc tgatgcgtcc ggtaagggtg tttttgatcg 7320
   cgttcggctg ttcgtagatc tctttctgca tgtagtgacg gtaaatgcct ttatcgcccg 7380
   cgtcatattg cagattggat tcgatatcct gacgttttac ttccgcgcca gttttatcga 7440
   agatgtttac cgaacggcga gtga 7464
<210> 70
   <211> 70
   <212> DNA
   <213> Artificial Sequence
<220>
   <223> Primer (APtacKKDyIv03_6038-36-5)
<400> 70
   gataaagtct tcagtctgat ttaaataagc gttgatattc agtcaattac tgaagcctgc 60
   ttttttatac 70
<210> 71
   <211> 71
   <212> DNA
   <213> Artificial Sequence
<220>
   <223> Primer (SPtacKKDyIv02_6038-36-3)
<400> 71
   tcaccaaaaa taataacctt tcccggtgca gaagttaaga acggtaatga catggcagtc   60
   tccttgtgtg a                                                         71
<210> 72
   <211> 803
   <212> PRT
   <213> Enterococcus faecalis
<400> 72
   Met Lys Thr Val Val Ile Ile Asp Ala Leu Arg Thr Pro Ile Gly Lys
   1               5                   10                  15
   Tyr Lys Gly Ser Leu Ser Gln Val Ser Ala Val Asp Leu Gly Thr His
               20                  25                  30
   Val Thr Thr Gln Leu Leu Lys Arg His Ser Thr Ile Ser Glu Glu Ile
           35                  40                  45
   Asp Gln Val Ile Phe Gly Asn Val Leu Gln Ala Gly Asn Gly Gln Asn
       50                  55                  60
   Pro Ala Arg Gln Ile Ala Ile Asn Ser Gly Leu Ser His Glu Ile Pro
   65                  70                  75                  80
   Ala Met Thr Val Asn Glu Val Cys Gly Ser Gly Met Lys Ala Val Ile
                   85                  90                  95
   Leu Ala Lys Gln Leu Ile Gln Leu Gly Glu Ala Glu Val Leu Ile Ala
               100                 105                 110
   Gly Gly Ile Glu Asn Met Ser Gln Ala Pro Lys Leu Gln Arg Phe Asn
           115                 120                 125
   Tyr Glu Thr Glu Ser Tyr Asp Ala Pro Phe Ser Ser Met Met Tyr Asp
       130                 135                 140
   Gly Leu Thr Asp Ala Phe Ser Gly Gln Ala Met Gly Leu Thr Ala Glu
   145                 150                 155                 160
   Asn Val Ala Glu Lys Tyr His Val Thr Arg Glu Glu Gln Asp Gln Phe
                   165                 170                 175
   Ser Val His Ser Gln Leu Lys Ala Ala Gln Ala Gln Ala Glu Gly Ile
               180                 185                 190
   Phe Ala Asp Glu Ile Ala Pro Leu Glu Val Ser Gly Thr Leu Val Glu
           195                 200                 205
   Lys Asp Glu Gly Ile Arg Pro Asn Ser Ser Val Glu Lys Leu Gly Thr
       210                 215                 220
   Leu Lys Thr Val Phe Lys Glu Asp Gly Thr Val Thr Ala Gly Asn Ala
   225 230 235 240
   Ser Thr Ile Asn Asp Gly Ala Ser Ala Leu Ile Ile Ala Ser Gln Glu
   245 250 255
   Tyr Ala Glu Ala His Gly Leu Pro Tyr Leu Ala Ile Ile Arg Asp Ser
   260 265 270
   Val Glu Val Gly Ile Asp Pro Ala Tyr Met Gly Ile Ser Pro Ile Lys
   275 280 285
   Ala Ile Gln Lys Leu Leu Ala Arg Asn Gln Leu Thr Thr Glu Glu Ile
   290 295 300
   Asp Leu Tyr Glu Ile Asn Glu Ala Phe Ala Ala Thr Ser Ile Val Val
   305 310 315 320
   Gln Arg Glu Leu Ala Leu Pro Glu Glu Lys Val Asn Ile Tyr Gly Gly
   325 330 335
   Gly Ile Ser Leu Gly His Ala Ile Gly Ala Thr Gly Ala Arg Leu Leu
   340 345 350
   Thr Ser Leu Ser Tyr Gln Leu Asn Gln Lys Glu Lys Lys Tyr Gly Val
   355 360 365
   Ala Ser Leu Cys Ile Gly Gly Gly Leu Gly Leu Ala Met Leu Leu Glu
   370 375 380
   Arg Pro Gln Gln Lys Lys Asn Ser Arg Phe Tyr Gln Met Ser Pro Glu
   385 390 395 400
   Glu Arg Leu Ala Ser Leu Leu Asn Glu Gly Gln Ile Ser Ala Asp Thr
   405 410 415
   Lys Lys Glu Phe Glu Asn Thr Ala Leu Ser Ser Gln Ile Ala Asn His
   420 425 430
   Met Ile Glu Asn Gln Ile Ser Glu Thr Glu Val Pro Met Gly Val Gly
   435 440 445
   Leu His Leu Thr Val Asp Glu Thr Asp Tyr Leu Val Pro Met Ala Thr
   450 455 460
   Glu Glu Pro Ser Val Ile Ala Ala Leu Ser Asn Gly Ala Lys Ile Ala
   465 470 475 480
   Gln Gly Phe Lys Thr Val Asn Gln Gln Arg Leu Met Arg Gly Gln Ile
   485 490 495
   Val Phe Tyr Asp Val Ala Asp Ala Glu Ser Leu Ile Asp Glu Leu Gln
   500 505 510
   Val Arg Glu Thr Glu Ile Phe Gln Gln Ala Glu Leu Ser Tyr Pro Ser
   515 520 525
   Ile Val Lys Arg Gly Gly Gly Leu Arg Asp Leu Gln Tyr Arg Ala Phe
   530 535 540
   Asp Glu Ser Phe Val Ser Val Asp Phe Leu Val Asp Val Lys Asp Ala
   545 550 555 560
   Met Gly Ala Asn Ile Val Asn Ala Met Leu Glu Gly Val Ala Glu Leu
   565 570 575
   Phe Arg Glu Trp Phe Ala Glu Gln Lys Ile Leu Phe Ser Ile Leu Ser
   580 585 590
   Asn Tyr Ala Thr Glu Ser Val Val Thr Met Lys Thr Ala Ile Pro Val
   595 600 605
   Ser Arg Leu Ser Lys Gly Ser Asn Gly Arg Glu Ile Ala Glu Lys Ile
   610 615 620
   Val Leu Ala Ser Arg Tyr Ala Ser Leu Asp Pro Tyr Arg Ala Val Thr
   625 630 635 640
   His Asn Lys Gly Ile Met Asn Gly Ile Glu Ala Val Val Leu Ala Thr
   645 650 655
   Gly Asn Asp Thr Arg Ala Val Ser Ala Ser Cys His Ala Phe Ala Val
   660 665 670
   Lys Glu Gly Arg Tyr Gln Gly Leu Thr Ser Trp Thr Leu Asp Gly Glu
   675 680 685
   Gln Leu Ile Gly Glu Ile Ser Val Pro Leu Ala Leu Ala Thr Val Gly
   690 695 700
   Gly Ala Thr Lys Val Leu Pro Lys Ser Gln Ala Ala Ala Asp Leu Leu
   705 710 715 720
   Ala Val Thr Asp Ala Lys Glu Leu Ser Arg Val Val Ala Ala Val Gly
   725 730 735
   Leu Ala Gln Asn Leu Ala Ala Leu Arg Ala Leu Val Ser Glu Gly Ile
               740                 745                 750
   Gln Lys Gly His Met Ala Leu Gln Ala Arg Ser Leu Ala Met Thr Val
           755                 760                 765
   Gly Ala Thr Gly Lys Glu Val Glu Ala Val Ala Gln Gln Leu Lys Arg
       770                 775                 780
   Gln Lys Thr Met Asn Gln Asp Arg Ala Leu Ala Ile Leu Asn Asp Leu
   785                 790                 795                 800
   Arg Lys Gln
<210> 73
   <211> 2412
   <212> DNA
   <213> Enterococcus faecalis
<400> 73
   atgaaaacag tagttattat tgatgcatta cgaacaccaa ttggaaaata taaaggcagc     60
   ttaagtcaag taagtgccgt agacttagga acacatgtta caacacaact tttaaaaaga    120
   cattccacta tttctgaaga aattgatcaa gtaatctttg gaaatgtttt acaagctgga    180
   aatggccaaa atcccgcacg acaaatagca ataaacagcg gtttgtctca tgaaattccc    240
   gcaatgacgg ttaatgaggt ctgcggatca ggaatgaagg ccgttatttt ggcgaaacaa    300
   ttgattcaat taggagaagc ggaagtttta attgctggcg ggattgagaa tatgtcccaa    360
   gcacctaaat tacaacgttt taattacgaa acagaaagct acgatgcgcc tttttctagt    420
   atgatgtatg atggattaac ggatgccttt agtggtcagg caatgggctt aactgctgaa    480
   aatgtggccg aaaagtatca tgtaactaga gaagagcaag atcaattttc tgtacattca    540
   caattaaaag cagctcaagc acaagcagaa gggatattcg ctgacgaaat agccccatta    600
   gaagtatcag gaacgcttgt ggagaaagat gaagggattc gccctaattc gagcgttgag    660
   aagctaggaa cgcttaaaac agtttttaaa gaagacggta ctgtaacagc agggaatgca    720
   tcaaccatta atgatggggc ttctgctttg attattgctt cacaagaata tgccgaagca    780
   cacggtcttc cttatttagc tattattcga gacagtgtgg aagtcggtat tgatccagcc    840
   tatatgggaa tttcgccgat taaagccatt caaaaactgt tagcgcgcaa tcaacttact    900
   acggaagaaa ttgatctgta tgaaatcaac gaagcatttg cagcaacttc aatcgtggtc    960
   caaagagaac tggctttacc agaggaaaag gtcaacattt atggtggcgg tatttcatta   1020
   ggtcatgcga ttggtgccac aggtgctcgt ttattaacga gtttaagtta tcaattaaat   1080
   caaaaagaaa agaaatatgg agtggcttct ttatgtatcg gcggtggctt aggactcgct   1140
   atgctactag agagacctca gcaaaaaaaa aacagccgat tttatcaaat gagtcctgag   1200
   gaacgcctgg cttctcttct taatgaaggc cagatttctg ctgatacaaa aaaagaattt   1260
   gaaaatacgg ctttatcttc gcagattgcc aatcatatga ttgaaaatca aatcagtgaa   1320
   acagaagtgc cgatgggcgt tggcttacat ttaacagtgg acgaaactga ttatttggta   1380
   ccaatggcga cagaagagcc ctcagttatt gcggctttga gtaatggtgc aaaaatagca   1440
   caaggattta aaacagtgaa tcaacaacgc ttaatgcgtg gacaaatcgt tttttacgat   1500
   gttgcagatc ccgagtcatt gattgataaa ctacaagtaa gagaagcgga agtttttcaa   1560
   caagcagagt taagttatcc atctatcgtt aaacggggcg gcggcttaag agatttgcaa   1620
   tatcgtactt ttgatgaatc atttgtatct gtcgactttt tagtagatgt taaggatgca   1680
   atgggggcaa atatcgttaa cgctatgttg gaaggtgtgg ccgagttgtt ccgtgaatgg   1740
   tttgcggagc aaaagatttt attcagtatt ttaagtaatt atgccacgga gtcggttgtt   1800
   acgatgaaaa cggctattcc agtttcacgt ttaagtaagg ggagcaatgg ccgggaaatt   1860
   gctgaaaaaa ttgttttagc ttcacgctat gcttcattag atccttatcg ggcagtcacg   1920
   cataacaaag gaatcatgaa tggcattgaa gctgtagttt tagctacagg aaatgataca   1980
   cgcgctgtta gcgcttcttg tcatgctttt gcggtgaagg aaggtcgcta ccaaggcttg   2040
   actagttgga cgctggatgg cgaacaacta attggtgaaa tttcagttcc gcttgcttta   2100
   gccacggttg gcggtgccac aaaagtctta cctaaatctc aagcagctgc tgatttgtta   2160
   gcagtgacgg atgcaaaaga actaagtcga gtagtagcgg ctgttggttt ggcacaaaat   2220
   ttagcggcgt tacgggcctt agtctctgaa ggaattcaaa aaggacacat ggctctacaa   2280
   gcacgttctt tagcgatgac ggtcggagct actggtaaag aagttgaggc agtcgctcaa   2340
   caattaaaac gtcaaaaaac gatgaaccaa gaccgagcca tggctatttt aaatgattta   2400
   agaaaacaat aa                                                        2412
<210> 74
   <211> 2412
   <212> DNA
   <213> Artificial Sequence
<220>
   <223> DNA having modified codons, which encodes mvaE derived from Enterococcus faecalis
<400> 74
   atgaaaaccg tggttattat cgatgcgctg cgcacgccga ttggtaaata taaaggcagc     60
   ctgtctcaag tgagcgccgt tgatctgggt acgcatgtga ccacgcagct gctgaaacgt    120
   cacagcacca tctctgaaga aattgatcag gtgatctttg gtaacgttct gcaagccggt    180
   aatggtcaga atccggcacg tcagattgca atcaacagtg gcctgagcca tgaaattccg 240
   gcgatgaccg tgaatgaagt ttgcggtagc ggcatgaaag cggttattct ggccaaacag 300
   ctgatccagc tgggtgaagc ggaagtgctg attgccggcg gtatcgaaaa catgagtcag 360
   gcaccgaaac tgcaacgttt taattatgaa accgaaagct acgatgcccc gttcagctct 420
   atgatgtatg atggcctgac cgatgcattt agcggtcagg cgatgggcct gacggcagaa 480
   aacgtggcgg aaaaatacca tgttacccgc gaagaacagg atcagttttc tgttcacagt 540
   cagctgaaag cggcccaggc ccaggcagaa ggtattttcg ccgatgaaat cgcaccgctg 600
   gaagtgtctg gtacgctggt tgaaaaagat gaaggcattc gtccgaatag tagcgtggaa 660
   aaactgggca ccctgaaaac ggtgttcaaa gaagatggca ccgttacggc gggcaatgca 720
   agcaccatca atgatggtgc gagtgccctg attatcgcga gccaggaata tgcagaagcg 780
   catggcctgc cgtacctggc cattatccgc gattctgtgg aagttggtat tgatccggca 840
   tatatgggca ttagtccgat caaagcgatt cagaaactgc tggcccgtaa ccagctgacc 900
   accgaagaaa ttgatctgta cgaaatcaat gaagcgtttg cagcgaccag tattgtggtt 960
   cagcgcgaac tggccctgcc ggaagaaaaa gttaacattt atggcggtgg catcagcctg 1020
   ggtcacgcaa ttggtgccac cggtgcacgt ctgctgacca gtctgagcta tcagctgaat 1080
   cagaaagaga aaaaatacgg tgtggcaagc ctgtgtattg gtggcggtct gggtctggcc 1140
   atgctgctgg aacgtccgca gcagaagaaa aactctcgtt tttaccagat gagtccggaa 1200
   gaacgtctgg ccagtctgct gaacgaaggc cagattagcg cagataccaa aaaagaattc 1260
   gaaaatacgg cactgtctag tcagatcgcg aaccatatga ttgaaaatca gatcagcgaa 1320
   accgaagtgc cgatgggtgt tggcctgcac ctgaccgtgg atgaaacgga ttatctggtt 1380
   ccgatggcga cggaagaacc gagcgttatt gccgcactgt ctaacggtgc aaaaatcgcg 1440
   cagggcttta aaaccgtgaa tcagcagcgt ctgatgcgcg gccagattgt gttctacgat 1500
   gttgcggatc cggaaagcct gatcgataaa ctgcaagtgc gcgaagccga agtttttcag 1560
   caggcagaac tgagctatcc gtctattgtg aaacgtggcg gtggcctgcg cgatctgcaa 1620
   taccgtacct ttgatgaaag tttcgtgagc gttgatttcc tggtggatgt taaagatgcc 1680
   atgggtgcaa acatcgtgaa tgcgatgctg gaaggcgttg ccgaactgtt tcgtgaatgg 1740
   ttcgcggaac agaaaatcct gttttctatc ctgagtaact acgcgaccga aagcgtggtt 1800
   accatgaaaa cggccattcc tgtgagccgc ctgtctaaag gtagtaatgg ccgtgaaatt 1860
   gcggaaaaaa tcgttctggc gagccgctat gcctctctgg atccgtaccg tgccgtgacc 1920
   cataacaaag gtattatgaa tggcatcgaa gcagtggttc tggcgaccgg taacgatacc 1980
   cgtgccgtgt ctgcaagttg ccatgcattc gcagttaaag aaggtcgtta tcagggcctg 2040
   accagctgga cgctggatgg tgaacagctg atcggcgaaa tttctgtgcc gctggccctg 2100
   gcaaccgtgg gtggcgcgac gaaagttctg ccgaaaagcc aggcggccgc agatctgctg   2160
   gcggtgaccg atgcaaaaga actgtctcgc gtggttgcgg ccgttggtct ggcacagaat   2220
   ctggcagcgc tgcgtgcgct ggtgtctgaa ggtattcaga aaggccacat ggcactgcaa   2280
   gcccgtagtc tggccatgac cgtgggtgca acgggcaaag aagtggaagc agttgcgcag   2340
   cagctgaaac gccagaaaac catgaaccag gatcgtgcca tggcaatcct gaatgatctg   2400
   cgcaaacagt aa                                                       2412
<210> 75
   <211> 383
   <212> PRT
   <213> Enterococcus faecalis
<400> 75
   Met Thr Ile Gly Ile Asp Lys Ile Ser Phe Phe Val Pro Pro Tyr Tyr
   1               5                   10                  15
   Ile Asp Met Thr Ala Leu Ala Glu Ala Arg Asn Val Asp Pro Gly Lys
               20                  25                  30
   Phe His Ile Gly Ile Gly Gln Asp Gln Met Ala Val Asn Pro Ile Ser
           35                  40                  45
   Gln Asp Ile Val Thr Phe Ala Ala Asn Ala Ala Glu Ala Ile Leu Thr
       50                  55                  60
   Lys Glu Asp Lys Glu Ala Ile Asp Met Val Ile Val Gly Thr Glu Ser
   65                  70                  75                  80
   Ser Ile Asp Glu Ser Lys Ala Ala Ala Val Val Leu His Arg Leu Met
                   85                  90                  95
   Gly Ile Gln Pro Phe Ala Arg Ser Phe Glu Ile Lys Glu Ala Cys Tyr
               100                 105                 110
   Gly Ala Thr Ala Gly Leu Gln Leu Ala Lys Asn His Val Ala Leu His
           115                 120                 125
   Pro Asp Lys Lys Val Leu Val Val Ala Ala Asp Ile Ala Lys Tyr Gly
       130                 135                 140
   Leu Asn Ser Gly Gly Glu Pro Thr Gln Gly Ala Gly Ala Val Ala Met
   145                 150                 155                 160
   Leu Val Ala Ser Glu Pro Arg Ile Leu Ala Leu Lys Glu Asp Asn Val
                   165                 170                 175
   Met Leu Thr Gln Asp Ile Tyr Asp Phe Trp Arg Pro Thr Gly His Pro
               180                 185                 190
   Tyr Pro Met Val Asp Gly Pro Leu Ser Asn Glu Thr Tyr Ile Gln Ser
           195                 200                 205
   Phe Ala Gln Val Trp Asp Glu His Lys Lys Arg Thr Gly Leu Asp Phe
       210                 215                 220
   Ala Asp Tyr Asp Ala Leu Ala Phe His Ile Pro Tyr Thr Lys Met Gly
   225                 230                 235                 240
   Lys Lys Ala Leu Leu Ala Lys Ile Ser Asp Gln Thr Glu Ala Glu Gln
                   245                 250                 255
   Glu Arg Ile Leu Ala Arg Tyr Glu Glu Ser Ile Ile Tyr Ser Arg Arg
               260                 265                 270
   Val Gly Asn Leu Tyr Thr Gly Ser Leu Tyr Leu Gly Leu Ile Ser Leu
           275                 280                 285
   Leu Glu Asn Ala Thr Thr Leu Thr Ala Gly Asn Gln Ile Gly Leu Phe
       290                 295                 300
   Ser Tyr Gly Ser Gly Ala Val Ala Glu Phe Phe Thr Gly Glu Leu Val
   305                 310                 315                 320
   Ala Gly Tyr Gln Asn His Leu Gln Lys Glu Thr His Leu Ala Leu Leu
                   325                 330                 335
   Asp Asn Arg Thr Glu Leu Ser Ile Ala Glu Tyr Glu Ala Met Phe Ala
               340                 345                 350
   Glu Thr Leu Asp Thr Asp Ile Asp Gln Thr Leu Glu Asp Glu Leu Lys
           355                 360                 365
   Tyr Ser Ile Ser Ala Ile Asn Asn Thr Val Arg Ser Tyr Arg Asn
       370                 375                 380
<210> 76
   <211> 1152
   <212> DNA
   <213> Enterococcus faecalis
<400> 76
   atgacaattg ggattgataa aattagtttt tttgtgcccc cttattatat tgatatgacg     60
   gcactggctg aagccagaaa tgtagaccct ggaaaatttc atattggtat tgggcaagac    120
   caaatggcgg tgaacccaat cagccaagat attgtgacat ttgcagccaa tgccgcagaa    180
   gcgatcttga ccaaagaaga taaagaggcc attgatatgg tgattgtcgg gactgagtcc    240
   agtatcgatg agtcaaaagc ggccgcagtt gtcttacatc gtttaatggg gattcaacct    300
   ttcgctcgct ctttcgaaat caaggaagct tgttacggag caacagcagg cttacagtta    360
   gctaagaatc acgtagcctt acatccagat aaaaaagtct tggtcgtagc ggcagatatt    420
   gcaaaatatg gcttaaattc tggcggtgag cctacacaag gagctggggc ggttgcaatg    480
   ttagttgcta gtgaaccgcg cattttggct ttaaaagagg ataatgtgat gctgacgcaa    540
   gatatctatg acttttggcg tccaacaggc cacccgtatc ctatggtcga tggtcctttg    600
   tcaaacgaaa cctacatcca atcttttgcc caagtctggg atgaacataa aaaacgaacc    660
   ggtcttgatt ttgcagatta tgatgcttta gcgttccata ttccttacac aaaaatgggc    720
   aaaaaagcct tattagcaaa aatctccgac caaactgaag cagaacagga acgaatttta    780
   gcccgttatg aagaaagtat cgtctatagt cgtcgcgtag gaaacttgta tacgggttca    840
   ctttatctgg gactcatttc ccttttagaa aatgcaacga ctttaaccgc aggcaatcaa    900
   attggtttat tcagttatgg ttctggtgct gtcgctgaat ttttcactgg tgaattagta    960
   gctggttatc aaaatcattt acaaaaagaa actcatttag cactgctgga taatcggaca   1020
   gaactttcta tcgctgaata tgaagccatg tttgcagaaa ctttagacac agacattgat   1080
   caaacgttag aagatgaatt aaaatatagt atttctgcta ttaataatac cgttcgttct   1140
   tatcgaaact aa                                                       1152
<210> 77
   <211> 1152
   <212> DNA
   <213> Artificial Sequence
<220>
   <223> DNA having modified codons, which encodes mvaS derived from Enterococcus faecalis
<400> 77
   atgaccattg gtatcgataa aattagcttt ttcgtgccgc cgtattacat cgatatgacg      60
   gcgctggccg aagcacgtaa cgttgatccg ggcaaatttc atattggcat cggtcaggat     120
   cagatggcgg tgaacccgat ttctcaggat atcgttacct tcgcggccaa tgcagcggaa     180
   gcaattctga cgaaagaaga taaagaagcg attgatatgg tgatcgttgg caccgaaagc     240
   tctatcgatg aaagtaaagc cgcagcggtg gttctgcacc gtctgatggg cattcagccg     300
   tttgcgcgca gcttcgaaat caaagaagcc tgctatggcg cgaccgccgg tctgcaactg     360
   gccaaaaacc atgtggcact gcacccggat aaaaaagttc tggtggttgc cgcagatatt     420
   gcgaaatacg gtctgaatag cggcggtgaa ccgacccagg gtgcaggtgc cgtggcaatg     480
   ctggttgcat ctgaaccgcg tattctggcg ctgaaagaag ataacgtgat gctgacccag     540
   gatatctatg atttttggcg tccgaccggt catccgtacc cgatggtgga tggcccgctg     600
   agtaatgaaa cctatattca gagcttcgcc caggtttggg atgaacataa aaaacgtacg     660
   ggtctggatt ttgcggatta tgatgcactg gcgttccaca ttccgtacac caaaatgggc     720
   aaaaaagcgc tgctggccaa aatcagcgat cagacggaag ccgaacagga acgtattctg     780
   gcacgctatg aagaaagcat cgtgtactct cgtcgcgttg gcaacctgta taccggttct     840
   ctgtacctgg gcctgattag tctgctggaa aacgcgacca cgctgacggc cggcaatcag     900
   atcggtctgt tttcttatgg cagtggtgcc gtggcagaat ttttcaccgg tgaactggtt     960
   gccggctacc agaaccatct gcaaaaagaa acccacctgg ccctgctgga taatcgcacg    1020
   gaactgtcta ttgcagaata tgaagcaatg tttgcggaaa ccctggatac ggatatcgat    1080
   cagaccctgg aagatgaact gaaatatagt attagcgcga tcaacaatac ggtgcgtagt    1140
   taccgcaatt aa                                                        1152
<210> 78
   <211> 40
   <212> DNA
   <213> Artificial Sequence
<220>
   <223> Primer for amplifying a fragment comprising Para composed of araC and ara BAD promoters from E.coli
<400> 78
   tgaattcgag ctcggtaccc actcttcctt tttcaatatt 40
<210> 79
   <211> 40
   <212> DNA
   <213> Artificial Sequence
<220>
   <223> Primer for amplifying a fragment comprising Para composed of araC and ara BAD promoters from E.coli
<400> 79
   ataataacca cggttttcat tttttataac ctccttagag 40
<210> 80
   <211> 40
   <212> DNA
   <213> Artificial Sequence
<220>
   <223> Primer for amplifying a fragment comprising EFmvaE gene
<400> 80
   ctctaaggag gttataaaaa atgaaaaccg tggttattat 40
<210> 81
   <211> 55
   <212> DNA
   <213> Artificial Sequence
<220>
   <223> Primer for amplifying a fragment comprising EFmvaE gene
<400> 81
   ttatcgatac caatggtcat gtttttttac ctcctttact gtttgcgcag atcat 55
<210> 82
   <211> 55
   <212> DNA
   <213> Artificial Sequence
<220>
   <223> Primer for amplifying a fragment comprising EFmvaS gene
<400> 82
   atgatctgcg caaacagtaa aggaggtaaa aaaacatgac cattggtatc gataa 55
<210> 83
   <211> 40
   <212> DNA
   <213> Artificial Sequence
<220>
   <223> Primer for amplifying a fragment comprising EFmvaS gene
<400> 83
   cagcggaact ggcggctccc ttaattgcgg taactacgca 40
<210> 84
   <211> 40
   <212> DNA
   <213> Artificial Sequence
<220>
   <223> Primer for amplifying a fragment comprising Ttrp
<400> 84
   tgcgtagtta ccgcaattaa gggagccgcc agttccgctg 40
<210> 85
   <211> 38
   <212> DNA
   <213> Artificial Sequence
<220>
   <223> Primer for amplifying a fragment comprising Ttrp
<400> 85
   gtcgactcta gaggatccct aatgagaatt agtcaaat 38
<210> 86
   <211> 108
   <212> DNA
   <213> Artificial Sequence
<220>
   <223> Primer 1 for pUC-mvk-pmk (KKDS1_6038-3-1)
<400> 86
   gaggaataaa ccatggatcc gagctcggat ccactagtaa cggccgccag tgtgctggaa 60
   ttcgccctta ggaggtaaaa aaacatgtca ttaccgttct taacttct 108
<210> 87
   <211> 55
   <212> DNA
   <213> Artificial Sequence
<220>
   <223> Primer 2 for pUC-mvk-pmk (KKMyIA_6038-2-9)
<400> 87
   aagggcgaat tctgcatgca gctaccttaa gttatttatc aagataagtt tccgg 55
<210> 88
   <211> 51
   <212> DNA
   <213> Artificial Sequence
<220>
   <223> Primer 1 for pTWV-dmd-yidi (KMS_6038-6-1)
<400> 88
   gcagaattcg cccttaagga ggaaaaaaaa atgaccgttt acacagcatc c 51
<210> 89
   <211> 47
   <212> DNA
   <213> Artificial Sequence
<220>
   <223> Primer 2 for pTWV-dmd-yidi (KDyIA_6038-3-3)
<400> 89
   ccatatggta ccagctgcag ttatagcatt ctatgaattt gcctgtc 47
<210> 90
   <211> 9329
   <212> DNA
   <213> Artificial Sequence
<220>
   <223> Nucleotide sequence of the expression vector, pTrc-KKDyI(alpha)
<400> 90
   gtttgacagc ttatcatcga ctgcacggtg caccaatgct tctggcgtca ggcagccatc     60
   ggaagctgtg gtatggctgt gcaggtcgta aatcactgca taattcgtgt cgctcaaggc    120
   gcactcccgt tctggataat gttttttgcg ccgacatcat aacggttctg gcaaatattc    180
   tgaaatgagc tgttgacaat taatcatccg gctcgtataa tgtgtggaat tgtgagcgga 240
   taacaatttc acacaggaaa cagcgccgct gagaaaaagc gaagcggcac tgctctttaa 300
   caatttatca gacaatctgt gtgggcactc gaccggaatt atcgattaac tttattatta 360
   aaaattaaag aggtatatat taatgtatcg attaaataag gaggaataaa ccatggatcc 420
   gagctcggat ccactagtaa cggccgccag tgtgctggaa ttcgccctta ggaggtaaaa 480
   aaacatgtca ttaccgttct taacttctgc accgggaaag gttattattt ttggtgaaca 540
   ctctgctgtg tacaacaagc ctgccgtcgc tgctagtgtg tctgcgttga gaacctacct 600
   gctaataagc gagtcatctg caccagatac tattgaattg gacttcccgg acattagctt 660
   taatcataag tggtccatca atgatttcaa tgccatcacc gaggatcaag taaactccca 720
   aaaattggcc aaggctcaac aagccaccga tggcttgtct caggaactcg ttagtctttt 780
   ggatccgttg ttagctcaac tatccgaatc cttccactac catgcagcgt tttgtttcct 840
   gtatatgttt gtttgcctat gcccccatgc caagaatatt aagttttctt taaagtctac 900
   tttacccatc ggtgctgggt tgggctcaag cgcctctatt tctgtatcac tggccttagc 960
   tatggcctac ttgggggggt taataggatc taatgacttg gaaaagctgt cagaaaacga 1020
   taagcatata gtgaatcaat gggccttcat aggtgaaaag tgtattcacg gtaccccttc 1080
   aggaatagat aacgctgtgg ccacttatgg taatgccctg ctatttgaaa aagactcaca 1140
   taatggaaca ataaacacaa acaattttaa gttcttagat gatttcccag ccattccaat 1200
   gatcctaacc tatactagaa ttccaaggtc tacaaaagat cttgttgctc gcgttcgtgt 1260
   gttggtcacc gagaaatttc ctgaagttat gaagccaatt ctagatgcca tgggtgaatg 1320
   tgccctacaa ggcttagaga tcatgactaa gttaagtaaa tgtaaaggca ccgatgacga 1380
   ggctgtagaa actaataatg aactgtatga acaactattg gaattgataa gaataaatca 1440
   tggactgctt gtctcaatcg gtgtttctca tcctggatta gaacttatta aaaatctgag 1500
   cgatgatttg agaattggct ccacaaaact taccggtgct ggtggcggcg gttgctcttt 1560
   gactttgtta cgaagagaca ttactcaaga gcaaattgac agcttcaaaa agaaattgca 1620
   agatgatttt agttacgaga catttgaaac agacttgggt gggactggct gctgtttgtt 1680
   aagcgcaaaa aatttgaata aagatcttaa aatcaaatcc ctagtattcc aattatttga 1740
   aaataaaact accacaaagc aacaaattga cgatctatta ttgccaggaa acacgaattt 1800
   accatggact tcataagcta atttgcgata ggcctgcacc cttaaggagg aaaaaaacat 1860
   gtcagagttg agagccttca gtgccccagg gaaagcgtta ctagctggtg gatatttagt 1920
   tttagataca aaatatgaag catttgtagt cggattatcg gcaagaatgc atgctgtagc 1980
   ccatccttac ggttcattgc aagggtctga taagtttgaa gtgcgtgtga aaagtaaaca 2040
   atttaaagat ggggagtggc tgtaccatat aagtcctaaa agtggcttca ttcctgtttc 2100
   gataggcgga tctaagaacc ctttcattga aaaagttatc gctaacgtat ttagctactt 2160
   taaacctaac atggacgact actgcaatag aaacttgttc gttattgata ttttctctga 2220
   tgatgcctac cattctcagg aggatagcgt taccgaacat cgtggcaaca gaagattgag 2280
   ttttcattcg cacagaattg aagaagttcc caaaacaggg ctgggctcct cggcaggttt 2340
   agtcacagtt ttaactacag ctttggcctc cttttttgta tcggacctgg aaaataatgt 2400
   agacaaatat agagaagtta ttcataattt agcacaagtt gctcattgtc aagctcaggg 2460
   taaaattgga agcgggtttg atgtagcggc ggcagcatat ggatctatca gatatagaag 2520
   attcccaccc gcattaatct ctaatttgcc agatattgga agtgctactt acggcagtaa 2580
   actggcgcat ttggttgatg aagaagactg gaatattacg attaaaagta accatttacc 2640
   ttcgggatta actttatgga tgggcgatat taagaatggt tcagaaacag taaaactggt 2700
   ccagaaggta aaaaattggt atgattcgca tatgccagaa agcttgaaaa tatatacaga 2760
   actcgatcat gcaaattcta gatttatgga tggactatct aaactagatc gcttacacga 2820
   gactcatgac gattacagcg atcagatatt tgagtctctt gagaggaatg actgtacctg 2880
   tcaaaagtat cctgaaatca cagaagttag agatgcagtt gccacaatta gacgttcctt 2940
   tagaaaaata actaaagaat ctggtgccga tatcgaacct cccgtacaaa ctagcttatt 3000
   ggatgattgc cagaccttaa aaggagttct tacttgctta atacctggtg ctggtggtta 3060
   tgacgccatt gcagtgatta ctaagcaaga tgttgatctt agggctcaaa ccgctaatga 3120
   caaaagattt tctaaggttc aatggctgga tgtaactcag gctgactggg gtgttaggaa 3180
   agaaaaagat ccggaaactt atcttgataa ataacttaag gtagctgcat gcagaattcg 3240
   cccttaagga ggaaaaaaaa atgaccgttt acacagcatc cgttaccgca cccgtcaaca 3300
   tcgcaaccct taagtattgg gggaaaaggg acacgaagtt gaatctgccc accaattcgt 3360
   ccatatcagt gactttatcg caagatgacc tcagaacgtt gacctctgcg gctactgcac 3420
   ctgagtttga acgcgacact ttgtggttaa atggagaacc acacagcatc gacaatgaaa 3480
   gaactcaaaa ttgtctgcgc gacctacgcc aattaagaaa ggaaatggaa tcgaaggacg 3540
   cctcattgcc cacattatct caatggaaac tccacattgt ctccgaaaat aactttccta 3600
   cagcagctgg tttagcttcc tccgctgctg gctttgctgc attggtctct gcaattgcta 3660
   agttatacca attaccacag tcaacttcag aaatatctag aatagcaaga aaggggtctg 3720
   gttcagcttg tagatcgttg tttggcggat acgtggcctg ggaaatggga aaagctgaag 3780
   atggtcatga ttccatggca gtacaaatcg cagacagctc tgactggcct cagatgaaag 3840
   cttgtgtcct agttgtcagc gatattaaaa aggatgtgag ttccactcag ggtatgcaat 3900
   tgaccgtggc aacctccgaa ctatttaaag aaagaattga acatgtcgta ccaaagagat 3960
   ttgaagtcat gcgtaaagcc attgttgaaa aagatttcgc cacctttgca aaggaaacaa 4020
   tgatggattc caactctttc catgccacat gtttggactc tttccctcca atattctaca 4080
   tgaatgacac ttccaagcgt atcatcagtt ggtgccacac cattaatcag ttttacggag 4140
   aaacaatcgt tgcatacacg tttgatgcag gtccaaatgc tgtgttgtac tacttagctg 4200
   aaaatgagtc gaaactcttt gcatttatct ataaattgtt tggctctgtt cctggatggg 4260
   acaagaaatt tactactgag cagcttgagg ctttcaacca tcaatttgaa tcatctaact 4320
   ttactgcacg tgaattggat cttgagttgc aaaaggatgt tgccagagtg attttaactc 4380
   aagtcggttc aggcccacaa gaaacaaacg aatctttgat tgacgcaaag actggtctac 4440
   caaaggaata agatcaattc gctgcatcgc ccttaggagg taaaaaaaaa tgactgccga 4500
   caacaatagt atgccccatg gtgcagtatc tagttacgcc aaattagtgc aaaaccaaac 4560
   acctgaagac attttggaag agtttcctga aattattcca ttacaacaaa gacctaatac 4620
   ccgatctagt gagacgtcaa atgacgaaag cggagaaaca tgtttttctg gtcatgatga 4680
   ggagcaaatt aagttaatga atgaaaattg tattgttttg gattgggacg ataatgctat 4740
   tggtgccggt accaagaaag tttgtcattt aatggaaaat attgaaaagg gtttactaca 4800
   tcgtgcattc tccgtcttta ttttcaatga acaaggtgaa ttacttttac aacaaagagc 4860
   cactgaaaaa ataactttcc ctgatctttg gactaacaca tgctgctctc atccactatg 4920
   tattgatgac gaattaggtt tgaagggtaa gctagacgat aagattaagg gcgctattac 4980
   tgcggcggtg agaaaactag atcatgaatt aggtattcca gaagatgaaa ctaagacaag 5040
   gggtaagttt cactttttaa acagaatcca ttacatggca ccaagcaatg aaccatgggg 5100
   tgaacatgaa attgattaca tcctatttta taagatcaac gctaaagaaa acttgactgt 5160
   caacccaaac gtcaatgaag ttagagactt caaatgggtt tcaccaaatg atttgaaaac 5220
   tatgtttgct gacccaagtt acaagtttac gccttggttt aagattattt gcgagaatta 5280
   cttattcaac tggtgggagc aattagatga cctttctgaa gtggaaaatg acaggcaaat 5340
   tcatagaatg ctataactgc agctggtacc atatgggaat tcgaagcttt ctagaacaaa 5400
   aactcatctc agaagaggat ctgaatagcg ccgtcgacca tcatcatcat catcattgag 5460
   tttaaacggt ctccagcttg gctgttttgg cggatgagag aagattttca gcctgataca 5520
   gattaaatca gaacgcagaa gcggtctgat aaaacagaat ttgcctggcg gcagtagcgc 5580
   ggtggtccca cctgacccca tgccgaactc agaagtgaaa cgccgtagcg ccgatggtag 5640
   tgtggggtct ccccatgcga gagtagggaa ctgccaggca tcaaataaaa cgaaaggctc 5700
   agtcgaaaga ctgggccttt cgttttatct gttgtttgtc ggtgaacgct ctcctgagta 5760
   ggacaaatcc gccgggagcg gatttgaacg ttgcgaagca acggcccgga gggtggcggg 5820
   caggacgccc gccataaact gccaggcatc aaattaagca gaaggccatc ctgacggatg 5880
   gcctttttgc gtttctacaa actctttttg tttatttttc taaatacatt caaatatgta 5940
   tccgctcatg agacaataac cctgataaat gcttcaataa tattgaaaaa ggaagagtat 6000
   gagtattcaa catttccgtg tcgcccttat tccctttttt gcggcatttt gccttcctgt 6060
   ttttgctcac ccagaaacgc tggtgaaagt aaaagatgct gaagatcagt tgggtgcacg 6120
   agtgggttac atcgaactgg atctcaacag cggtaagatc cttgagagtt ttcgccccga 6180
   agaacgtttt ccaatgatga gcacttttaa agttctgcta tgtggcgcgg tattatcccg 6240
   tgttgacgcc gggcaagagc aactcggtcg ccgcatacac tattctcaga atgacttggt 6300
   tgagtactca ccagtcacag aaaagcatct tacggatggc atgacagtaa gagaattatg 6360
   cagtgctgcc ataaccatga gtgataacac tgcggccaac ttacttctga caacgatcgg 6420
   aggaccgaag gagctaaccg cttttttgca caacatgggg gatcatgtaa ctcgccttga 6480
   tcgttgggaa ccggagctga atgaagccat accaaacgac gagcgtgaca ccacgatgcc 6540
   tgtagcaatg gcaacaacgt tgcgcaaact attaactggc gaactactta ctctagcttc 6600
   ccggcaacaa ttaatagact ggatggaggc ggataaagtt gcaggaccac ttctgcgctc 6660
   ggcccttccg gctggctggt ttattgctga taaatctgga gccggtgagc gtgggtctcg 6720
   cggtatcatt gcagcactgg ggccagatgg taagccctcc cgtatcgtag ttatctacac 6780
   gacggggagt caggcaacta tggatgaacg aaatagacag atcgctgaga taggtgcctc 6840
   actgattaag cattggtaac tgtcagacca agtttactca tatatacttt agattgattt 6900
   aaaacttcat ttttaattta aaaggatcta ggtgaagatc ctttttgata atctcatgac 6960
   caaaatccct taacgtgagt tttcgttcca ctgagcgtca gaccccgtag aaaagatcaa 7020
   aggatcttct tgagatcctt tttttctgcg cgtaatctgc tgcttgcaaa caaaaaaacc 7080
   accgctacca gcggtggttt gtttgccgga tcaagagcta ccaactcttt ttccgaaggt 7140
   aactggcttc agcagagcgc agataccaaa tactgtcctt ctagtgtagc cgtagttagg 7200
   ccaccacttc aagaactctg tagcaccgcc tacatacctc gctctgctaa tcctgttacc 7260
   agtggctgct gccagtggcg ataagtcgtg tcttaccggg ttggactcaa gacgatagtt 7320
   accggataag gcgcagcggt cgggctgaac ggggggttcg tgcacacagc ccagcttgga 7380
   gcgaacgacc tacaccgaac tgagatacct acagcgtgag ctatgagaaa gcgccacgct 7440
   tcccgaaggg agaaaggcgg acaggtatcc ggtaagcggc agggtcggaa caggagagcg 7500
   cacgagggag cttccagggg gaaacgcctg gtatctttat agtcctgtcg ggtttcgcca 7560
   cctctgactt gagcgtcgat ttttgtgatg ctcgtcaggg gggcggagcc tatggaaaaa 7620
   cgccagcaac gcggcctttt tacggttcct ggccttttgc tggccttttg ctcacatgtt 7680
   ctttcctgcg ttatcccctg attctgtgga taaccgtatt accgcctttg agtgagctga 7740
   taccgctcgc cgcagccgaa cgaccgagcg cagcgagtca gtgagcgagg aagcggaaga   7800
   gcgcctgatg cggtattttc tccttacgca tctgtgcggt atttcacacc gcatatggtg   7860
   cactctcagt acaatctgct ctgatgccgc atagttaagc cagtatacac tccgctatcg   7920
   ctacgtgact gggtcatggc tgcgccccga cacccgccaa cacccgctga cgcgccctga   7980
   cgggcttgtc tgctcccggc atccgcttac agacaagctg tgaccgtctc cgggagctgc   8040
   atgtgtcaga ggttttcacc gtcatcaccg aaacgcgcga ggcagcagat caattcgcgc   8100
   gcgaaggcga agcggcatgc atttacgttg acaccatcga atggtgcaaa acctttcgcg   8160
   gtatggcatg atagcgcccg gaagagagtc aattcagggt ggtgaatgtg aaaccagtaa   8220
   cgttatacga tgtcgcagag tatgccggtg tctcttatca gaccgtttcc cgcgtggtga   8280
   accaggccag ccacgtttct gcgaaaacgc gggaaaaagt ggaagcggcg atggcggagc   8340
   tgaattacat tcccaaccgc gtggcacaac aactggcggg caaacagtcg ttgctgattg   8400
   gcgttgccac ctccagtctg gccctgcacg cgccgtcgca aattgtcgcg gcgattaaat   8460
   ctcgcgccga tcaactgggt gccagcgtgg tggtgtcgat ggtagaacga agcggcgtcg   8520
   aagcctgtaa agcggcggtg cacaatcttc tcgcgcaacg cgtcagtggg ctgatcatta   8580
   actatccgct ggatgaccag gatgccattg ctgtggaagc tgcctgcact aatgttccgg   8640
   cgttatttct tgatgtctct gaccagacac ccatcaacag tattattttc tcccatgaag   8700
   acggtacgcg actgggcgtg gagcatctgg tcgcattggg tcaccagcaa atcgcgctgt   8760
   tagcgggccc attaagttct gtctcggcgc gtctgcgtct ggctggctgg cataaatatc   8820
   tcactcgcaa tcaaattcag ccgatagcgg aacgggaagg cgactggagt gccatgtccg   8880
   gttttcaaca aaccatgcaa atgctgaatg agggcatcgt tcccactgcg atgctggttg   8940
   ccaacgatca gatggcgctg ggcgcaatgc gcgccattac cgagtccggg ctgcgcgttg   9000
   gtgcggatat ctcggtagtg ggatacgacg ataccgaaga cagctcatgt tatatcccgc   9060
   cgtcaaccac catcaaacag gattttcgcc tgctggggca aaccagcgtg gaccgcttgc   9120
   tgcaactctc tcagggccag gcggtgaagg gcaatcagct gttgcccgtc tcactggtga   9180
   aaagaaaaac caccctggcg cccaatacgc aaaccgcctc tccccgcgcg ttggccgatt   9240
   cattaatgca gctggcacga caggtttccc gactggaaag cgggcagtga gcgcaacgca   9300
   attaatgtga gttagcgcga attgatctg                                     9329
<210> 91
   <211> 68
   <212> DNA
   <213> Artificial Sequence
<220>
   <223> Primer for amplifying IspSK gene (pTrcKKDyIkSS_6038-10-1)
<400> 91
   atagaatgct ataacaacgc gtcctgcatt cgcccttagg aggtaaaaaa acatgtgtgc 60
   gacctctt 68
<210> 92
   <211> 46
   <212> DNA
   <213> Artificial Sequence
<220>
   <223> Primer for amplifying IspSK gene (pTrcKKDyIkSS_6038-10-2)
<400> 92
   ccatatggta ccagctgcag ttagacatac atcagctggt taatcg 46
<210> 93
   <211> 11062
   <212> DNA
   <213> Artificial Sequence
<220>
   <223> Nucleotide sequence of the expression vector, pTrc-KKDyI(alpha)-ispS(K)
<400> 93
   gtttgacagc ttatcatcga ctgcacggtg caccaatgct tctggcgtca ggcagccatc     60
   ggaagctgtg gtatggctgt gcaggtcgta aatcactgca taattcgtgt cgctcaaggc    120
   gcactcccgt tctggataat gttttttgcg ccgacatcat aacggttctg gcaaatattc    180
   tgaaatgagc tgttgacaat taatcatccg gctcgtataa tgtgtggaat tgtgagcgga    240
   taacaatttc acacaggaaa cagcgccgct gagaaaaagc gaagcggcac tgctctttaa    300
   caatttatca gacaatctgt gtgggcactc gaccggaatt atcgattaac tttattatta    360
   aaaattaaag aggtatatat taatgtatcg attaaataag gaggaataaa ccatggatcc    420
   gagctcggat ccactagtaa cggccgccag tgtgctggaa ttcgccctta ggaggtaaaa    480
   aaacatgtca ttaccgttct taacttctgc accgggaaag gttattattt ttggtgaaca    540
   ctctgctgtg tacaacaagc ctgccgtcgc tgctagtgtg tctgcgttga gaacctacct    600
   gctaataagc gagtcatctg caccagatac tattgaattg gacttcccgg acattagctt    660
   taatcataag tggtccatca atgatttcaa tgccatcacc gaggatcaag taaactccca    720
   aaaattggcc aaggctcaac aagccaccga tggcttgtct caggaactcg ttagtctttt    780
   ggatccgttg ttagctcaac tatccgaatc cttccactac catgcagcgt tttgtttcct    840
   gtatatgttt gtttgcctat gcccccatgc caagaatatt aagttttctt taaagtctac    900
   tttacccatc ggtgctgggt tgggctcaag cgcctctatt tctgtatcac tggccttagc    960
   tatggcctac ttgggggggt taataggatc taatgacttg gaaaagctgt cagaaaacga   1020
   taagcatata gtgaatcaat gggccttcat aggtgaaaag tgtattcacg gtaccccttc   1080
   aggaatagat aacgctgtgg ccacttatgg taatgccctg ctatttgaaa aagactcaca 1140
   taatggaaca ataaacacaa acaattttaa gttcttagat gatttcccag ccattccaat 1200
   gatcctaacc tatactagaa ttccaaggtc tacaaaagat cttgttgctc gcgttcgtgt 1260
   gttggtcacc gagaaatttc ctgaagttat gaagccaatt ctagatgcca tgggtgaatg 1320
   tgccctacaa ggcttagaga tcatgactaa gttaagtaaa tgtaaaggca ccgatgacga 1380
   ggctgtagaa actaataatg aactgtatga acaactattg gaattgataa gaataaatca 1440
   tggactgctt gtctcaatcg gtgtttctca tcctggatta gaacttatta aaaatctgag 1500
   cgatgatttg agaattggct ccacaaaact taccggtgct ggtggcggcg gttgctcttt 1560
   gactttgtta cgaagagaca ttactcaaga gcaaattgac agcttcaaaa agaaattgca 1620
   agatgatttt agttacgaga catttgaaac agacttgggt gggactggct gctgtttgtt 1680
   aagcgcaaaa aatttgaata aagatcttaa aatcaaatcc ctagtattcc aattatttga 1740
   aaataaaact accacaaagc aacaaattga cgatctatta ttgccaggaa acacgaattt 1800
   accatggact tcataagcta atttgcgata ggcctgcacc cttaaggagg aaaaaaacat 1860
   gtcagagttg agagccttca gtgccccagg gaaagcgtta ctagctggtg gatatttagt 1920
   tttagataca aaatatgaag catttgtagt cggattatcg gcaagaatgc atgctgtagc 1980
   ccatccttac ggttcattgc aagggtctga taagtttgaa gtgcgtgtga aaagtaaaca 2040
   atttaaagat ggggagtggc tgtaccatat aagtcctaaa agtggcttca ttcctgtttc 2100
   gataggcgga tctaagaacc ctttcattga aaaagttatc gctaacgtat ttagctactt 2160
   taaacctaac atggacgact actgcaatag aaacttgttc gttattgata ttttctctga 2220
   tgatgcctac cattctcagg aggatagcgt taccgaacat cgtggcaaca gaagattgag 2280
   ttttcattcg cacagaattg aagaagttcc caaaacaggg ctgggctcct cggcaggttt 2340
   agtcacagtt ttaactacag ctttggcctc cttttttgta tcggacctgg aaaataatgt 2400
   agacaaatat agagaagtta ttcataattt agcacaagtt gctcattgtc aagctcaggg 2460
   taaaattgga agcgggtttg atgtagcggc ggcagcatat ggatctatca gatatagaag 2520
   attcccaccc gcattaatct ctaatttgcc agatattgga agtgctactt acggcagtaa 2580
   actggcgcat ttggttgatg aagaagactg gaatattacg attaaaagta accatttacc 2640
   ttcgggatta actttatgga tgggcgatat taagaatggt tcagaaacag taaaactggt 2700
   ccagaaggta aaaaattggt atgattcgca tatgccagaa agcttgaaaa tatatacaga 2760
   actcgatcat gcaaattcta gatttatgga tggactatct aaactagatc gcttacacga 2820
   gactcatgac gattacagcg atcagatatt tgagtctctt gagaggaatg actgtacctg 2880
   tcaaaagtat cctgaaatca cagaagttag agatgcagtt gccacaatta gacgttcctt 2940
   tagaaaaata actaaagaat ctggtgccga tatcgaacct cccgtacaaa ctagcttatt 3000
   ggatgattgc cagaccttaa aaggagttct tacttgctta atacctggtg ctggtggtta 3060
   tgacgccatt gcagtgatta ctaagcaaga tgttgatctt agggctcaaa ccgctaatga 3120
   caaaagattt tctaaggttc aatggctgga tgtaactcag gctgactggg gtgttaggaa 3180
   agaaaaagat ccggaaactt atcttgataa ataacttaag gtagctgcat gcagaattcg 3240
   cccttaagga ggaaaaaaaa atgaccgttt acacagcatc cgttaccgca cccgtcaaca 3300
   tcgcaaccct taagtattgg gggaaaaggg acacgaagtt gaatctgccc accaattcgt 3360
   ccatatcagt gactttatcg caagatgacc tcagaacgtt gacctctgcg gctactgcac 3420
   ctgagtttga acgcgacact ttgtggttaa atggagaacc acacagcatc gacaatgaaa 3480
   gaactcaaaa ttgtctgcgc gacctacgcc aattaagaaa ggaaatggaa tcgaaggacg 3540
   cctcattgcc cacattatct caatggaaac tccacattgt ctccgaaaat aactttccta 3600
   cagcagctgg tttagcttcc tccgctgctg gctttgctgc attggtctct gcaattgcta 3660
   agttatacca attaccacag tcaacttcag aaatatctag aatagcaaga aaggggtctg 3720
   gttcagcttg tagatcgttg tttggcggat acgtggcctg ggaaatggga aaagctgaag 3780
   atggtcatga ttccatggca gtacaaatcg cagacagctc tgactggcct cagatgaaag 3840
   cttgtgtcct agttgtcagc gatattaaaa aggatgtgag ttccactcag ggtatgcaat 3900
   tgaccgtggc aacctccgaa ctatttaaag aaagaattga acatgtcgta ccaaagagat 3960
   ttgaagtcat gcgtaaagcc attgttgaaa aagatttcgc cacctttgca aaggaaacaa 4020
   tgatggattc caactctttc catgccacat gtttggactc tttccctcca atattctaca 4080
   tgaatgacac ttccaagcgt atcatcagtt ggtgccacac cattaatcag ttttacggag 4140
   aaacaatcgt tgcatacacg tttgatgcag gtccaaatgc tgtgttgtac tacttagctg 4200
   aaaatgagtc gaaactcttt gcatttatct ataaattgtt tggctctgtt cctggatggg 4260
   acaagaaatt tactactgag cagcttgagg ctttcaacca tcaatttgaa tcatctaact 4320
   ttactgcacg tgaattggat cttgagttgc aaaaggatgt tgccagagtg attttaactc 4380
   aagtcggttc aggcccacaa gaaacaaacg aatctttgat tgacgcaaag actggtctac 4440
   caaaggaata agatcaattc gctgcatcgc ccttaggagg taaaaaaaaa tgactgccga 4500
   caacaatagt atgccccatg gtgcagtatc tagttacgcc aaattagtgc aaaaccaaac 4560
   acctgaagac attttggaag agtttcctga aattattcca ttacaacaaa gacctaatac 4620
   ccgatctagt gagacgtcaa atgacgaaag cggagaaaca tgtttttctg gtcatgatga 4680
   ggagcaaatt aagttaatga atgaaaattg tattgttttg gattgggacg ataatgctat 4740
   tggtgccggt accaagaaag tttgtcattt aatggaaaat attgaaaagg gtttactaca 4800
   tcgtgcattc tccgtcttta ttttcaatga acaaggtgaa ttacttttac aacaaagagc 4860
   cactgaaaaa ataactttcc ctgatctttg gactaacaca tgctgctctc atccactatg 4920
   tattgatgac gaattaggtt tgaagggtaa gctagacgat aagattaagg gcgctattac 4980
   tgcggcggtg agaaaactag atcatgaatt aggtattcca gaagatgaaa ctaagacaag 5040
   gggtaagttt cactttttaa acagaatcca ttacatggca ccaagcaatg aaccatgggg 5100
   tgaacatgaa attgattaca tcctatttta taagatcaac gctaaagaaa acttgactgt 5160
   caacccaaac gtcaatgaag ttagagactt caaatgggtt tcaccaaatg atttgaaaac 5220
   tatgtttgct gacccaagtt acaagtttac gccttggttt aagattattt gcgagaatta 5280
   cttattcaac tggtgggagc aattagatga cctttctgaa gtggaaaatg acaggcaaat 5340
   tcatagaatg ctataacaac gcgtcctgca ttcgccctta ggaggtaaaa aaacatgtgt 5400
   gcgacctctt ctcaatttac tcagattacc gagcataatt cccgtcgttc cgcaaactat 5460
   cagccaaacc tgtggaattt cgaattcctg caatccctgg agaacgacct gaaagtggaa 5520
   aagctggagg agaaagcgac caaactggag gaagaagttc gctgcatgat caaccgtgta 5580
   gacacccagc cgctgtccct gctggagctg atcgacgatg tgcagcgcct gggtctgacc 5640
   tacaaatttg aaaaagacat cattaaagcc ctggaaaaca tcgtactgct ggacgaaaac 5700
   aaaaagaaca aatctgacct gcacgcaacc gctctgtctt tccgtctgct gcgtcagcac 5760
   ggtttcgagg tttctcagga tgtttttgag cgtttcaagg ataaagaagg tggtttcagc 5820
   ggtgaactga aaggtgacgt ccaaggcctg ctgagcctgt atgaagcgtc ttacctgggt 5880
   ttcgagggtg agaacctgct ggaggaggcg cgtacctttt ccatcaccca cctgaagaac 5940
   aacctgaaag aaggcattaa taccaaggtt gcagaacaag tgagccacgc cctggaactg 6000
   ccatatcacc agcgtctgca ccgtctggag gcacgttggt tcctggataa atacgaaccg 6060
   aaagaaccgc atcaccagct gctgctggag ctggcgaagc tggattttaa catggtacag 6120
   accctgcacc agaaagagct gcaagatctg tcccgctggt ggaccgagat gggcctggct 6180
   agcaaactgg attttgtacg cgaccgcctg atggaagttt atttctgggc actgggtatg 6240
   gcgccagacc cgcagtttgg tgaatgtcgc aaagctgtta ctaaaatgtt tggtctggtg 6300
   acgatcatcg atgacgtgta tgacgtttat ggcactctgg acgaactgca actgttcacc 6360
   gatgctgtag agcgctggga cgttaacgct attaacaccc tgccggacta tatgaaactg 6420
   tgtttcctgg cactgtacaa caccgttaac gacacgtcct attctattct gaaagagaaa 6480
   ggtcataaca acctgtccta tctgacgaaa agctggcgtg aactgtgcaa agcctttctg 6540
   caagaggcga aatggtccaa caacaaaatt atcccggctt tctccaagta cctggaaaac 6600
   gccagcgttt cctcctccgg tgtagcgctg ctggcgccgt cttacttttc cgtatgccag 6660
   cagcaggaag acatctccga ccacgcgctg cgttccctga ccgacttcca tggtctggtg 6720
   cgttctagct gcgttatctt ccgcctgtgc aacgatctgg ccacctctgc ggcggagctg 6780
   gaacgtggcg agactaccaa ttctatcatt agctacatgc acgaaaacga tggtaccagc 6840
   gaggaacagg cccgcgaaga actgcgtaaa ctgatcgacg ccgaatggaa aaagatgaat 6900
   cgtgaacgcg ttagcgactc caccctgctg cctaaagcgt tcatggaaat cgcagttaac 6960
   atggcacgtg tttcccactg cacctaccag tatggcgatg gtctgggtcg cccagactac 7020
   gcgactgaaa accgcatcaa actgctgctg attgaccctt tcccgattaa ccagctgatg 7080
   tatgtctaac tgcagctggt accatatggg aattcgaagc tttctagaac aaaaactcat 7140
   ctcagaagag gatctgaata gcgccgtcga ccatcatcat catcatcatt gagtttaaac 7200
   ggtctccagc ttggctgttt tggcggatga gagaagattt tcagcctgat acagattaaa 7260
   tcagaacgca gaagcggtct gataaaacag aatttgcctg gcggcagtag cgcggtggtc 7320
   ccacctgacc ccatgccgaa ctcagaagtg aaacgccgta gcgccgatgg tagtgtgggg 7380
   tctccccatg cgagagtagg gaactgccag gcatcaaata aaacgaaagg ctcagtcgaa 7440
   agactgggcc tttcgtttta tctgttgttt gtcggtgaac gctctcctga gtaggacaaa 7500
   tccgccggga gcggatttga acgttgcgaa gcaacggccc ggagggtggc gggcaggacg 7560
   cccgccataa actgccaggc atcaaattaa gcagaaggcc atcctgacgg atggcctttt 7620
   tgcgtttcta caaactcttt ttgtttattt ttctaaatac attcaaatat gtatccgctc 7680
   atgagacaat aaccctgata aatgcttcaa taatattgaa aaaggaagag tatgagtatt 7740
   caacatttcc gtgtcgccct tattcccttt tttgcggcat tttgccttcc tgtttttgct 7800
   cacccagaaa cgctggtgaa agtaaaagat gctgaagatc agttgggtgc acgagtgggt 7860
   tacatcgaac tggatctcaa cagcggtaag atccttgaga gttttcgccc cgaagaacgt 7920
   tttccaatga tgagcacttt taaagttctg ctatgtggcg cggtattatc ccgtgttgac 7980
   gccgggcaag agcaactcgg tcgccgcata cactattctc agaatgactt ggttgagtac 8040
   tcaccagtca cagaaaagca tcttacggat ggcatgacag taagagaatt atgcagtgct 8100
   gccataacca tgagtgataa cactgcggcc aacttacttc tgacaacgat cggaggaccg 8160
   aaggagctaa ccgctttttt gcacaacatg ggggatcatg taactcgcct tgatcgttgg 8220
   gaaccggagc tgaatgaagc cataccaaac gacgagcgtg acaccacgat gcctgtagca 8280
   atggcaacaa cgttgcgcaa actattaact ggcgaactac ttactctagc ttcccggcaa 8340
   caattaatag actggatgga ggcggataaa gttgcaggac cacttctgcg ctcggccctt 8400
   ccggctggct ggtttattgc tgataaatct ggagccggtg agcgtgggtc tcgcggtatc 8460
   attgcagcac tggggccaga tggtaagccc tcccgtatcg tagttatcta cacgacgggg 8520
   agtcaggcaa ctatggatga acgaaataga cagatcgctg agataggtgc ctcactgatt 8580
   aagcattggt aactgtcaga ccaagtttac tcatatatac tttagattga tttaaaactt 8640
   catttttaat ttaaaaggat ctaggtgaag atcctttttg ataatctcat gaccaaaatc 8700
   ccttaacgtg agttttcgtt ccactgagcg tcagaccccg tagaaaagat caaaggatct 8760
   tcttgagatc ctttttttct gcgcgtaatc tgctgcttgc aaacaaaaaa accaccgcta 8820
   ccagcggtgg tttgtttgcc ggatcaagag ctaccaactc tttttccgaa ggtaactggc 8880
   ttcagcagag cgcagatacc aaatactgtc cttctagtgt agccgtagtt aggccaccac 8940
   ttcaagaact ctgtagcacc gcctacatac ctcgctctgc taatcctgtt accagtggct 9000
   gctgccagtg gcgataagtc gtgtcttacc gggttggact caagacgata gttaccggat 9060
   aaggcgcagc ggtcgggctg aacggggggt tcgtgcacac agcccagctt ggagcgaacg 9120
   acctacaccg aactgagata cctacagcgt gagctatgag aaagcgccac gcttcccgaa 9180
   gggagaaagg cggacaggta tccggtaagc ggcagggtcg gaacaggaga gcgcacgagg 9240
   gagcttccag ggggaaacgc ctggtatctt tatagtcctg tcgggtttcg ccacctctga 9300
   cttgagcgtc gatttttgtg atgctcgtca ggggggcgga gcctatggaa aaacgccagc 9360
   aacgcggcct ttttacggtt cctggccttt tgctggcctt ttgctcacat gttctttcct 9420
   gcgttatccc ctgattctgt ggataaccgt attaccgcct ttgagtgagc tgataccgct 9480
   cgccgcagcc gaacgaccga gcgcagcgag tcagtgagcg aggaagcgga agagcgcctg 9540
   atgcggtatt ttctccttac gcatctgtgc ggtatttcac accgcatatg gtgcactctc 9600
   agtacaatct gctctgatgc cgcatagtta agccagtata cactccgcta tcgctacgtg 9660
   actgggtcat ggctgcgccc cgacacccgc caacacccgc tgacgcgccc tgacgggctt 9720
   gtctgctccc ggcatccgct tacagacaag ctgtgaccgt ctccgggagc tgcatgtgtc 9780
   agaggttttc accgtcatca ccgaaacgcg cgaggcagca gatcaattcg cgcgcgaagg 9840
   cgaagcggca tgcatttacg ttgacaccat cgaatggtgc aaaacctttc gcggtatggc 9900
   atgatagcgc ccggaagaga gtcaattcag ggtggtgaat gtgaaaccag taacgttata 9960
   cgatgtcgca gagtatgccg gtgtctctta tcagaccgtt tcccgcgtgg tgaaccaggc 10020
   cagccacgtt tctgcgaaaa cgcgggaaaa agtggaagcg gcgatggcgg agctgaatta 10080
   cattcccaac cgcgtggcac aacaactggc gggcaaacag tcgttgctga ttggcgttgc 10140
   cacctccagt ctggccctgc acgcgccgtc gcaaattgtc gcggcgatta aatctcgcgc 10200
   cgatcaactg ggtgccagcg tggtggtgtc gatggtagaa cgaagcggcg tcgaagcctg 10260
   taaagcggcg gtgcacaatc ttctcgcgca acgcgtcagt gggctgatca ttaactatcc 10320
   gctggatgac caggatgcca ttgctgtgga agctgcctgc actaatgttc cggcgttatt 10380
   tcttgatgtc tctgaccaga cacccatcaa cagtattatt ttctcccatg aagacggtac 10440
   gcgactgggc gtggagcatc tggtcgcatt gggtcaccag caaatcgcgc tgttagcggg 10500
   cccattaagt tctgtctcgg cgcgtctgcg tctggctggc tggcataaat atctcactcg 10560
   caatcaaatt cagccgatag cggaacggga aggcgactgg agtgccatgt ccggttttca 10620
   acaaaccatg caaatgctga atgagggcat cgttcccact gcgatgctgg ttgccaacga 10680
   tcagatggcg ctgggcgcaa tgcgcgccat taccgagtcc gggctgcgcg ttggtgcgga 10740
   tatctcggta gtgggatacg acgataccga agacagctca tgttatatcc cgccgtcaac 10800
   caccatcaaa caggattttc gcctgctggg gcaaaccagc gtggaccgct tgctgcaact 10860
   ctctcagggc caggcggtga agggcaatca gctgttgccc gtctcactgg tgaaaagaaa 10920
   aaccaccctg gcgcccaata cgcaaaccgc ctctccccgc gcgttggccg attcattaat 10980
   gcagctggca cgacaggttt cccgactgga aagcgggcag tgagcgcaac gcaattaatg 11040
   tgagttagcg cgaattgatc tg 11062
<210> 94
   <211> 301
   <212> PRT
   <213> Methanosarcina mazei
<400> 94
   Met Val Ser Cys Ser Ala Pro Gly Lys Ile Tyr Leu Phe Gly Glu His
   1 5 10 15
   Ala Val Val Tyr Gly Glu Thr Ala Ile Ala Cys Ala Val Glu Leu Arg
   20 25 30
   Thr Arg Val Arg Ala Glu Leu Asn Asp Ser Ile Thr Ile Gln Ser Gln
   35 40 45
   Ile Gly Arg Thr Gly Leu Asp Phe Glu Lys His Pro Tyr Val Ser Ala
   50 55 60
   Val Ile Glu Lys Met Arg Lys Ser Ile Pro Ile Asn Gly Val Phe Leu
   65 70 75 80
   Thr Val Asp Ser Asp Ile Pro Val Gly Ser Gly Leu Gly Ser Ser Ala
   85 90 95
   Ala Val Thr Ile Ala Ser Ile Gly Ala Leu Asn Glu Leu Phe Gly Phe
   100 105 110
   Gly Leu Ser Leu Gln Glu Ile Ala Lys Leu Gly His Glu Ile Glu Ile
   115 120 125
   Lys Val Gln Gly Ala Ala Ser Pro Thr Asp Thr Tyr Val Ser Thr Phe
   130 135 140
   Gly Gly Val Val Thr Ile Pro Glu Arg Arg Lys Leu Lys Thr Pro Asp
   145                 150                 155                 160
   Cys Gly Ile Val Ile Gly Asp Thr Gly Val Phe Ser Ser Thr Lys Glu
                   165                 170                 175
   Leu Val Ala Asn Val Arg Gln Leu Arg Glu Ser Tyr Pro Asp Leu Ile
               180                 185                 190
   Glu Pro Leu Met Thr Ser Ile Gly Lys Ile Ser Arg Ile Gly Glu Gln
           195                 200                 205
   Leu Val Leu Ser Gly Asp Tyr Ala Ser Ile Gly Arg Leu Met Asn Val
       210                 215                 220
   Asn Gln Gly Leu Leu Asp Ala Leu Gly Val Asn Ile Leu Glu Leu Ser
   225                 230                 235                 240
   Gln Leu Ile Tyr Ser Ala Arg Ala Ala Gly Ala Phe Gly Ala Lys Ile
                   245                 250                 255
   Thr Gly Ala Gly Gly Gly Gly Cys Met Val Ala Leu Thr Ala Pro Glu
               260                 265                 270
   Lys Cys Asn Gln Val Ala Glu Ala Val Ala Gly Ala Gly Gly Lys Val
           275                 280                 285
   Thr Ile Thr Lys Pro Thr Glu Gln Gly Leu Lys Val Asp
       290                 295                 300
<210> 95
   <211> 906
   <212> DNA
   <213> Methanosarcina mazei
<400> 95
   atggtttcat gttctgcgcc cgggaaaatc tatcttttcg gagaacatgc ggttgtttac 60
   ggagaaaccg caatagcgtg tgcagtagag ttaaggaccc gggtgcgggc ggagttaaat 120
   gactccataa ctatccagtc tcagatcggc aggacaggtc ttgattttga aaaacatccc 180
   tatgtctctg cagtgattga aaaaatgaga aaatctatcc ccataaatgg tgttttttta 240
   actgttgatt ccgacattcc tgttgggtca gggctcggct catctgctgc tgttacgatt 300
   gcaagcatag gagctctcaa cgaacttttc ggattcgggc tttcgcttca ggaaattgcg 360
   aaactggggc atgaaattga gataaaagtt cagggtgcag cgagccctac tgacacctat 420
   gtttctactt tcggaggagt cgttaccatc cctgaaagga gaaagcttaa gactcctgac 480
   tgtggaattg ttatagggga caccggagtt ttttcttcta caaaagagct tgtggcaaac 540
   gtcaggcagc tccgcgaaag ttaccctgat cttatcgaac ctcttatgac ttctattggc 600
   aaaatctcca gaatcggtga gcaacttgta ctttccgggg actatgcttc tattggcagg 660
   cttatgaatg taaatcaggg actgcttgat gcacttggag ttaatatcct tgagctttca 720
   cagcttatct attctgcaag ggcagcagga gctttcgggg caaaaattac tggagcagga 780
   ggcggtggtt gtatggttgc gctaactgca ccggagaaat gtaatcaggt agcggaagcc 840
   gttgcaggtg cagggggcaa agtgaccatt acaaaaccca cggaacaggg gttgaaggtc 900
   gattga 906
<210> 96
   <211> 906
   <212> DNA
   <213> Artificial Sequence
<220>
   <223> DNA having modified codons, which encodes mevalonate kinase derived from Methanosarcina mazei (Mmamvk)
<400> 96
   atggtatcct gttctgcgcc gggtaagatt tacctgttcg gtgaacacgc cgtagtttat      60
   ggcgaaactg caattgcgtg tgcggtggaa ctgcgtaccc gtgttcgcgc ggaactcaat     120
   gactctatca ctattcagag ccagatcggc cgcaccggtc tggatttcga aaagcaccct     180
   tatgtgtctg cggtaattga gaaaatgcgc aaatctattc ctattaacgg tgttttcttg     240
   accgtcgatt ccgacatccc ggtgggctcc ggtctgggta gcagcgcagc cgttactatc     300
   gcgtctattg gtgcgctgaa cgagctgttc ggctttggcc tcagcctgca agaaatcgct     360
   aaactgggcc acgaaatcga aattaaagta cagggtgccg cgtccccaac cgatacgtat     420
   gtttctacct tcggcggcgt ggttaccatc ccggaacgtc gcaaactgaa aactccggac     480
   tgcggcattg tgattggcga taccggcgtt ttctcctcca ccaaagagtt agtagctaac     540
   gtacgtcagc tgcgcgaaag ctacccggat ttgatcgaac cgctgatgac ctctattggc     600
   aaaatctctc gtatcggcga acaactggtt ctgtctggcg actacgcatc catcggccgc     660
   ctgatgaacg tcaaccaggg tctcctggac gccctgggcg ttaacatctt agaactgagc     720
   cagctgatct attccgctcg tgcggcaggt gcgtttggcg ctaaaatcac gggcgctggc     780
   ggcggtggct gtatggttgc gctgaccgct ccggaaaaat gcaaccaagt ggcagaagcg     840
   gtagcaggcg ctggcggtaa agtgactatc actaaaccga ccgagcaagg tctgaaagta     900
   gattaa                                                                906
<210> 97
   <211> 79
   <212> DNA
   <213> Artificial Sequence
<220>
   <223> Primer for amplifying IspSK gene
<400> 97
   gataacaatt tcacacaata attttgttta actttaagaa ggagatataa tgtgtgcgac      60
   ctcttctcaa tttactcag                                                   79
<210> 98
   <211> 42
   <212> DNA
   <213> Artificial Sequence
<220>
   <223> Primer for amplifying IspSK gene
<400> 98
   acggccagtg aattcttaga catacatcag ctggttaatc gg 42
<210> 99
   <211> 29
   <212> DNA
   <213> Artificial Sequence
<220>
   <223> Primer for amplifying pSTV28-Ptac-Ttrp construct
<400> 99
   gtgtgaaatt gttatccgct cacaattcc 29
<210> 100
   <211> 27
   <212> DNA
   <213> Artificial Sequence
<220>
   <223> Primer for amplifying pSTV28-Ptac-Ttrp construct
<400> 100
   gaattcactg gccgtcgttt tacaacg 27
<210> 101
   <211> 53
   <212> DNA
   <213> Artificial Sequence
<220>
   <223> Primer for amplifying Mmamvk gene
<400> 101
   ctgatgtatg tctaactgca taaaggaggt aaaaaaacat ggtatcctgt tct 53
<210> 102
   <211> 50
   <212> DNA
   <213> Artificial Sequence
<220>
   <223> Primer for amplifying Mmamvk gene
<400> 102
   ttgtaaaacg acggccagtg aattcttaat ctactttcag accttgctcg 50
<210> 103
   <211> 20
   <212> DNA
   <213> Artificial Sequence
<220>
   <223> Primer for amplifying pSTV28-Ptac-IspSK
<400> 103
   gaattcactg gccgtcgttt 20
<210> 104
   <211> 20
   <212> DNA
   <213> Artificial Sequence
<220>
   <223> Primer for amplifying pSTV28-Ptac-IspSK
<400> 104
   ttagacatac atcagctggt 20
<210> 105
   <211> 40
   <212> DNA
   <213> Artificial Sequence
<220>
   <223> Primer 1 for amplifying Mclmvk gene (mcl_mvk_N)
<400> 105
   tttcacacaa ggagactccc atgacgatgg cttccgctcc 40
<210> 106
   <211> 40
   <212> DNA
   <213> Artificial Sequence
<220>
   <223> Primer 2 for amplifying Mclmvk gene (mcl_mvk_C)
<400> 106
   cagcggaact ggcggctccc ttacgcgact tccaggcgaa 40
<210> 107
   <211> 20
   <212> DNA
   <213> Artificial Sequence
<220>
   <223> Primer 1 for amplifying pMW219-Ptac-Ttrp (PtTt219f)
<400> 107
   gggagccgcc agttccgctg 20
<210> 108
   <211> 21
   <212> DNA
   <213> Artificial Sequence
<220>
   <223> Primer 2 for amplifying pMW219-Ptac-Ttrp (PtTt219r)
<400> 108
   gggagtctcc ttgtgtgaaa t 21
<210> 109
   <211> 68
   <212> DNA
   <213> Artificial Sequence
<220>
   <223> Primer
<400> 109
   ctgatgaact gtcacctgaa tgagtgctga tgaaaatata gaaaggtcat ttttcctgaa 60
   tatgctca 68
<210> 110
   <211> 64
   <212> DNA
   <213> Artificial Sequence
<220>
   <223> Primer
<400> 110
   attcgccagc ataacgatgc cgctgttgag ctgaggaaca cgtttgttga cagctggtcc 60
   aatg 64
<210> 111
   <211> 70
   <212> DNA
   <213> Artificial Sequence
<220>
   <223> Primer
<400> 111
   atgcgcactc cttacgtact ggctctactg gtttctttgc gaaaggtcat ttttcctgaa 60
   tatgctcaca 70
<210> 112
   <211> 64
   <212> DNA
   <213> Artificial Sequence
<220>
   <223> Primer
<400> 112
   ttaaggaatc gcctggacca tcatcggcga gccgttctga cgtttgttga cagctggtcc 60
   aatg 64
<210> 113
   <211> 68
   <212> DNA
   <213> Artificial Sequence
<220>
   <223> Primer
<400> 113
   atgttgtgga tttggaatgc cctgatcgtt ttcgttaccg gaaaggtcat ttttcctgaa 60
   tatgctca 68
<210> 114
   <211> 64
   <212> DNA
   <213> Artificial Sequence
<220>
   <223> Primer
<400> 114
   atgacggtct gcgcaaaaaa acacgttcat ctcactcgcg cgtttgttga cagctggtcc 60
   aatg 64
<210> 115
   <211> 21
   <212> DNA
   <213> Artificial Sequence
<220>
   <223> Primer
<400> 115
   gattcccact tcaccgagcc g 21
<210> 116
   <211> 21
   <212> DNA
   <213> Artificial Sequence
<220>
   <223> Primer
<400> 116
   ggcaggtatg gtgctctgac g 21
<210> 117
   <211> 18
   <212> DNA
   <213> Artificial Sequence
<220>
   <223> Primer
<400> 117
   gcgaagccct ctccgttg 18
<210> 118
   <211> 21
   <212> DNA
   <213> Artificial Sequence
<220>
   <223> Primer
<400> 118
   agccagtcag cctcatcagc g 21
<210> 119
   <211> 21
   <212> DNA
   <213> Artificial Sequence
<220>
   <223> Primer
<400> 119
   ccgtgtggtt ctgaaagccg a 21
<210> 120
   <211> 21
   <212> DNA
   <213> Artificial Sequence
<220>
   <223> Primer
<400> 120
   cgttgccgta aatgtatccg t 21
<210> 121
   <211> 27
   <212> DNA
   <213> Artificial Sequence
<220>
   <223> Primer
<400> 121
   ggatgtaaac cataacactc tgcgaac 27
<210> 122
   <211> 25
   <212> DNA
   <213> Artificial Sequence
<220>
   <223> Primer
<400> 122
   gattggtggt tgaattgtcc gtaac 25
<210> 123
   <211> 22
   <212> DNA
   <213> Artificial Sequence
<220>
   <223> Primer
<400> 123
   tggaaggatt cggatagttg ag 22
<210> 124
   <211> 20
   <212> DNA
   <213> Artificial Sequence
<220>
   <223> Primer
<400> 124
   ttactgacct gttgatcggc 20
<210> 125
   <211> 20
   <212> DNA
   <213> Artificial Sequence
<220>
   <223> Primer
<400> 125
   ctgaggatgt tttccgcctg 20
<210> 126
   <211> 89
   <212> DNA
   <213> Artificial Sequence
<220>
   <223> Primer
<400> 126
   ctgctgccgc ctctcctgca cgacatcagc tgaactctcc tcctctgtct gacgtcatcc 60
   tgaagcctgc ttttttatac taagttggc 89
<210> 127
   <211> 90
   <212> DNA
   <213> Artificial Sequence
<220>
   <223> Primer
<400> 127
   aacggcggca gaggcggtga taaaaaagag acaagcaaaa agcggtttta ccaggttcat 60
   tatatctcct tcttaaagtt aaacaaaatt 90
<210> 128
   <211> 22
   <212> DNA
   <213> Artificial Sequence
<220>
   <223> Primer
<400> 128
   tggggcacac gcgtttatga gc 22
<210> 129
   <211> 22
   <212> DNA
   <213> Artificial Sequence
<220>
   <223> Primer
<400> 129
   gtaagcgccc ggtccgacct ta 22
<210> 130
   <211> 20
   <212> DNA
   <213> Artificial Sequence
<220>
   <223> Primer
<400> 130
   tgctgcgggc attgtttccc 20
<210> 131
   <211> 20
   <212> DNA
   <213> Artificial Sequence
<220>
   <223> Primer
<400> 131
   tcggcattgg ccgggatctc 20
<210> 132
   <211> 89
   <212> DNA
   <213> Artificial Sequence
<220>
   <223> Primer
<400> 132
   tgttcatcac ggccaaatag cgttgaggag cattccgaaa cgccaaaaag ggggcgtact 60
   tgaagcctgc ttttttatac taagttggc 89
<210> 133
   <211> 90
   <212> DNA
   <213> Artificial Sequence
<220>
   <223> Primer
<400> 133
   aataattacg tagatatctt ctggcaggtc tttacctgct ggcacctggt tcaaactcat 60
   tatatctcct tcttaaagtt aaacaaaatt 90
<210> 134
   <211> 22
   <212> DNA
   <213> Artificial Sequence
<220>
   <223> Primer
<400> 134
   catgaaaaga ggatgggtgt tg 22
<210> 135
   <211> 22
   <212> DNA
   <213> Artificial Sequence
<220>
   <223> Primer
<400> 135
   gttatctacc ttaagatcct gt 22
<210> 136
   <211> 176
   <212> PRT
   <213> Escherichia coli
<400> 136
   Met Ser Leu Leu Asn Val Pro Ala Gly Lys Asp Leu Pro Glu Asp Ile
   1               5                   10                  15
   Tyr Val Val Ile Glu Ile Pro Ala Asn Ala Asp Pro Ile Lys Tyr Glu
               20                  25                  30
   Ile Asp Lys Glu Ser Gly Ala Leu Phe Val Asp Arg Phe Met Ser Thr
           35                  40                  45
   Ala Met Phe Tyr Pro Cys Asn Tyr Gly Tyr Ile Asn His Thr Leu Ser
       50                  55                  60
   Leu Asp Gly Asp Pro Val Asp Val Leu Val Pro Thr Pro Tyr Pro Leu
   65                  70                  75                  80
   Gln Pro Gly Ser Val Ile Arg Cys Arg Pro Val Gly Val Leu Lys Met
                   85                  90                  95
   Thr Asp Glu Ala Gly Glu Asp Ala Lys Leu Val Ala Val Pro His Ser
               100                 105                 110
   Lys Leu Ser Lys Glu Tyr Asp His Ile Lys Asp Val Asn Asp Leu Pro
           115                 120                 125
   Glu Leu Leu Lys Ala Gln Ile Ala His Phe Phe Glu His Tyr Lys Asp
       130                 135                 140
   Leu Glu Lys Gly Lys Trp Val Lys Val Glu Gly Trp Glu Asn Ala Glu
   145                 150                 155                 160
   Ala Ala Lys Ala Glu Ile Val Ala Ser Phe Glu Arg Ala Lys Asn Lys
                   165                 170                 175
<210> 137
   <211> 199
   <212> PRT
   <213> Pantoea ananatis
<400> 137
   Met Asn Leu Val Lys Pro Leu Phe Ala Cys Leu Phe Phe Ile Thr Ala
   1               5                   10                  15
   Ser Ala Ala Val Gln Ala Glu Asn Ile Leu Ser Phe Pro Gln Pro Asp
               20                  25                  30
   Ser Leu Pro Asp Glu Phe Tyr Ala Val Thr Glu Ile Pro Ala Gly Gly
           35                  40                  45
   Ile Ile Lys Tyr Glu Thr Asp Ala Lys Thr Gly Phe Ile Val Ala Asp
       50                  55                  60
   Arg Phe Gln Ser Met Pro Val Ala Tyr Pro Ala Asn Tyr Gly Ser Leu
   65                  70                  75                  80
   Thr Gln Ser Leu Gly Gly Asp Asn Asp Pro Leu Asp Val Ile Phe Tyr
                   85                  90                  95
   Thr Arg Ala Pro Leu Ala Pro Gly Thr Leu Ile Lys Leu Arg Pro Ile
   100 105 110
   Gly Val Leu Lys Met Ile Asp Gly Gly Glu Val Asp Asp Lys Ile Val
   115 120 125
   Ala Val Pro Ala Ser Lys Val Asp Pro Thr Tyr Asp Ala Ile Gln Thr
   130 135 140
   Val Ala Asp Leu Pro Lys Leu Glu Gln Gln Arg Leu Glu Ala Phe Phe
   145 150 155 160
   Arg Val Tyr Lys Ala Leu Pro Glu Gly Arg Lys Asn Val Glu Leu Lys
   165 170 175
   Gly Phe Glu Asp Ala Ser Ala Ala Lys Ala Glu Ile Lys Gln Ala Phe
   180 185 190
   Glu Ala Trp Lys Ala Lys Gln
   195
<210> 138
   <211> 176
   <212> PRT
   <213> Pantoea ananatis
<400> 138
   Met Ser Leu Asn Gln Val Pro Ala Gly Lys Asp Leu Pro Glu Asp Ile
   1 5 10 15
   Tyr Val Ile Ile Glu Ile Pro Ala Asn Ala Asp Pro Ile Lys Tyr Glu
   20 25 30
   Val Asp Lys Asp Ser Gly Ala Leu Phe Val Asp Arg Phe Met Ser Thr
   35 40 45
   Ala Met Phe Tyr Pro Cys Asn Tyr Gly Tyr Ile Asn His Thr Leu Ser
   50 55 60
   Leu Asp Gly Asp Pro Val Asp Val Leu Val Pro Thr Gln Tyr Pro Leu
   65 70 75 80
   Glu Pro Gly Ser Val Ile Arg Cys Arg Pro Val Gly Val Leu Lys Met
   85 90 95
   Thr Asp Glu Ser Gly Glu Asp Ala Lys Leu Val Ala Val Pro His Thr
   100 105 110
   Lys Leu Thr Lys Glu Tyr Asp His Ile Lys Asp Val Asn Asp Leu Pro
   115 120 125
   Glu Leu Leu Arg Ala Gln Ile Thr His Phe Phe Glu His Tyr Lys Asp
   130 135 140
   Leu Glu Lys Gly Lys Trp Val Lys Val Asp Gly Trp Asp Asn Ala Glu
   145 150 155 160
   Ala Ala Lys Ala Glu Ile Ile Ser Ser Phe Glu Arg Ala Ala Lys Lys
   165 170 175
<210> 139
   <211> 2661
   <212> DNA
   <213> Artificial Sequence
<220>
   <223> EFMvaE gene
<400> 139
   ttgacaatta atcatccggc tcgtataatg tgtggaattg tgagcggata acaatttcac 60
   acaggaaaca gcgccgctga gaaaaagcga agcggcactg ctctttaaca atttatcaga 120
   caatctgtgt gggcactcga ccggaattat cgattaactt tattattaaa aattaaagag 180
   gtatatatta atgtatcgat taaataagga ggaataaacc atggatccga gctcaggagg 240
   taaaaaaaca tgaaaaccgt ggttattatc gatgcgctgc gcacgccgat tggtaaatat 300
   aaaggcagcc tgtctcaagt gagcgccgtt gatctgggta cgcatgtgac cacgcagctg 360
   ctgaaacgtc acagcaccat ctctgaagaa attgatcagg tgatctttgg taacgttctg 420
   caagccggta atggtcagaa tccggcacgt cagattgcaa tcaacagtgg cctgagccat 480
   gaaattccgg cgatgaccgt gaatgaagtt tgcggtagcg gcatgaaagc ggttattctg 540
   gccaaacagc tgatccagct gggtgaagcg gaagtgctga ttgccggcgg tatcgaaaac 600
   atgagtcagg caccgaaact gcaacgtttt aattatgaaa ccgaaagcta cgatgccccg 660
   ttcagctcta tgatgtatga tggcctgacc gatgcattta gcggtcaggc gatgggcctg 720
   acggcagaaa acgtggcgga aaaataccat gttacccgcg aagaacagga tcagttttct 780
   gttcacagtc agctgaaagc ggcccaggcc caggcagaag gtattttcgc cgatgaaatc 840
   gcaccgctgg aagtgtctgg tacgctggtt gaaaaagatg aaggcattcg tccgaatagt 900
   agcgtggaaa aactgggcac cctgaaaacg gtgttcaaag aagatggcac cgttacggcg 960
   ggcaatgcaa gcaccatcaa tgatggtgcg agtgccctga ttatcgcgag ccaggaatat 1020
   gcagaagcgc atggcctgcc gtacctggcc attatccgcg attctgtgga agttggtatt 1080
   gatccggcat atatgggcat tagtccgatc aaagcgattc agaaactgct ggcccgtaac 1140
   cagctgacca ccgaagaaat tgatctgtac gaaatcaatg aagcgtttgc agcgaccagt 1200
   attgtggttc agcgcgaact ggccctgccg gaagaaaaag ttaacattta tggcggtggc 1260
   atcagcctgg gtcacgcaat tggtgccacc ggtgcacgtc tgctgaccag tctgagctat 1320
   cagctgaatc agaaagagaa aaaatacggt gtggcaagcc tgtgtattgg tggcggtctg 1380
   ggtctggcca tgctgctgga acgtccgcag cagaagaaaa actctcgttt ttaccagatg 1440
   agtccggaag aacgtctggc cagtctgctg aacgaaggcc agattagcgc agataccaaa 1500
   aaagaattcg aaaatacggc actgtctagt cagatcgcga accatatgat tgaaaatcag 1560
   atcagcgaaa ccgaagtgcc gatgggtgtt ggcctgcacc tgaccgtgga tgaaacggat 1620
   tatctggttc cgatggcgac ggaagaaccg agcgttattg ccgcactgtc taacggtgca 1680
   aaaatcgcgc agggctttaa aaccgtgaat cagcagcgtc tgatgcgcgg ccagattgtg 1740
   ttctacgatg ttgcggatcc ggaaagcctg atcgataaac tgcaagtgcg cgaagccgaa 1800
   gtttttcagc aggcagaact gagctatccg tctattgtga aacgtggcgg tggcctgcgc 1860
   gatctgcaat accgtacctt tgatgaaagt ttcgtgagcg ttgatttcct ggtggatgtt 1920
   aaagatgcca tgggtgcaaa catcgtgaat gcgatgctgg aaggcgttgc cgaactgttt 1980
   cgtgaatggt tcgcggaaca gaaaatcctg ttttctatcc tgagtaacta cgcgaccgaa 2040
   agcgtggtta ccatgaaaac ggccattcct gtgagccgcc tgtctaaagg tagtaatggc 2100
   cgtgaaattg cggaaaaaat cgttctggcg agccgctatg cctctctgga tccgtaccgt 2160
   gccgtgaccc ataacaaagg tattatgaat ggcatcgaag cagtggttct ggcgaccggt 2220
   aacgataccc gtgccgtgtc tgcaagttgc catgcattcg cagttaaaga aggtcgttat 2280
   cagggcctga ccagctggac gctggatggt gaacagctga tcggcgaaat ttctgtgccg 2340
   ctggccctgg caaccgtggg tggcgcgacg aaagttctgc cgaaaagcca ggcggccgca 2400
   gatctgctgg cggtgaccga tgcaaaagaa ctgtctcgcg tggttgcggc cgttggtctg 2460
   gcacagaatc tggcagcgct gcgtgcgctg gtgtctgaag gtattcagaa aggccacatg 2520
   gcactgcaag cccgtagtct ggccatgacc gtgggtgcaa cgggcaaaga agtggaagca 2580
   gttgcgcagc agctgaaacg ccagaaaacc atgaaccagg atcgtgccat ggcaatcctg 2640
   aatgatctgc gcaaacagta a 2661
<210> 140
   <211> 1401
   <212> DNA
   <213> Artificial Sequence
<220>
   <223> EFMvaS gene
<400> 140
   ttgacaatta atcatccggc tcgtataatg tgtggaattg tgagcggata acaatttcac      60
   acaggaaaca gcgccgctga gaaaaagcga agcggcactg ctctttaaca atttatcaga     120
   caatctgtgt gggcactcga ccggaattat cgattaactt tattattaaa aattaaagag     180
   gtatatatta atgtatcgat taaataagga ggaataaacc atggatccga gctcaggagg     240
   taaaaaaaca tgaccattgg tatcgataaa attagctttt tcgtgccgcc gtattacatc     300
   gatatgacgg cgctggccga agcacgtaac gttgatccgg gcaaatttca tattggcatc     360
   ggtcaggatc agatggcggt gaacccgatt tctcaggata tcgttacctt cgcggccaat     420
   gcagcggaag caattctgac gaaagaagat aaagaagcga ttgatatggt gatcgttggc     480
   accgaaagct ctatcgatga aagtaaagcc gcagcggtgg ttctgcaccg tctgatgggc     540
   attcagccgt ttgcgcgcag cttcgaaatc aaagaagcct gctatggcgc gaccgccggt     600
   ctgcaactgg ccaaaaacca tgtggcactg cacccggata aaaaagttct ggtggttgcc     660
   gcagatattg cgaaatacgg tctgaatagc ggcggtgaac cgacccaggg tgcaggtgcc     720
   gtggcaatgc tggttgcatc tgaaccgcgt attctggcgc tgaaagaaga taacgtgatg     780
   ctgacccagg atatctatga tttttggcgt ccgaccggtc atccgtaccc gatggtggat     840
   ggcccgctga gtaatgaaac ctatattcag agcttcgccc aggtttggga tgaacataaa     900
   aaacgtacgg gtctggattt tgcggattat gatgcactgg cgttccacat tccgtacacc     960
   aaaatgggca aaaaagcgct gctggccaaa atcagcgatc agacggaagc cgaacaggaa    1020
   cgtattctgg cacgctatga agaaagcatc gtgtactctc gtcgcgttgg caacctgtat    1080
   accggttctc tgtacctggg cctgattagt ctgctggaaa acgcgaccac gctgacggcc    1140
   ggcaatcaga tcggtctgtt ttcttatggc agtggtgccg tggcagaatt tttcaccggt    1200
   gaactggttg ccggctacca gaaccatctg caaaaagaaa cccacctggc cctgctggat    1260
   aatcgcacgg aactgtctat tgcagaatat gaagcaatgt ttgcggaaac cctggatacg    1320
   gatatcgatc agaccctgga agatgaactg aaatatagta ttagcgcgat caacaatacg    1380
   gtgcgtagtt accgcaatta a                                               1401
<210> 141
   <211> 5314
   <212> DNA
   <213> Artificial Sequence
<220>
   <223> pUC57-EFmvaE
<400> 141
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt accttgacaa 420
   ttaatcatcc ggctcgtata atgtgtggaa ttgtgagcgg ataacaattt cacacaggaa 480
   acagcgccgc tgagaaaaag cgaagcggca ctgctcttta acaatttatc agacaatctg 540
   tgtgggcact cgaccggaat tatcgattaa ctttattatt aaaaattaaa gaggtatata 600
   ttaatgtatc gattaaataa ggaggaataa accatggatc cgagctcagg aggtaaaaaa 660
   acatgaaaac cgtggttatt atcgatgcgc tgcgcacgcc gattggtaaa tataaaggca 720
   gcctgtctca agtgagcgcc gttgatctgg gtacgcatgt gaccacgcag ctgctgaaac 780
   gtcacagcac catctctgaa gaaattgatc aggtgatctt tggtaacgtt ctgcaagccg 840
   gtaatggtca gaatccggca cgtcagattg caatcaacag tggcctgagc catgaaattc 900
   cggcgatgac cgtgaatgaa gtttgcggta gcggcatgaa agcggttatt ctggccaaac 960
   agctgatcca gctgggtgaa gcggaagtgc tgattgccgg cggtatcgaa aacatgagtc 1020
   aggcaccgaa actgcaacgt tttaattatg aaaccgaaag ctacgatgcc ccgttcagct 1080
   ctatgatgta tgatggcctg accgatgcat ttagcggtca ggcgatgggc ctgacggcag 1140
   aaaacgtggc ggaaaaatac catgttaccc gcgaagaaca ggatcagttt tctgttcaca 1200
   gtcagctgaa agcggcccag gcccaggcag aaggtatttt cgccgatgaa atcgcaccgc 1260
   tggaagtgtc tggtacgctg gttgaaaaag atgaaggcat tcgtccgaat agtagcgtgg 1320
   aaaaactggg caccctgaaa acggtgttca aagaagatgg caccgttacg gcgggcaatg 1380
   caagcaccat caatgatggt gcgagtgccc tgattatcgc gagccaggaa tatgcagaag 1440
   cgcatggcct gccgtacctg gccattatcc gcgattctgt ggaagttggt attgatccgg 1500
   catatatggg cattagtccg atcaaagcga ttcagaaact gctggcccgt aaccagctga 1560
   ccaccgaaga aattgatctg tacgaaatca atgaagcgtt tgcagcgacc agtattgtgg 1620
   ttcagcgcga actggccctg ccggaagaaa aagttaacat ttatggcggt ggcatcagcc 1680
   tgggtcacgc aattggtgcc accggtgcac gtctgctgac cagtctgagc tatcagctga 1740
   atcagaaaga gaaaaaatac ggtgtggcaa gcctgtgtat tggtggcggt ctgggtctgg 1800
   ccatgctgct ggaacgtccg cagcagaaga aaaactctcg tttttaccag atgagtccgg 1860
   aagaacgtct ggccagtctg ctgaacgaag gccagattag cgcagatacc aaaaaagaat 1920
   tcgaaaatac ggcactgtct agtcagatcg cgaaccatat gattgaaaat cagatcagcg 1980
   aaaccgaagt gccgatgggt gttggcctgc acctgaccgt ggatgaaacg gattatctgg 2040
   ttccgatggc gacggaagaa ccgagcgtta ttgccgcact gtctaacggt gcaaaaatcg 2100
   cgcagggctt taaaaccgtg aatcagcagc gtctgatgcg cggccagatt gtgttctacg 2160
   atgttgcgga tccggaaagc ctgatcgata aactgcaagt gcgcgaagcc gaagtttttc 2220
   agcaggcaga actgagctat ccgtctattg tgaaacgtgg cggtggcctg cgcgatctgc 2280
   aataccgtac ctttgatgaa agtttcgtga gcgttgattt cctggtggat gttaaagatg 2340
   ccatgggtgc aaacatcgtg aatgcgatgc tggaaggcgt tgccgaactg tttcgtgaat 2400
   ggttcgcgga acagaaaatc ctgttttcta tcctgagtaa ctacgcgacc gaaagcgtgg 2460
   ttaccatgaa aacggccatt cctgtgagcc gcctgtctaa aggtagtaat ggccgtgaaa 2520
   ttgcggaaaa aatcgttctg gcgagccgct atgcctctct ggatccgtac cgtgccgtga 2580
   cccataacaa aggtattatg aatggcatcg aagcagtggt tctggcgacc ggtaacgata 2640
   cccgtgccgt gtctgcaagt tgccatgcat tcgcagttaa agaaggtcgt tatcagggcc 2700
   tgaccagctg gacgctggat ggtgaacagc tgatcggcga aatttctgtg ccgctggccc 2760
   tggcaaccgt gggtggcgcg acgaaagttc tgccgaaaag ccaggcggcc gcagatctgc 2820
   tggcggtgac cgatgcaaaa gaactgtctc gcgtggttgc ggccgttggt ctggcacaga 2880
   atctggcagc gctgcgtgcg ctggtgtctg aaggtattca gaaaggccac atggcactgc 2940
   aagcccgtag tctggccatg accgtgggtg caacgggcaa agaagtggaa gcagttgcgc 3000
   agcagctgaa acgccagaaa accatgaacc aggatcgtgc catggcaatc ctgaatgatc 3060
   tgcgcaaaca gtaaaagctt ggcgtaatca tggtcatagc tgtttcctgt gtgaaattgt 3120
   tatccgctca caattccaca caacatacga gccggaagca taaagtgtaa agcctggggt 3180
   gcctaatgag tgagctaact cacattaatt gcgttgcgct cactgcccgc tttccagtcg 3240
   ggaaacctgt cgtgccagct gcattaatga atcggccaac gcgcggggag aggcggtttg 3300
   cgtattgggc gctcttccgc ttcctcgctc actgactcgc tgcgctcggt cgttcggctg 3360
   cggcgagcgg tatcagctca ctcaaaggcg gtaatacggt tatccacaga atcaggggat 3420
   aacgcaggaa agaacatgtg agcaaaaggc cagcaaaagg ccaggaaccg taaaaaggcc 3480
   gcgttgctgg cgtttttcca taggctccgc ccccctgacg agcatcacaa aaatcgacgc 3540
   tcaagtcaga ggtggcgaaa cccgacagga ctataaagat accaggcgtt tccccctgga 3600
   agctccctcg tgcgctctcc tgttccgacc ctgccgctta ccggatacct gtccgccttt 3660
   ctcccttcgg gaagcgtggc gctttctcat agctcacgct gtaggtatct cagttcggtg 3720
   taggtcgttc gctccaagct gggctgtgtg cacgaacccc ccgttcagcc cgaccgctgc 3780
   gccttatccg gtaactatcg tcttgagtcc aacccggtaa gacacgactt atcgccactg 3840
   gcagcagcca ctggtaacag gattagcaga gcgaggtatg taggcggtgc tacagagttc 3900
   ttgaagtggt ggcctaacta cggctacact agaagaacag tatttggtat ctgcgctctg 3960
   ctgaagccag ttaccttcgg aaaaagagtt ggtagctctt gatccggcaa acaaaccacc 4020
   gctggtagcg gtggtttttt tgtttgcaag cagcagatta cgcgcagaaa aaaaggatct 4080
   caagaagatc ctttgatctt ttctacgggg tctgacgctc agtggaacga aaactcacgt 4140
   taagggattt tggtcatgag attatcaaaa aggatcttca cctagatcct tttaaattaa 4200
   aaatgaagtt ttaaatcaat ctaaagtata tatgagtaaa cttggtctga cagttaccaa 4260
   tgcttaatca gtgaggcacc tatctcagcg atctgtctat ttcgttcatc catagttgcc 4320
   tgactccccg tcgtgtagat aactacgata cgggagggct taccatctgg ccccagtgct 4380
   gcaatgatac cgcgagaccc acgctcaccg gctccagatt tatcagcaat aaaccagcca 4440
   gccggaaggg ccgagcgcag aagtggtcct gcaactttat ccgcctccat ccagtctatt 4500
   aattgttgcc gggaagctag agtaagtagt tcgccagtta atagtttgcg caacgttgtt 4560
   gccattgcta caggcatcgt ggtgtcacgc tcgtcgtttg gtatggcttc attcagctcc 4620
   ggttcccaac gatcaaggcg agttacatga tcccccatgt tgtgcaaaaa agcggttagc 4680
   tccttcggtc ctccgatcgt tgtcagaagt aagttggccg cagtgttatc actcatggtt 4740
   atggcagcac tgcataattc tcttactgtc atgccatccg taagatgctt ttctgtgact 4800
   ggtgagtact caaccaagtc attctgagaa tagtgtatgc ggcgaccgag ttgctcttgc 4860
   ccggcgtcaa tacgggataa taccgcgcca catagcagaa ctttaaaagt gctcatcatt 4920
   ggaaaacgtt cttcggggcg aaaactctca aggatcttac cgctgttgag atccagttcg 4980
   atgtaaccca ctcgtgcacc caactgatct tcagcatctt ttactttcac cagcgtttct 5040
   gggtgagcaa aaacaggaag gcaaaatgcc gcaaaaaagg gaataagggc gacacggaaa 5100
   tgttgaatac tcatactctt cctttttcaa tattattgaa gcatttatca gggttattgt 5160
   ctcatgagcg gatacatatt tgaatgtatt tagaaaaata aacaaatagg ggttccgcgc 5220
   acatttcccc gaaaagtgcc acctgacgtc taagaaacca ttattatcat gacattaacc 5280
   tataaaaata ggcgtatcac gaggcccttt cgtc 5314
<210> 142
   <211> 4054
   <212> DNA
   <213> Artificial Sequence
<220>
   <223> pUC57-EFmvaS
<400> 142
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt accttgacaa 420
   ttaatcatcc ggctcgtata atgtgtggaa ttgtgagcgg ataacaattt cacacaggaa 480
   acagcgccgc tgagaaaaag cgaagcggca ctgctcttta acaatttatc agacaatctg 540
   tgtgggcact cgaccggaat tatcgattaa ctttattatt aaaaattaaa gaggtatata 600
   ttaatgtatc gattaaataa ggaggaataa accatggatc cgagctcagg aggtaaaaaa 660
   acatgaccat tggtatcgat aaaattagct ttttcgtgcc gccgtattac atcgatatga 720
   cggcgctggc cgaagcacgt aacgttgatc cgggcaaatt tcatattggc atcggtcagg 780
   atcagatggc ggtgaacccg atttctcagg atatcgttac cttcgcggcc aatgcagcgg 840
   aagcaattct gacgaaagaa gataaagaag cgattgatat ggtgatcgtt ggcaccgaaa 900
   gctctatcga tgaaagtaaa gccgcagcgg tggttctgca ccgtctgatg ggcattcagc 960
   cgtttgcgcg cagcttcgaa atcaaagaag cctgctatgg cgcgaccgcc ggtctgcaac 1020
   tggccaaaaa ccatgtggca ctgcacccgg ataaaaaagt tctggtggtt gccgcagata 1080
   ttgcgaaata cggtctgaat agcggcggtg aaccgaccca gggtgcaggt gccgtggcaa 1140
   tgctggttgc atctgaaccg cgtattctgg cgctgaaaga agataacgtg atgctgaccc 1200
   aggatatcta tgatttttgg cgtccgaccg gtcatccgta cccgatggtg gatggcccgc 1260
   tgagtaatga aacctatatt cagagcttcg cccaggtttg ggatgaacat aaaaaacgta 1320
   cgggtctgga ttttgcggat tatgatgcac tggcgttcca cattccgtac accaaaatgg 1380
   gcaaaaaagc gctgctggcc aaaatcagcg atcagacgga agccgaacag gaacgtattc 1440
   tggcacgcta tgaagaaagc atcgtgtact ctcgtcgcgt tggcaacctg tataccggtt 1500
   ctctgtacct gggcctgatt agtctgctgg aaaacgcgac cacgctgacg gccggcaatc 1560
   agatcggtct gttttcttat ggcagtggtg ccgtggcaga atttttcacc ggtgaactgg 1620
   ttgccggcta ccagaaccat ctgcaaaaag aaacccacct ggccctgctg gataatcgca 1680
   cggaactgtc tattgcagaa tatgaagcaa tgtttgcgga aaccctggat acggatatcg 1740
   atcagaccct ggaagatgaa ctgaaatata gtattagcgc gatcaacaat acggtgcgta 1800
   gttaccgcaa ttaaaagctt ggcgtaatca tggtcatagc tgtttcctgt gtgaaattgt 1860
   tatccgctca caattccaca caacatacga gccggaagca taaagtgtaa agcctggggt 1920
   gcctaatgag tgagctaact cacattaatt gcgttgcgct cactgcccgc tttccagtcg 1980
   ggaaacctgt cgtgccagct gcattaatga atcggccaac gcgcggggag aggcggtttg 2040
   cgtattgggc gctcttccgc ttcctcgctc actgactcgc tgcgctcggt cgttcggctg 2100
   cggcgagcgg tatcagctca ctcaaaggcg gtaatacggt tatccacaga atcaggggat 2160
   aacgcaggaa agaacatgtg agcaaaaggc cagcaaaagg ccaggaaccg taaaaaggcc 2220
   gcgttgctgg cgtttttcca taggctccgc ccccctgacg agcatcacaa aaatcgacgc 2280
   tcaagtcaga ggtggcgaaa cccgacagga ctataaagat accaggcgtt tccccctgga 2340
   agctccctcg tgcgctctcc tgttccgacc ctgccgctta ccggatacct gtccgccttt 2400
   ctcccttcgg gaagcgtggc gctttctcat agctcacgct gtaggtatct cagttcggtg 2460
   taggtcgttc gctccaagct gggctgtgtg cacgaacccc ccgttcagcc cgaccgctgc 2520
   gccttatccg gtaactatcg tcttgagtcc aacccggtaa gacacgactt atcgccactg 2580
   gcagcagcca ctggtaacag gattagcaga gcgaggtatg taggcggtgc tacagagttc 2640
   ttgaagtggt ggcctaacta cggctacact agaagaacag tatttggtat ctgcgctctg 2700
   ctgaagccag ttaccttcgg aaaaagagtt ggtagctctt gatccggcaa acaaaccacc 2760
   gctggtagcg gtggtttttt tgtttgcaag cagcagatta cgcgcagaaa aaaaggatct 2820
   caagaagatc ctttgatctt ttctacgggg tctgacgctc agtggaacga aaactcacgt 2880
   taagggattt tggtcatgag attatcaaaa aggatcttca cctagatcct tttaaattaa 2940
   aaatgaagtt ttaaatcaat ctaaagtata tatgagtaaa cttggtctga cagttaccaa 3000
   tgcttaatca gtgaggcacc tatctcagcg atctgtctat ttcgttcatc catagttgcc 3060
   tgactccccg tcgtgtagat aactacgata cgggagggct taccatctgg ccccagtgct 3120
   gcaatgatac cgcgagaccc acgctcaccg gctccagatt tatcagcaat aaaccagcca 3180
   gccggaaggg ccgagcgcag aagtggtcct gcaactttat ccgcctccat ccagtctatt 3240
   aattgttgcc gggaagctag agtaagtagt tcgccagtta atagtttgcg caacgttgtt 3300
   gccattgcta caggcatcgt ggtgtcacgc tcgtcgtttg gtatggcttc attcagctcc 3360
   ggttcccaac gatcaaggcg agttacatga tcccccatgt tgtgcaaaaa agcggttagc 3420
   tccttcggtc ctccgatcgt tgtcagaagt aagttggccg cagtgttatc actcatggtt 3480
   atggcagcac tgcataattc tcttactgtc atgccatccg taagatgctt ttctgtgact 3540
   ggtgagtact caaccaagtc attctgagaa tagtgtatgc ggcgaccgag ttgctcttgc 3600
   ccggcgtcaa tacgggataa taccgcgcca catagcagaa ctttaaaagt gctcatcatt 3660
   ggaaaacgtt cttcggggcg aaaactctca aggatcttac cgctgttgag atccagttcg 3720
   atgtaaccca ctcgtgcacc caactgatct tcagcatctt ttactttcac cagcgtttct 3780
   gggtgagcaa aaacaggaag gcaaaatgcc gcaaaaaagg gaataagggc gacacggaaa 3840
   tgttgaatac tcatactctt cctttttcaa tattattgaa gcatttatca gggttattgt 3900
   ctcatgagcg gatacatatt tgaatgtatt tagaaaaata aacaaatagg ggttccgcgc 3960
   acatttcccc gaaaagtgcc acctgacgtc taagaaacca ttattatcat gacattaacc 4020
   tataaaaata ggcgtatcac gaggcccttt cgtc 4054
<210> 143
   <211> 38
   <212> DNA
   <213> Artificial Sequence
<220>
   <223> Primer to amplify trc promoter (Ptrc)
<400> 143
   gaattcgagc tcggtaccct tgacaattaa tcatccgg 38
<210> 144
   <211> 45
   <212> DNA
   <213> Artificial Sequence
<220>
   <223> Primer to amplify trc promoter (Ptrc)
<400> 144
   aaccacggtt ttcatgtttt tttacctcct gagctcggat ccatg 45
<210> 145
   <211> 45
   <212> DNA
   <213> Artificial Sequence
<220>
   <223> Primer to amplify mvaE
<400> 145
   catggatccg agctcaggag gtaaaaaaac atgaaaaccg tggtt 45
<210> 146
   <211> 55
   <212> DNA
   <213> Artificial Sequence
<220>
   <223> Primer to amplify mvaE, mvaS
<400> 146
   ttatcgatac caatggtcat gtttttttac ctcctttact gtttgcgcag atcat 55
<210> 147
   <211> 55
   <212> DNA
   <213> Artificial Sequence
<220>
   <223> Primer to amplify mvaS
<400> 147
   atgatctgcg caaacagtaa aggaggtaaa aaaacatgac cattggtatc gataa 55
<210> 148
   <211> 40
   <212> DNA
   <213> Artificial Sequence
<220>
   <223> Primer to amplify tryptophane terminator (Ttrp)
<400> 148
   cagcggaact ggcggctccc ttaattgcgg taactacgca 40
<210> 149
   <211> 40
   <212> DNA
   <213> Artificial Sequence
<220>
   <223> Primer to amplify tryptophane terminator (Ttrp)
<400> 149
   tgcgtagtta ccgcaattaa gggagccgcc agttccgctg 40
<210> 150
   <211> 38
   <212> DNA
   <213> Artificial Sequence
<220>
   <223> Primer
<400> 150
   gtcgactcta gaggatccct aatgagaatt agtcaaat 38
<210> 151
   <211> 8053
   <212> DNA
   <213> Artificial Sequence
<220>
   <223> pMW-Ptrc-mvaES-Ttrp
<400> 151
   gacagtaaga cgggtaagcc tgttgatgat accgctgcct tactgggtgc attagccagt 60
   ctgaatgacc tgtcacggga taatccgaag tggtcagact ggaaaatcag agggcaggaa 120
   ctgcagaaca gcaaaaagtc agatagcacc acatagcaga cccgccataa aacgccctga 180
   gaagcccgtg acgggctttt cttgtattat gggtagtttc cttgcatgaa tccataaaag 240
   gcgcctgtag tgccatttac ccccattcac tgccagagcc gtgagcgcag cgaactgaat 300
   gtcacgaaaa agacagcgac tcaggtgcct gatggtcgga gacaaaagga atattcagcg 360
   atttgcccga gcttgcgagg gtgctactta agcctttagg gttttaaggt ctgttttgta 420
   gaggagcaaa cagcgtttgc gacatccttt tgtaatactg cggaactgac taaagtagtg 480
   agttatacac agggctggga tctattcttt ttatcttttt ttattctttc tttattctat    540
   aaattataac cacttgaata taaacaaaaa aaacacacaa aggtctagcg gaatttacag    600
   agggtctagc agaatttaca agttttccag caaaggtcta gcagaattta cagataccca    660
   caactcaaag gaaaaggact agtaattatc attgactagc ccatctcaat tggtatagtg    720
   attaaaatca cctagaccaa ttgagatgta tgtctgaatt agttgttttc aaagcaaatg    780
   aactagcgat tagtcgctat gacttaacgg agcatgaaac caagctaatt ttatgctgtg    840
   tggcactact caaccccacg attgaaaacc ctacaaggaa agaacggacg gtatcgttca    900
   cttataacca atacgctcag atgatgaaca tcagtaggga aaatgcttat ggtgtattag    960
   ctaaagcaac cagagagctg atgacgagaa ctgtggaaat caggaatcct ttggttaaag   1020
   gctttgagat tttccagtgg acaaactatg ccaagttctc aagcgaaaaa ttagaattag   1080
   tttttagtga agagatattg ccttatcttt tccagttaaa aaaattcata aaatataatc   1140
   tggaacatgt taagtctttt gaaaacaaat actctatgag gatttatgag tggttattaa   1200
   aagaactaac acaaaagaaa actcacaagg caaatataga gattagcctt gatgaattta   1260
   agttcatgtt aatgcttgaa aataactacc atgagtttaa aaggcttaac caatgggttt   1320
   tgaaaccaat aagtaaagat ttaaacactt acagcaatat gaaattggtg gttgataagc   1380
   gaggccgccc gactgatacg ttgattttcc aagttgaact agatagacaa atggatctcg   1440
   taaccgaact tgagaacaac cagataaaaa tgaatggtga caaaatacca acaaccatta   1500
   catcagattc ctacctacat aacggactaa gaaaaacact acacgatgct ttaactgcaa   1560
   aaattcagct caccagtttt gaggcaaaat ttttgagtga catgcaaagt aagcatgatc   1620
   tcaatggttc gttctcatgg ctcacgcaaa aacaacgaac cacactagag aacatactgg   1680
   ctaaatacgg aaggatctga ggttcttatg gctcttgtat ctatcagtga agcatcaaga   1740
   ctaacaaaca aaagtagaac aactgttcac cgttacatat caaagggaaa actgtccata   1800
   tgcacagatg aaaacggtgt aaaaaagata gatacatcag agcttttacg agtttttggt   1860
   gcattcaaag ctgttcacca tgaacagatc gacaatgtaa cagatgaaca gcatgtaaca   1920
   cctaatagaa caggtgaaac cagtaaaaca aagcaactag aacatgaaat tgaacacctg   1980
   agacaacttg ttacagctca acagtcacac atagacagcc tgaaacaggc gatgctgctt   2040
   atcgaatcaa agctgccgac aacacgggag ccagtgacgc ctcccgtggg gaaaaaatca   2100
   tggcaattct ggaagaaata gcgccattcg ccattcaggc tgcgcaactg ttgggaaggg   2160
   cgatcggtgc gggcctcttc gctattacgc cagctggcga aggggggatg tgctgcaagg   2220
   cgattaagtt gggtaacgcc agggttttcc cagtcacgac gttgtaaaac gacggccagt   2280
   gaattcgagc tcggtaccct tgacaattaa tcatccggct cgtataatgt gtggaattgt   2340
   gagcggataa caatttcaca caggaaacag cgccgctgag aaaaagcgaa gcggcactgc 2400
   tctttaacaa tttatcagac aatctgtgtg ggcactcgac cggaattatc gattaacttt 2460
   attattaaaa attaaagagg tatatattaa tgtatcgatt aaataaggag gaataaacca 2520
   tggatccgag ctcaggaggt aaaaaaacat gaaaaccgtg gttattatcg atgcgctgcg 2580
   cacgccgatt ggtaaatata aaggcagcct gtctcaagtg agcgccgttg atctgggtac 2640
   gcatgtgacc acgcagctgc tgaaacgtca cagcaccatc tctgaagaaa ttgatcaggt 2700
   gatctttggt aacgttctgc aagccggtaa tggtcagaat ccggcacgtc agattgcaat 2760
   caacagtggc ctgagccatg aaattccggc gatgaccgtg aatgaagttt gcggtagcgg 2820
   catgaaagcg gttattctgg ccaaacagct gatccagctg ggtgaagcgg aagtgctgat 2880
   tgccggcggt atcgaaaaca tgagtcaggc accgaaactg caacgtttta attatgaaac 2940
   cgaaagctac gatgccccgt tcagctctat gatgtatgat ggcctgaccg atgcatttag 3000
   cggtcaggcg atgggcctga cggcagaaaa cgtggcggaa aaataccatg ttacccgcga 3060
   agaacaggat cagttttctg ttcacagtca gctgaaagcg gcccaggccc aggcagaagg 3120
   tattttcgcc gatgaaatcg caccgctgga agtgtctggt acgctggttg aaaaagatga 3180
   aggcattcgt ccgaatagta gcgtggaaaa actgggcacc ctgaaaacgg tgttcaaaga 3240
   agatggcacc gttacggcgg gcaatgcaag caccatcaat gatggtgcga gtgccctgat 3300
   tatcgcgagc caggaatatg cagaagcgca tggcctgccg tacctggcca ttatccgcga 3360
   ttctgtggaa gttggtattg atccggcata tatgggcatt agtccgatca aagcgattca 3420
   gaaactgctg gcccgtaacc agctgaccac cgaagaaatt gatctgtacg aaatcaatga 3480
   agcgtttgca gcgaccagta ttgtggttca gcgcgaactg gccctgccgg aagaaaaagt 3540
   taacatttat ggcggtggca tcagcctggg tcacgcaatt ggtgccaccg gtgcacgtct 3600
   gctgaccagt ctgagctatc agctgaatca gaaagagaaa aaatacggtg tggcaagcct 3660
   gtgtattggt ggcggtctgg gtctggccat gctgctggaa cgtccgcagc agaagaaaaa 3720
   ctctcgtttt taccagatga gtccggaaga acgtctggcc agtctgctga acgaaggcca 3780
   gattagcgca gataccaaaa aagaattcga aaatacggca ctgtctagtc agatcgcgaa 3840
   ccatatgatt gaaaatcaga tcagcgaaac cgaagtgccg atgggtgttg gcctgcacct 3900
   gaccgtggat gaaacggatt atctggttcc gatggcgacg gaagaaccga gcgttattgc 3960
   cgcactgtct aacggtgcaa aaatcgcgca gggctttaaa accgtgaatc agcagcgtct 4020
   gatgcgcggc cagattgtgt tctacgatgt tgcggatccg gaaagcctga tcgataaact 4080
   gcaagtgcgc gaagccgaag tttttcagca ggcagaactg agctatccgt ctattgtgaa 4140
   acgtggcggt ggcctgcgcg atctgcaata ccgtaccttt gatgaaagtt tcgtgagcgt 4200
   tgatttcctg gtggatgtta aagatgccat gggtgcaaac atcgtgaatg cgatgctgga 4260
   aggcgttgcc gaactgtttc gtgaatggtt cgcggaacag aaaatcctgt tttctatcct 4320
   gagtaactac gcgaccgaaa gcgtggttac catgaaaacg gccattcctg tgagccgcct 4380
   gtctaaaggt agtaatggcc gtgaaattgc ggaaaaaatc gttctggcga gccgctatgc 4440
   ctctctggat ccgtaccgtg ccgtgaccca taacaaaggt attatgaatg gcatcgaagc 4500
   agtggttctg gcgaccggta acgatacccg tgccgtgtct gcaagttgcc atgcattcgc 4560
   agttaaagaa ggtcgttatc agggcctgac cagctggacg ctggatggtg aacagctgat 4620
   cggcgaaatt tctgtgccgc tggccctggc aaccgtgggt ggcgcgacga aagttctgcc 4680
   gaaaagccag gcggccgcag atctgctggc ggtgaccgat gcaaaagaac tgtctcgcgt 4740
   ggttgcggcc gttggtctgg cacagaatct ggcagcgctg cgtgcgctgg tgtctgaagg 4800
   tattcagaaa ggccacatgg cactgcaagc ccgtagtctg gccatgaccg tgggtgcaac 4860
   gggcaaagaa gtggaagcag ttgcgcagca gctgaaacgc cagaaaacca tgaaccagga 4920
   tcgtgccatg gcaatcctga atgatctgcg caaacagtaa aggaggtaaa aaaacatgac 4980
   cattggtatc gataaaatta gctttttcgt gccgccgtat tacatcgata tgacggcgct 5040
   ggccgaagca cgtaacgttg atccgggcaa atttcatatt ggcatcggtc aggatcagat 5100
   ggcggtgaac ccgatttctc aggatatcgt taccttcgcg gccaatgcag cggaagcaat 5160
   tctgacgaaa gaagataaag aagcgattga tatggtgatc gttggcaccg aaagctctat 5220
   cgatgaaagt aaagccgcag cggtggttct gcaccgtctg atgggcattc agccgtttgc 5280
   gcgcagcttc gaaatcaaag aagcctgcta tggcgcgacc gccggtctgc aactggccaa 5340
   aaaccatgtg gcactgcacc cggataaaaa agttctggtg gttgccgcag atattgcgaa 5400
   atacggtctg aatagcggcg gtgaaccgac ccagggtgca ggtgccgtgg caatgctggt 5460
   tgcatctgaa ccgcgtattc tggcgctgaa agaagataac gtgatgctga cccaggatat 5520
   ctatgatttt tggcgtccga ccggtcatcc gtacccgatg gtggatggcc cgctgagtaa 5580
   tgaaacctat attcagagct tcgcccaggt ttgggatgaa cataaaaaac gtacgggtct 5640
   ggattttgcg gattatgatg cactggcgtt ccacattccg tacaccaaaa tgggcaaaaa 5700
   agcgctgctg gccaaaatca gcgatcagac ggaagccgaa caggaacgta ttctggcacg 5760
   ctatgaagaa agcatcgtgt actctcgtcg cgttggcaac ctgtataccg gttctctgta 5820
   cctgggcctg attagtctgc tggaaaacgc gaccacgctg acggccggca atcagatcgg 5880
   tctgttttct tatggcagtg gtgccgtggc agaatttttc accggtgaac tggttgccgg 5940
   ctaccagaac catctgcaaa aagaaaccca cctggccctg ctggataatc gcacggaact 6000
   gtctattgca gaatatgaag caatgtttgc ggaaaccctg gatacggata tcgatcagac 6060
   cctggaagat gaactgaaat atagtattag cgcgatcaac aatacggtgc gtagttaccg 6120
   caattaaggg agccgccagt tccgctggcg gcattttaac tttctttaat gaagccggaa 6180
   aaatcctaaa ttcatttaat atttatcttt ttaccgtttc gcttaccccg gtcgaacgtc 6240
   aacttacgtc atttttccgc ccaacagtaa tataatcaaa caaattaatc ccgcaacata 6300
   acaccagtaa aatcaataat tttctctaag tcacttattc ctcaggtaat tgttaatata 6360
   tccagaatgt tcctcaaaat atattttccc tctatcttct cgttgcgctt aatttgacta 6420
   attctcatta gggatcctct agagtcgacc tgcaggcatg caagcttggc gtaatcatgg 6480
   tcatagctgt ttcctgtgtg aaattgttat ccgctcacaa ttccacacaa catacgagcc 6540
   ggaagcataa agtgtaaagc ctggggtgcc taatgagtga gctaactcac attaattgcg 6600
   ttgcgctcac tgcccgcttt ccagtcggga aacctgtcgt gccagctgca ttaatgaatc 6660
   ggccaacgcg cggggagagg cggtttgcgt attgggcgct ttctcatagc tcacgctgta 6720
   ggtatctcag ttcggtgtag gtcgttcgct ccaagctggg ctgtgtgccg aaccccagag 6780
   tcccgctcag aagaactcgt caagaaggcg atagaaggcg atgcgctgcg aatcgggagc 6840
   ggcgataccg taaagcacga ggaagcggtc agcccattcg ccgccaagct cttcagcaat 6900
   atcacgggta gccaacgcta tgtcctgata gcggtccgcc acacccagcc ggccacagtc 6960
   gatgaatcca gaaaagcggc cattttccac catgatattc ggcaagcagg catcgccatg 7020
   ggtcacgacg agatcctcgc cgtcgggcat gcgcgccttg agcctggcga acagttcggc 7080
   tggcgcgagc ccctgatgct cttcgtccag atcatcctga tcgacaagac cggcttccat 7140
   ccgagtacgt gctcgctcga tgcgatgttt cgcttggtgg tcgaatgggc aggtagccgg 7200
   atcaagcgta tgcagccgcc gcattgcatc agccatgatg gatactttct cggcaggagc 7260
   aaggtgagat gacaggagat cctgccccgg cacttcgccc aatagcagcc agtcccttcc 7320
   cgcttcagtg ccaacgtcga gcacagctgc gcaaggaacg cccgtcgtgg ccagccacga 7380
   tagccgcgct gcctcgtcct gcagttcatt cagggcaccg gacaggtcgg tcttgacaaa 7440
   aagaaccggg cgcccctgcg ctgacagccg gaacacggcg gcatcagagc agccgattgt 7500
   ctgttgtgcc cagtcatagc cgaatagcct ctccacccaa gcggccggag aacctgcgtg 7560
   caatccatct tgttcaatca tgcgaaacga tcctcatcct gtctcttgat cactaccgca 7620
   ttaaagcata tcgatgataa gctgtcaaac atgagcgttc ttcggggcga aaactctcaa 7680
   ggatcttacc gctgttgaga tccagttcga tgtaacccac tcgtgcaccc aactgatctt 7740
   cagcatcttt tactttcacc agcgtttctg ggtgagcaaa aacaggaagg caaaatgccg 7800
   caaaaaaggg aataagggcg acacggaaat gttgaatact catactcttc ctttttcaat 7860
   attattgaag catttatcag ggttattgtc tcatgagcgg atacatattt gaatgtattt 7920
   agaaaaataa acaaataggg gttccgcgca catttccccg aaaagtgcca cctgacgtct 7980
   aagaaaccat tattatcatg acattaacct ataaaaatag gcgtatcacg aggccctttc 8040
   gtcttcaaga att 8053
<210> 152
   <211> 40
   <212> DNA
   <213> Artificial Sequence
<220>
   <223> Primer to amplify pMW-Ptrc-mvaES-Ttrp
<400> 152
   ctctaaggag gttataaaaa atgaaaaccg tggttattat 40
<210> 153
   <211> 40
   <212> DNA
   <213> Artificial Sequence
<220>
   <223> Primer to amplify pMW-Ptrc-mvaES-Ttrp
<400> 153
   aatattgaaa aaggaagagt gggtaccgag ctcgaattca 40
<210> 154
   <211> 40
   <212> DNA
   <213> Artificial Sequence
<220>
   <223> Primer to amplify pKD46
<400> 154
   tgaattcgag ctcggtaccc actcttcctt tttcaatatt 40
<210> 155
   <211> 40
   <212> DNA
   <213> Artificial Sequence
<220>
   <223> Primer to amplify pKD46
<400> 155
   ataataacca cggttttcat tttttataac ctccttagag 40
<210> 156
   <211> 9180
   <212> DNA
   <213> Artificial Sequence
<220>
   <223> pMW-Para-mvaES-Ttrp
<400> 156
   gacagtaaga cgggtaagcc tgttgatgat accgctgcct tactgggtgc attagccagt 60
<210> 157
   <211> 42
   <212> DNA
   <213> Artificial Sequence
<220>
   <223> Primer (Linker-F)
<400> 157
   agctttaggg ataacagggt aatctcgagc tgcaggcatg ca 42
<210> 158
   <211> 42
   <212> DNA
   <213> Artificial Sequence
<220>
   <223> Primer (Linker-R)
<400> 158
   agcttgcatg cctgcagctc gagattaccc tgttatccct aa 42
<210> 159
   <211> 54
   <212> DNA
   <213> Artificial Sequence
<220>
   <223> Primer (phoC5\201fCAS)
<400> 159
   tttttaagct ttagggataa cagggtaatc tcgagtggat aacctcatgt aaac 54
<210> 160
   <211> 44
   <212> DNA
   <213> Artificial Sequence
<220>
   <223> Primer (phoC3\201fCAS)
<400> 160
   tttttaagct tgcatgcctg cagttgatgt ctgattatct ctga 44
<210> 161
   <211> 33
   <212> DNA
   <213> Artificial Sequence
<220>
   <223> Primer (n67)
<400> 161
   tgcgaagacg tcctcgtgaa gaaggtgttg ctg 33
<210> 162
   <211> 36
   <212> DNA
   <213> Artificial Sequence
<220>
   <223> Primer (n68)
<400> 162
   tgcgaagggc cccgttgtgt ctcaaaatct ctgatg 36
<210> 163
   <211> 70
   <212> DNA
   <213> Artificial Sequence
<220>
   <223> Primer (ampH-attL-phi80)
<400> 163
   atgcgcactc cttacgtact ggctctactg gtttctttgc gaaaggtcat ttttcctgaa 60
   tatgctcaca 70
<210> 164
   <211> 64
   <212> DNA
   <213> Artificial Sequence
<220>
   <223> Primer (ampH-attR-phi80)
<400> 164
   ttaaggaatc gcctggacca tcatcggcga gccgttctga cgtttgttga cagctggtcc 60
   aatg 64
<210> 165
   <211> 68
   <212> DNA
   <213> Artificial Sequence
<220>
   <223> Primer (DampC-phL)
<400> 165
   ctgatgaact gtcacctgaa tgagtgctga tgaaaatata gaaaggtcat ttttcctgaa 60
   tatgctca 68
<210> 166
   <211> 64
   <212> DNA
   <213> Artificial Sequence
<220>
   <223> Primer (DampC-phR)
<400> 166
   attcgccagc ataacgatgc cgctgttgag ctgaggaaca cgtttgttga cagctggtcc 60
   aatg 64
<210> 167
   <211> 18
   <212> DNA
   <213> Artificial Sequence
<220>
   <223> Primer (ampH-t1)
<400> 167
   gcgaagccct ctccgttg 18
<210> 168
   <211> 21
   <212> DNA
   <213> Artificial Sequence
<220>
   <223> Primer (ampH-t2)
<400> 168
   agccagtcag cctcatcagc g 21
<210> 169
   <211> 21
   <212> DNA
   <213> Artificial Sequence
<220>
   <223> Primer (ampC-t1)
<400> 169
   gattcccact tcaccgagcc g 21
<210> 170
   <211> 21
   <212> DNA
   <213> Artificial Sequence
<220>
   <223> Primer (ampC-t2)
<400> 170
   ggcaggtatg gtgctctgac g 21
<210> 171
   <211> 64
   <212> DNA
   <213> Artificial Sequence
<220>
   <223> Primer (crtE-attRphi80)
<400> 171
   atgacggtct gcgcaaaaaa acacgttcat ctcactcgcg cgtttgttga cagctggtcc 60
   aatg 64
<210> 172
   <211> 68
   <212> DNA
   <213> Artificial Sequence
<220>
   <223> Primer (crtZ-attLphi80)
<400> 172
   atgttgtgga tttggaatgc cctgatcgtt ttcgttaccg gaaaggtcat ttttcctgaa 60
   tatgctca 68
<210> 173
   <211> 21
   <212> DNA
   <213> Artificial Sequence
<220>
   <223> Primer (crtZ-test)
<400> 173
   ccgtgtggtt ctgaaagccg a 21
<210> 174
   <211> 21
   <212> DNA
   <213> Artificial Sequence
<220>
   <223> Primer (crtE-test)
<400> 174
   cgttgccgta aatgtatccg t 21
<210> 175
   <211> 40
   <212> DNA
   <213> Artificial Sequence
<220>
   <223> Primer (pTac-MGppa-F)
<400> 175
   cacacaagga gactcccatg agcttactca acgtccctgc 40
<210> 176
   <211> 40
   <212> DNA
   <213> Artificial Sequence
<220>
   <223> Primer (Ttrp-MGppa-R)
<400> 176
   cggaactggc ggctccctta tttattcttt gcgcgctcga 40
<210> 177
   <211> 45
   <212> DNA
   <213> Artificial Sequence
<220>
   <223> Primer (Tac-CRIM-km-NF3)
<400> 177
   cgactctaga ggatcagatc tccctgttga caattaatca tcggc 45
<210> 178
   <211> 45
   <212> DNA
   <213> Artificial Sequence
<220>
   <223> Primer (Ttrp_CRIM_Km-CR2)
<400> 178
   tcaaacatga gaattccggc ttcattaaag aaagttaaaa tgccg 45
<210> 179
   <211> 80
   <212> DNA
   <213> Artificial Sequence
<220>
   <223> Primer (ydcI_phi80-F)
<400> 179
   atggaaaaaa atatcctttt caatcagcgc attcgcttgc gtcatttaca gaaaggtcat 60
   ttttcctgaa tatgctcaca 80
<210> 180
   <211> 80
   <212> DNA
   <213> Artificial Sequence
<220>
   <223> Primer (ydcI_phi80-R)
<400> 180
   ctgtgaaatt tacgaatagt actcatcaga acctcagcgc tcaggcttaa tcgtttgttg 60
   acagctggtc caatgccagc 80
<210> 181
   <211> 22
   <212> DNA
   <213> Artificial Sequence
<220>
   <223> Primer (ydcI_F)
<400> 181
   cccgataaaa agtacacatt gt 22
<210> 182
   <211> 22
   <212> DNA
   <213> Artificial Sequence
<220>
   <223> Primer (ydcI_R)
<400> 182
   agttatctgg atcgttggct ac 22
<210> 183
   <211> 22
   <212> DNA
   <213> Artificial Sequence
<220>
   <223> Primer (MG-ppa-seq-1)
<400> 183
   gccggtgaag atgcgaaact gg 22
<210> 184
   <211> 3471
   <212> DNA
   <213> Artificial Sequence
<220>
   <223> Sequence of the chemically synthesized DNA fragment retaining artificial KDyI operon with optimized codons
<400> 184
   gcatgcagga ggtatgaatg tcagagttgc gtgccttcag tgccccaggg aaagcgttac      60
   tcgctggtgg atatttagtt ttagatacaa aatatgaagc atttgtagtc ggattatcgg     120
   cacgtatgca cgctgtagcc catccttacg gttcattgca agggtctgat aagtttgaag     180
   tgcgtgtgaa aagtaaacaa tttaaagatg gggagtggct gtaccatata agtcctaaaa     240
   gtggcttcat tcctgtttcg ataggcggat ctaagaaccc tttcattgaa aaagttatcg     300
   ctaacgtatt tagctacttt aaacctaaca tggacgacta ctgcaatcgt aacttgttcg     360
   ttattgatat tttctctgat gatgcctacc attctcagga ggatagcgtt accgaacatc     420
   gtggcaaccg ccgtttgagt tttcattcgc accgtattga agaagttccc aaaacagggc     480
   tgggctcctc ggcaggttta gtcacagttt taactacagc tttggcctcc ttttttgtat     540
   cggacctgga aaataatgta gacaaatatc gtgaagttat tcataattta gcacaagttg     600
   ctcattgtca agctcagggt aaaattggaa gcgggtttga tgtagcggcg gcagcatatg     660
   gatctatccg ttatcgccgt ttcccacccg cattaatctc taatttgcca gatattggaa     720
   gtgctactta cggcagtaaa ctggcgcatt tggttgatga agaagactgg aatattacga     780
   ttaaaagtaa ccatttacct tcgggattaa ctttatggat gggcgatatt aagaatggtt     840
   cagaaacagt aaaactggtc cagaaggtaa aaaattggta tgattcgcat atgccagaaa     900
   gcctcaaaat atatacagaa ctcgatcatg caaattctcg ttttatggat ggactctcta     960
   aactcgatcg cttacacgag actcatgacg attacagcga tcagatattt gagtctcttg    1020
   agcgtaatga ctgtacctgt caaaagtatc ctgaaatcac agaagttcgt gatgcagttg    1080
   ccacaattcg tcgttccttt cgtaaaataa ctaaagaatc tggtgccgat atcgaacctc    1140
   ccgtacaaac tagcttattg gatgattgcc agaccttaaa aggagttctt acttgcttaa 1200
   tacctggtgc tggtggttat gacgccattg cagtgattac taagcaagat gttgatcttc 1260
   gtgctcaaac cgctaatgac aaacgttttt ctaaggttca atggctggat gtaactcagg 1320
   ctgactgggg tgttcgtaaa gaaaaagatc cggaaactta tcttgataaa taactgcaga 1380
   ggaggtatga atgaccgttt acacagcatc cgttaccgca cccgtcaaca tcgcaaccct 1440
   taagtattgg gggaaacgtg acacgaagtt gaatctgccc accaattcgt ccatatcagt 1500
   gactttatcg caagatgacc tccgtacgtt gacctctgcg gctactgcac ctgagtttga 1560
   acgcgacact ttgtggttaa atggagaacc acacagcatc gacaatgaac gtactcaaaa 1620
   ttgtctgcgc gacctccgcc aattacgtaa ggaaatggaa tcgaaggacg cctcattgcc 1680
   cacattatct caatggaaac tccacattgt ctccgaaaat aactttccta cagcagctgg 1740
   tttagcttcc tccgctgctg gctttgctgc attggtctct gcaattgcta agttatacca 1800
   attaccacag tcaacttcag aaatatctcg tatagcacgt aaggggtctg gttcagcttg 1860
   tcgttcgttg tttggcggat acgtggcctg ggaaatggga aaagctgaag atggtcatga 1920
   ttccatggca gtacaaatcg cagacagctc tgactggcct cagatgaaag catgtgtcct 1980
   tgttgtcagc gatattaaaa aggatgtgag ttccactcag ggtatgcaat tgaccgtggc 2040
   aacctccgaa ctctttaaag aacgtattga acatgtcgta ccaaagcgtt ttgaagtcat 2100
   gcgtaaagcc attgttgaaa aagatttcgc cacctttgca aaggaaacaa tgatggattc 2160
   caactctttc catgccacat gtttggactc tttccctcca atattctaca tgaatgacac 2220
   ttccaagcgt atcatcagtt ggtgccacac cattaatcag ttttacggag aaacaatcgt 2280
   tgcatacacg tttgatgcag gtccaaatgc tgtgttgtac tacttagctg aaaatgagtc 2340
   gaaactcttt gcatttatct ataaattgtt tggctctgtt cctggatggg acaagaaatt 2400
   tactactgag cagcttgagg ctttcaacca tcaatttgaa tcatctaact ttactgcacg 2460
   tgaattggat cttgagttgc aaaaggatgt tgcccgtgtg attttaactc aagtcggttc 2520
   aggcccacaa gaaacaaacg aatctttgat tgacgcaaag actggtctcc caaaggaata 2580
   aggatccagg aggtatgaat gactgccgac aacaatagta tgccccatgg tgcagtatct 2640
   agttacgcca aattagtgca aaaccaaaca cctgaagaca ttttggaaga gtttcctgaa 2700
   attattccat tacaacaacg tcctaatacc cgctctagtg agacgtcaaa tgacgaaagc 2760
   ggagaaacat gtttttctgg tcatgatgag gagcaaatta agttaatgaa tgaaaattgt 2820
   attgttttgg attgggacga taatgctatt ggtgccggca ccaagaaagt ttgtcattta 2880
   atggaaaata ttgaaaaggg tttacttcat cgtgcattct ccgtctttat tttcaatgaa 2940
   caaggtgaat tacttttaca acaacgtgcc actgaaaaaa taactttccc tgatctttgg 3000
   actaacacat gctgctctca tccactttgt attgatgacg aattaggttt gaagggtaag 3060
   ctcgacgata agattaaggg cgctattact gcggcggtgc gtaaactcga tcatgaatta   3120
   ggtattccag aagatgaaac taagacacgt ggtaagtttc actttttaaa ccgtatccat   3180
   tacatggcac caagcaatga accatggggt gaacatgaaa ttgattacat cctcttttat   3240
   aagatcaacg ctaaagaaaa cttgactgtc aacccaaacg tcaatgaagt tcgtgacttc   3300
   aaatgggttt caccaaatga tttgaaaact atgtttgctg acccaagtta caagtttacg   3360
   ccttggttta agattatttg cgagaattac ttattcaact ggtgggagca attagatgac   3420
   ctttctgaag tggaaaatga ccgtcaaatt catcgtatgc tctaaggtac c            3471

## Description

### TECHNICAL FIELD

The present invention relates to an isoprene synthase-expressing microorganism that exhibits improved expression of pyrophosphate phosphatase, and a method of producing an isoprene monomer using the isoprene synthase-expressing microorganism.

### BACKGROUND ART

Natural rubbers are very important raw materials in tire industries and rubber industries. While demands for rubbers will expand in motorization mainly in developing countries in future, increase of farm plantations is not easy due to regulation to deforestation and competition with palms. Thus, it is predicted that the increase of natural rubber yields is difficult to be anticipated and a balance of demands and supplies will become tight. Synthesized polyisoprene is available as a material in place of the natural rubber, and its raw material monomer (isoprene(2-methyl-1,3-butadiene)) is obtained by extracting from a C5 fraction obtained by cracking of naphtha. However in recent years, with lightening of a field of a cracker, a production amount of isoprene has tended to decrease, and its supply has been apprehended. Also in recent years, due to strong influence of variation in oil prices, establishment of a system for inexpensively producing isoprene derived from non-oil resource has been required for stably securing an isoprene monomer.

For such a request, a method of producing the isoprene monomer using a transformant obtained by integrating an isolated isoprene synthase gene derived from kudzu or poplar and its mutant into a bacterium for fermentation production has been disclosed (see Patent Literature 1 to 4) .

A reaction mechanism of isoprene synthase has been already demonstrated, and the isoprene synthase acts upon DMAPP (dimethylallyl pyrophosphate) as a substrate to form pyrophosphate and isoprene (Non-patent Literature 1). As an effect of pyrophosphate that is a product on an activity of isoprene synthase, it has been known that an enzyme activity of isoprene synthase derived from willow (*Salix discolor L*.) is decreased by 1 mM sodium pyrophosphate (Non-patent Literature 2). It has been known that an intracellular concentration of pyrophosphate is kept at about 0.5 mM by a protein having a pyrophosphate phosphatase activity in wild type strains of *Escherichia coli* and the like widely used as fermentative production bacteria (Non-patent Literature 3).

However, there is no finding for the concentrations of pyrophosphate in microorganisms that produce excessive isoprene. Therefore, it has been unknown whether pyrophosphate reduces an activity of isoprene synthase in such an microorganism. Also, it has been unknown whether pyrophosphate has an effect on an ability to produce isoprene.

### PRIOR ART REFERENCES

### PATENT LITERARURES

Patent Literature 1: Japanese Laid-Open Publication No. 2011-505841
Patent Literature 2: Japanese Laid-Open Publication No. 2011-518564
Patent Literature 3: International Publication WO2010/031076
Patent Literature 4: International Publication WO2014/052054

WO 2013 166211 discloses isoprene synthase variants with improved properties.

JP 2013216850 discloses polymerizing isoprene monomers.

### NON-PATENT LITERATURES

Non-patent Literature 1: Gary M. Silver and Ray Fall, Plant Physiol., (1991) 97:1588-1591
Non-patent Literature 2: Mary C. Wildermuth and Ray Fall, Plant Physiol., (1998) 116:1111-1123
Non-patent Literature 3: Kukko-Kalske E., et al., J. Bacteriol., (1989) 171:4498-4500

### SUMMARY OF INVENTION

### PROBLEM TO BE SOLVED BY THE INVENTION

It is an object of the present invention to provide a biological method that is excellent in production of isoprene.

### MEANS FOR SOLVING PROBLEM

As a result of an extensive study for solving the above problems, the present inventors have found that an ability to produce an isoprene monomer is improved by improving an expression amount of pyrophosphate phosphatase in an isoprene synthase-expressing microorganism, and completed the present invention.

Namely, the present invention is as follows.
1. An isoprene synthase-expressing microorganism that exhibits improved expression of pyrophosphate phosphatase,
   wherein an expression unit comprising a polynucleotide encoding the isoprene synthase is introduced into the isoprene synthase-expressing microorganism, and
   the expression of pyrophosphate phosphatase is improved by modifying a promoter region of a pyrophosphate phosphatase gene inherent to the isoprene synthase-expressing microorganism, or by transforming the isoprene synthase-expressing microorganism with a pyrophosphate phosphatase expression vector to introduce an expression unit comprising a polynucleotide encoding pyrophosphate phosphatase into the isoprene synthase-expressing microorganism.
2. The isoprene synthase-expressing microorganism according to embodiment 1, wherein said microorganism is a microorganism transformed with an expression vector for isoprene synthase.
3. The isoprene synthase-expressing microorganism according to embodiment 1 or 2, wherein pyrophosphate phosphatase is homologous to said microorganism.
4. The isoprene synthase-expressing microorganism according to embodiment 3, wherein expression of the pyrophosphate phosphatase is improved by modification of a promoter region of a pyrophosphate phosphatase gene inherent to said microorganism.
5. The isoprene synthase-expressing microorganism according to any one of embodiments 1 to 4, wherein the expression of the pyrophosphate phosphatase is improved by increased copy number of the pyrophosphate phosphatase gene on a chromosome.
6. The isoprene synthase-expressing microorganism according to any one of embodiments 1 to 5, wherein said microorganism is a microorganism belonging to *Enterobacteriaceae.*
7. The isoprene synthase-expressing microorganism according to any one of embodiments 1 to 6, wherein said microorganism has an ability to synthesize dimethylallyl diphosphate via a methylerythritol diphosphate pathway,
   wherein said microorganism is preferably a bacterium belonging to genus *Escherichia,* more preferably *Escherichia coli.*
8. The isoprene synthase-expressing microorganism according to any one of embodiments 1 to 7, wherein said microorganism has an ability to synthesize dimethylallyl diphosphate via a mevalonate pathway.
9. The isoprene synthase-expressing microorganism according to any one of embodiments 1 to 8, wherein said microorganism is a bacterium belonging to genus *Pantoea*.
10. The isoprene synthase-expressing microorganism according to embodiment 9, wherein said bacterium belonging to genus *Pantoea* is *Pantoea ananatis.*
11. A method of producing an isoprene monomer, comprising producing the isoprene monomer by culturing the isoprene synthase-expressing microorganism according to any one of embodiments 1 to 10 in culture medium.
12. A method of producing an isoprene polymer, comprising
   (I) producing an isoprene monomer by the method according to embodiment 11; and
   (II) polymerizing the isoprene monomer to produce the isoprene polymer.
13. A method of producing a rubber composition, comprising:
   (A) preparing an isoprene polymer by the method of embodiment 12; and
   (B) mixing said isoprene polymer with one or more rubber composition components.
14. A method of producing a tire, comprising:
   (i) producing a rubber composition by the method of embodiment 13; and
   (ii) applying said rubber composition to manufacture a tire.

### EFFECT OF THE INVENTION

The isoprene synthase-expressing microorganism of the present invention is excellent in ability to produce isoprene, and also remarkably ameliorates a glucose yield.

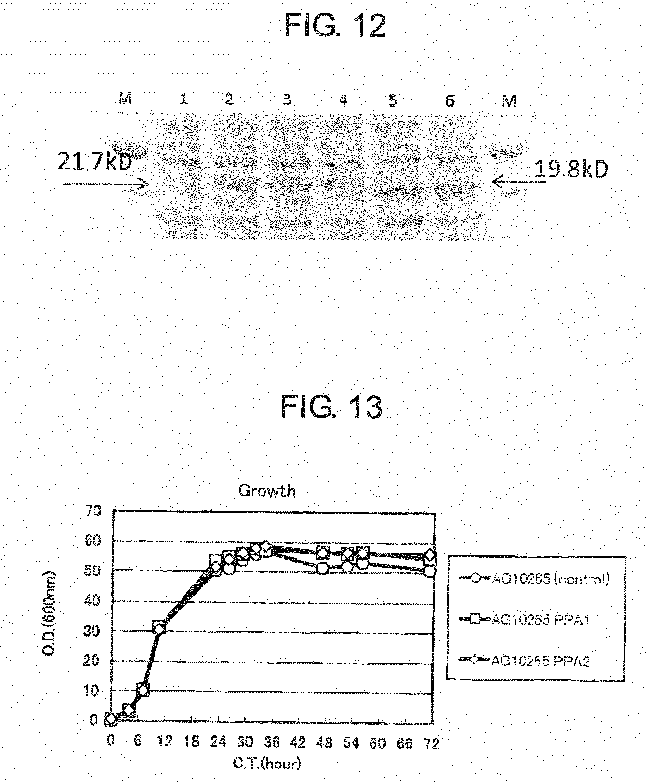

In the isoprene synthase-expressing microorganism of the present invention, its growth is also ameliorated with improvement of the expression of pyrophosphate phosphatase (see FIG. 13).

### BRIEF DESCRIPTION OF DRAWINGS

FIG. 1 shows analysis of PPA expression by SDS-PAGE. Controls in lanes 1 and 2 denote samples prepared from MG1655 Ptac-KKDyI strain. Ptac-ppa in lanes 3 and 4 denotes samples prepared from MG1655 Ptac-KKDyI Ptac-ppa strain. M denotes protein molecular weight markers;
FIG. 2 shows amounts of isoprene generated per unit weight of dry leaves from various plants;
FIG. 3 shows amounts of isoprene generated per total protein mass extracted from leaves of various plants;
FIG. 4 shows an outline of mevalonate pathway downstream and its surrounding region in chromosome fixation;
FIG. 5 shows an outline of mevalonate pathway downstream and its surrounding region controlled by a tac promoter on a chromosome;
FIG. 6 shows a map of the plasmid pAH162-Para-mvaES;
FIG. 7 shows a map of the plasmid pAH162-KKDyI-ispS(K);
FIG. 8 shows a map of pAH162-Ptac-ispS(M)-mvk(Mma);
FIG. 9 shows construction of a modified chromosome ΔampC::KKDyI-ispS(K). (A) λRed dependent substitution of ampC gene with PCR formed DNA fragment attLphi80-kan-attRphi80. (B) phi80 Int dependent integration of plasmid pAH162-KKDyI-ispS(K). (C) phi80Int/Xis dependent removal of vector portion of pAH162-KKDyI-ispS(K);
FIG. 10 shows construction of a modified chromosome ΔampC::Para-mvaES. (A) λRed dependent substitution of ampH gene with PCR formed DNA fragment attLphi80-kan-attRphi80. (B) phi80 Int dependent integration of plasmid pAH162-Para-mvaES. (C) phi80 Int/Xis dependent removal of vector portion of pAH162-Para-mvaES;
FIG. 11 shows construction of the modified genome Δcrt::KKDyI-ispS(K) of megaplasmid pEA320. (A) Structure of *P*. *ananatis* crt locus arranged in megaplasmid pEA320. (B) λRed dependent substitution of crt operon with PCR formed DNA fragment attLphi80-kan-attRphi80. (C) phi80 Int dependent integration of plasmid pAH162-Ptac-ispS(M)-mvk(Mma). (D) phi80 Int/Xis dependent removal of vector portion of pAH162-Ptac-ispS(M)-mvk(Mma);
FIG. 12 shows amounts of protein expression of pyrophosphate phosphatase in AG10265 strain. 21.7 kD denotes an assumed molecular weight of pyrophosphate phosphatase encoded by a PAJ_2344(ppa-1) gene, and 19.8 kD denotes an assumed molecular weight of pyrophosphate phosphatase encoded by a PAJ_2736(ppa-2) gene. Lane 1: soluble protein derived from AG10265 strain, Lanes 2 to 4: soluble protein derived from AG10265 Ptac-ϕ10-ppa1 strain, lanes 5 to 6: soluble protein derived from AG10265 Ptac-ϕ10-ppa2 strain, and M: molecular weight markers.
FIG. 13 shows growth change of *P. ananatis* isoprene-producing bacterium in jar cultivation;
FIG. 14 shows amounts of isoprene produced by *P*. *ananatis* isoprene-producing bacterium in jar cultivation; The amount (mg) of isoprene produced per batch is shown;
FIG. 15 shows a map of pAH162-mvaES;
FIG. 16 shows a plasmid pAH162-MCS-mvaES for chromosome fixation;
FIG. 17 shows a set of plasmid for chromosome fixation holding a mvaES gene under transcription control by P_{phoC};
FIG. 18 shows construction of an integrative vector pAH162-λattL-KmR-λattR;
FIG. 19 shows an integrative expression vector pAH162-Ptac;
FIG. 20 shows optimization of codons in chemically synthesized operon KDyI;
FIG. 21 shows integrative plasmids (A) pAH162-Tc-Ptac-KDyI and (B) pAH162-Km-Ptac-KDyI holding operon KDyI having optimized codons;
FIG. 22 shows an integrative plasmid holding a mevalonate kinase gene derived from *M. paludicola;*
FIG. 23 shows maps of the modified genomes: (A) ΔampC::attBₚₕᵢ₈₀, (B) ΔampH::attBₚₕᵢ₈₀ and (C) Δcrt::attBₚₕᵢ₈₀;
FIG. 24 shows maps of the modified genomes: (A) Δcrt::pAH162-P_{tac}-mvk(X) and (B) Δcrt::P_{tac}-mvk(X);
FIG. 25 shows maps of the modified genomes: (A) ΔampH::pAH162-Km-P_{tac}-KDyI, and (B) ΔampC::pAH162-Km-P_{tac}-KDyI;
FIG. 26 shows maps of the modified genomes (A) ΔampH::pAH162-Para-mvaES and (B) ΔampC::pAH162-Para-mvaES; and
FIG. 27 shows confirmation of PPA expression in SWITCH-PphoC-1(S)ΔydcI::Ptac-MG-ppa. Cont and MG-ppa denote that a sample derived from SWITCH-PphoC-1(S)ΔydcI strain and a sample derived from SWITCH-PphoC-1(S)ΔydcI::Ptac-MG-ppa strain were electrophoresed, respectively.

### EMBODIMENTS FOR CARRYING OUT THE INVENTION

The present invention provides an isoprene-expressing microorganism that exhibits improved expression of pyrophosphate phosphatase.

Pyrophosphate phosphatase is an enzyme that hydrolyzes a pyrophosphoric acid into a bimolecular phosphoric acid. Examples of the pyrophosphate phosphatase include pyrophosphate phosphatase derived from a microorganism as a host as described below. For the pyrophosphate phosphatase, pyrophosphate phosphatase derived from a microorganism belonging to the family *Enterobacteriaceae,* in particular, a microorganism belonging to the family *Enterobacteriaceae* among microorganisms as described below, is also preferred.

Specifically, the pyrophosphate phosphatase may be a protein consisting of the amino acid sequence of SEQ ID NO:136, SEQ ID NO:137 or SEQ ID NO:138.

Further, the pyrophosphate phosphatase may be a protein that comprises an amino acid sequence having 70% or more amino acid sequence identity to the amino acid sequence of SEQ ID NO:136, SEQ ID NO:137 or SEQ ID NO:138, and has a pyrophosphate phosphatase activity. The amino acid sequence percent identity may be, for example, 75% or more, 80% or more, 85% or more, 90% or more, 95% or more, 96% or more, 97% or more, 98% or more, or 99% or more. The pyrophosphate phosphatase activity refers to an activity of hydrolyzing a pyrophosphoric acid into a bimolecular phosphoric acid.

Further, the pyrophosphate phosphatase may be a protein that comprises an amino acid sequence having a mutation of one or several amino acid residues in the amino acid sequence of SEQ ID NO:136, SEQ ID NO:137 or SEQ ID NO:138, and has a pyrophosphate phosphatase activity. Examples of the mutation of the amino acid residues may include deletion, substitution, addition and insertion of amino acid residues. The mutation of one or several amino acid residues may be introduced into one region or multiple different regions in the amino acid sequence. The term "one or several" indicates a range in which a three-dimensional structure and an activity of the protein are not impaired greatly. In the case of the protein, the number represented by "one or several" is, for example, 1 to 50, preferably 1 to 40, more preferably 1 to 30, 1 to 20, 1 to 10, or 1 to 5.

The pyrophosphate phosphatase preferably has a pyrophosphate phosphatase activity that is 50% or more, 60% or more, 70% or more, 80% or more, 90% or more, or 95% or more of the pyrophosphate phosphatase activity of the protein consisting of the amino acid sequence of SEQ ID NO:136, SEQ ID NO:137 or SEQ ID NO:138 when measured under the same conditions (e.g., buffer, concentration, temperature, and reaction time).

The identity of the amino acid sequences can be determined, for example, using the algorithm BLAST (Pro. Natl. Acad. Sci. USA, 90, 5873 (1993)) by Karlin and Altschul, and the FASTA algorithm (Methods Enzymol., 183, 63 (1990)) by Pearson. The program referred to as BLASTP was developed based on the algorithm BLAST (see http://www.ncbi.nlm.nih.gov). Thus, the identity of the amino acid sequences may be calculated using this program with default setting. Also, for example, a numerical value obtained by calculating similarity as a percentage at a setting of "unit size to compare=2" using the full length of a polypeptide portion encoded in ORF with the software GENETYX Ver. 7.0.9 from Genetyx Corporation employing the Lipman-Pearson method may be used as the identity of the amino acid sequences. The lowest value among the values derived from these calculations may be employed as the identity of the amino acid sequences.

In the pyrophosphate phosphatase, the mutation may be introduced into sites in a catalytic domain and sites other than the catalytic domain as long as an objective activity is retained. The positions of amino acid residues to be mutated which are capable of retaining the objective activity are understood by a person skilled in the art. Specifically, a person skilled in the art can recognize a correlation between structure and function, since a person skilled in the art can 1) compare the amino acid sequences of multiple proteins having the same type of activity, 2) clarify regions that are relatively conserved and regions that are not relatively conserved, and then 3) predict regions capable of playing a functionally important role and regions incapable of playing a functionally important role from the regions that are relatively conserved and the regions that are not relatively conserved, respectively. Therefore, a person skilled in the art can identify the positions of the amino acid residues to be mutated in the amino acid sequence of the pyrophosphate phosphatase.

When the mutation of the amino acid residue is substitution, the substitution of the amino acid residue may be conservative substitution. The term "conservative substitution" refers to substitution of a certain amino acid residue with an amino acid residue having a similar side chain. Families of amino acid residues having similar side chains are well-known in the art. Examples of such families may include amino acids having a basic side chain (e.g., lysine, arginine, histidine), amino acids having an acidic side chain (e.g., aspartic acid, glutamic acid), amino acids having a non-charged polar side chain (e.g., asparagine, glutamine, serine, threonine, tyrosine, cysteine), amino acids having a non-polar side chain (e.g., glycine, alanine, valine, leucine, isoleucine, proline, phenylalanine, methionine, tryptophan), amino acids having a branched side chain at position β (e.g., threonine, valine, isoleucine), amino acids having an aromatic side chain (e.g., tyrosine, phenylalanine, tryptophan, histidine), amino acids having a hydroxyl group-containing (e.g., alcoholic, phenolic) side chain (e.g., serine, threonine, tyrosine), and amino acids having a sulfur-containing side chain (e.g., cysteine, methionine). Preferably, the conservative substitution of the amino acids may be the substitution between aspartic acid and glutamic acid, the substitution among arginine, lysine and histidine, the substitution between tryptophan and phenylalanine, the substitution between phenylalanine and valine, the substitution among leucine, isoleucine and alanine, and the substitution between glycine and alanine.

The expression of pyrophosphate phosphatase in the isoprene synthase-expressing microorganism can be improved as indicated in the claims. Pyrophosphate phosphatase, the expression of which is to be improved in the isoprene synthase-expressing microorganism is pyrophosphate phosphatase that is homologous or heterologous to isoprene synthase and/or the isoprene synthase-expressing microorganism. Pyrophosphate phosphatase homologous to the isoprene synthase-expressing microorganism may be pyrophosphate phosphatase inherent to the isoprene synthase-expressing microorganism or foreign pyrophosphate phosphatase. One or a plurality (e.g., 2 or 3) of pyrophosphate phosphatase, the expression of which is to be improved in the isoprene synthase-expressing microorganism may be available.

The expression of pyrophosphate phosphatase in the isoprene synthase-expressing microorganism is improved, by modifying a promoter region of a pyrophosphate phosphatase gene inherent to the isoprene synthase-expressing microorganism, or by transforming the isoprene synthase-expressing microorganism with a pyrophosphate phosphatase expression vector to introduce an expression unit comprising a polynucleotide encoding pyrophosphate phosphatase into the isoprene synthase-expressing microorganism. The expression vector used in the present invention may further comprise one or more region that allows for homologous recombination with genome of a host cell when introduced into the host cell. For example, the expression vector may be designed such that an expression unit comprising a given polynucleotide is positioned between a pair of homologous regions (e.g., homology arm homologous to a certain sequence in host genome, loxP, FRT). The expression unit refers to a unit that comprises a given polynucleotide to be expressed and a promoter (homologous promoter, heterologous promoter) operably linked thereto and allows for transcription of the polynucleotide and further production of a polypeptide encoded by the polynucleotide. The expression unit may further comprise elements such as a terminator, a ribosome binding site and a drug resistant gene.

Examples of the expression vector used in the present invention may include vectors that expresses a protein in a host. The expression vector may also be a plasmid, a viral vector, a phage or an artificial chromosome. The expression vector may further be a DNA vector or an RNA vector. The expression vector may be an integrative vector or a non-integrative vector. The integrative vector may be a vector of a type where the vector is entirely integrated into genome of a host cell. Alternatively, the integrative vector may be a vector of a type where the vector is partially (e.g., the aforementioned expression unit) integrated into genome of a host cell.

Examples of the modification of the promoter region may include one or several (e.g., 1 to 500, 1 to 300, 1 to 200 or 1 to 100) nucleotide substitutions, insertions or deletions of the promoter region. Preferably, the modification of the promoter region is the substitution of the promoter region and if necessary the substitution of the SD sequence. Examples of a promoter to be introduced after the substitution may include inducible promoters such as a tac promoter (Ptac), a trc promoter (Ptrc) and a lac promoter (Plac).

In the present invention, it is desirable that a copy number of the pyrophosphate phosphatase gene on a chromosome is increased thereby enhancing an activity. It is preferable that a plurality of copies, desirably 2 copies and more preferably 3 copies are carried on the chromosome. Increase of the copy number can be accomplished by introducing a plasmid carrying the pyrophosphate phosphatase gene into a host cell. The increase of the copy number can also be accomplished by utilizing transposon or Mu phage to transfer the pyrophosphate phosphatase gene onto the genome of the host.

The isoprene synthase-expressing microorganism is a microorganism that produces the isoprene synthase. Preferably, the isoprene synthase-expressing microorganism is a microorganism obtained by transforming a host cell with an isoprene synthase-expressing vector to introduce an expression unit comprising a polynucleotide encoding the isoprene synthase into the host cell. The expression unit and the expression vector are as described above. It is preferable that a plurality of copies, desirably 2 copies and more preferably 3 copies of an isoprene synthase gene are carried on the chromosome in the isoprene synthase-expressing microorganism. Such an isoprene synthase-expressing microorganism can be obtained by introducing an isoprene synthase-expressing vector into a host. Also such an isoprene synthase-expressing microorganism can be obtained by utilizing transposon or Mu phage to transfer the isoprene synthase gene onto the genome of the host. The host cell may be homologous or heterologous to the isoprene synthase, but is preferably heterologous. Examples of the isoprene synthase gene contained in the isoprene synthase-expressing vector may include isoprene synthase genes derived from kudzu (*Pueraria montana var. lobata*), poplar (*Populus alba x Populus tremula*), Mucuna (*Mucuna bracteata*), willow (*Salix*), false acacia (*Robinia pseudoacacia*), wisteria (*Wisterria*), eucalyptus (*Eucalyptus globulus*), and tea plant (*Melaleuca alterniflora*) (see, e.g., Evolution 67 (4), 1026-1040 (2013)). The isoprene synthase-expressing vector may be an integrative vector or a non-integrative vector. A gene encoding the isoprene synthase can be arranged under control of a constitutive promoter or an inducible promoter (e.g., a promoter as described below which is inversely depending on the growth promoting agent) in the expression vector. Preferably, the gene encoding the isoprene synthase can be arranged under the control of the constitutive promoter. Examples of the constitutive promoter may include a tac promoter, a lac promoter, a trp promoter, a trc promoter, a T7 promoter, a T5 promoter, a T3 promoter, and an Sp6 promoter.

In one embodiment, the isoprene synthase may be, for example, a protein as follows:
1) a full-length protein which may be derived from Kudzu (the amino acid sequence of SEQ ID NO:8);
2) a protein obtained by deleting a chloroplast localization signal from the full-length protein in 1) above (amino acid sequence obtained by deleting amino acid residues at positions 1 to 45 in the amino acid sequence of SEQ ID NO:8);
3) a full-length protein which may be derived from Poplar (the amino acid sequence of SEQ ID NO:11);
4) a protein obtained by deleting a chloroplast localization signal from the full-length protein in 3) above (amino acid sequence obtained by deleting amino acid residues at positions 1 to 37 in the amino acid sequence of SEQ ID NO: 11);
5) a full-length protein which may be derived from Mucuna (the amino acid sequence of SEQ ID NO:7); and
6) a protein obtained by deleting a chloroplast localization signal from the full-length protein in 5) above (amino acid sequence obtained by deleting amino acid residues at positions 1 to 44 in the amino acid sequence of SEQ ID NO:146).

In a preferred embodiment, the isoprene synthase may be derived from Kudzu. In another preferred embodiment, the isoprene synthase may be derived from Poplar. In still another preferred embodiment, the isoprene synthase may be derived from Mucuna.

In another embodiment, the isoprene synthase is a protein that comprises an amino acid sequence having 70% or more amino acid sequence identity to the amino acid sequence of the proteins of 1) to 6) above, and has an isoprene synthase activity. The amino acid sequence percent identity may be, for example, 75% or more, 80% or more, 85% or more, 90% or more, 95% or more, 96% or more, 97% or more, 98% or more, or 99% or more. The amino acid sequence percent identity can be determined in the aforementioned manner. The isoprene synthase activity refers to an activity of forming isoprene from dimethylallyl diphosphate (DMAPP).

In still another embodiment, the isoprene synthase is a protein that comprises an amino acid sequence having a mutation of one or several amino residues in the amino acid sequence of the protein of 1) to 6) above, and has an isoprene synthase activity. Examples of the mutation of the amino acid residues may include deletion, substitution, addition and insertion of amino acid residues. The mutation of one or several amino acid residues may be introduced into one region or multiple different regions in the amino acid sequence. The term "one or several" indicates a range in which a three-dimensional structure and an activity of the protein are not impaired greatly. In the case of the protein, the number represented by "one or several" is, for example, 1 to 100, preferably 1 to 80, more preferably 1 to 50, 1 to 30, 1 to 20, 1 to 10, or 1 to 5.

The isoprene synthase preferably has an isoprene synthase activity that is 50% or more, 60% or more, 70% or more, 80% or more, 90% or more, or 95% or more of the isoprene synthase activity of the protein of 1) to 6) above when measured under the same conditions (e.g., buffer, concentration, temperature, and reaction time). In terms of stability, it is also preferable that the isoprene synthase has a remaining activity that is 30% or more, 40% or more, 50% or more, 60% or more or 65% or more of the original activity when stored in a certain buffer [e.g., a solution of 50 mM Tris-HCl (pH 8.0), and 0.15 mM MgCl₂] at 4°C for 48 hours.

In the isoprene synthase, the mutation may be introduced into sites in a catalytic domain and sites other than the catalytic domain as long as an objective activity is retained. The positions of amino acid residues to be mutated which are capable of retaining the objective activity are understood by a person skilled in the art. Specifically, a person skilled in the art can recognize a correlation between structure and function, since a person skilled in the art can 1) compare the amino acid sequences of multiple proteins having the same type of activity, 2) clarify regions that are relatively conserved and regions that are not relatively conserved, and then 3) predict regions capable of playing a functionally important role and regions incapable of playing a functionally important role from the regions that are relatively conserved and the regions that are not relatively conserved, respectively. Therefore, a person skilled in the art can identify the positions of the amino acid residues to be mutated in the amino acid sequence of the isoprene synthase. When an amino acid residue is mutated by substitution, the substitution of the amino acid residue may be the conservative substitution as described above.

Preferably, the isoprene synthase-expressing microorganism may be a microorganism that further expresses a mevalonate kinase in addition to the isoprene synthase. Therefore, in the isoprene synthase-expressing microorganism, a mevalonate kinase expression vector may be introduced into a host. Examples of the mevalonate kinase gene to be introduced into the host by the mevalonate kinase expression vector may include genes from microorganisms belonging to the genus *Methanosarcina* such as *Methanosarcina mazei,* the genus Methanocella such as *Methanocella paludicola,* the genus Corynebacterium such as *Corynebacterium variabile,* the genus *Methanosaeta* such as *Methanosaeta concilii*, and the genus *Nitrosopumilus* such as *Nitrosopumilus maritimus.* The mevalonate kinase expression vector may be an integrative vector or a non-integrative vector. In the expression vector, the gene encoding the mevalonate kinase may be placed under the control of the constitutive promoter as described above or an inducible promoter (e.g., a promoter as described below which is inversely dependent on the growth promoting agent). Preferably, the gene encoding the mevalonate kinase may be placed under the control of the constitutive promoter.

For the isoprene synthase-expressing microorganism (host cell) used in the present invention, a bacterium or a fungus is preferred. The bacterium may be a gram-positive bacterium or a gram-negative bacterium. For the isoprene synthase-expressing microorganism, a microorganism belonging to the family *Enterobacteriaceae,* in particular, a microorganism belonging to the family *Enterobacteriaceae* among microorganisms as described below, is also preferred.

Examples of the gram-positive bacterium may include bacteria belonging to the genera *Bacillus, Listeria, Staphylococcus, Streptococcus, Enterococcus, Clostridium, Corynebacterium,* and *Streptomyces.* Bacteria belonging to the genera *Bacillus* and *Corynebacterium* are preferable.

Examples of the bacteria belonging to the genus *Bacillus* may include *Bacillus subtilis, Bacillus anthracis,* and *Bacillus cereus. Bacillus subtilis* is more preferable.

Examples of the bacteria belonging to genus the *Corynebacterium* may include *Corynebacterium glutamicum, Corynebacterium efficiens,* and *Corynebacterium callunae. Corynebacterium glutamicum* is more preferable.

Examples of the gram-negative bacterium may include bacteria belonging to the genera *Escherichia, Pantoea, Salmonella, Vivrio, Serratia,* and *Enterobacter.* The bacteria belonging to the genera *Escherichia, Pantoea* and *Enterobacter* are preferable.

*Escherichia coli* is preferable as the bacteria belonging to the genus *Escherichia.*

Examples of the bacteria belonging to the genus *Pantoea* may include *Pantoea ananatis, Pantoea stewartii, Pantoea agglomerans,* and *Pantoea citrea. Pantoea ananatis* and *Pantoea citrea* are preferable. Strains exemplified in EP 0 952 221 may be used as the bacteria belonging to the genus *Pantoea*. Examples of representative strains of the bacteria belonging to genus *Pantoea* may include Pantoea *ananatis* AJ13355 strain (FERM BP-6614) and *Pantoea ananatis* AJ13356 strain (FERM BP-6615), both of which are disclosed in EP 0 952 221, and *Pantoea ananatis* SC17(0) strain. SC17(0) was deposited to Russian National Collection of Industrial Microorganisms (VKPM), GNII Genetika (address: Russia, 117545 Moscow, 1 Dorozhny proezd. 1) as of September 21, 2005, with the deposit number of VKPM B-9246.

Examples of the bacteria belonging to the genus *Enterobacter* may include *Enterobacter agglomerans* and *Enterobacter aerogenes. Enterobacter* aerogenes is preferable. The bacterial strains exemplified in EP 0 952 221 may be used as the bacteria belonging to the genus *Enterobacter.* Examples of representative strains of the bacteria belonging to the genus *Enterobacter* may include *Enterobacter agglomerans* ATCC12287 strain, *Enterobacter aerogenes* TACC13048 strain, Enterobacter aerogenes NBRC12010 strain (Biotechnol. Bioeng., 2007 Mar 27;98(2): 340-348), and *Enterobacter aerogenes* AJ110637 (FERM BP-10955). The *Enterobacter aerogenes* AJ110637 strain was deposited to International Patent Organism Depositary (IPOD), National Institute of Advanced Industrial Science and Technology (AIST) (Chuo No. 6, Higashi 1-1-1, Tsukuba City, Ibaraki Pref., JP, Postal code 305-8566) as of August 22, 2007, with the deposit number of FERM P-21348 and was transferred to the international deposition based on Budapest Treaty on March 13, 2008, and the receipt number FERM BP-10955 was given thereto.

Examples of the fungus may include microorganisms belonging to the genera *Saccharomyces, Schizosaccharomyces, Yarrowia, Trichoderma, Aspergillus, Fusarium,* and *Mucor.* The microorganisms belonging to the genera *Saccharomyces, Schizosaccharomyces, Yarrowia,* or *Trichoderma* are preferable.

Examples of the microorganisms belonging to the genus *Saccharomyces* may include *Saccharomyces carlsbergensis, Saccharomyces cerevisiae, Saccharomyces diastaticus, Saccharomyces douglasii, Saccharomyces kluyveri, Saccharomyces norbensis,* and *Saccharomyces oviformis. Saccharomyces cerevisiae* is preferable.

*Schizosaccharomyces pombe* is preferable as a microorganism belonging to the genus *Schizosaccharomyces.*

*Yarrowia lypolytica* is preferable as a microorganism belonging to the genus *Yarrowia.*

Examples of the microorganisms belonging to the genus *Trichoderma* may include *Trichoderma harzianum, Trichoderma koningii, Trichoderma longibrachiatum, Trichoderma reesei,* and *Trichoderma viride. Trichoderma reesei* is preferable.

In the isoprene synthase-expressing microorganism of the present invention, the pathway to synthesize dimethylallyl diphosphate (DMAPP) that is the substrate of the isoprene synthase may be further enhanced. For such an enhancement, an expression vector that expresses an isopentenyl-diphosphate delta isomerase having an ability to convert isopentenyl diphosphate (IPP) into dimethylallyl diphosphate (DMAPP) may be introduced into the isoprene synthase-expressing microorganism of the present invention. An expression vector that expresses one or more enzymes involved in the mevalonate pathway and/or methylerythritol phosphate pathway associated with formation of IPP and/or DMAPP may also be introduced into the isoprene synthase-expressing microorganism of the present invention. The expression vector for such an enzyme may be an integrative vector or a non-integrative vector. The expression vector for such an enzyme may further express at a time or separately a plurality of enzymes (e.g., one, two, three or four or more) involved in the mevalonate pathway and/or the methylerythritol phosphate pathway, and may be, for example, an expression vector for polycistronic mRNA. Origin of one or more enzymes involved in the mevalonate pathway and/or the methylerythritol phosphate pathway may be homologous or heterologous to the host. When the origin of the enzyme involved in the mevalonate pathway and/or the methylerythritol phosphate pathway is heterologous to the host, for example, the host may be a bacterium as described above (e.g., *Escherichia coli*) and the enzyme involved in the mevalonate pathway may be derived from a fungus (e.g., *Saccharomyces cerevisiae*). In addition, when the host inherently produces the enzyme involved in the methylerythritol phosphate pathway, an expression vector to be introduced into the host may express an enzyme involved in the mevalonate pathway.

Examples of isopentenyl-diphosphate delta isomerase (EC: 5.3.3.2) may include Idilp (ACCESSION ID NP_015208), AT3G02780 (ACCESSION ID NP_186927), AT5G16440 (ACCESSION ID NP_197148) and Idi (ACCESSION ID NP_417365). In the expression vector, a gene encoding an isopentenyl-diphosphate delta isomerase may be placed under the control of a promoter as described below which is inversely dependent on a growth promoting agent.

Examples of the enzymes involved in the mevalonate (MVA) pathway may include mevalonate kinase (EC: 2.7.1.36; example 1, Erg12p, ACCESSION ID NP_013935; example 2, AT5G27450, ACCESSION ID NP_001190411), phosphomevalonate kinase (EC: 2.7.4.2; example 1, Erg8p, ACCESSION ID NP_013947; example 2, AT1G31910, ACCESSION ID NP_001185124), diphosphomevalonate decarboxylase (EC: 4.1.1.33; example 1, Mvd1p, ACCESSION ID NP_014441; example 2, AT2G38700, ACCESSION ID NP_181404; example 3, AT3G54250, ACCESSION ID NP_566995), acetyl-CoA-C-acetyltransferase (EC: 2.3.1.9; example 1, Erg10p, ACCESSION ID NP_015297; example 2, AT5G47720, ACCESSION ID NP_001032028; example 3, AT5G48230, ACCESSION ID NP_568694), hydroxymethylglutaryl-CoA synthase (EC: 2.3.3.10; example 1, Erg13p, ACCESSION ID NP_013580; example 2, AT4G11820, ACCESSION ID NP_192919; example 3, MvaS, ACCESSION ID AAG02438), hydroxymethylglutaryl-CoA reductase (EC: 1.1.1.34; example 1, Hmg2p, ACCESSION ID NP_013555; example 2, Hmg1p, ACCESSION ID NP_013636; example 3, AT1G76490, ACCESSION ID NP_177775; example 4, AT2G17370, ACCESSION ID NP_179329, EC: 1.1.1.88, example, MvaA, ACCESSION ID P13702), and acetyl-CoA-C-acetyltransferase/hydroxymethylglutaryl-CoA reductase (EC: 2.3.1.9/1.1.1.34, example, MvaE, ACCESSION ID AAG02439). In the expression vector, a gene encoding one or more enzymes involved in the mevalonate (MVA) pathway (e.g., phosphomevalonate kinase, diphosphomevalonate decarboxylase, acetyl-CoA-C-acetyltransferase/hydroxymethylglutaryl-CoA reductase, hydroxymethylglutaryl-CoA synthase) may be placed under the control of a promoter as described below which is inversely dependent on the growth promoting agent.

Examples of the enzymes involved in the methylerythritol phosphate (MEP) pathway may include 1-deoxy-D-xylulose-5-phosphate synthase (EC: 2.2.1.7, example 1, Dxs, ACCESSION ID NP_414954; example 2, AT3G21500, ACCESSION ID NP_566686; example 3, AT4G15560, ACCESSION ID NP_193291; example 4, AT5G11380, ACCESSION ID NP_001078570), 1-deoxy-D-xylulose-5-phosphate reductoisomerase (EC: 1.1.1.267; example 1, Dxr, ACCESSION ID NP_414715; example 2, AT5G62790, ACCESSION ID NP_001190600), 4-diphosphocytidyl-2-C-methyl-D-erythritol synthase (EC: 2.7.7.60; example 1, IspD, ACCESSION ID NP_417227; example 2, AT2G02500, ACCESSION ID NP_565286), 4-diphosphocytidyl-2-C-methyl-D-erythritol kinase (EC: 2.7.1.148; example 1, IspE, ACCESSION ID NP_415726; example 2, AT2G26930, ACCESSION ID NP_180261), 2-C-methyl-D-erythritol-2,4-cyclodiphosphate synthase (EC: 4.6.1.12; example 1, IspF, ACCESSION ID NP_417226; example 2, AT1G63970, ACCESSION ID NP_564819), 1-hydroxy-2-methyl-2-(E)-butenyl-4-diphosphate synthase (EC: 1.17.7.1; example 1, IspG, ACCESSION ID NP_417010; example 2, AT5G60600, ACCESSION ID NP_001119467), and 4-hydroxy-3-methyl-2-butenyl diphosphate reductase (EC: 1.17.1.2; example 1, IspH, ACCESSION ID NP_414570; example 2, AT4G34350, ACCESSION ID NP_567965). In the expression vector, a gene encoding one or more enzymes involved in the methylerythritol phosphate (MEP) pathway may be placed under the control of a promoter as described below which is inversely dependent on the growth promoting agent.

Transformation of the host cell by the expression vector in which the gene is incorporated can be carried out using known methods. Examples of such a method may include a competent cell method using a microbial cell treated with calcium and an electroporation method. The gene may be introduced by infecting the microbial cell with a phage vector rather than the plasmid vector.

Further, a gene encoding the enzyme involved in the mevalonate pathway or the methylerythritol phosphate pathway that synthesizes dimethylallyl diphosphate that is the substrate of the isoprene synthase may also be introduced into the isoprene synthase-expressing microorganism of the present invention. Examples of such an enzyme may include 1-deoxy-D-xylose-5-phosphate synthase that converts a pyruvate and D-glycelaldehyde-3-phosphate into 1-deoxy-D-xylose-5-phosphate, and isopentyl diphosphate isomerase that converts isopentenyl diphosphate into dimethylallyl diphosphate. In the expression vector, a gene encoding the enzyme involved in the mevalonate pathway or the methylerythritol phosphate pathway that synthesizes dimethylallyl diphosphate may be placed under the control of the constitutive promoters as described above or an inducible promoter (e.g., a promoter as described below, which is inversely dependent on the growth promoting agent).

DMAPP (dimethylallyl diphosphate) that is a substrate of isoprene synthesis has been known to be a precursor of peptide glycan and an electron acceptor, such as menaquinone and the like, and to be essential for growth of microorganisms (Fujisaki et al., J. Biochem., 1986; 99: 1137-1146). In the light of efficient production of isoprene, the isoprene synthase-expressing microorganism of the present invention may be used in the method of producing isoprene, in which a step 1) corresponding to a growth phase of a microorganism (culturing an isoprene-expressing microorganism in the presence of a growth promoting agent at a sufficient concentration to grow the isoprene-expressing microorganism) and a step 3) corresponding to a formation phase of the isoprene (culturing the isoprene-expressing microorganism to form an isoprene monomer) are separated. The method may also comprise a step 2) corresponding to an induction phase of isoprene production for transferring the growth phase of the microorganism to the formation phase of the isoprene (decreasing the sufficient concentration of the growth promoting agent to induce production of the isoprene monomer by the isoprene-expressing microorganism).

In the method of the present invention, the growth promoting agent can refer to a factor essential for the growth of a microorganism or a factor having an activity of promoting the growth of the microorganism, which can be consumed by the microorganism, the consumption of which causes reduction of its amount in a culture medium, consequently lost or reduction of the growth of the microorganism. For example, when the growth promoting agent in a certain amount is used, a microorganism continues to grow until the growth promoting agent in that amount is consumed, but once the growth promoting agent is entirely consumed, the microorganism cannot grow or the growth rate can decrease. Therefore, the degree of the growth of the microorganism can be regulated by the growth promoting agent. Examples of such a growth promoting agent may include substances such as oxygen (gas); minerals such as ions of iron, magnesium, potassium and calcium; phosphorus compounds such as monophosphoric acid, diphosphoric acid and polyphosphoric acid, or salt thereof; nitrogen compounds (gas) such as ammonia, nitrate, nitrite, and urea; sulfur compounds such as ammonium sulfate and thiosulfuric acid; and nutrients such as vitamins (e.g., vitamin A, vitamin D, vitamin E, vitamin K, vitamin B1, vitamin B2, vitamin B6, vitamin B12, niacin, pantothenic acid, biotin, ascorbic acid), and amino acids (e.g., alanine, arginine, asparagine, aspartic acid, cysteine, glutamine, glutamic acid, glycine, histidine, leucine, isoleucine, lysine, methionine, phenylalanine, proline, serine, threonine, tryptophan, tyrosine, valine, selenocysteine). One growth promoting agent may be used or two or more growth promoting agents may be used in combination in the method of the present invention.

When the growth promoting agent is used, the isoprene-expressing microorganism of the present invention may have an ability to grow depending on the growth promoting agent and an ability to form isoprene depending on a promoter which is inversely dependent on the growth promoting agent. An isoprene-producing microorganism can grow in the presence of the growth promoting agent at concentration sufficient for the growth of the isoprene-producing microorganism. Here, the "sufficient concentration" can refer to that the growth promoting agent is used at concentration which is effective for the growth of the isoprene-producing microorganism. The expression "ability to produce an(the) isoprene depending on a promoter which is inversely depending on the growth promoting agent" can mean that the isoprene cannot be produced or a producing efficiency of the isoprene is low in the presence of the growth promoting agent at relatively high concentration whereas the isoprene can be produced or the producing efficiency of the isoprene is high in the presence of the growth promoting agent at relatively low concentration or in the absence of the growth promoting agent. Therefore, the isoprene-producing microorganism used in the present invention can grow well but cannot produce the isoprene or exhibits low producing efficiency of the isoprene in the presence of the growth promoting agent at sufficient concentration. The isoprene-producing microorganism cannot grow well but can produce the isoprene and exhibits high producing efficiency of the isoprene in the presence of the growth promoting agent at insufficient concentration or in the absence of the growth promoting agent.

In such an isoprene-producing microorganism, a gene encoding the above-described enzyme can be present under the control of a promoter which is inversely dependent on the growth promoting agent. The expression "promoter which is inversely dependent on the growth promoting agent" can mean a promoter not having at all or having low transcription activity in the presence of the growth promoting agent at relatively high concentration but having some or high transcription activity in the presence of the growth promoting agent at relatively low concentration or in the absence of the growth promoting agent. Therefore, the promoter which is inversely dependent on the growth promoting agent can suppress the expression of the gene encoding the above-described enzyme in the presence of the growth promoting agent at a concentration sufficient for the growth of the isoprene-producing microorganism whereas it can promote the expression of the gene encoding the above-described enzyme in the presence of the growth promoting agent at the concentration insufficient for the growth of the isoprene-producing microorganism or in the absence of the growth promoting agent. Preferably, the isoprene-producing microorganism is a microorganism transformed with an expression vector comprising the gene encoding the above-described enzyme under the control of the promoter which is inversely dependent on the growth promoting agent.

For example, when the growth promoting agent is oxygen, a microaerobically inducible promoter can be utilized. The microaerobically inducible promoter can refer to a promoter that can promote the expression of a downstream gene under a microaerophilic condition. In general, the saturated concentration of dissolved oxygen is 7.22 ppm (under the air condition: 760 mmHg, 33°C, 20.9% oxygen and saturated water vapor). The microaerophilic condition can refer to a condition where a (dissolved) oxygen concentration is 0.35 ppm or less. The (dissolved) oxygen concentration under the microaerophilic condition may be 0.30 ppm or less, 0.25 ppm or less, 0.20 ppm or less, 0.15 ppm or less, 0.10 ppm or less, or 0.05 ppm or less. Examples of the microaerobically inducible promoter may include a promoter of the gene encoding a D- or L-lactate dehydrogenase (e.g., 11d, ldhA), a promoter of the gene encoding an alcohol dehydrogenase (e.g., adhE), a promoter of the gene encoding a pyruvate formate lyase (e.g., pflB), and a promoter of the gene encoding an α-acetolactate decarboxylase (e.g., budA).

When the growth promoting agent is a phosphorus compound, a phosphorus deficiency-inducible promoter can be utilized. The expression "phosphorus deficiency-inducible promoter" can refer to a promoter that can promote the expression of a downstream gene at low concentration of phosphorus compound. The low concentration of phosphorus compound can refer to a condition where a (free) phosphorus concentration is 100 mg/L or less. The expression "phosphorus" is synonymous to the expression "phosphorus compound", and they can be used in exchangeable manner. The (free) phosphorus concentration under a phosphorus deficient condition may be 50 mg/L or less, 10 mg/L or less, 5 mg/L or less, 1 mg/L or less, 0.1 mg/L or less, or 0.01 mg/L or less. Examples of the phosphorus deficiency-inducible promoter may include a promoter of the gene encoding alkali phosphatase (e.g., phoA), a promoter of the gene encoding an acid phosphatase (e.g., phoC), a promoter of the gene encoding a sensor histidine kinase (phoR), a promoter of the gene encoding a response regulator (e.g., phoB), and a promoter of the gene encoding a phosphorus uptake carrier (e.g., pstS).

When the growth promoting agent is an amino acid, an amino acid deficiency-inducible promoter can be utilized. The amino acid deficiency-inducible promoter can refer to a promoter that can promote the expression of a downstream gene at low concentration of an amino acid. The low concentration of the amino acid can refer to a condition where a concentration of a (free) amino acid or a salt thereof is 100 mg/L or less. The concentration of the (free) amino acid or a salt thereof under the amino acid deficient condition may be 50 mg/L or less, 10 mg/L or less, 5 mg/L or less, 1 mg/L or less, 0.1 mg or less or 0.01 mg/L or less. Examples of the amino acid deficiency-inducible promoter may include a promoter of the gene encoding a tryptophan leader peptide (e.g., trpL) and a promoter of the gene encoding an N-acetylglutamate synthase (e.g., ArgA).

### <Method of producing isoprene monomer and isoprene polymer>

The present invention provides a method of producing an isoprene monomer. The method of producing an isoprene monomer of the present invention includes culturing an isoprene synthase-expressing microorganism in a culture medium so as to form an isoprene monomer.

The method of producing the isoprene monomer of the present invention can be performed by culturing the isoprene synthase-expressing microorganism of the present invention. Dimethylallyl diphosphate that is a raw material of the isoprene monomer is efficiently supplied from a carbon source in a culture medium by the isoprene synthase-expressing microorganism of the present invention. The isoprene synthase-expressing microorganism of the present invention produces the isoprene monomer mainly as an outgas from the carbon source in the culture medium. Thus, the isoprene monomer is recovered by collecting gas produced from the transformant. Dimethylallyl diphosphate that is the substrate of the isoprene synthase is synthesized from the carbon source in the culture medium via the mevalonate pathway or the methylerythritol phosphate pathway in the host cells.

The culture medium for culturing the isoprene synthase-expressing microorganism of the present invention preferably contains the carbon source to be converted into isoprene. The carbon source may include carbohydrates such as monosaccharides, disaccharides, oligosaccharides and polysaccharides; invert sugars obtained by hydrolyzing sucrose; glycerol; compounds having one carbon atom (hereinafter referred to as a C1 compound) such as methanol, formaldehyde, formate, carbon monoxide and carbon dioxide; oils such as corn oil, palm oil and soybean oil; acetate; animal fats; animal oils; fatty acids such as saturated fatty acids and unsaturated fatty acids; lipids; phospholipids; glycerolipids; glycerine fatty acid esters such as monoglyceride, diglyceride and triglyceride; polypeptides such as microbial proteins and plant proteins; renewable carbon sources such as hydrolyzed biomass carbon sources; yeast extracts, or combinations thereof. For a nitrogen source, inorganic ammonium salts such as ammonium sulfate, ammonium chloride and ammonium phosphate, organic nitrogen such as hydrolyzed soybeans, ammonia gas, ammonia water, and the like can be used. It is desirable to include required substances such as vitamin B1 and L-homoserine, or yeast extract and the like in an appropriate amount as an organic trace nutrient source. In addition thereto, potassium phosphate, magnesium sulfate, iron ion, manganese ion, and the like may be added in small amounts if necessary. The culture medium used in the present invention may be a natural medium or a synthesized medium as long as the culture medium contains a carbon source, a nitrogen source, inorganic ions, and optionally other organic trace ingredients.

Examples of the monosaccharides may include triose such as ketotriose (dihydroxyacetone) and aldotriose (glyceraldehyde); tetrose such as ketotetrose (erythrulose) and aldotetrose (erythrose, threose); pentose such as ketopentose (ribulose, xylulose), aldopentose (ribose, arabinose, xylose, lyxose) and deoxysaccharide (deoxyribose); hexose such as ketohexose (psychose, fructose, sorbose, tagatose), aldohexose (allose, altrose, glucose, mannose, gulose, idose, galactose, tallose), and deoxysaccharide (fucose, fucrose, rhamnose); and heptose such as sedoheptulose. C6 sugars such as fructose, mannose, galactose and glucose; and C5 sugars such as xylose and arabinose are preferable.

Examples of the disaccharides may include sucrose, lactose, maltose, trehalose, turanose, and cellobiose. Sucrose and lactose are preferable.

Examples of the oligosaccharides may include trisaccharides such as raffinose, melezitose and maltotriose; tetrasaccharides such as acarbose and stachyose; and other oligosaccharides such as fructooligosaccharide (FOS), galactooligosaccharide (GOS) and mannan-oligosaccharide (MOS).

Examples of the polysaccharides may include glycogen, starch (amylose, amylopectin), cellulose, dextrin, and glucan (β1,3-glucan). Starch and cellulose are preferable.

Examples of the microbial protein may include polypeptides obtainable from a yeast or bacterium.

Examples of the plant protein may include polypeptides obtainable from soybean, corn, canola, Jatropha, palm, peanut, sunflower, coconut, mustard, cotton seed, palm kernel oil, olive, safflower, sesame and linseed.

Examples of the lipid may include substances containing one or more saturated or unsaturated fatty acids of C4 or more.

The oil is preferably the lipid that contains one or more saturated or unsaturated fatty acids of C4 or more and is liquid at room temperature, and examples of the oil may include lipids obtainable from soybean, corn, canola, Jatropha, palm, peanut, sunflower, coconut, mustard, cotton seed, Palm kernel oil, olive, safflower, sesame, linseed, oily microbial cells, Chinese tallow tree, and a combination of two or more thereof.

Examples of the fatty acid may include compounds represented by a formula RCOOH ("R" represents a hydrocarbon group).

The unsaturated fatty acid is a compound having at least one double bond between two carbon atoms in "R", and examples of the unsaturated fatty acid may include oleic acid, vaccenic acid, linoleic acid, palmitelaidic acid and arachidonic acid.

The saturated fatty acid is a compound where the "R" is a saturated aliphatic group, and examples of the saturated fatty acid may include docosanoic acid, eicosanoic acid, octadecanoic acid, hexadecanoic acid, tetradecanoic acid, and dodecanoic acid.

Among them, those containing one or more C2 to C22 fatty acids are preferable as the fatty acid, and those containing C12 fatty acid, C14 fatty acid, C16 fatty acid, C18 fatty acid, C20 fatty acid and C22 fatty acid are more preferable.

The carbon source may include salts and derivatives of these fatty acids and salts of these derivatives. Examples of the salt may include lithium salts, potassium salts and sodium salts.

Examples of the carbon source may also include combinations of carbohydrate such as glucose with the lipid(s), the oil(s), the fats, the fatty acid(s) and glycerin fatty acid(s) ester(s).

Examples of the renewable carbon source may include hydrolyzed biomass carbon sources.

Examples of the biomass carbon source may include cellulose-based substrates such as waste materials of woods, papers and pulps, leafy plants, and fruit pulps; and partial plants such as stalks, grain particles, roots and tubers.

Examples of the plants to be used as the biomass carbon source may include corn, wheat, rye, sorghum, triticale, rice, millet, barley, cassava, legumes such as peas, potato, sweet potato, banana, sugar cane and tapioca.

When the renewable carbon source such as biomass is added to the culture medium, the carbon source is preferably pretreated. Examples of the pretreatment may include an enzymatic pretreatment, a chemical pretreatment, and a combination of the enzymatic pretreatment and the chemical pretreatment.

It is preferred that the renewable carbon source is entirely or partially hydrolyzed before being added to the culture medium.

Examples of the carbon source may also include the yeast extract and a combination of the yeast extract with the other carbon source such as glucose. The combination of the yeast extract with the C1 compound such as carbon dioxide and methanol is preferable.

In the method of culturing the transformant according to the present invention, it is preferable the cell is cultured in a standard medium containing saline and nutrients.

The culture medium is not particularly limited, and examples of the culture medium may include ready-made general media that are commercially available such as Luria Bertani (LB) broth, Sabouraud dextrose (SD) broth, and yeast medium (YM) broth. The medium suitable for the cultivation of the specific host can be selected appropriately for the use.

It is desirable to include appropriate minerals, salts, supplemental elements, buffers, and ingredients known for those skilled in the art to be suitable for the cultivation and to facilitate the production of isoprene in addition to the appropriate carbon source in the cell medium.

A culture condition for the isoprene synthase-expressing microorganism of the present invention is not particularly limited as long as the isoprene formation ability by the isoprene synthase-expressing microorganism can be improved as a result of enhancement in the expression of the pyrophosphate phosphatase, and a standard cell culture condition can be used.

A culture temperature is preferably 20 to 37°C, a gas composition is preferably about 6 to about 84% of CO₂ concentration, and a pH value is preferably about 5 to about 9.

It is preferable that the culturing is performed under an aerobic, oxygen-free, or anaerobic condition depending on a nature of the host cell.

Examples of methods of culturing the transformant include a method using a known fermentation method such as a batch cultivation method, a feeding cultivation method or a continuous cultivation method.

In the batch cultivation method, a medium composition is added at start of the fermentation, the host cell is inoculated in the medium composition and the transformant is cultured while pH and an oxygen concentration are controlled.

In the cultivation of the transformant by the batch cultivation method, the growth of the transformant starts from a mild induction phase, passes through a logarithmic growth phase and finally goes to a stationary phase in which a growth speed is reduced or stopped. Isoprene is produced by the transformant in the logarithmic growth phase and the stationary phase.

In the feeding cultivation method, in addition to the above batch method, the carbon source is gradually added according to the progress of a fermentation process. The feeding cultivation method is effective when an amount of the carbon source is to be restricted in the medium because metabolism of the transformant tends to be reduced due to catabolite suppression. The feed cultivation can be performed using a restricted amount or an excessive amount of the carbon source such as glucose.

In the continuous cultivation method, a certain amount of the medium is continuously supplied to a bioreactor at a constant rate while the same amount of the medium is removed. In the continuous cultivation method, the culture can be kept constantly at high concentration and the transformant in the culture medium is generally in the logarithmic growth phase.

The nutrition can be supplemented by entirely or partly exchanging the medium appropriately, and accumulation of metabolic byproducts that potentially have adverse effects on the growth of the transformant, and the accumulation of dead cells can be prevented.

Examples of the promoter possessed by the expression vector to be introduced into the isoprene synthase-expressing microorganism of the present invention may include the promoters as described above. When the expression vector to be introduced into the isoprene synthase-expressing microorganism of the present invention has the inducible promoter such as a lac promoter, the expression of protein may be induced by, for example, adding IPTG (isopropyl-p-thiogalactopyranoside) into the culture medium.

Examples of the method of evaluating an amount of isoprene monomer produced by culturing the isoprene synthase-expressing microorganism of the present invention may include a method in which a gas phase is collected by a headspace method and this gas phase is analyzed by gas chromatography.

In detail, the isoprene monomer in a headspace which is obtained by culturing the transformant in a sealed vial with shaking the culture medium is analyzed by standard gas chromatography. Then, an area calculated by a curve measured by gas chromatography is converted into the amount of the isoprene monomer produced with the transformant using a standard curve.

Examples of the method of collecting the isoprene monomer obtained by culturing the isoprene synthase-expressing microorganism of the present invention may include gas stripping, fractional distillation, or dissociation of the isoprene monomer adsorbed to a solid phase by heat or vacuum, or extraction with a solvent.

In the gas stripping, isoprene gas is continuously removed from the outgas. Such removal of the isoprene gas can be performed by various methods. Examples of the removal may include adsorption to the solid phase, separation into a liquid phase, and a method in which the isoprene gas is directly condensed.

The isoprene monomer can be collected by a single step or multiple steps. When the isoprene monomer is collected by the single step, the isoprene monomer is converted into the liquid phase simultaneously with separating the isoprene monomer from the outgas. The isoprene monomer can also be directly condensed from the outgas to make the liquid phase. When the isoprene monomer is collected by the multiple stages, the isoprene monomer is separated from off-gas and subsequently converted into the liquid phase. For example, the isoprene monomer is adsorbed to the solid phase, and extracted from the solid phase with the solvent.

Exemplary methods of collecting the isoprene monomer may comprise further purifying the isoprene monomer. Examples of the purification may include separation from a liquid phase extract by distillation and various chromatographic methods.

The present invention provides further a method of producing an isoprene polymer. The method of producing the isoprene polymer according to the present invention comprises the following (I) and (II):
(I) producing an isoprene monomer by the method of the present invention; and
(II) polymerizing the isoprene monomer to form an isoprene polymer.

The step (I) can be performed in the same manner as in the method of producing the isoprene monomer according to the present invention described above. The polymerization of the isoprene monomer in the step (II) can be performed by any method such as addition polymerization known in the art (e.g., synthesis methods in organic chemistry).

The rubber composition produced according to the present invention comprises a polymer derived from isoprene produced by a method for producing isoprene according to the present invention. The polymer derived from isoprene may be a homopolymer (i.e., isoprene polymer) or a heteropolymer comprising isoprene and one or more monomer units other than the isoprene (e.g., a copolymer such as a block copolymer). Preferably, the polymer derived from isoprene is a homopolymer (i.e., isoprene polymer) produced by a method for producing isoprene polymer according to the present invention. The rubber composition produced according to the present invention may further comprise one or more polymers other than the above polymer, one or more rubber components, and/or other components. The rubber composition produced according to the present invention can be manufactured using a polymer derived from isoprene. For example, the rubber composition can be prepared by mixing a polymer derived from isoprene with one or more polymers other than the above polymer, one or more rubber components, and/or other components such as a reinforcing filler, a crosslinking agent, a vulcanization accelerator and an antioxidant.

The tire produced according to the present invention is manufactured using the rubber composition produced according to the present invention. The rubber composition may be applied to any portion of the tire without limitation, which may be selected as appropriate depending on the application thereof. For example, the rubber composition may be used in a tread, a base tread, a sidewall, a side reinforcing rubber and a bead filler of a tire. The tire can be manufactured by a conventional method. For example, a carcass layer, a belt layer, a tread layer, which are composed of unvulcanized rubber, and other members used for the production of usual tires may be successively laminated on a tire molding drum, then the drum may be withdrawn to obtain a green tire. Thereafter, the green tire may be heated and vulcanized in accordance with an ordinary method, to thereby obtain a desired tire (e.g., a pneumatic tire).

### EXAMPLES

Subsequently, the present invention will be described in more detail with reference to Examples, but the present invention is not limited to the following Examples.

### Example 1: Enhancement of ppa gene expression in MG1655 Ptac-KKDyI strain

A strain in which a promoter inherent to an endogenous ppa gene (pyrophosphate phosphatase gene) was substituted with another strong promoter to augment the expression of the endogenous ppa gene in *E. coli* strain was made by the following procedure.

First, competent cells of MG1655 Ptac-KKDyI strain (see Reference Example 7-4. This strain is a transformant of *E. coli*) for electroporation were prepared as follows. Cells of MG1655 Ptac-KKDyI strain were cultured with shaking in 5 mL of LB medium at 37°C overnight. Subsequently, 50 µL of the resulting cultured medium was inoculated to new 5 mL LB medium and cultured with shaking at 37°C until absorbance at OD600 became around 0.6. Then, the microbial cells were collected, washed three times with ice-cooled 10% glycerol, and finally suspended in 0.5 mL of 10% glycerol to use as the competent cells.

Next, pKD46 was introduced into the competent cells of MG1655 Ptac-KKDyI strain by electroporation. The electroporation was carried out under the condition of an electric field intensity of 18 kV/cm, a condenser volume of 25 µF, and a resistance value of 200 Ω using GENE PULSER II (supplied from BioRad). Subsequently, 1 mL of SOC medium (20 g/L of bacto tryptone, 5 g/L of yeast extract, 0.5 g/L of NaCl, 10 g/L of glucose) was added to the microbial cells having pKD46 introduced by the electroporation, the cells were cultured with shaking at 30°C for 2 hours, and then applied onto LB agar medium containing 100 mg/L of ampicillin. After culturing at 30°C overnight, a grown colony was refined on the same agar medium to obtain a strain MG1655 Ptac-KKDI/pKD46.

Competent cells of the obtained strain MG1655 Ptac-KKDI/pKD46 for the electroporation were prepared as follows. Cells of the strain MG1655 Ptac-KKDI/pKD46 were cultured with shaking in 5 mL of LB medium containing 100 mg/L of ampicillin at 30°C overnight. Subsequently, 50 µL of the resulting cultured medium was inoculated to 5 mL of LB medium containing 100 mg/L of ampicillin, and the cells were cultured with shaking at 30°C until absorbance at OD600 became around 0.6. Subsequently the microbial cells were collected, washed three times with ice-cooled 10% glycerol, and then finally suspended in 0.3 mL of 10% glycerol to use as the competent cells.

Next, a gene fragment for substituting a promoter region of the ppa gene on the chromosome was prepared. A nucleotide sequence of the ppa gene and its promoter region are available from existing database (NCBI Reference Sequences NC_000913.2, ppa gene locus tag: b4225, Range: 4447145..4447675, complement). Substitution of the promoter region of the ppa gene was carried out by a λ-red method. A genomic fragment having λattL-Tet-λattR-Ptac was used as a template for PCR. This includes a tac promoter (Ptac), a tetracycline resistant drug marker (Tet) and λattL and λattR that are attachment sites of λ phage. These nucleotide sequences are shown in SEQ ID NO:1. A PCR was carried out using primers consisting of the nucleotide sequences of SEQ ID NO:2 and SEQ ID NO:3. LA-Taq polymerase sold by TaKaRa Bio was utilized as DNA polymerase, and the reaction was carried out under a condition of 92°C for one minute, 40 cycles (92°C for 10 seconds, 54°C for 20 seconds and 72°C for 2 minutes) and 72°C for 5 minutes. A gene fragment where sequences of upstream 60 bp and downstream 60 bp of the promoter region of the ppa gene had been added to each outer side of λattL-Tet-λattR-Ptac, respectively was amplified by PCR above. This gene fragment was purified using Wizard PCR Prep DNA Purification System (supplied from Promega). Hereinafter, the resulting gene fragment was designated as Tet-Ptac-ppa.

Next, Tet-Ptac-ppa was introduced into the competent cells of the strain MG1655 Ptac-KKDYI/pKD46 by the electroporation. The electroporation was carried out under the condition of the electric field intensity of 18 kV/cm, the condenser volume of 25 µF, and the resistance value of 200 Ω using GENE PULSER II (supplied from BioRad). Subsequently, 1 mL of SOC medium was added to the competent cells, which were then cultured with shaking at 30°C for 2 hours, and then applied onto LB agar medium containing 25 mg/L of tetracycline. After culturing at 37°C overnight, a grown colony was refined using the same agar medium. Subsequently, colony PCR was carried out using primers consisting of the nucleotide sequences of SEQ ID NO:4 and SEQ ID NO:5 to confirm that the promoter region of the ppa gene was substituted with the tac promoter. The strain where the promoter region of the ppa gene had been substituted with the tac promoter was designated as a strain MG1655 Ptac-KKDyI Ptac-ppa.

### Example 2: Analysis of PPA expression in MG1655 Ptac-KKDyI Ptac-ppa strain

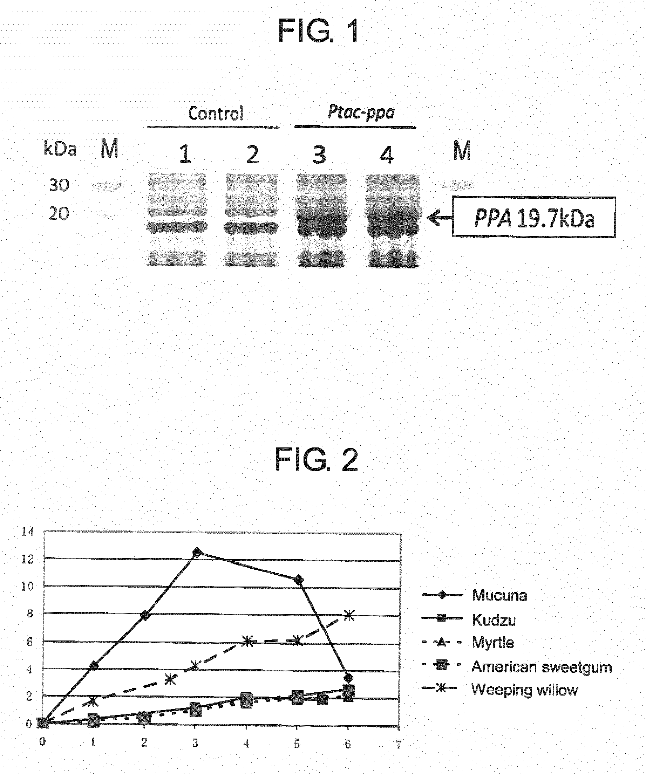

An expression amount of a protein of the pyrophosphate phosphatase (PPA) in MG1655 Ptac-KKDyI Ptac-ppa strain was confirmed by SDS-PAGE. Cells of MG1655 Ptac-KKDyI strain and MG1655 Ptac-KKDyI Ptac-ppa strain were cultured with shaking in 5 mL of LB medium at 37°C overnight. The microbial cells after being collected were washed three times with ice-cooled 50 mM Tris buffer (Tris-HCl, pH 8.0), and disrupted using a sonicator (Bio-ruptor: ON for 30 seconds and OFF for 30 seconds for 20 minutes). The disrupted cell solution was centrifuged at 15,000 rpm for 10 minutes to remove cell debris. The resulting supernatant fraction was used as a soluble protein fraction. The soluble protein fraction was quantified by Bradford method, and 5 µg of the soluble protein was electrophoresed on SDS-PAGE (NuPAGE: SDS-PAGE Gel System supplied from Invitrogen). Subsequently, CBB staining and decoloration were carried out according to standard methods. A photograph of a gel showing bands around a PPA protein mass was shown in FIG. 1. As a result, increase of the expression amount of a protein presumed to be PPA was confirmed in MG1655 Ptac-KKDyI Ptac-ppa strain (FIG. 1). The expression amount of the protein presumed to be PPA in MG1655 Ptac-KKDyI Ptac-ppa strain was estimated to be about 2 to 5 folds larger than that of the original bacterial strain (control) from density of the bands on SDS-PAGE after the electrophoresis.

### Example 3: Construction of MG1655 Ptac-KKDyI Ptac-ppa/pSTV28-Ptac-ispSK/pMW-Para-mvaES strain

Competent cells of MG1655 Ptac-KKDyI Ptac-ppa strain for electroporation were prepared as follows. Cells of MG1655 Ptac-KKDyI Ptac-ppa strain were cultured with shaking in 5 mL of LB medium at 37°C overnight. Subsequently, 50 µL of the resulting cultured medium was inoculated to new 5 mL LB medium and cultured with shaking at 37°C until absorbance at OD600 became around 0.6. Then, the microbial cells were collected, washed three times with ice-cooled 10% glycerol, and finally suspended in 0.5 mL of 10% glycerol to use as the competent cells.

An isoprene synthase-expressing plasmid derived from kudzu, pSTV28-Ptac-ispSK (see Reference Example 3-5) was introduced into the competent cells of MG1655 Ptac-KKDyI Ptac-ppa strain by the electroporation under the above condition. Subsequently, 1 mL of SOC medium was added to the competent cells, which were then cultured at 30°C for 2 hours, and then applied onto LB agar medium containing 60 mg/mL of chloramphenicol. After culturing at 37°C overnight, a grown colony was refined in the same agar medium to obtain MG1655 Ptac-KKDyI Ptac-ppa/pSTV28-Ptac-ispSK strain having introduced pSTV28-Ptac-ispSK.

Subsequently, pMW-Para-mvaES-Ttrp (see Reference Example 7-3) was introduced into MG1655 Ptac-KKDyI Ptac-ppa/pSTV28-Ptac-ispSK strain. As with above, competent cells of MG1655 Ptac-KKDyI Ptac-ppa/pSTV28-Ptac-ispSK strain were prepared, and then pMW-Para-mvaES-Ttrp was introduced by the electroporation under the above condition. Subsequently, 1 mL of SOC medium was added to the competent cells, which were then cultured with shaking at 30°C for 2 hours, and then applied onto LB agar medium containing 60 mg/mL of chloramphenicol and 100 mg/L of kanamycin. After culturing at 37°C overnight, a grown colony was refined in the same agar medium to obtain MG1655 Ptac-KKDyI Ptac-ppa/pSTV28-Ptac-ispSK/pMW-Para-mvaES-Ttrp strain having introduced pMW-Para-mvaES-Ttrp. Hereinafter, MG1655 Ptac-KKDyI Ptac-ppa/pSTV28-Ptac-ispSK/pMW-Para-mvaES-Ttrp strain where the expression of the ppa gene was enhanced is described as a ppa expression-enhanced strain.

### Example 4: Evaluation of jar cultivation of ppa expression-enhanced strain

Jar cultivation of the ppa expression-enhanced strain and a control strain (MG1655 Ptac-KKDyI/pSTV28-Ptac-ispSK/pMW-Para-mvaES-Ttrp) was evaluated. Cells were applied onto the LB agar medium containing 60 mg/mL of chloramphenicol and 100 mg/L of kanamycin, and cultured at 34°C for 16 hours. Subsequently, 0.3 L of glucose medium described in Table 1 was placed in a 1 L volume fermenter, and the microbial cells sufficiently grown on one plate were inoculated thereto and cultured. A culture condition was pH 7.0 (controlled with ammonia gas), 30°C, ventilation of 150 mL/minute, and stirring such that an oxygen concentration in the medium was 5% or higher. After absorbance at OD600 reached around 20, L-arabinose at final concentration of 20 mM was added to the medium, and the cultivation was carried out for 45 hours. During the cultivation, a glucose solution prepared at 500 g/L was appropriately added such that a glucose concentration in the medium was kept at 10 g/L or higher. Evolved gas was collected in a 1 L gas bag with time, and a concentration of isoprene gas contained in the evolved gas was measured. An analysis condition for the isoprene gas was described below. An analysis condition for gas chromatography is the same as described in Reference Example 4-3.

**Table 1. Composition of glucose medium**

| Group A | Final concentration |
|---|---|
| Glucose | 80 g/L |
| MgSO₄. 7aq | 2.0 g/L |
| | |

| Group B | |
|---|---|
| (NH₄)₂SO₄ | 2.0 g/L |
| KH₂PO₄ | 2.0 g/L |
| FeS0₄.7aq | 20 mg/L |
| MnSO₄.5aq | 20 mg/L |
| Yeast Extract | 4.0 g/L |

After preparing 0.15 L of Group A and 0.15 L of Group B, they were heated and sterilized at 115°C for 10 minutes. After cooling, Group A and Group B were mixed, and 60 mg/mL of chloramphenicol and 100 mg/L of kanamycin were added thereto to use as the medium.

An amount of isoprene per jar (mg/B) and a glucose consumption rate (%) after the cultivation for 45 hours in the control strain and the ppa expression-enhanced strain were described in Table 2. Both the amount of isoprene produced per jar (mg/B) and the glucose consumption rate (%) could be confirmed to be higher in the ppa expression-enhanced strain than in the control strain.

**Table 2**

| Strain name | Amount of isoprene produced per jar (mg/B) | Glucose consumption rate (%) |
|---|---|---|
| Control strain | 436 | 2.87 |
| ppa Expression-enhanced strain | 657 | 4.59 |

### Reference Example 1: Evaluation of ability to produce isoprene in plants

### 1-1) Measurement of amount of isoprene formed per unit weight of dry leaves

First, an amount of isoprene formed per 1 g of dry leaves in the plant was measured for evaluating an ability to produce isoprene in plants. Mucuna (*Mucuna bracteata*), Weeping willow (*Salix babylonica*), American sweetgum (*Liquidambar styraciflua*), Myrtle (*Myrtus communis*), and Kudzu (*Pueraria lobata*) were used as the plants.

In the measurement of an amount of formed isoprene, a gas replaceable desiccator (trade name: Vacuum Desiccator, manufactured by AS ONE Corporation) was housed in an incubator (trade name: Growth Chamber MLR-351H, manufactured by SANYO), and the incubator was set to a high temperature induction condition (an illuminance of 100 µmol E/m²/s at 40°C) while a fan for stirring the gas, which was provided in the gas replaceable desiccator, was driven to stir an atmosphere in space in the gas replaceable desiccator. After the temperature of the atmosphere in the gas replaceable desiccator reached 40°C, a plant body of Mucuna planted in a planter was housed therein and kept for 3 hours in a state where the gas replaceable desiccator was sealed. Then, a gas component released from Mucuna was aspirated from the space in the gas replaceable desiccator by an aspiration pump through a silicon tube, an adsorption tube and a gas collection tube. Thereby, water vapor (water content) contained in the gas component released from Mucuna was adsorbed and separated in the adsorption tube, the gas component from which the water vapor had been separated was led to the gas collection tube, and the gas component was collected in the gas collection tube. Subsequently, isoprene contained in the gas component collected in the gas collection tube was quantitatively analyzed using gas chromatograph (trade name: GC-FID6890, manufactured by Agilent).

For the weight of dry leaves, a leaf area of a fresh individual leaf, and a dry weight when the fresh individual leaf is dried by a dryer at 80°C for 8 hours establish a very good positive correlation. Thus, a formula for converting from the leaf area to the dry weight was derived, and the dry weight was estimated from the entire leaf area from the plant body of Mucuna used for the measurement of an amount of formed isoprene.

The amount of formed isoprene per 1 g of the dry leaf was obtained by dividing the amount of formed isoprene from the entire plant body of Mucuna by the estimated weight of the entire plant body.

As a result, it was demonstrated that Mucuna was excellent in amount of formed isoprene per unit weight of the dry leaf (FIG. 2).

### 1-2) Measurement of amount of formed isoprene per amount of total protein

Then, the amount of formed isoprene per amount of total protein extracted from leaves of various plants was measured. Mucuna (samples 1 and 2), Weeping willow, American sweetgum, Myrtle, and Kudzu were used as the plants.

For extraction of the protein, a buffer solution (50 mM Tris-HCl, 20 mM MgCl, 5% glycerol, 0.02% Triton-XlOO, pH 8.0) was made, and 10% Polyclar AT, 20 mM DTT, protease complete tablet (one tablet/50 mL), and 1 mM benzamidine HCl (final concentrations, each) were added just before the use, and was used as a protein extraction buffer. 50 mL of the protein extraction buffer was added to 5 g of the sample, then the mixture was ground well in a cold mortar on ice and filtrated though doubly overlapped Miracloth. A filtrate was centrifuged at 12,000 G for 20 minutes and 40,000 G for 40 minutes to obtain a supernatant, and the supernatant was used as a crude extract.

Subsequently, this crude extract was fractionated with ammonium sulfate. Proteins precipitated in a range of 40% to 55% of final concentrations of ammonium sulfate were centrifuged at 40,000 G for 40 minutes, and an obtained pellet was re-dissolved in the protein extraction buffer to obtain an ammonium sulfate fraction.

A total (ammonium sulfate fraction) protein mass was calculated by measuring the ammonium sulfate fraction using Bradford assay. A Bradford reagent was reacted with the standard protein, bovine serum albumin, and absorbance at a wavelength of 595 nm was measured using a spectrophotometer. A standard curve for the protein was made using the obtained absorbance values. The absorbance at a wavelength of 595 nm was also measured in the ammonium sulfate fraction diluted to 50 times, and the amount of the total (ammonium sulfate fraction) protein was estimated from the standard curve for the standard protein.

In the measurement of the amount of formed isoprene, 100 µL of the crude extract or 100 µL of a crude enzyme solution boiled at 100°C was placed in a 4 mL glass vial, and then 2 µL of a 0.5 M MgCl₂ solution and 5 µL of a 0.2 M DMAPP solution were added thereto. The vial was tightly closed with a screw cap with a septum, and then the vial was gently vortexed and set in an incubator at 40°C. After 0.5, 1 and 2 hours, 0.5 to 2 mL of a gas layer in a headspace was sampled by a gas-tight syringe, and the amount of formed isoprene was measured using gas chromatograph (trade name: GC-FID6890, manufactured by Agilent). The amount of formed isoprene using the crude enzyme after 0.5, 1 and 2 hours was calculated by subtracting a measured value in the case of using the crude enzyme solution boiled at 100°C from a measured value in the case of using the crude enzyme. An enzymatic activity per 1 mg of the total protein (specific activity) was calculated from the amount of the formed isoprene per one hour. The amount of formed isoprene was measured with keeping the amount of DMAPP that was the substrate of the isoprene synthase constant.

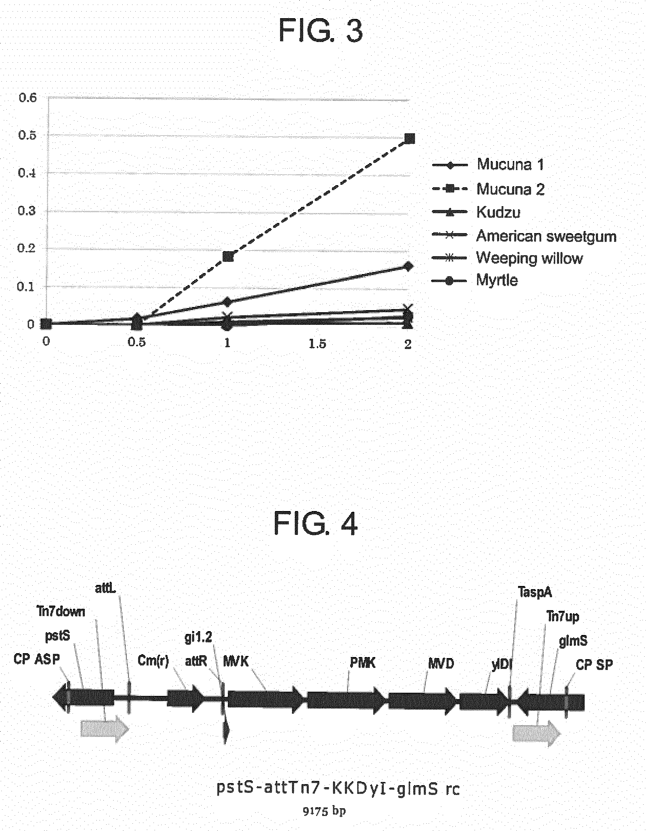

As a result, it was demonstrated that Mucuna was excellent in amount of formed isoprene per amount of total protein (FIG. 3, Table 3). As described above, it was shown that Mucuna was excellent in ability to produce isoprene.

**Table 3. Amount of formed isoprene per amount of total protein (index numbers relative to case of Kudzu)**

| | 0 hour* | 0.5 hour* | 1 hour* | 2 hours* | Specific activity index (Value from Kudzu was set to 1) |
|---|---|---|---|---|---|
| Mucuna 1 | 0 | 16.947 | 61.895 | 160.632 | 16.87842808 |
| Mucuna 2 | 0 | 0 | 183.587 | 449.514 | 47.23274141 |
| American sweetgum | 0 | 0 | 22.063 | 46.132 | 4.847325838 |
| Weeping willow | 0 | 0 | 9.756 | 24.39 | 2.562782389 |
| Myrtle | 0 | 0 | 0 | 27.451 | 2.884417358 |
| Kudzu | 0 | 0 | 6.662 | 9.517 | 1 |

| | | | | | |
|---|---|---|---|---|---|
| *Unit is µg isoprene/mg protein | | | | | |

### Reference Example 2: Cloning of isoprene synthase gene derived from Mucuna

### 2-1) Evaluation of sampling time

Isoprene gas released from leaves of Mucuna illuminated with light for 1, 2, 3 and 5 hours at temperature of 40°C was sampled and the amount of produced isoprene was quantified by gas chromatography described later, and production of 4, 8, 12 and 10 µg of isoprene/g DW leaf was confirmed. Thus, it was confirmed that an optimal light illumination time was 3 hours.

### 2-2) Extraction of total RNA lysis solution

A total RNA was extracted from leaves of Mucuna with total RNA lysis solution according to the following procedures.
(1) The leaves of Mucuna illuminated with light for 3 hours at temperature of 40°C were sampled.
(2) 100 mg of leaf tissue was pulverized in a mortar with rapidly freezing the leaf tissue with liquid nitrogen, then the leaf tissue together with the liquid nitrogen was dispensed in an RNA-free 2 mL Eppendorf tube, and the liquid nitrogen was gasified.
(3) To this Eppendorf tube, 450 µL of a dissolution buffer RLT (containing 2-mercaptoethanol) attached to RNeasy Plant Kit (manufactured by Qiagen), and mixed vigorously with Vortex to obtain a leaf tissue lysate.
(4) This leaf tissue lysate was applied to QIAshredder spin column attached to RNeasy Plant Kit, and centrifuged at 15,000 rpm for 2 minutes.
(5) A supernatant alone of a column eluate was transferred to a new RNA-free 2 mL Eppendorf tube, then special grade ethanol in a half volume of the supernatant was added to the supernatant, and the obtained solution was mixed by pipetting to obtain about 650 µL of a solution.
(6) This solution was applied to RNeasy spin column attached to RNeasy Plant Kit, centrifuged at 10,000 rpm for 15 seconds, and a filtrate was discarded.
(7) 700 µL of RW1 buffer attached to RNeasy Plant Kit was added to this RNeasy spin column, centrifuged at 10,000 rpm for 15 seconds, and a filtrate was discarded.
(8) 500 µL of BPE buffer attached to RNeasy Plant Kit was added to this RNeasy spin column, centrifuged at 10,000 rpm for 15 seconds, and a filtrate was discarded.
(9) 500 µL of BPE buffer was again added to this RNeasy spin column, centrifuged at 10,000 rpm for 2 minutes, and a filtrate was discarded.
(10) This RNeasy spin column was set to a 2 mL collective tube attached to RNeasy Plant Kit, centrifuged at 15,000 rpm for one minute, and a filtrate was discarded.
(11) This RNeasy spin column was set to a 1.5 mL collective tube attached to RNeasy Plant Kit.
(12) RNA-free distilled water attached to RNeasy Plant Kit was directly added to a membrane of this RNeasy spin column using a Pipetman, centrifuged at 10,000 rpm for one minute, and total RNA was collected. This step was repeated twice to obtain about 100 µg of total RNA.

### 2-3) Analysis of nucleotide sequence of isoprene synthase gene derived from Mucuna

Quality of RNA in the extracted total RNA solution was checked using nano-chips for RNA provided by BioAnalyzer (Agilent Technologies, Inc.), and it was confirmed that the solution was not contaminated with genomic DNA and RNA was not decomposed in the solution.

This total RNA was converted into a double strand using reverse transcriptase, and then fragmented using a nebulizer. Nucleotide sequences of 198,179 fragments having a poly A sequence at a 3' end were analyzed using 454 titanium FLX high performance sequencer (manufactured by Roche Applied Science). Overlapped sequences in the obtained fragment sequences were aligned to obtain 13,485 contig sequences. BLAST search was performed for these contig sequences, and 6 contig sequences having the homology (identity of nucleotide sequences) to registered and known isoprene synthase gene sequences from Kudzu and Poplar were extracted. These sequences were further analyzed in detail, and 3 sequences in these 6 contig sequences were found to be derived from the same gene. Thus, a partial sequence of the isoprene synthase gene derived from Mucuna was obtained. 5' RACE was performed based on this partial sequence to obtain a full length nucleotide sequence of the isoprene synthase cDNA derived from Mucuna, which was represented by SEQ ID NO:6.

### Reference Example 3: Preparation of expression plasmid for isoprene synthase derived from various plants

### 3-1) Chemical synthesis of isoprene synthase derived from Pueraria montana var. lobata (Kudzu)

The nucleotide sequence and the amino acid sequence of the isoprene synthase cDNA derived from *Pueraria montana var. lobata* were already known (ACCESSION: AAQ84170: P. *montana var. lobata* isoprene synthase (IspS)). The amino acid sequence of the IspS protein derived from *P. montana* and the nucleotide sequence of its cDNA are represented by SEQ ID NO:8 and SEQ ID NO:9, respectively. The IspS gene was optimized for codon usage frequency in *E. coli* in order to efficiently express the IspS gene in *E. coli,* and further designed to cut off the chloroplast localization signal. The designed gene was designated as IspSK. A nucleotide sequence of IspSK is represented by SEQ ID NO:10. The IspSK gene was chemically synthesized, then cloned into pUC57 (manufactured by GenScript), and the resulting plasmid was designated as pUC5-IspSK.

### 3-2) Chemical synthesis of isoprene synthase derived from Populus alba x Populus tremula (Poplar)

The nucleotide sequence of the isoprene synthase cDNA and the amino acid sequence of the isoprene synthase derived from *P. alba* x *P. tremula* were already known (ACCESSION: CAC35696: *P. alba x P. tremula* (Poplar) isoprene synthase). The amino acid sequence of the IspS protein derived from *P. alba x P. tremula* and the nucleotide sequence of its cDNA are represented by SEQ ID NO:11 and SEQ ID NO:12, respectively. An IspS gene that was optimized for the codon usage frequency in *E. coli* in the same manner as above and in which the chloroplast localization signal was cut off was designed and designated as IspSP. A nucleotide sequence of IspSP is represented by SEQ ID NO:13. The IspSP gene was chemically synthesized, then cloned into pUC57 (manufactured by GenScript), and the resulting plasmid was designated as pUC57-IspSP.

### 3-3) Chemical synthesis of isoprene synthase derived from Mucuna bracteata (Mucuna)

Based on the nucleotide sequence of the isoprene synthase cDNA derived from *Mucuna bracteata,* an IspS gene that was optimized for the codon usage frequency in *E. coli* was designed in the same manner as above. One in which the chloroplast localization signal had been conferred was designated as IspSM (L), and one in which the chloroplast localization signal had been cut off was designated as IspSM. Nucleotide sequences for IspSM (L) and IspSM are represented by SEQ ID NO:14 and SEQ ID NO:15, respectively. The IspSM gene and the IspSM (L) gene were chemically synthesized, then cloned into pUC57 (manufactured by GenScript), and the resulting plasmids were designated as pUC57-IspSM and pUC57-IspSM (L).

### 3-4) Construction of expression plasmid, pSTV28-Ptac-Ttrp

An expression plasmid pSTV28-Ptac-Ttrp for expressing IspS derived from various plants in *E. coli* was constructed. First, a DNA fragment comprising a tac promoter (synonym: Ptac) region (deBoer, et al.,(1983) Proc. Natl. Acad. Sci. U.S.A., 80, 21-25) and a terminator region of tryptophan operon (synonym: Ttrp) derived from *E. coli* (Wu et al., (1978) Proc. Natl. Acad. Sci. U.S.A., 75, 442-5446) and having a *Kpn*I site at a 5' terminus and a *BamH*I site at a 3' end was synthesized chemically (the nucleotide sequence of Ptac-Ttrp is represented by SEQ ID NO:16). The resulting Ptac-Ttrp DNA fragment was digested with *Kpn*I and *BamH*I, and ligated to pSTV28 (manufactured by Takara Bio Inc.) similarly digested with *Kpn*I and *BamH*I by a ligation reaction with DNA ligase. The resulting plasmid was designated as pSTV28-Ptac-Ttrp (its nucleotide sequence is represented by SEQ ID NO:17). This plasmid can amplify the expression of the IspS gene by cloning the IspS gene downstream of Ptac.

### 3-5) Construction of plasmid for expressing IspS gene derived from various plants

Plasmids for expressing the IspSK gene, the IspSP gene, the IspSM gene and the IspSM (L) gene in *E. coli* were constructed by the following procedure. PCR was performed with Prime Star polymerase (manufactured by Takara Bio Inc.) using synthesized oligonucleotides consisting of the nucleotide sequences of SEQ ID NOs:18 and 19 as primers with pUC57-IspSK as a template, synthesized oligonucleotides consisting of the nucleotide sequences of SEQ ID NOs:20 and 21 as primers with pUC57-IspSP as a template, synthesized oligonucleotides consisting of the nucleotide sequences of SEQ ID NOs:22 and 23 as primers with pUC57-IspSM as a template, or further synthesized oligonucleotides consisting of the nucleotide sequences of SEQ ID NOs:24 and 25 as primers with pUC57-IspSM (L) as a template. A reaction solution was prepared according to a composition attached to the kit, and a reaction at 98°C for 10 seconds, 54°C for 20 seconds and 68°C for 120 seconds was performed in 40 cycles. As a result, a PCR product containing the IspSK gene, the IspSP gene, the IspSM gene or the IspSM (L) gene was obtained. Likewise, PCR was performed with Prime Star polymerase (manufactured by Takara Bio Inc.) using synthesized oligonucleotides consisting of the nucleotide sequences of SEQ ID NOs:26 and 27 as primers with pSTV28-Ptac-Ttrp as a template. A reaction solution was prepared according to a composition attached to the kit, and a reaction at 98°C for 10 seconds, 54°C for 20 seconds and 68°C for 210 seconds was performed in 40 cycles. As a result, a PCR product containing pSTV28-Ptac-Ttrp was obtained. Subsequently, the purified IspSK gene, IspSP gene, IspSM gene, and IspSM (L) gene fragments were ligated to the PCR product for pSTV28-Ptac-Ttrp using In-Fusion HD Cloning Kit (manufactured by Clontech). The resulting plasmids for expressing the IspSK gene, the IspSP gene, IspSM gene and IspSM (L) gene were designated as pSTV28-Ptac-IspSK, pSTV28-Ptac-IspSP, pSTV28-Ptac-IspSM, and pSTV28-Ptac-IspSM (L), respectively.

**Table 4. Primer sequences used for construction of plasmids for expressing IspS genes derived from various plants**

| Subject for amplification | Sequence name | Sequence (5'-) |
|---|---|---|
| IspSK | Ptac-IspS(K)F | |
| IspSK | IspS(K)R-MCSR | |
| IspSP | Ptac-IspS(P)F | |
| IspSP | IspS(P)R-MCSR | |
| IspSM | Ptac-IspS(M)F | |
| IspSM | IspS(M)R-MCSR | ACGGCCAGTGAATTCTTAGTTAATCGGGAACGGGT (SEQ ID NO:23) |
| IspSM(L) | Ptac-IspS(M(L))F | |
| IspSM(L) | IspS(M(L))R-MCSR | ACGGCCAGTGAATTCTCAGTTAATCGGGAACGGGT (SEQ ID NO:25) |
| pSTV28-Ptac-Ttrp | pSTV28-F | GTGTGAAATTGTTATCCGCTCACAATTCC (SEQ ID NO:26) |
| pSTV28-Ptac-Ttrp | pSTV28-R | GAATTCACTGGCCGTCGTTTTACAACG (SEQ ID NO:27) |

### Reference Example 4: Measurement of enzymatic activity of isoprene synthase derived from various plants using crude enzyme extract derived from E. coli

### 4-1) Construction of E. coli MG1655 strain having ability to produce isoprene

Competent cells of *E. coli* MG1655 strain (ATCC 700926) were prepared, and then pSTV28-Ptac-Ttrp, pSTV28-Ptac-IspSK, pSTV28-Ptac-IspSP, pSTV28-Ptac-IspSM, or further pSTV28-Ptac-IspSM (L) was introduced therein by an electroporation method. A suspension of the cells was evenly applied onto an LB plate containing 60 mg/L of chloramphenicol, and cultured at 37°C for 18 hours. Subsequently, transformants that were resistant to chloramphenicol were obtained from the resulting plate. A strain in which pSTV28-Ptac-Ttrp, pSTV28-Ptac-IspSK, pSTV28-Ptac-IspSP, pSTV28-Ptac-IspSM, or further pSTV28-Ptac-IspSM (L) was introduced into *E. coli* MG1655 strain were designated as MG1655/pSTV28-Ptac-Ttrp, MG1655/pSTV28-Ptac-IspSK, MG1655/pSTV28-Ptac-IspSP, MG1655/pSTV28-Ptac-IspSM, or further MG1655/pSTV28-Ptac-IspSM (L) strain, respectively.

### 4-2) Method of preparing crude enzyme extract

Microbial cells of MG1655/pSTV28-Ptac-Ttrp, MG1655/pSTV28-Ptac-IspSK, MG1655/pSTV28-Ptac-IspSP, MG1655/pSTV28-Ptac-IspSM, or MG1655/pSTV28-Ptac-IspSM (L) strain were evenly applied onto the LB plate containing 60 mg/L of chloramphenicol, and cultured at 37°C for 18 hours. The microbial cells corresponding to 1/6 of the resulting plate were inoculated to a Sakaguchi flask in which 20 mL of LB containing 60 mg/L of chloramphenicol had been added, and cultured at 37°C for 6 hours. The microbial cells from the culture medium were centrifuged at 5000 rpm at 4°C for 5 minutes, and washed twice with ice-cold isoprene synthase buffer (50 mM Tris-HCl, pH 8.0, 20 mM MgCl₂, 5% glycerol). The washed microbial cells were suspended in 1.8 mL of the same buffer. About 0.9 mL of beads for disruption (YBG01, diameter 0.1 mm) and 0.9 mL of the microbial cell suspension were placed in a 2 mL tube specific for a multibead shocker, and the microbial cells were disrupted using the multibead shocker manufactured by Yasui Kikai Corporation at 2500 rpm at 4°C for 3 cycles of ON for 30 seconds/OFF for 30 seconds. After the disruption, the tube was centrifuged at 20,000 g at 4°C for 20 minutes, and a supernatant was used as a crude enzyme extract.

### 4-3) Measurement of isoprene synthase activity

The crude enzyme extract from MG1655/pSTV28-Ptac-Ttrp, MG1655/pSTV28-Ptac-IspSK, MG1655/pSTV28-Ptac-IspSP, MG1655/pSTV28-Ptac-IspSM, or MG1655/pSTV28-Ptac-IspSM (L) strain (containing 2 mg as amount of total protein) together with the isoprene buffer in a total volume of 0.5 mL was placed in a headspace vial (22 mL CLEAR CRIMP TOP VIAL (cat #B0104236) manufactured by Perkin Elmer), then 0.025 mL of a 0.5 M MgCl₂ solution and 0.01 mL of a 0.2 M DMAPP (manufactured by Cayman, catalog No. 63180) solution were added thereto, and the mixture was lightly vortexed. Then immediately, the vial was tightly sealed with a cap with a butyl rubber septum for the headspace vial (CRIMPS (cat #B0104240) manufactured by Perkin Elmer), and kept at 37°C for 2 hours.

After completion of the reaction, a concentration of isoprene in the headspace of the vial was measured by gas chromatography. An analysis condition for the gas chromatography will be described below.

Headspace sampler (manufactured by Perkin Elmer, Turbo Matrix 40)
Temperature for keeping vial warm: 40°C
Time period for keeping vial warm: 30 minutes
Pressurization time: 3.0 minutes
Injection time: 0.02 minute
Needle temperature: 70°C
Transfer temperature: 80°C
Carrier gas pressure (high purity helium): 124 kPa
Gas chromatography (manufactured by Shimadzu Corporation, GC-2010 Plus AF)
Column (Rxi (registered trademark)-1 ms: length 30 m, internal diameter 0.53 mm, liquid phase film thickness 1.5 µm, cat #13370)
Column temperature: 37°C
Pressure: 24.8 kPa
Column flow: 5 mL/minute
Influx method: Split 1:0 (actually measured 1:18) Transfer flow: 90 mL
GC injection volume: 1.8 mL (transfer flow × injection time)
Injection volume of sample into column: 0.1 mL
Inlet temperature: 250°C
Detector: FID (hydrogen 40 mL/minute, air 400 mL/minute, makeup gas helium 30 mL/minute)
Detector temperature: 250°C

### Preparation of isoprene standard sample

A reagent isoprene (specific gravity 0.681) was diluted to 10, 100, 1000, 10000 and 100000 times with cold methanol to prepare standard solutions for addition. Subsequently, 1 µL of each standard solution for addition was added to a headspace vial in which 1 mL of water had been added, and used as a standard sample.

The amount of formed isoprene after the reaction of each microbial strain for 2 hours is described in Table 5.

**Table 5. Amount of formed isoprene after reaction for 2 hours**

| Name of microbial strain | Amount of formed isoprene (mg/L) |
|---|---|
| MG1655/pSTV28-Ptac-Ttrp | 0.10 ± 0.01 |
| MG1655/pSTV28-Ptac-IspSK | 0.45 ± 0.02 |
| MG1655/pSTV28-Ptac-IspSM | 28.93 ± 6.04 |
| MG1655/pSTV28-Ptac-IspSM(L) | 5.06 ± 0.13 |
| MG1655/pSTV28-Ptac-IspSP | 0.10 ± 0.01 |

From the result in Table 5, the amount of formed isoprene was larger in order of MG1655/pSTV28-Ptac-IspSM, MG1655/pSTV28-Ptac-IspSM (L) and MG1655/pSTV28-Ptac-IspSK strains, and was almost equal in MG1655/pSTV28-Ptac-IspSP and MG1655/pSTV28-Ptac-Ttrp strains. From the above result, the crude enzyme extract from the strain introduced with the isoprene synthase derived from Mucuna exhibited the highest activity to form isoprene.

### Reference Example 5: Effects of introduction of isoprene synthase derived from various plants on E. coli MG1655 strain

From the result of the crude enzymatic activity in Reference Example 4, the highest activity was confirmed in the isoprene synthase derived from Mucuna that deleted the chloroplast localization signal. Thus, an ability to produce isoprene from glucose was compared in all isoprene synthase-introduced strains in which the chloroplast localization signal had been deleted. Microbial cells of MG1655/pSTV28-Ptac-Ttrp, MG1655/pSTV28-Ptac-IspSK, MG1655/pSTV28-Ptac-IspSP, or MG1655/pSTV28-Ptac-IspSM strain were evenly applied onto the LB plate containing 60 mg/L of chloramphenicol, and cultured at 37°C for 18 hours. One loopful of the microbial cells from the resulting plate was inoculated to 1 mL of M9 glucose medium in a headspace vial. The vial was tightly sealed with the cap with the butyl rubber septum for the headspace vial (CRIMPS (cat #B0104240) manufactured by Perkin Elmer), and the microbial cells were cultured at 30°C for 24 hours using a reciprocal shaking cultivation apparatus (120 rpm). A composition of the M9 glucose medium is as described in Table 6.

**Table 6. Composition of M9 glucose medium**

| | |
|---|---|
| Glucose | 1.0 g/L |
| Na₂HPO₄ | 6.0 g/L |
| KH₂PO₄ | 3.0 g/L |
| NaCl | 0.5 g/L |
| NH₄Cl | 1.0 g/L |
| 1 M MgSO₄ (autoclaved) | 1.0 mL |
| 1 M CaCl₂ (autoclaved) | 0.1 mL |

Further, chloramphenicol was added at a final concentration of 60 mg/L. The volume was adjusted to 1 L and the medium was then sterilized by filtration.

After completion of the cultivation, the concentration of isoprene in the headspace in the vial was measured by the gas chromatography. An OD value was also measured at 600 nm using a spectrophotometer (HITACHI U-2900). The concentration of isoprene and the OD value in each microbial strain at the time of completing the cultivation are described in Table 7.

**Table 7. OD value, and amount (µg/L) of isoprene produced by MG1655/pSTV28-Ptac-Ttrp, MG1655/pSTV28-Ptac-IspSK, MG1655/pSTV28-Ptac-IspSP and MG1655/pSTV28-Ptac-IspSM strains at the time of completing cultivation**

| Name of microbial strain | OD value | Amount (µg/L) of formed isoprene |
|---|---|---|
| MG1655/pSTV28-Ptac-Ttrp | 1.68±0.04 | ND |
| MG1655/pSTV28-Ptac-IspSK | 1.60±0.09 | 43±6 |
| MG1655/pSTV28-Ptac-IspSM | 1.45±0.03 | 56±7 |
| MG1655/pSTV28-Ptac-IspSP | 1.59±0.07 | 26±3 |

From the results in Table 7, it was found that the amount of produced isoprene was larger in order of MG1655/pSTV28-Ptac-IspSM, MG1655/pSTV28-Ptac-IspSK, MG1655/pSTV28-Ptac-IspSP and MG1655/pSTV28-Ptac-Ttrp strains. From the above results, the strain introduced with the isoprene synthase derived from Mucuna exhibited the highest activity to produce isoprene in the wild strains.

### Reference Example 6: Effects of introduction of isoprene synthase derived from various plants on E. coli MG1655 strain in which MEP (methylerythritol) pathway is enhanced 6-1) Construction of plasmid for expressing dxs gene (pMW219-dxs)

It was already reported that the amount of formed isoprene was enhanced (Appl. Microbiol. Biotechnol., (2011) 90, 1915-1922), when the expression of a dxs (1-deoxy-D-xylulose-5-phosphate synthase) gene that constitutes the MEP pathway was enhanced in *E. coli* strain in which the isoprene synthase was introduced. Thus, it was confirmed whether an ability to produce isoprene was also different due to an origin of the isoprene synthase in the strain in which the expression of the dxs gene was enhanced. The entire genomic nucleotide sequence of *E. coli* K-12 strain was already shown (GenBank Accession No. U00096) (Science, (1997) 277, 1453-1474). pMW219 (manufactured by Nippon Gene Co., Ltd.) was used for amplifying the gene. This plasmid can increase an expression level of an objective gene when isopropyl-β-thiogalactopyranoside (IPTG) is added by introducing the objective gene into a multicloning site. Synthesized oligonucleotides were synthesized from the nucleotide sequences of SEQ ID NOs:28 and 29 based on the nucleotide sequence of the dxs gene in the genomic nucleotide sequence of *E*. *coli.* Subsequently, PCR was performed with Prime Star polymerase (manufactured by Takara Bio Inc.) using the synthesized oligonucleotides consisting of the nucleotide sequences of SEQ ID NOs:28 and 29 as the primers with MR1655 strain genomic DNA as the template. A reaction solution was prepared according to the composition attached to the kit, and a reaction at 98°C for 10 seconds, 54°C for 20 seconds and 68°C for 120 seconds was performed in 40 cycles. As a result, a PCR product containing the dxs gene was obtained. Likewise, PCR was performed with Prime Star polymerase (manufactured by Takara Bio Inc.) using the synthesized oligonucleotides consisting of the nucleotide sequences of SEQ ID NOs:30 and 31 as the primers with pMW219 as the template. A reaction solution was prepared according to the composition attached to the kit, and a reaction at 98°C for 10 seconds, 54°C for 20 seconds and 68°C for 240 seconds was performed in 40 cycles. As a result, a PCR product containing pMW219 was obtained. Subsequently, the purified dxs gene fragment was ligated to the PCR product of pMW219 using In-Fusion HD Cloning Kit (manufactured by Clontech). The resulting plasmid for expressing the dxs gene was designated as pMW219-dxs.

**Table 8. Primer sequences used for construction of plasmid for expressing dxs gene**

| Sequence name | Sequence (5'-) |
|---|---|
| *dxs*-F | CAGGAAACAGCTATGAGTTTTGATATTGCCAAATACCCGAC (SEQ ID NO:28) |
| *dxs*-R | GCTGCCACTCCTGCTATACTCGTCATAC (SEQ ID NO:29) |
| pMW219-F | CATAGCTGTTTCCTGTGTGAAATTGTTATC (SEQ ID NO:30) |
| pMW219-R | AGCAGGAGTGGCAGCGAATTCGAGCTCGGTACCCGGGGAT (SEQ ID NO:31) |

### 6-2) Introduction of pMW219-dxs into E. coli MG1655 strain having ability to produce isoprene

Competent cells of MG1655/pSTV28-Ptac-Ttrp, MG1655/pSTV28-Ptac-IspSK, MG1655/pSTV28-Ptac-IspSM, or further MG1655/pSTV28-Ptac-IspSP strain were prepared, and pMW219-dxs was introduced therein by an electroporation method. The cells were evenly applied onto the LB plate containing 60 mg/L of chloramphenicol and 50 mg/L of kanamycin hydrochloride, and the cells were cultured at 37°C for 18 hours. Transformants that were resistant to chloramphenicol and kanamycin were obtained from the resulting LB plates. Strains in which pMW219-dxs had been introduced into MG1655/pSTV28-Ptac-Ttrp, MG1655/pSTV28-Ptac-IspSK, MG1655/pSTV28-Ptac-IspSM, or further MG1655/pSTV28-Ptac-IspSP strain were designated as MG1655/pSTV28-Ptac-Ttrp/pMW219-dxs, MG1655/pSTV28-Ptac-IspSK/pMW219-dxs, MG1655/pSTV28-Ptac-IspSM/pMW219-dxs, or further MG1655/pSTV28-Ptac-IspSP/pMW219-dxs strain, respectively.

### 6-3) Effects of introduction of isoprene synthase derived from various plants on E. coli MG1655 strain in which expression of DXS is enhanced

MG1655/pSTV28-Ptac-Ttrp/pMW219-dxs, MG1655/pSTV28-Ptac-IspSK/pMW219-dxs, MG1655/pSTV28-Ptac-IspSM/pMW219-dxs, or further MG1655/pSTV28-Ptac-IspSP/pMW219-dxs strain were evenly applied onto the LB plate containing 60 mg/L of chloramphenicol and 50 mg/L of kanamycin hydrochloride, and were cultured at 37°C for 18 hours. Subsequently, the cultivation in the headspace vial was evaluated as described in Reference Example 5. The amount (µg/L) of produced isoprene and the OD value upon completion of the cultivation are described in Table 9.

**Table 9. Amount (µg/L) of produced isoprene and OD value when the cultivation was completed in various strains having enhanced isoprene synthase which are prepared from E. coli MG1655 strain having enhanced DXS as host**

| Name of microbial strain | OD value | Amount (µg/L) of produced isoprene |
|---|---|---|
| MG1655/pSTV28-Ptac-Ttrp/pMW219-dxs | 1.46±0.04 | ND |
| MG1655/pSTV28-Ptac-IspSK/pMW219-dxs | 1.13±0.02 | 101±28 |
| MG1655/pSTV28-Ptac-IspSM/pMW219-dxs | 1.76±0.06 | 126±23 |
| MG1655/pSTV28-Ptac-IspSP/pMW219-dxs | 2.21±0.12 | 42±17 |

From the results in Table 9, the amount of produced isoprene was larger in order of MG1655/pSTV28-Ptac-IspSM/pMW219-dxs, MG1655/pSTV28-Ptac-IspSK/pMW219-dxs, MG1655/pSTV28-Ptac-IspSP/pMW219-dxs and MG1655/pSTV28-Ptac-Ttrp/pMW219-dxs strains. From the above results, the strain introduced with the isoprene synthase derived from Mucuna also exhibited the highest ability to produce isoprene in the MEP pathway-enhanced strains.

### Reference Example 7: Effects of introduction of isoprene synthase derived from various plants on E. coli MG1655 strain in which MVA (mevalonate) pathway is introduced 7-1) Cloning gene downstream of mevalonate pathway which is derived from yeast

A downstream region of the mevalonate pathway was obtained from *Saccharomyces cerevisiae* (WO2009076676, *Saccharomyces* Genome database http://www.yeastgenome.org/# Nucleic Acids Res., Jan 2012; 40: D700-D705). An ERG12 gene encoding mevalonate kinase, an ERG8 gene encoding phosphomevalonate kinase, an ERG19 gene encoding diphosphomevalonate decarboxylase, and an IDI1 gene encoding isopentenyl-diphosphate delta isomerase were amplified by PCR with genomic DNA of S. *cerevisiae* as the template using the primer shown below (Table 10). Prime Star Max Premix sold by Takara Bio Inc. was used for a PCR enzyme, and the reaction was performed at 98°C for 2 minutes and for 30 cycles of 98°C for 10 seconds, 55°C for 5 seconds and 72°C for 5 seconds/kb. Cloning and construction of an expression vector were performed by introducing the PCR fragment into the pSTV28-Ptac-Ttrp vector (SEQ ID NO:17) treated with the restriction enzyme *Sma*I by an in-fusion cloning method. *E. coli* DH5α was transformed with the expression vector, clones having assumed sequence length from each gene were selected, a plasmid was extracted according to standard methods, and its sequence was confirmed. The nucleotide sequences of these amplified genes and the amino acid sequences of the enzymes encoded by these genes are available on *Saccharomyces* Genome database http://www.yeastgenome.org/#.

**Table 10. Primer sequences used for cloning of genes downstream of mevalonate pathway**

| Amplified gene | Sequence name | Sequence (5'-) |
|---|---|---|
| ERG12 | MVK-IFS_5742-33-1 | |
| ERG12 | MVK-IFA_5742-33-2 | |
| ERG8 | PMK-IFS_5742-33-3 | |
| ERG8 | PMK-IFA_5742-33-4 | |
| ERG19 | MVD-IFS_5742-33-5 | |
| ERG19 | MVD-IFA_5742-33-6 | |
| IDI1 | yIDI-IFS_5742-33-7 | |
| IDI1 | yIDI-IFA_5742-33-8 | |

### 7-2) Construction of artificial operon downstream of mevalonate pathway

A sequence in which the gene encoding the mevalonate kinase and the gene encoding the phosphomevalonate kinase were arranged in straight was constructed by the in-fusion cloning method. The ERG12 gene encoding the mevalonate kinase and the ERG8 gene encoding the phosphomevalonate kinase were amplified by PCR with genomic DNA from *Saccharomyces cerevisiae* as the template using the primers shown in Table 11. KOD plus sold by Toyobo was used for the PCR enzyme, and the reaction was performed at 94°C for 2 minutes and for 30 cycles of 94°C for 15 seconds, 45°C for 30 seconds and 68°C for 1 minute/kb. The cloning and the construction of an expression vector were performed by inserting the PCR fragment into pUC118 vector treated with the restriction enzyme *Sma*I by the in-fusion cloning method. *E*. *coli* JM109 was transformed with the expression vector, clones having assumed sequence length of each gene were selected, a plasmid was extracted according to standard methods, and its sequence was confirmed. The produced plasmid was designated as pUC-mvk-pmk. The nucleotide sequence of pUC-mvk-pmk is represented by SEQ ID NO:40.

**Table 11. Primer sequences used for ligating mevalonate kinase and phosphomevalonate kinase**

| Amplified gene | Sequence name | Sequence (5'-) |
|---|---|---|
| ERG12 | KKS1-6038-2-1 | TCGAGCTCGGTACCCATGTCATTACCGTTCTTAACTTCT (SEQ ID NO:41) |
| ERG12 | KKA1-6038-2-2 | |
| ERG8 | KKS2-6083-2-3 | |
| ERG8 | KKA2-6083-2-4 | CTCTAGAGGATCCCCTTATTTATCAAGATAAGTTTCCGG (SEQ ID NO:44) |

A sequence in which a gene encoding diphosphomevalonate decarboxylase and a gene encoding isopentenyl-diphosphate delta isomerase were arranged in straight was constructed by the in-fusion cloning method. The ERG19 gene encoding the diphosphomevalonate decarboxylase and the IDI1 gene encoding the isopentenyl-diphosphate delta isomerase were amplified by PCR with genomic DNA of *Saccharomyces cerevisiae* as the template using the primers shown in Table 12. KOD plus sold by Toyobo was used for the PCR enzyme, and the reaction was performed at 94°C for 2 minutes and for 30 cycles of 94°C for 15 seconds, 45°C for 30 seconds and 68°C for 1 minute/kb, and then at 68°C for 10 minutes. The cloning and the construction of an expression vector were performed by inserting the PCR fragment into TWV228 vector treated with the restriction enzyme *Sma*I by the in-fusion cloning method. *E. coli* DH5α was transformed with the expression vector, clones having assumed sequence length of each gene were selected, a plasmid was extracted according to standard methods, and its sequence was confirmed. The produced plasmid was designated as pTWV-dmd-yidi. The nucleotide sequence of pTWV-dmd-yidi is represented by SEQ ID NO:45.

**Table 12. Primer sequences used for ligating diphosphomevalonate decarboxylase and isopentenyl-diphosphate delta isomerase**

| Amplified gene | Sequence name | Sequence (5'-) |
|---|---|---|
| ERG19 | DyIS1-6083-2-5 | TCGAGCTCGGTACCCATGACCGTTTACACAGCATCC (SEQ ID NO:46) |
| ERG19 | DyIA1-6083-2-6 | |
| IDI1 | DyIS2-6083-2-7 | |
| IDI1 | DyIA2-6083-2-8 | |

A sequence in which the gene encoding the mevalonate kinase, the gene encoding the phosphomevalonate kinase, the gene encoding the diphosphomevalonate decarboxylase and the gene encoding the isopentenyl-diphosphate delta isomerase were arranged in straight was constructed by the in-fusion cloning method. An expression vector in which these four enzyme genes were arranged in straight was constructed by amplifying the gene encoding the mevalonate kinase and the gene encoding the phosphomevalonate kinase by PCR with pUC-mvk-pmk as the template using the primers shown in Table 13 and amplifying the gene encoding the diphosphomevalonate decarboxylase and the gene encoding the isopentenyl-diphosphate delta isomerase by PCR with pTWV-dmd-yidi as the template using the primers shown in Table 13, followed by cloning the amplified products into pTrcHis2B vector by the in-fusion cloning method. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. The PCR fragment was inserted into pTrcHis2B vector treated with the restriction enzymes *Nco*I and PstI to construct the expression vector. *E*. *coli* JM109 was transformed with the expression vector, clones having an objective sequence length were selected, a plasmid was extracted according to standard methods, and its sequence was confirmed. The constructed expression vector was designated as pTrc-KKDyI (β). The nucleotide sequence of pTrc-KKDyI (β) is represented by SEQ ID NO:50.

**Table 13. Primer sequences used for amplifying genes for constructing pTrc-KKDyI (β)**

| Template plasmid | Sequence name | Sequence (5'-) |
|---|---|---|
| pUC-mvk-pmk | KKDS2_6038-3-2 | |
| pUC-mvk-pmk | KKMyIA_6038-2-9 | |
| pTWV-dmd-yidi | KMS_6038-6-1 | |
| pTWV-dmd-yidi | KDyIA_6038-3-3 | |

### 7-3) Fixation of downstream region of mevalonate pathway on chromosome

The sequence in which the gene encoding the mevalonate kinase, the gene encoding the phosphomevalonate kinase, the gene encoding the diphosphomevalonate decarboxylase and the gene encoding the isopentenyl-diphosphate delta isomerase were arranged in straight was expressed on a chromosome. A glucose isomerase promoter was used for the expression of the gene, and a transcription termination region of aspA gene in *E. coli* was used for the termination of the transcription (WO2010031062). A translocation site of Tn7 was used as a chromosomal site to be fixed (Mol Gen Genet., 1981; 183 (2): 380-7). A cat gene was used as a drug marker after the fixation of the chromosome. A Tn7 downstream region in the chromosome region to be fixed was amplified by PCR with genomic DNA of *E. coli* as the template using the primers shown in Table 14. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. A cat gene region containing a λ phage attachment site was amplified by PCR with pMW118-attL-Cm-attR plasmid as the template using the primers shown in Table 14 (WO2010-027022). Prime Star HS DNA polymerase sold by Takara Bio Inc. was used for the PCR enzyme, and the reaction was carried out at 95°C for 3 minutes followed by in 2 cycles of 95°C for 1 minute, 34°C for 30 seconds and 72°C for 40 seconds, 2 cycles of 95°C for 30 seconds, 50°C for 30 seconds and 72°C for 40 seconds, and then at 72°C for 5 minutes. A sequence downstream of the mevalonate pathway to which a promoter and a transcription termination region had been added (hereinafter abbreviated as KKDyI) was amplified with pTrc-KKDyI (β) as the template using the primers shown in Table 14. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. A vector was constructed using these PCR products and pMW219 treated with the restriction enzyme *Sma*I by the in-fusion cloning method. *E*. *coli* JM109 was transformed with the expression vector, clones having an objective sequence length were selected, a plasmid was extracted according to standard methods, and its sequence was confirmed. The resulting plasmid was designated as pMW219-KKDyI-TaspA. The nucleotide sequence of pMW219-KKDyI-TaspA is represented by SEQ ID NO:55.

Subsequently, a Tn7 upstream region in the chromosome region to be fixed was amplified by PCR with the genomic DNA of *E. coli* as the template using the primers shown in Table 14. Prime Star HS DNA polymerase sold by Takara Bio Inc. was used for the PCR enzyme, and the reaction was carried out at 98°C for 2 minutes followed by in 30 cycles of 98°C for 10 seconds, 52°C for 5 seconds and 72°C for 1 minute/kb, and then at 72°C for 10 minutes. A vector was constructed using the PCR product and pMW219-KKDyI-TaspA treated with the restriction enzyme *Sal*I by the in-fusion cloning method. *E. coli* JM109 was transformed with the expression vector, clones having an objective sequence length were selected, a plasmid was extracted according to standard methods, and its sequence was confirmed. The resulting plasmid was designated as pMW-Tn7-Pgi-KKDyI-TaspA-Tn7. The sequence of the constructed plasmid is represented by SEQ ID NO:56.

Subsequently, a chromosome having a region including the chloramphenicol resistance gene, the glucose isomerase promoter, the operon downstream of the mevalonate pathway, and the aspA gene transcription termination region was fixed using λ-Red method. A fragment for chromosome fixation was prepared by extracting the plasmid pMW-Tn7-Pgi-KKDyI-TaspA-Tn7 and then treating it with the restriction enzymes *Pvu*I and *Sal*I followed by purifying it. *E. coli* MG1655 containing a plasmid pKD46 having a temperature-sensitive replication capacity (hereinafter referred to as MG1655/pKD46) was used for the electroporation. The plasmid pKD46 [Proc. Natl. Acad. Sci. USA, 2000, vol. 97, No.12, p6640-6645] contains a DNA fragment of total 2154 nucleotides (GenBank/EMBL Accession No. J02459, 31088th to 33241st) of λ phage containing λ Red system genes (λ, β, exo genes) controlled by an arabinose-inducible ParaB Promoter. After the electroporation, a colony that had acquired the resistance to chloramphenicol was obtained, subsequently genomic DNA was extracted, and it was confirmed by PCR using the primers shown in Table 16 that the objective region was fixed on the chromosome. Further, the sequence of the objective region was confirmed by confirming the sequence of the PCR fragment. The nucleotide sequence of the mevalonate pathway downstream and its proximal region fixed on the chromosome is represented by SEQ ID NO:57, and its construction outline is shown in FIG. 4. The resulting mutant was designated as MG1655 cat-Pgi-KKDyI.

The drug marker in MG1655 cat-Pgi-KKDyI was removed by the following procedure. Competent cells of MG1655 cat-Pgi-KKDyI was made, and then pMW-int-xis was introduced therein. pMW-int-xis is a plasmid containing a gene encoding integrase (Int) of the λ phage and a gene encoding excisionase (Xis) of the λ phage and having the temperature-sensitive replication capacity (WO2007/037460, JP Publication No. 2005-058827).

The chloramphenicol-resistant gene located in a region sandwiched with attL and attR that are the attachment site of the λ phage is dropped off from the chromosome by introducing pMW-int-xis. As a result, it is known that the host loses the resistance to chloramphenicol. And, a chloramphenicol-sensitive strain was obtained from the resulting colony, and subsequently cultured on the LB medium at 42°C for 6 hours. The cultured microbial cells were applied onto the LB plate medium to allow colonies to appear. A colony that had lost the resistance to ampicillin was selected from these colonies to remove the drug resistance. The mutant obtained as above was designated as MG1655 Pgi-KKDyI.

**Table 14. Primers for making PCR fragments used for construction of pMW219-KKDyI-TaspA**

| Template DNA | Amplified region | Sequence name | Sequence (5'-) |
|---|---|---|---|
| E. coli genome | Tn7 downstream | Tn7dS_6038-7-1 | |
| E. coli genome | Tn7 downstream | Tn7dA_6038-7-2 | AGGCTTCATTTTAATCAAACATCCTGCCAACTC (SEQ ID NO:59) |
| pMW-attL -Cm-attR | attL-cat-attR | Tn7dattLcm S_6038-7-4 | ATTAAAATGAAGCCTGCTTTTTTAT (SEQ ID NO:60) |
| pMW-attL -Cm-attR | attL-cat-attR | PgiattRcmA 6038-7-5 | GGCATCGTCAAGGGCCGCTCAAGTTAGTATAA (SEQ ID NO:61) |
| pTrc-KKDyI(β) | KKDyI | gil.2-MVK-S_6038-7-6 | |
| pTrc-KKDyI(β) | KKDyI | pMW-TaspA-yIDIA_6038-7-7 | |

**Table 15. Primers for making PCR fragments used for construction of pMW-Tn7-Pgi-KKDyI-TaspA-Tn7**

| Template DNA | Amplified region | Sequence name | Sequence (5'-) |
|---|---|---|---|
| E. coli genome | Tn7 upstream | Tn7upSv02_6038-24-1 | |
| E. coli genome | Tn7 upstream | Tn7upAv02_6038-24-2 | |

**Table 16. PCR primers for confirming chromosome fixation of mevalonate pathway downstream**

| Sequence name | Sequence (5'-) |
|---|---|
| Tn7v02-F_6038-22-5 | ACGAACTGCTGTCGAAGGTT (SEQ ID NO:66) |
| Tn7v02-R_6038-22-6 | GGTGTACGCCAGGTTGTTCT (SEQ ID NO:67) |

### 7-4) Substitution of promoter downstream of mevalonate pathway on chromosome

The promoter of the operon downstream of the mevalonate pathway on the chromosome was substituted by the λ-red method. A genomic fragment having attL-Tet-attR-Ptac was used as the template for PCR. This is one in which the tac promoter, and attL and attR that are the attachment sites for a tetracycline resistant drug marker and the λ phage are aligned. This sequence is represented by SEQ ID NO:68. A PCR fragment was prepared using the promoter shown in Table 17. LA-Taq polymerase sold by Takara Bio Inc. was used for the PCR enzyme, and the reaction was carried out at 92°C for 1 minute, then for 40 cycles of 92°C for 10 seconds, 50°C for 20 seconds and 72°C for 1 minute/kb, and further at 72°C for 7 minutes. The PCR product was purified. MG1655 Pgi-KKDyI containing the plasmid pKD46 (hereinafter referred to as MG1655 Pgi-KKDyI/pKD46) having the temperature-sensitive replication capacity was used for the electroporation. The plasmid pKD46 [Proc. Natl. Acad. Sci. USA, 2000, vol. 97, No.12, p6640-6645] contains a DNA fragment of total 2154 nucleotides (GenBank/EMBL Accession No. J02459, 31088th to 33241st) of λ phage containing λ Red system genes (λ, β, exo genes) controlled by an arabinose-inducible ParaB Promoter. The plasmid pKD46 is required for incorporating the PCR product into MG1655 Pgi-KKDyI.

Competent cells for the electroporation were prepared as follows. MG1655 Pgi-KKDyI/pKD46 cultured in the LB medium containing 100 mg/L of ampicillin at 30°C overnight were diluted to 100 times with 5 mL of LB medium containing ampicillin and L-arabinose (1 mM). The resulting cells in diluted suspension were grown until OD600 reached about 0.6 with ventilating at 30°C, and subsequently washed three times with ice-cold 10% glycerol solution to use for the electroporation. The electroporation was performed using 50 µL of the competent cells and about 100 ng of the PCR product. The cells after the electroporation in 1 mL of SOC medium [Molecular Cloning: Laboratory Manuals, 2nd Edition, Sambrook, J. et al., Cold Spring Harbor Laboratory Press (1989)] were cultured at 37°C for one hour, and subjected to a plate culture on LB agar medium at 37°C to select a chloramphenicol-resistant transformant. Subsequently, in order to remove the pKD46 plasmid, the transformant was subcultured on the LB agar medium containing tetracycline at 37°C. The ampicillin resistance was examined in the obtained colonies, and an ampicillin-resistant strain having no pKD46 was obtained. A mutant containing the tac promoter substitution that could be distinguished by the tetracycline-resistant gene was obtained. The obtained mutant was designated as MG1655 tet-Ptac-KKDyI.

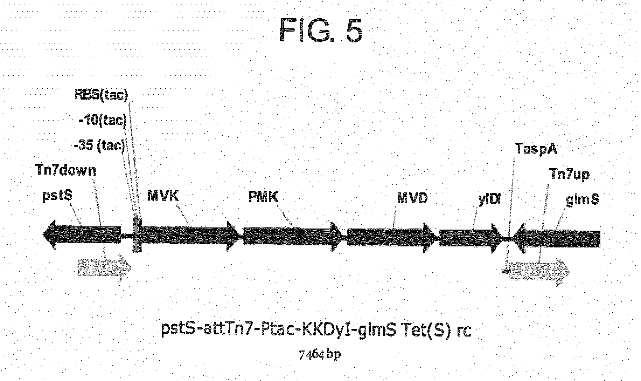

The antibiotic marker was removed by the following procedure. Competent cells of MG1655 tet-Ptac-KKDyI were made, and then pMW-int-xis was introduced therein. pMW-int-xis is a plasmid containing the genes encoding integrase (Int) and excisionase (Xis) of the λ phage and having the temperature-sensitive replication capacity (WO2007/037460, JP Publication No. 2005-058827). The tetracycline-resistant gene located in a region sandwiched with attL and attR that are the attachment site of the λ phage is dropped off from the chromosome by introducing pMW-int-xis. As a result, it is known that the host loses the resistance to tetracycline. Thus, a tetracycline-sensitive strain was obtained from the resulting colonies. Cells of this strain were cultured on the LB medium at 42°C for 6 hours, and the cultured cells were applied onto the LB plate medium to allow colonies to appear. A clone that had lost the resistance to ampicillin was selected to remove the drug resistance. The resulting mutant was designated as MG1655 Ptac-KKDyI. The nucleotide sequence of the mevalonate pathway downstream and its proximal region controlled by the tac promoter on the chromosome is represented by SEQ ID NO:69, and its outline is shown in FIG. 5.

**Table 17. Primers for making PCR fragments for promoter substitution**

| Sequence name | Sequence (5'-) |
|---|---|
| APtacKKDyIv03_6038-36-5 | |
| SPtacKKDyIv02_6038-36-3 | |

### 7-5) Introduction of isoprene synthase derived from various plants into MG1655 Ptac-KKDyI strain

Competent cells of MG1655 Ptac-KKDyI strain were prepared, and then pSTV28-Ptac-Ttrp, pSTV28-Ptac-IspSK, pSTV28-Ptac-IspSM, or further pSTV28-Ptac-SP was introduced therein. The cells were evenly applied onto the LB plate containing 60 mg/L of chloramphenicol, and the cells were cultured at 37°C for 18 hours. Transformants that exhibited the chloramphenicol resistance were obtained from the resulting plate. A strain in which pSTV28-Ptac-Ttrp, pSTV28-Ptac-IspSK, pSTV28-Ptac-IspSM, or pSTV28-Ptac-IspSP had been introduced into MG1655 Ptac-KKDyI strain was designated as MG1655 Ptac-KKDyI/pSTV28-Ptac-Ttrp, MG1655 Ptac-KKDyI/pSTV28-Ptac-IspSK, MG1655 Ptac-KKDyI/pSTV28-Ptac-IspSM, or MG1655 Ptac-KKDyI/pSTV28-Ptac-IspSP, respectively.

### 7-6) Effects of introduction of isoprene synthase derived from various plants on MG1655 strain in which MVA pathway is enhanced

Microbial cells of MG1655 Ptac-KKDyI/pSTV28-Ptac-Ttrp, MG1655 Ptac-KKDyI/pSTV28-Ptac-IspSK, MG1655 Ptac-KKDyI/pSTV28-Ptac-IspSM, or further MG1655 Ptac-KKDyI/pSTV28-Ptac-IspSP strain were evenly applied onto the LB plate containing 60 mg/L of chloramphenicol, and the cells were cultured at 37°C for 18 hours. One loopful of the microbial cells from the resulting LB plate was inoculated to 1 mL of M9 glucose (containing mevalonic acid) medium in a headspace vial (22 mL CLEAR CRIMP TOP VIAL (cat #B0104236) manufactured by Perkin Elmer), and subsequently the cultivation was evaluated according to the method described in Reference Example 2. A composition of the M9 glucose (containing mevalonic acid) medium is described in Table 18. The amount of produced isoprene and the OD value upon completion of the cultivation are described in Table 19.

**Table 18. Composition of M9 glucose (containing mevalonic acid) medium**

| | |
|---|---|
| Glucose | 2.0 g/L |
| Na₂HPO₄ | 6.0 g/L |
| KH₂PO₄ | 3.0 g/L |
| NaCl | 0.5 g/L |
| NH₄Cl | 1.0 g/L |
| Mevalonic acid (manufactured by ADEKA) | 1.0 g/L |
| 1 M MgSO₄ (autoclaved) | 1.0 mL |
| 1 M CaCl₂ (autoclaved) | 0.1 mL |

Chloramphenicol was added at a final concentration of 60 mg/L.

A total volume was adjusted to 1 L, and the medium was sterilized by filtration.

**Table 19. Amount (mg/L) of produced isoprene and OD value when cultivation of MG1655 Ptac-KKDyI/pSTV28-Ptac-Ttrp, MG1655 Ptac-KKDyI/pSTV28-Ptac-IspSK, MG1655 Ptac-KKDyI/pSTV28-Ptac-IspSM, or further MG1655 Ptac-KKDyI/pSTV28-Ptac-IspSP was completed**

| Name of microbial strain | OD value | Amount (mg/L) of produced isoprene |
|---|---|---|
| MG1655 Ptac-KKDyI/pSTV28-Ptac-Ttrp | 2.08 ± 0.07 | 0.07 ± 0.01 |
| MG1655 Ptac-KKDyI/pSTV28-Ptac-IspSK | 2.48 ± 0.13 | 30.96 ± 3.04 |
| MG1655 Ptac-KKDyI/pSTV28-Ptac-IspSM | 2.48 ± 0.09 | 57.13 ± 15.00 |
| MG1655 Ptac-KKDyI/pSTV28-Ptac-IspSP | 1.95 ± 0.09 | 0.52 ± 0.01 |

From the results in Table 19, the amount of produced isoprene was larger in order of MG1655 Ptac-KKDyI/pSTV28-Ptac-IspSM, MG1655 Ptac-KKDyI/pSTV28-Ptac-IspSK, MG1655 Ptac-KKDyI/pSTV28-Ptac-IspSP, and MG1655 Ptac-KKDyI/pSTV28-Ptac-Ttrp strains. From the above results, the strain introduced with the isoprene synthase derived from Mucuna also exhibited the highest ability to produce isoprene in the strains introduced with the MVA pathway.

### Example 5: Construction of P. ananatis AG10265

### (5-1) Construction of pMW-Para-mvaES-Ttrp

### (5-1-1) Chemical synthesis of mvaE gene derived from Enterococcus faecalis

The nucleotide sequence and amino acid sequence of mvaE derived from *Enterococcus faecalis* and encoding acetyl-CoA acetyltransferase and hydroxymethlglutaryl-CoA reductase have been already known (ACCESSION number of nucleotide sequence: AF290092.1 (1479..3890), ACCESSION number of amino acid sequence: AAG02439) (J. Bacteriol. 182(15), 4319-4327(2000)). The amino acid sequence of the mvaE protein derived from *Enterococcus faecalis* and the nucleotide sequence of its gene are shown in SEQ ID NO:72 and SEQ ID NO:73, respectively. In order to efficiently express the mvaE gene in *E. coli,* a mvaE gene in which codon usage was optimized for *E. coli* was designed and designated as EFmvaE. Its nucleotide sequence is shown in SEQ ID NO:74. This mvaE gene was chemically synthesized, cloned into pUC57 (supplied from GenScript), and the resulting plasmid was designated as pUC57-EFmvaE.

### (5-1-2) Chemical synthesis of mvaS gene derived from Enterococcus faecalis

The nucleotide sequence and amino acid sequence of mvaS derived from *Enterococcus faecalis* and encoding hydroxymethylglutaryl-CoA synthase have been already known (ACCESSION number of nucleotide sequence: AF290092.1, complement(142..1293), ACCESSION number of amino acid sequence: AAG02438) (J. Bacteriol. 182(15), 4319-4327 (2000)). The amino acid sequence of the mvaS protein derived from *Enterococcus faecalis* and the nucleotide sequence of its gene are shown in SEQ ID NO:75 and SEQ ID NO:76, respectively. In order to efficiently express the mvaS gene in *E*. *coli,* a mvaS gene in which codon usage was optimized for *E. coli* was designed and designated as EFmvaS. Its nucleotide sequence is shown in SEQ ID NO:77. This mvaS gene was chemically synthesized, cloned into pUC57 (supplied from GenScript), and the resulting plasmid was designated as pUC57-EFmvaS.

### (5-1-3) Construction of arabinose-inducible mvaES expression vector

An arabinose-inducible expression vector for a gene upstream of a mevalonate pathway was constructed by the following procedure. A PCR fragment comprising Para composed of araC and araBAD promoter sequences derived from *E. coli* was obtained by PCR using the plasmid pKD46 as a template and using synthesized oligonucleotides represented by SEQ ID NO:78 and SEQ ID NO:79 as primers. A PCR fragment comprising the EFmvaE gene was obtained by PCR using the plasmid pUC57-EFmvaE as the template and using synthesized oligonucleotides represented by SEQ ID NO:80 and SEQ ID NO:81 as the primers. A PCR fragment comprising the EFmvaS gene was obtained by PCR using the plasmid pUC57-EFmvaS as the template and using synthesized oligonucleotides represented by SEQ ID NO:82 and SEQ ID NO:83 as the primers. A PCR fragment comprising a Ttrp sequence was obtained by PCR using the plasmid pSTV-Ptac-Ttrp as the template and using synthesized oligonucleotides represented by SEQ ID NO:84 and SEQ ID NO:85 as the primers. PrimeStar polymerase (supplied from TaKaRa Bio) was used for PCR for obtaining these 4 PCR fragments. A reaction solution was prepared according to a composition attached to the kit, and the reaction by a cycle of 98°C for 10 seconds, 55°C for 5 seconds and 72°C for one minute/kb was repeated 30 times. Using the purified PCR product comprising Para and the purified PCR product comprising the EFmvaE gene as the template, PCR was carried out using the synthesized oligonucleotides represented by SEQ ID NO:78 and SEQ ID NO:81 as the primers. Using the purified PCR product comprising the EFmvaS gene and the purified PCR product comprising Ttrp as the template, PCR was carried out using the synthesized oligonucleotides represented by SEQ ID NO:82 and SEQ ID NO:85 as the primers. As a result, the PCR product comprising Para and the EFmvaE gene and the PCR product comprising the EFmvaS gene and Ttrp were obtained. A plasmid pMW219 (supplied from Nippon Gene) was digested with *Sma*I according to the standard method. After digestion with *Sma*I, pMW219, the purified PCR product comprising Para and the EFmvaE gene and the purified PCR product comprising the EFmvaS gene and Ttrp were ligated using In-Fusion HD Cloning Kit (supplied from Clontech). The resulting plasmid was designated as pMW-Para-mvaES-Ttrp.

### (5-2) Construction of pTrc-KKDyI-ispS(K)

First, an expression vector comprising a sequence where mevalonate kinase, phosphomevalonate kinase, diphosphomevalonate decarboxylase and isopentenyl diphosphate delta isomerase were aligned in line was constructed by In-fusion cloning. Sequences of mevalonate kinase and phosphomevalonate kinase were amplified by PCR using pUC-mvk-pmk (see Reference Example 7-2) (SEQ ID NO:40) as the template and using primers consisting of the nucleotide sequences of SEQ ID NOs:86 to 89. Sequences of diphosphomevalonate decarboxylase and isopentenyl diphosphate delta isomerase were amplified by PCR using pTWV-dmd-yidi (see Reference Example 7-2) ((SEQ ID NO:45) as the template and using primers consisting of the nucleotide sequences of SEQ ID NOs:86 to 89. Subsequently, the expression vector where these four enzyme genes was aligned in line was constructed by cloning them into pTrcHis2B vector using the in-fusion cloning method. PrimeStar HS DNA polymerase sold by TaKaRa Bio was used for PCR, and the reaction was carried out under the condition of 98°C for 2 minutes, 30 cycles (98°C for 10 seconds, 52°C for 5 seconds and 72°C for one minute/kb) and 72°C for 10 minutes. The resulting PCR fragment was inserted into the pTrcHis2B vector digested with restriction enzymes *Nco*I and PstI using the in-fusion cloning method to construct the expression vector. *E. coli* JM109 was transformed with this expression vector, a clone having an objective length was selected, subsequently the plasmid was extracted according to the standard methods, and its sequence was confirmed. The constructed expression vector was designated as pTrc-KKDyI(α). A nucleotide sequence of pTrc-KKDyI(α) is shown in SEQ ID NO:90.

Then, a plasmid pTrc-KKDyI-ispS(K) where IspS(K) was added to the obtained expression vector pTrc-KKDyI(α) (SEQ ID NO:90) was constructed by the following procedure.

pTrc-KKDyI(α) was digested with the restriction enzyme PstI (supplied from TaKaRa Bio) to obtain pTrc-KKDyI(α)/PstI. Using pUC57-ispSK as the template, PCR was carried out using pTrcKKDyIkSS_6083-10-1 (SEQ ID NO:91) and pTrcKKDyIkSA_6083-10-2 (SEQ ID NO:92) as the primers and using PrimeStar polymerase (supplied from TaKaRa Bio). A reaction solution was prepared according to the composition attached to the kit, and the reaction of a cycle of 98°C for 10 seconds, 54°C for 20 seconds and 68°C for 120 seconds was repeated 30 times. As a result, a PCR product comprising an IspSK gene was obtained. Subsequently, the purified IspSK gene fragment and pTrc-KKDyI(α)/PstI were ligated using In-Fusion HD Cloning Kit (supplied from Clontech). The resulting plasmid was designated as pTrc-KKDyI-ispS(K) (SEQ ID NO:93).

### (5-3) Construction of pSTV-Ptac-ispS-Mmamvk

### (5-3-1) Chemical synthesis of mevalonate kinase derived from Methanosarcina mazei

The nucleotide sequence and amino acid sequence of mevalonate kinase derived from *Methanosarcina mazei* Gol have been already known (ACCESSION number of nucleotide sequence: NC_003901.1 (2101873..2102778, LOCUS TAG MM_1762, ACCESSION number of amino acid sequence: NP_633786.1)).

The amino acid sequence of the MVK protein derived from *Methanosarcina mazei* and the nucleotide sequence of its gene are shown in SEQ ID NO:94 and SEQ ID NO:95, respectively. In order to efficiently express the MVK gene in *E. coli,* the MVK gene in which the codon usage was optimized for *E. coli* was designed and designated as Mmamvk. A nucleotide sequence of Mmamvk is shown in SEQ ID NO:96. The Mmamvk gene was chemically synthesized, cloned into pUC57 (supplied from GenScript), and the resulting plasmid was designated as pUC57-Mmamvk.

### (5-3-2) Construction of plasmid for expressing isoprene synthase gene derived from Pueraria montana var. lobata (kudzu) and MVK gene

A plasmid for expressing the IspSK gene and the Mmamvk gene in *E. coli* was constructed by the following procedure. Using pUC57-IspSK as the template, PCR was carried out using synthesized oligonucleotide consisting of the nucleotide sequences of SEQ ID NO:97 and SEQ ID NO:98 as the primers and using PrimeStar polymerase (supplied from TaKaRa Bio). A reaction solution was prepared according to the composition attached to the kit, and the reaction of a cycle of 98°C for 10 seconds, 54°C for 20 seconds and 68°C for 120 seconds was repeated 40 times. As a result, a PCR product comprising the IspSK gene was obtained. Likewise, using pSTV28-Ptac-Ttrp as the template, PCR was carried out using synthesized oligonucleotide consisting of the nucleotide sequences of SEQ ID NO:99 and SEQ ID NO:100 as the primers and using PrimeStar polymerase (supplied from TaKaRa Bio). A reaction solution was prepared according to the composition attached to the kit, and the reaction of a cycle of 98°C for 10 seconds, 54°C for 20 seconds and 68°C for 210 seconds was repeated 40 times. As a result, a PCR product comprising pSTV28-Ptac-Ttrp was obtained. Subsequently, the IspSK gene fragment and the PCR product comprising pSTV28-Ptac-Ttrp were ligated using In-Fusion HD Cloning Kit (supplied from Clontech). The resulting plasmid for expressing the IspSK gene was designated as pSTV28-Ptac-IspSK. Then, using pUC57-Mmamvk as the template, PCR was carried out using synthesized oligonucleotide consisting of the nucleotide sequences of SEQ ID NO:101 and SEQ ID NO:102 as the primers and using PrimeStar polymerase (supplied from TaKaRa Bio). A reaction solution was prepared according to the composition attached to the kit, and the reaction of a cycle of 98°C for 10 seconds, 55°C for 5 seconds and 72°C for one minute/kb was repeated 30 times. As a result, a PCR product comprising the Mmamvk gene was obtained. Likewise, using pSTV28-Ptac-IspSK as the template, PCR was carried out using synthesized oligonucleotide consisting of the nucleotide sequences of SEQ ID NO:103 and SEQ ID NO:104 as the primers and using PrimeStar polymerase (supplied from TaKaRa Bio). A reaction solution was prepared according to the composition attached to the kit, and the reaction of a cycle of 98°C for 10 seconds, 55°C for 5 seconds and 72°C for one minute/kb was repeated 30 times. As a result, a PCR product comprising pSTV28-Ptac-IspSK was obtained. Subsequently, the purified Mmamvk gene and the PCR product comprising pSTV28-Ptac-IspSK were ligated using In-Fusion HD Cloning Kit (supplied from Clontech). The resulting plasmid for expressing the IspSK gene and the Mmamvk gene was designated as pSTV28-Ptac-IspSK-Mmamvk.

### (5-4) Construction of pMW-Ptac-Mclmvk-Ttrp

Next, a plasmid for expressing MVK (PMW-Ptac-mvk-Ttrp) was constructed.

Using pUC57-Mclmvk as the template, PCR was carried out using synthesized oligonucleotides consisting of the nucleotide sequences of Mcl_mvk_N (SEQ ID NO:105) and Mcl_mvk_C (SEQ ID NO:106) as the primers and using PrimeStar polymerase (supplied from TaKaRa Bio). A reaction solution was prepared according to the composition attached to the kit, and the reaction of a cycle of 98°C for 10 seconds, 55°C for 5 seconds and 72°C for one minute/kb was repeated 30 times. As a result, a PCR product comprising the Mclmvk gene was obtained. Likewise, using pMW219-Ptac-Ttrp (see WO2013069634A1) as the template, PCR was carried out using synthesized oligonucleotides consisting of the nucleotide sequences of PtTt219f (SEQ ID NO:107) and PtTt219r (SEQ ID NO:108) as the primers and using PrimeStar polymerase (supplied from TaKaRa Bio). A reaction solution was prepared according to the composition attached to the kit, and the reaction of a cycle of 98°C for 10 seconds, 55°C for 5 seconds and 72°C for one minute/kb was repeated 30 times. As a result, a PCR product comprising pMW219-Ptac-Ttrp was obtained. Subsequently, the purified Mclmvk gene and the PCR product comprising pMW219-Ptac-Ttrp were ligated using In-Fusion HD Cloning kit (supplied from Clontech). The resulting plasmid for expressing the Mclmvk gene was designated as pMW-Ptac-Mclmvk-Ttrp.

### (5-5) Integrative conditional replication plasmid having upstream gene and downstream gene of mevalonate pathway

In order to construct an integrative plasmid possessing an upstream gene and a downstream gene of the mevalonate pathway, a vector pAH162-λattL-TcR-λattR (Minaeva NI et al., BMC Biotechnol. 2008; 8:63) was used.

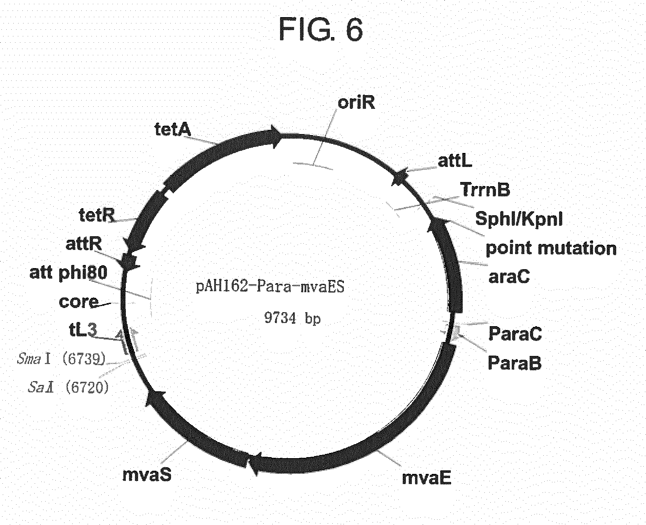

A KpnI-SalI fragment of pMW-Para-mvaES-Ttrp was cloned into a recognition site for *Sph*I*-Sal*I in pAH162-λattL-TcR-λattR. As a result, a plasmid pAH162-Para-mvaES possessing an operon mvaES derived from *E. faecalis* under the control of the Para promoter and a repressor gene AraC from *E. coli* was constructed (FIG. 6).

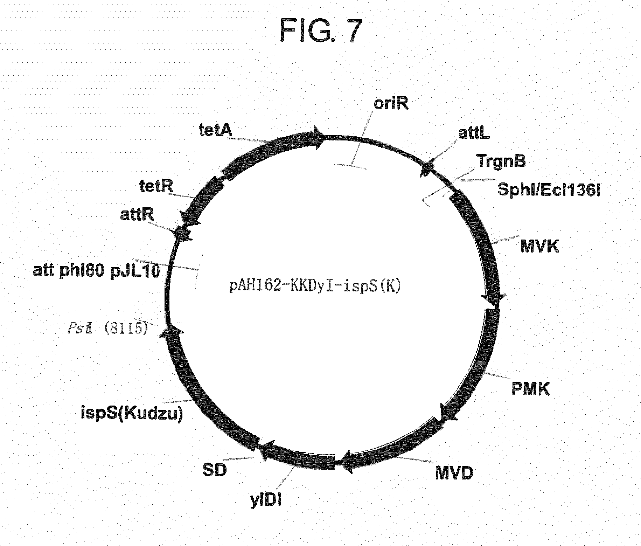

An Ecl136II-SalI fragment of a plasmid pTrc-KKDyI-ispS(K) comprising a mevalonate kinase gene, a phosphomevalonate kinase gene, a diphosphomevalonate decarboxylase gene and an IPP isomerase gene derived from *S. cerevisiae* and a coding portion of the ispS gene derived from kudzu was subcloned into a *Sph*I*-Sal*I site of pAH162-λattL-TcR-λattR, and the resulting plasmid was designated as pAH162-KKDyI-ispS(K) (FIG. 7).

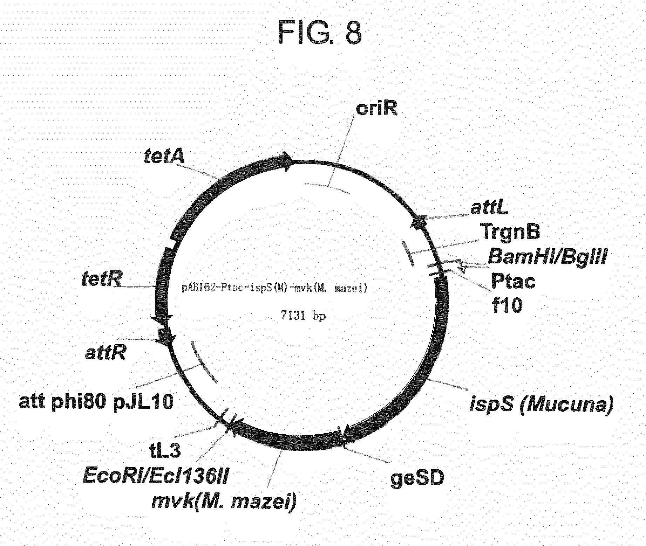

A BglII-EcoRI fragment of pSTV28-Ptac-ispS-Mmamvk comprising the ispS gene (Mucuna) and the mvk gene (*M*. *mazei*) under the control of Ptac was subcloned into a recognition site for BamHI-Ecl136II in the integrative vector pAH162-λattL-TcR-λattR. The resulting plasmid pAH162-Ptac-ispS(M)-mvk(Mma) is shown in FIG. 8.

### (5-6) Construction of P. ananatis SC17(0) derivative possessing attB site of phi80 phage at different site in genome

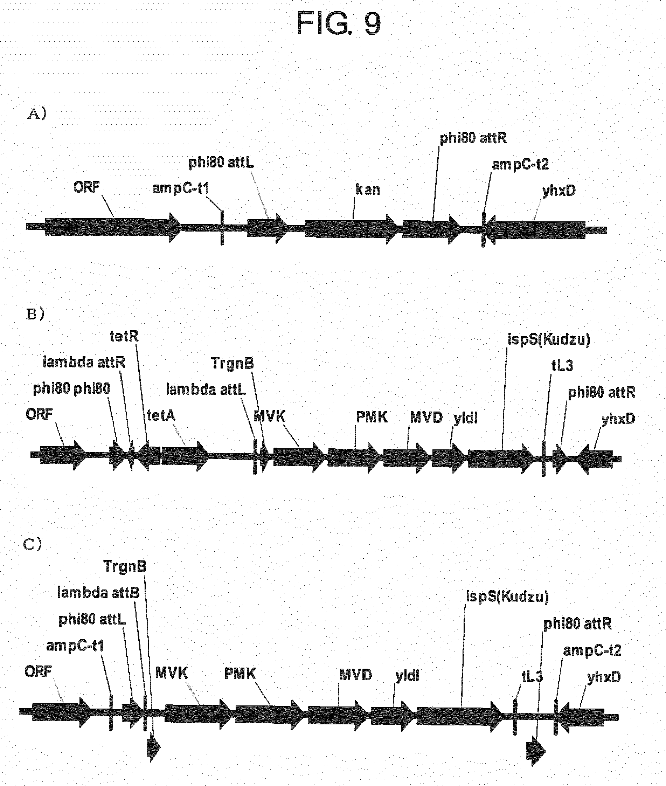

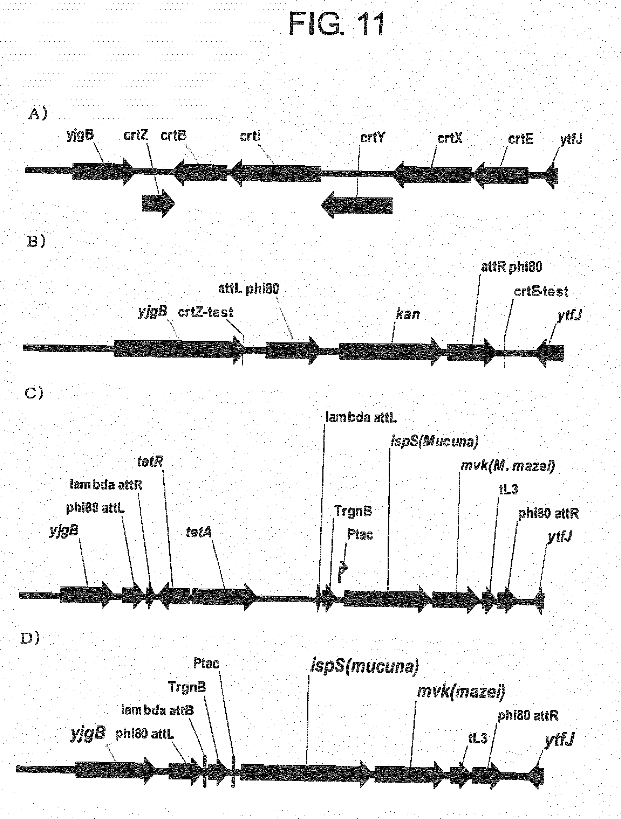

A *P*. *ananatis* SC17(0) derivative possessing an attB site of phi80 phage that substituted an ampC gene, an ampH gene or a crt operon was constructed (annotated complete genome sequence of *P. ananatis* AJ13355 is available as PRJDA162073 or GenBank accession number AP01232.1 and AP012033.1). In order to obtain these strains, λRed dependent integration of a PCR-amplified DNA fragment possessing attLphi80-kan-attRphi80 flanking to a 40 bp region homologous to a target site in the genome was carried out according to the previously reported technique (Katashkina JI et al., BMC Mol Biol. 2009; 10:34). After the electroporation, cells were cultured on L-agar containing 50 mg/L of kanamycin. DNA fragments used for the substitution of the ampC gene and the ampH gene and the crt operon with attLphi80-kan-attRphi80 were amplified by the reaction using oligonucleotides 1 and 2, 3 and 4, and 5 and 6 (Table 20), respectively. A plasmid PMWattphi (Minaeva NI et al., BMC Biotechnol. 2008; 8:63) was used as the template in these reactions. The resulting integrants were designated as SC17(0)ΔampC::attLphi80-kan-attRphi80, SC17(0)ΔampH::attLphi80-kan-attRphi80, and SC17(0)Δcrt::attLphi80-kan-attRphi80. Oligonucleotides 7 and 8, 9 and 10, and 11 and 12 (Table 20) were used for PCR validation of SC17(0)ΔampC::attLphi80-kan-attRphi80, SC17(0)ΔampH::attLphi80-kan-attRphi80, and SC17(0)Δcrt::attLphi80-kan-attRphi80, respectively. Maps of the resulting modified genomes ΔampC::attLphi80-kan-attRphi80, ΔampH::attLphi80-kan-attRphi80 and Δcrt::attLphi80-kan-attRphi80 are shown in FIG. 9A, FIG, 10A and FIG. 11A, respectively.

Curing of the constructed strains from a kanamycin resistant marker was carried out using a helper plasmid pAH129-cat according to the previously reported technique (Andreeva IG et al., FEMS Microbiol Lett. 2011; 318(1):55-60). Oligonucleotides 7 and 8, 9 and 10, and 11 and 12 (Table 20) were used for PCR validation of the resulting strains SC17(0)ΔampC::attBphi80, C17(0)ΔampH::attBphi80, and SC17(0)Δcrt::attBphiB0, respectively.

### (5-7) Construction of ISP3-S strain

The plasmid pAH162-KKDyI-ispS(K) described in (5-5) was integrated in SC17(0)ΔampC::attBphi80 described in (5-6) using a helper plasmid pAH123-cat according to the previously reported technique (Andreeva IG et al., FEMS Microbiol Lett. 2011; 318(1):55-60). An oligonucleotide pair 13-7 and 14-8 (Table 20) were used for PCR validation of the resulting integrant. The resulting SC17(0)ΔampC::pAH162-KKDyI-ispS(K) was cured from a vector portion of pAH162-KKDyI-ispS(K) using a helper plasmid pMWintxis-cat possessing an int gene and a xis gene of λ phage according to the previously reported technique (Katashkina JI et al., BMC Mol Biol. 2009; 10:34). As a result, a strain SC17(0)ΔampC:: KKDyI-ispS(K) was obtained. Oligonucleotides 7 and 15 (Table 20) were used for PCR validation of the kanamycin sensitive derivative. The construction of SC17(0)ΔampC:: KKDyI-ispS(K) is shown in FIG. 9.

Genomic DNA isolated from the above strain SC17(0)ΔampH::attLphi80-kan-attRphi80 using GeneElute Bacterial genome DNA Kit (Sigma) was electroporated into SC17(0)ΔampH::KKDyI-ispS(K) according to the method of chromosome electroporation previously reported (Katashkina JI et al., BMC Mol Biol. 2009; 10:34). Transfer of the mutation ΔampH::attLphi80-kan-attRphi80 was confirmed by PCR using the primers 9 and 10.

The final strain was cured from the kanamycin resistant marker using phi80 Int/Xis dependent technique (Andreeva IG et al., FEMS Microbiol Lett. 2011; 318(1):55-60). The modified genome ΔampH::KKDyI-ispS(K) was verified in obtained KmS integrants by PCR using the primers 7 and 15, and subsequently a strain SC17(0)ΔampC::KKDyI-ispS(K) ΔampH::attBphi80 was selected.

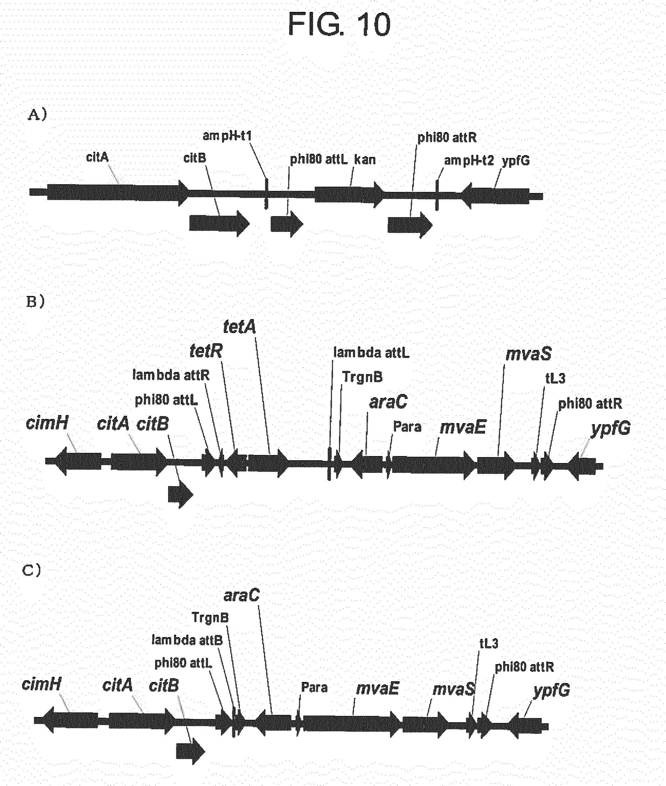

The above plasmid pAH162-Para-mvaES was integrated into SC17(0)ΔampC::KKDyI-ispS(K) ΔampH::attBphi80 using the helper plasmid pAH123-cat (Andreeva IG et al., FEMS Microbiol Lett. 2011; 318(1):55-60). Oligonucleotides 13 and 9 and oligonucleotides 14 and 10 (Table 20) were used for validation of the resulting integrant by PCR. This integrant was cured from a vector portion of pAH162-Para-mvaES using phage λ Int/Xis-dependent technique (Katashkina JI et al., BMC Mol Biol. 2009; 10:34). Removal of the vector from the chromosome was confirmed by PCR using the primers 9 and 16. As a result, SC17(0)ΔampC::KKDyI-ispS(K)ΔampH::Para-mvaES containing no marker was obtained. A construct of the modified chromosome ΔampH::Para-mvaES is shown in FIG. 10.

The plasmid pAH162-Ptac-ispS(K)-mvk(Mma) described in (5-5) was integrated into genome of SC17(0)Δcrt::attBphiB0 using the protocol described in the previous report (Andreeva IG et al., FEMS Microbiol Lett. 2011; 318(1): 55-60). The integration of the plasmid was confirmed by the polymerase chain reaction using the primers 11-13 and 12-14.

The modified chromosome SC17(0)Δcrt::pAH162-Ptac-ispS(K)-mvk(Mma) thus constructed was transferred into strain SC17(0)ΔampC::KKDyI-ispS(K)ΔampH::Para-mvaES using the electroporation by genomic DNA in the previous report (Katashkina JI et al., BMC Mol Biol. 2009; 10:34). The resulting integrant was cured from a vector portion of pAH162-Ptac-ispS(K)-mvk(Mma) using phage λ Int/Xis-dependent technique (Katashkina JI et al., BMC Mol Biol. 2009; 10:34). The structure of a final construct Δcrt::Ptac-ispS(K)-mvk(Mma) (FIG. 11) was confirmed by PCR using the primers 11 and 17.

An integrative expression cassette introduced into this final strain was verified by PCR, and it was found that some of a 5' portion of KKDyI operon comprising the MVK, PMK, MVD and yldI genes derived from *S*. *cerevisiae* were unexpectedly rearranged. In order to repair this cassette, this strain was electroporated with genomic DNA isolated from strain SC17(0)ΔampC::pAH162-KKDyI-ispS(K) using GeneElute Bacterial Genome DNA Kit (Sigma) according to the technique in the previous report (Katashkina JI et al., BMC Mol Biol. 2009; 10:34). The resulting strain contained all genes required for the production of isoprene.

After curing from a vector portion of pAH162-KKDyI-ispS(K) (see the above) using phage λ Int/Xis dependency, strain ISP3-S (*P. ananatis* SC17(0) ΔampC::attLphi80-KKDyI-ispS(K)-attRphi80 ΔampH::attLphiB0-Para-mvaES-attRphi80 Δcrt::attLphi80-Ptac-ispS(K)-mvk(Mma)-attRphi80) containing no marker was obtained.

### (5-8) Construction of integrative plasmid possessing different mevalonate kinase gene

A KpnI-BamHI fragment of pMW-Ptac-Mclmvk-Ttrp was subcloned into a recognition site for KpnI-Ecl136II in the integrative vector pAH162-λattL-TcR-λattR. As a result, a plasmid pAH162-Ptac-Mclmvk possessing the MVK gene derived from *M. concilii* and under the control of the tac promoter was constructed.

### (5-9) Construction of AG10265 strain

The plasmid pAH162-Ptac-Mclmvk described in (5-8) was integrated into the genome of SC17(0)Δcrt::attBphi80 using the helper plasmid pAH123-cat according to the protocol in the previous report (Andreeva IG et al., FEMS Microbiol Lett. 2011; 318(1): 55-60).

The modified chromosome Δcrt::pAH123-Ptac-mvk (M. paludicola) was transferred into the strain ISP3-S (5-7) via the electroporation of genomic DNA isolated from Δcrt::pAH162-Ptac-Mclmvk. The resulting integrant was designated as AG10265 (*P*. *ananatis* SC17(0) ΔampC::attLphi80-KKDyI-ispS(K)-attRphi80 ΔampH::attLphi80-Para-mvaES-attRphi80 Δcrt::attLphi80-λattL-TcR-λattR-Ptac-Mclmvk-attRphi80).

**Table 20. Oligonucleotides used as primers**

| | | | |
|---|---|---|---|
| 1 | DampC-phL | | |
| 2 | DampC-phR | | |
| 3 | ampH-attL-phi80 | | |
| 4 | ampH-attR-phi80 | | |
| 5 | crtZ-attLphi80 | | |
| 6 | crtE-attRphi80 | | |
| 7 | ampC-t1 | 5'-GATTCCCACTTCACCGAGCCG-3' | (SEQ ID NO:115) |
| 8 | ampC-t2 | 5'-GGCAGGTATGGTGCTCTGACG-3' | (SEQ ID NO:116) |
| 9 | ampH-t1 | 5'-GCGAAGCCCTCTCCGTTG-3' | (SEQ ID NO:117) |
| 10 | ampH-t2 | 5'-AGCCAGTCAGCCTCATCAGCG-3' | (SEQ ID NO:118) |
| 11 | crtZ-test | 5'-CCGTGTGGTTCTGAAAGCCGA-3' | (SEQ ID NO:119) |
| 12 | crtE-test | 5'-CGTTGCCGTAAATGTATCCGT-3' | (SEQ ID NO:120) |
| 13 | ag-phL-test | 5'-GGATGTAAACCATAACACTCTGCGAAC-3' | (SEQ ID NO:121) |
| 14 | ag-phR-test | 5'-GATTGGTGGTTGAATTGTCCGTAAC-3' | (SEQ ID NO:122) |
| 15 | KKDyI-s-3' | 5'-TGGAAGGATTCGGATAGTTGAG-3' | (SEQ ID NO:123) |

### Example 6: Preparation of Pantoea strain having improved expression of ppa gene and production of isoprene by cultivation using the same

A strain where a promoter inherent to an endogenous ppa gene (pyrophosphate phosphatase gene) was substituted with another strong promoter to augment the expression of the endogenous ppa gene in *P ananatis* strain was made by the following procedure.

### (6-1) Enhancement of ppa gene expression in AG10265 strain

### (6-1-1) Preparation of strain having substituted ppa gene promoter in wild type SC17(0)

There are two genes encoding pyrophosphate phosphatase, PAJ_2344(ppa-1) and PAJ_2736(ppa-2) in *P. ananatis.* In order to enhance the expression of the PAJ_2344(ppa-1) gene and the PAJ_2736(ppa-2) gene, a strain where a promoter sequence and an SD sequence of the PAJ_2344(ppa-1) gene and the PAJ_2736(ppa-2) gene were substituted with Ptac and ϕ10, respectively in P. ananatis SC17(0) was prepared by the λRed method. The λRed method was carried out according to the method described in BMC Mol Biol. 2009; 10:34. Annotated genome sequence information for *P*. *ananatis* AJ13355 is available as GenBank accession numbers AP012032.1 and AP012033.1.

Genomic DNA was extracted from *P. ananatis* SC17(0) strain Ptac-lacZ (RU application 2006134574, WO2008/090770, US2010062496), and used as the template for PCR. λattL-Km^{r}-λattR-Ptac where the Ptac promoter was ligated downstream of λattL-Km^{r}-λattR has been integrated upstream of the lacZ gene in *P*. *ananatis* SC17(0) strain Ptac-lacZ (see WO2011/87139 A1).

PCR was carried out using synthesized oligonucleotides consisting of the nucleotide sequences of PAJ_2344-F and PAJ_2344-R as the primers in order to substitute the promoter region of PAJ_2344 (ppa-1) gene, using synthesized oligonucleotides consisting of the nucleotide sequences of PAJ_2736-F and PAJ_2736-R as the primers in order to substitute the promoter region of PAJ_2736 (ppa-2) gene, and using PrimeStar MAX DNA polymerase (supplied from TaKaRa Bio) A reaction solution was prepared according to the composition attached to the kit, and the reaction of a cycle of 98°C for 10 seconds, 55°C for 5 seconds, 72°C for 20 seconds was repeated 30 times. As a result, a fragment comprising λattL-Km^{r}-λattR-ϕ10 for substituting the promoter was obtained.

A plasmid RSF-Red-TER (US7919284B2) was introduced into SC17(0) by the electroporation according to the standard method. The resulting strain was designated as SC17(0)/RSF-Red-TER. Competent cells of SC17(0)/RSF-Red-TER were prepared and then the purified fragment comprising λattL-Km^{r}-λattR-ϕ10 for substituting the promoter was introduced thereto by the electroporation. After the electroporation, a colony that had acquired kanamycin resistance was obtained.

Next, by PCR using two synthesized DNA primers represented by ppa_p1-R (Table 21) and ppa_p1-F (Table 21) or ppa_p2-R (Table 21) and ppa_p2-F (Table 21), it was confirmed that a sequence derived from a PCR fragment had been inserted into an upstream region of PAJ_2344 (ppa-1) gene or an upstream region of PAJ_2736 (ppa-2) gene. Bacterial strains where the insertion of the fragment had been confirmed were obtained. The bacterial strains thus obtained were designated as *P. ananatis* SC17(0)Ptac-ϕ10-ppa1 and *P. ananatis* SC17(0)Ptac-ϕ10-ppa2.

### (6-1-2) Preparation of AG10265 strain with ppa gene having substituted promoter

Genomic DNA was extracted from SC17(0)Ptac-ϕ10-ppa1 using PurElute Bacterial Genomic kit (EdgeBio). Using this as the template, a fragment from 1 kb upstream of to 1 kb downstream of the PAJ_2344 (ppa-1) gene was amplified by PCR using synthesized nucleotides consisting of the nucleotide sequences of ppa-1-g1000-F and ppa-1-g1000-R primer (Table 21) as the primers. Also, using genomic DNA extracted from SC17(0)Ptac-ϕ10-ppa2 as the template, a fragment from 1 kb upstream of to 1 kb downstream of the PAJ_2736 (ppa-2) gene was amplified by PCR using synthesized nucleotides consisting of the nucleotide sequences of ppa-2-g1000-F and ppa-2-g1000-R primer (Table 21) as the primers. PCR was carried out using PrimeStar GXL DNA polymerase (supplied from TaKaRa Bio), a reaction solution was prepared according to the composition attached to the kit, and the reaction of a cycle of 98°C for 10 seconds, 60°C for 15 seconds, 68°C for 5 minutes was repeated 40 times. As a result, the fragment comprising from 1 kb upstream of to 1 kb downstream of the PAJ_2344 (ppa-1) gene or the PAJ_2736 (ppa-2) gene was obtained. The resulting fragment was purified, 600 ng of the PCR product was introduced into AG10265 by electroporation to perform transformation, and colonies that had acquired the kanamycin resistance were obtained.

By colony PCR using synthesized nucleotides consisting of the nucleotide sequences of Ppa_p1-F and Ppa_p1-R in Table 21 as the primers, it was confirmed that the promoter of the PAJ_2344 (ppa-1) gene was substituted in the resulting transformant. Likewise, by colony PCR using synthesized nucleotides consisting of the nucleotide sequences of Ppa_p2-F and Ppa_p2-R in Table 21 as the primers, it was confirmed that the promoter of the PAJ_2736 (ppa-2) gene was substituted. In these strains, the promoter region of the PAJ_2344 (ppa-1) gene or the PAJ_2736 (ppa-2) gene has been substituted with Ptac-ϕ10. Of these, each one clone was selected, and designated as AG10265 Ptac-ϕ10-ppa1, or AG10265 Ptac-ϕ10-ppa2.

**Table 21. List of primers used**

| Primer name | Sequence (5'-) | |
|---|---|---|
| Ppa_p1-F | TTACTGACCTGTTGATCGGC | (SEQ ID NO:124) |
| Ppa_p1-R | CTGAGGATGTTTTCCGCCTG | (SEQ ID NO:125) |
| PAJ_2344-F | | |
| PAJ_2344-R | | |
| ppa-1-g1000-F | TGGGGCACACGCGTTTATGAGC | (SEQ ID NO:128) |
| ppa-1-g1000-R | GTAAGCGCCCGGTCCGACCTTA | (SEQ ID NO:129) |
| Ppa_ p2-F | TGCTGCGGGCATTGTTTCCC | (SEQ ID NO:130) |
| Ppa_p2-R | TCGGCATTGGCCGGGATCTC | (SEQ ID NO:131) |
| PAJ_2736-F | | |
| PAJ_2736-R | | |
| ppa-2-g1000-F | CATGAAAAGAGGATGGGTGTTG | (SEQ ID NO:134) |
| ppa-2-g1000-R | GTTATCTACCTTAAGATCCTGT | (SEQ ID NO:135) |

### (6-1-3) Confirmation of amount of expressed pyrophosphate phosphatase

An amount of an expressed protein of pyrophosphate phosphatase (PPA) in the AG10265 Ptac-ϕ10-ppa1 strain and the AG10265 Ptac-ϕ10-ppa2 strain was confirmed by SDS-PAGE. Cells of the AG10265 strain, the AG10265 Ptac-ϕ10-ppa1 strain and AG10265 Ptac-ϕ10-ppa2 strain were cultured with shaking in 3 mL of LB medium containing 50 mg/L of kanamycin at 30°C overnight. The microbial cells were collected, then washed twice with ice-cooled 50 mM Tris buffer (Tris-HCl, pH 8.0), and disrupted using a multibead shocker (Yasui Kikai, Japan, 4°C, 5 cycles of ON for 60 seconds and OFF for 60 seconds, 2500 rpm). A solution of the disrupted microbial cells was centrifuged at 14,000 rpm for 20 minutes to remove cell debris. The resulting supernatant fraction was handled as a soluble protein fraction. The soluble protein fraction was quantified by the BCA method, and 5 µg of the soluble protein was electrophoresed on SDS-PAGE (supplied from Invitrogen, NuPAGE: SDS-PAGE Gel System). Subsequently, CBB staining and decoloration were carried out according to the standard methods. A photograph of a gel around a mass of the PPA protein was shown in FIG. 12. As a result, increase of the expression amount of the protein each presumed to be PPA was confirmed in the AG10265 Ptac-ϕ10-ppa1 strain and the AG10265 Ptac-ϕ10-ppa2 strain. From density of bands on SDS-PAGE after the electrophoresis, the expression amount of the protein each presumed to be PPA in the AG10265 Ptac-ϕ10-ppa1 strain and the AG10265 Ptac-ϕ10-ppa2 strain was estimated to be about 1.5 to 2.0 folds higher than that in the original strain (AG10265).

### (6-1-4) Introduction of plasmid for expression of isoprene synthase

Electrocells of the AG10265 strain, the AG10265 Ptac-ϕ10-ppa1 strain and the AG10265 Ptac-ϕ10-ppa2 strain were prepared, and pSTV28-Ptac-IspSM (see Reference Example 3-5) was introduced thereto by the electroporation. The resulting isoprene-producing bacterial strains were designated as AG10265 (Control), AG10265 PPA1 and AG10265 PPA2, respectively.

### (6-2) Conditions for Jar cultivation of P. ananatis isoprene-producing bacteria

A one liter volume fermenter was used for the cultivation of *P. ananatis* isoprene-producing bacteria. Glucose medium was prepared as the composition shown in Table 22. Microbial cells of the isoprene-producing bacterial strain were applied onto an LB plate containing 60 mg/L of chloramphenicol and cultured at 34°C for 16 hours. 0.3 L of the glucose medium was placed in the 1 L volume fermenter, and microbial cells sufficiently grown on one plate were inoculated thereto to start the cultivation. A culture condition was pH 7.0 (controlled with ammonia gas), 30°C, ventilation of 150 mL/minute, and stirring such that an oxygen concentration in the medium was 5% or higher. During the cultivation, a glucose solution adjusted to 500 g/L was continuously added to the medium such that a glucose concentration in the medium was 10 g/L or higher. Finally, AG10265 (Control), AG10265 PPA1 and AG10265 PPA2 consumed 92.2g, 95.8 g and 85.7 g of glucose in the cultivation for 71 hours.

**Table 22. Composition of glucose medium**

| Group A | (Final concentration) |
|---|---|
| Glucose | 80 g/L |
| MgSO₄.7aq | 2.0 g/L |

| Group B | |
|---|---|
| (NH₄)₂SO₄ | 2.0 g/L |
| KH₂PO₄ | 2.0 g/L |
| FeSO₄.7aq | 20 mg/L |
| MnSO₄.5aq | 20 mg/L |
| Yeast Extract | 4.0 g/L |

After preparing 0.15 L of Group A and 0.15 L of Group B, they were heated and sterilized at 115°C for 10 minutes. After cooling, Group A and Group B were mixed, and chloramphenicol (60 mg/L) was added thereto to use as the medium.

### (6-3) Method of inducing to isoprene-producing phase

Since *P. ananatis* isoprene-producing bacterial strain expresses a gene upstream of the mevalonate pathway with an arabinose-inducible promoter, a production amount of isoprene is remarkably improved in the presence of L-arabinose (Wako Pure Chemical Industries). For inducing to an isoprene-producing phase, broth in the fermenter was analyzed with time, and when absorbance at 600 nm became 16, L-arabinose at a final concentration of 20 mM was added.

### (6-4) Method of measuring isoprene gas concentration in fermentation gas

From immediately after starting the cultivation, evolved gas was collected in a 1 L gas bag with time, and a concentration of the isoprene gas contained in the evolved gas was measured using gas chromatography based on the condition described in Reference Example 4-3.

### (6-5) Amount of isoprene formed in jar cultivation

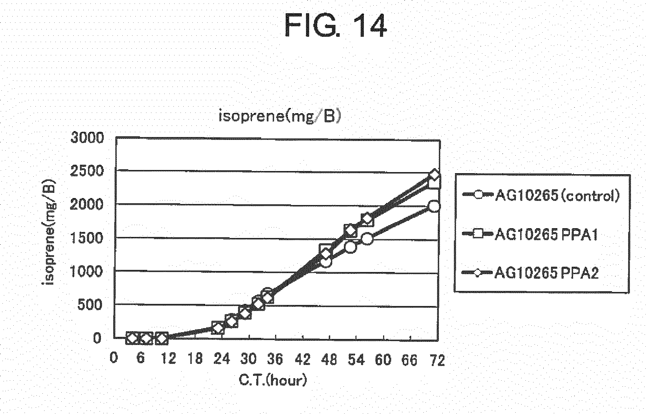

Microbial cells of AG10265 (control), AG10265 PPA1 and AG10265 PPA2 were cultured under the above jar cultivation condition, and an amount of formed isoprene was measured. Profiles of growth of the microbial cells and amounts of isoprene produced until 71 hours after starting the cultivation are shown in FIG. 13 and FIG. 14, respectively. The descending order of the production amount of total isoprene was AG10265 PPA2, AG10265 PPA1, then AG10265 Control (FIG. 3). The amounts of total isoprene produced by AG10265 PPA2, AG10265 PPA1, and AG10265 (Control) were 2478 mg, 2365 mg and 2013 mg, respectively. This has indicated that the *P*. *ananatis* isoprene-producing bacterial strain where the expression amount of pyrophosphate phosphatase was enhanced exhibited the more excellent ability to produce isoprene than *P. ananatis* isoprene-producing bacterial strain where the expression amount of pyrophosphate phosphatase was not enhanced.

### Reference Example 8: Preparation of SWITCH-PphosC-1(S) strain

### <1. Preparation of plasmid for upstream of MVA pathway for chromosome fixation >

A plasmid for upstream of MVA pathway for chromosome fixation, pAH162-PphoC-mvaES was constructed as follows.

### (1-1) Construction of arabinose-inducible plasmid for expressing mevalonate pathway upstream gene (mvaES [Enterococcus faecalis (E. faecalis)]) derived from E. faecalis, pMW-Para-mvaES-Ttrp

### (1-1-1) Chemical synthesis and cloning of mvaES (E. faecalis) gene

A nucleotide sequence (GenBank/EMBL/DDBJ accession ID AF290092.1) and an amino acid sequence (mvaS, GenPept accession ID AAG02438.1, mvaE, GenPept accession ID AAG02439.1) of the mvaES gene encoding the mevalonate pathway upstream gene (mvaES [*Enterococcus faecalis* (*E. faecalis*)]) derived from *E. faecalis* are known publicly (see Wilding, EI et al., J. Bacteriol. 182 (15), 4319-4327 (2000)). Based on these information, a mvaE gene and a mvaS gene in which their codon usages had been optimized for *E. coli* were designed and designated as EFmvaE and EF mvaS, respectively. Nucleotide sequences of EFmvaE and EFmvaS are shown in SEQ ID NO:139 and SEQ ID NO:140, respectively. DNA sequences of EFmvaE and EFmvaS prepared by chemical synthesis were cloned into a plasmid for expression pUC57 (supplied from GenScript), and designated as pUC57-EFmvaE and pUC57-EFmvaS, respectively. Nucleotide sequences of pUC57-EFmvaE and pUC57-EFmvaS are shown in SEQ ID NO:141 and SEQ ID NO:142, respectively.

### (1-1-2) Construction of plasmid pMW-P_{trc}-nwaES-Tₜᵣₚ used for In-Fusion method

A plasmid pMW-P_{trc}-mvaES-Tₜᵣₚ used for the In-fusion method was constructed according to the following procedure.

A plasmid pMW219 (supplied from Nippon Gene, Part number: 310-02571) was digested with *Sma*I, and this digested plasmid was purified. The resulting plasmid was designated as pMW219/SmaI.

In order to obtain a gene of a trc promoter (P_{trc}) region, PCR was carried out using a plasmid pTrcHis2B having the P_{trc} region as the template and using synthesized oligonucleotides consisting of the nucleotide sequences of SEQ ID NO:143 and SEQ IDNO:144 as primers.

In order to obtain a mvaE gene portion, PCR was carried out using the plasmid pUC57-EFmvaE as the template and using synthesized oligonucleotides consisting of the nucleotide sequences of SEQ ID NO:145 and SEQ IDNO:146 as the primers.

In order to obtain a mvaS gene portion, PCR was carried out using the plasmid pUC57-EFmvaS as the template and using synthesized oligonucleotides consisting of the nucleotide sequences of SEQ ID NO:146 and SEQ IDNO:147 as the primers.

In order to obtain a gene of a trp terminator (Tₜᵣₚ) region, PCR was carried out using a plasmid pSTV-P_{tac}-Tₜᵣₚ having the Tₜᵣₚ region as the template and using synthesized oligonucleotides consisting of the nucleotide sequences of SEQ ID NO:148 and SEQ IDNO:149 as the primers.

In the above four PCR cases, PrimeStar polymerase (supplied from TaKaRa Bio) was used as the enzyme, a reaction solution was prepared according to instructions provided by the supplier of the enzyme, and the reaction of a cycle of 98°C for 10 seconds, 55°C for 5 seconds and 72°C for 60 seconds/kb was repeated 30 times. As a result, the PCR product comprising the gene of the P_{trc} region, the mvaE gene, the mvaS gene and the gene of the Tₜᵣₚ region was obtained.

### (1-1-3) Construction of plasmid pMW-P_{trc}-mvaES-Tₜᵣₚ used for In-fusion method

Subsequently, PCR was carried out using the purified PCR product comprising P_{trc} and the purified PCR product comprising the mvaE gene as the template and using synthesized oligonucleotides consisting of SEQ ID NO:143 and SEQ IDNO:146 as the primers. Also PCR was carried out using the purified PCR product comprising the mvaS gene and the purified PCR product comprising Tₜᵣₚ as the template and using synthesized oligonucleotides consisting of SEQ ID NO:147 and SEQ IDNO:150 as the primers.

As a result, the PCR product comprising the gene of the P_{trc} region and the mvaE gene, and the PCR product comprising the mvaS gene and the gene of the Tₜᵣₚ region were obtained.

Subsequently, the PCR product comprising the gene of the P_{trc} region and the mvaE gene, the PCR product comprising the mvaS gene and the gene of the Tₜᵣₚ region, and the plasmid pMW219/SmaI digested above were ligated using In-Fusion HD Cloning Kit (supplied from Clontech). The resulting plasmid was designated as pMW-P_{trc}-mvaES-Tₜᵣₚ. A sequence of obtained pMW-P_{trc}-mvaES-Tₜᵣₚ is shown in SEQ ID NO:151.

### (1-1-4) Construction of plasmid pMW-Pₐᵣₐ-mvaES-Ttrp

The arabinose-inducible plasmid for expression of a mevalonate pathway upstream gene, pMW-Pₐᵣₐ-mvaES-Ttrp was constructed according to the following procedure.

PCR was carried out using the plasmid pMW-P_{trc}-mvaES-Tₜᵣₚ prepared in (1-1-3) as the template and using synthesized oligonucleotides consisting of the nucleotide sequences of SEQ ID NO:152 and SEQ IDNO:153 as the primers.

PCR was carried out using a plasmid pKD46 (see Proc. Natl. Acad. Sci. USA, 2000, vol.97, No.12, p6640-6645) comprising a gene of a P_{araC} region, an araC gene and a gene of a P_{araBAD} region (hereinafter also collectively referred to as a "gene of a Pₐᵣₐ region") as the template and using synthesized oligonucleotides consisting of the nucleotide sequences of SEQ ID NO:154 and SEQ IDNO:155 as the primers.

As a result, the PCR product comprising the plasmid pMW and the mvaES gene and the PCR product comprising the gene of the Para region were obtained. Purified these PCR products were ligated using In-Fusion HD Cloning Kit (supplied from Clontech). The resulting arabinose-inducible plasmid for expression of the mevalonate pathway upstream gene derived from *E*. *faecalis* (mvaES (*E*. *faecalis*)) was designated as PMW-Pₐᵣₐ-mvaES-Ttrp. A nucleotide sequence of PMW-Pₐᵣₐ-mvaES-Ttrp is shown in SEQ ID NO:156.

### (1-2) Construction of integrative conditional replication plasmid possessing mevalonate pathway upstream

A vector pAH162-λattL-TcR-λattR (Minaeva NI et al., BMC Biotechnol. 2008; 8:63) was used in order to construct an integrative plasmid possessing an upstream gene and a downstream gene of the mevalonate pathway.

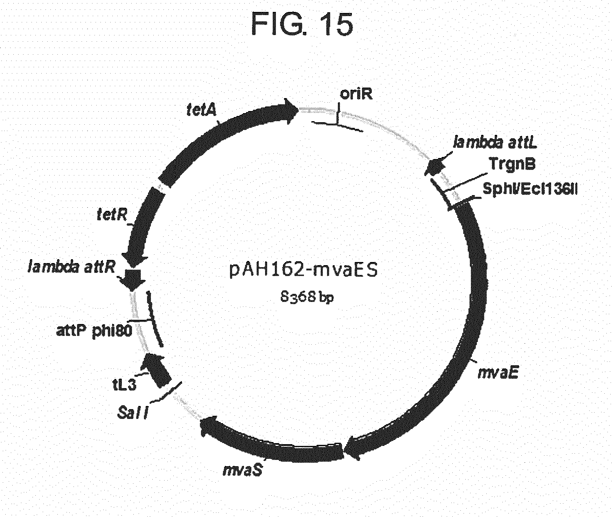

In order to obtain a promoter deletion mutant of an operon, an Ecl136II-SalI fragment of PMW-Para-mvaES-Ttrp was subcloned into the vector pAH162-λattL-TcR-λattR. A map of the resulting plasmid is shown in FIG. 15.

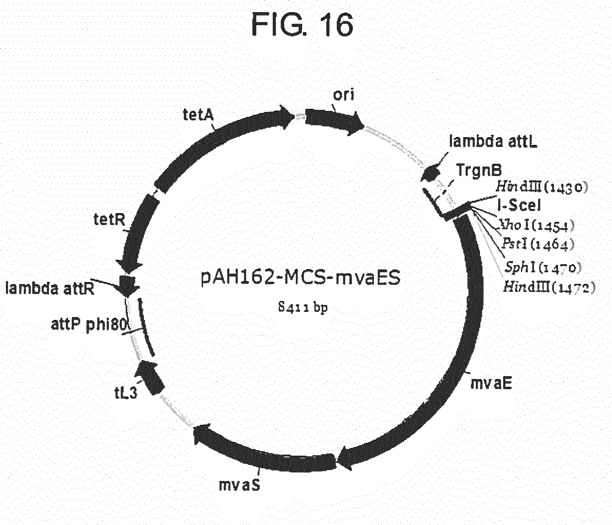

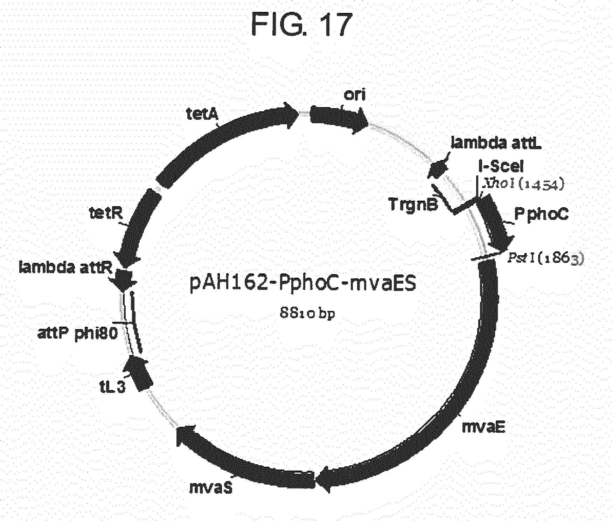

A set of plasmids for chromosome fixation holding the mvaES gene under the control of a different promoter was constructed. For this purpose, a polylinker comprising recognition sites for I-*Sce*I, *Xho*I, *Pst*I and *Sph*I was inserted into only one recognition site for *Hind*III located upstream of the mvaES gene. In order to accomplish this purpose, annealing was carried out using synthesized oligonucleotides consisting of the nucleotide sequences of SEQ ID NO:157 and SEQ ID NO:158 and polynucleotide kinase, and the resulting fragment was inserted into a plasmid pAH162-mvaES cleaved with *Hind*III by a ligation reaction. The resulting plasmid pAH162-MCS-mvaES (FIG. 16) is convenient for cloning a promoter while keeping a desired orientation before the mvaES gene. A DNA fragment holding a regulatory region of a phoC gene was formed by PCR using Genomic DNA from *P*. *ananatis* SC17(0) strain (Katashkina JI et al. BMC Mol Biol. 2009; 10:34) as the template and using oligonucleotides consisting of the nucleotide sequences of SEQ ID NO:159 and SEQ ID NO:160, and the resulting fragment was cloned into a recognition site for an appropriate restriction enzyme in pAH162-MCS-mvaES. The resulting plasmid is shown in FIG. 17. The cloned promoter fragment was sequenced, and confirmed to precisely correspond to a predicted nucleotide sequence. The resulting plasmid was designated as pAH162-PphoC-mvaES.

### <2. Preparation of plasmid for MVA pathway downstream for chromosome fixation>

An integrative plasmid pAH162-Km-Ptac-KDyI was constructed as follows, as a plasmid for MVA pathway downstream for chromosome fixation.

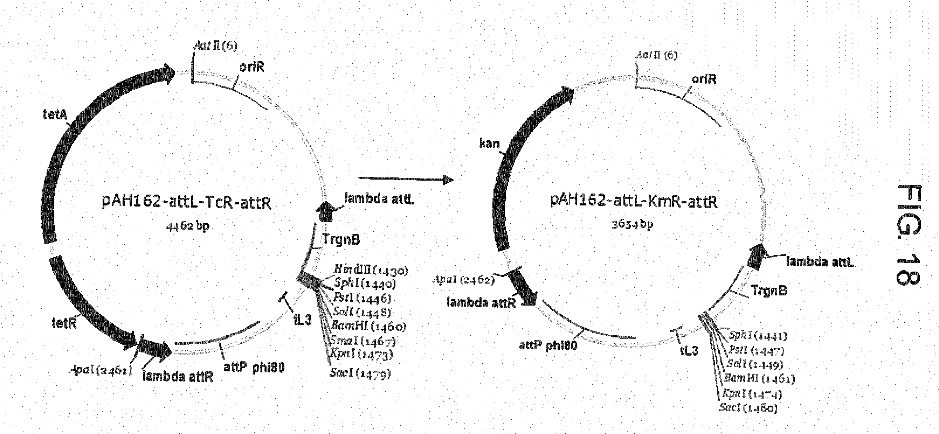

An AatII-ApaI fragment of pAH162-λattL-Tc^{R}-λattR (Minaeva NI et al. BMC Biotechnol. 2008; 8:63) comprising a tetAR gene was substituted with a DNA fragment obtained by PCR using synthesized oligonucleotides consisting of the nucleotide sequences of SEQ ID NO:161 (primer 11) and SEQ IDNO:162 (primer 12) as the primers and using a plasmid pUC4K (Taylor LA and Rose RE. Nucleic Acids Res. 16, 358, 1988) as the template. As a result, pAH162-λattL-Km^{R}-λattR was obtained (FIG. 18).

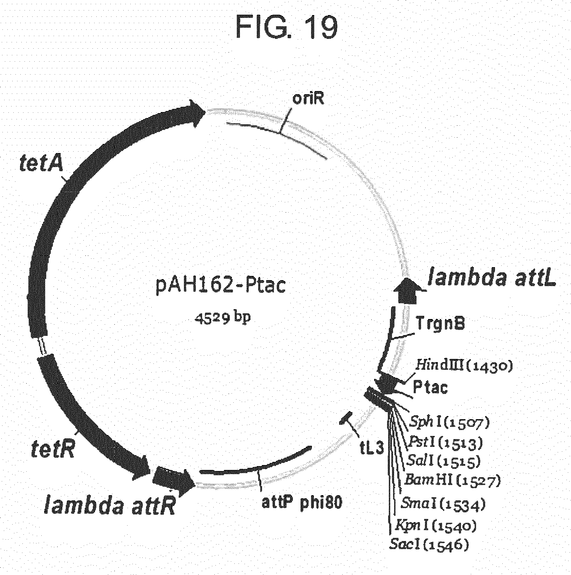

The P_{tac} promoter was inserted into a recognition site for *Hind*III-*Sph*I in the integrative vector pAH162-λattL-Tc^{R}-λattR (Minaeva NI et al. BMC Biotechnol. 2008; 8:63). As a result, an integrative vector pAH162-P_{tac} was constructed. The cloned promoter fragment was sequenced. A map of pAH162-P_{tac} is shown in FIG. 19.

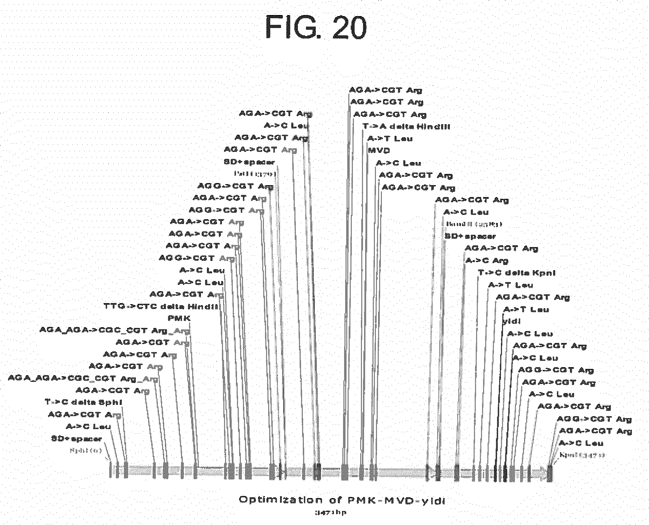

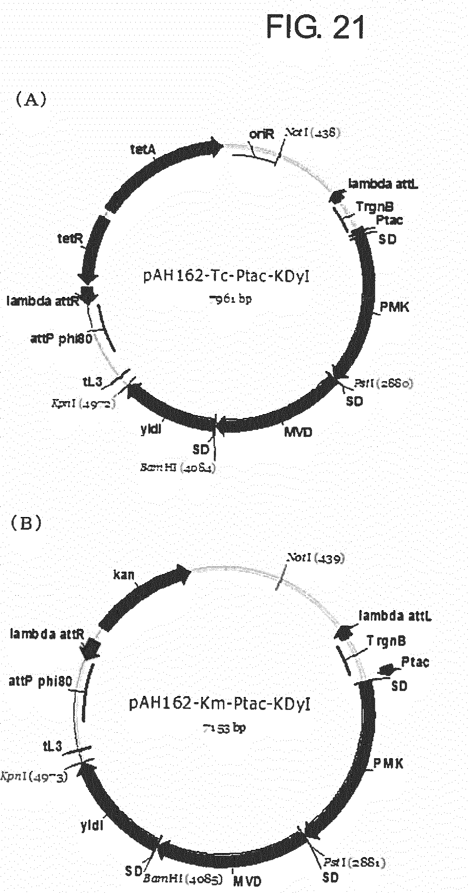

A DNA fragment chemically synthesized by ATG Service Gene (Russia) and holding the PMK gene, the MVD gene and the yldI gene derived from *S. cerevisiae* having substituted rare codons (FIG. 20) was subcloned into a recognition site for *Sph*I-KpnI restriction endonuclease in the integrative plasmid pAH162-Ptac. A chemically synthesized DNA sequence comprising a KDyI operon is shown in SEQ ID NO:184. The resulting plasmid pAH162-Tc-Ptac-KDyI holding an expression cassette Ptac-KDyI is shown in FIG. 21A. Subsequently, a NotI-KpnI fragment of pAH162-Tc-Ptac-KDyI holding a tetAR gene was substituted with a corresponding fragment of pAH162-λattL-KmR-λattR. As a result, a plasmid pAH162-Km-Ptac-KDyI having a kanamycin resistant gene kan as a marker was obtained (FIG. 21B).

### <3. Preparation of plasmid integrating MVA gene>

A chemically synthesized DNA fragment comprising a coding portion of a presumed mvk gene derived from SANAE [for complete genome sequence, see GenBank Accession Number AP011532] that was *Methanocella paludicola* and ligated to a classical SD sequence was cloned into a recognition site for PstI-KpnI in the above integrative expression vector pAH162-P_{tac}.

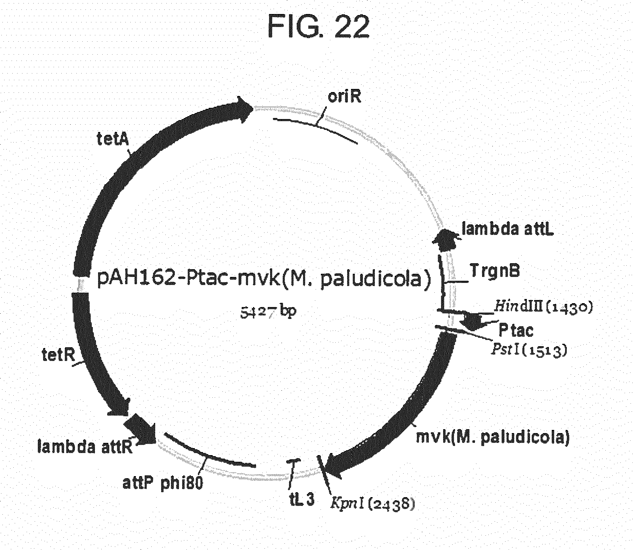

A map of the integrative plasmid holding the mvk gene is shown in FIG. 22.

### <4. Construction of recipient strain SC17(0) ΔampC: : attBₚₕᵢ₈₀ ΔampH: : attBₚₕᵢ₈₀ Δcrt: : P_{tac}-mvk (M. paludicola)>

Chromosomal modifications ΔampH: : attBₚₕᵢ₈₀ and ΔampC: : attBₚₕᵢ₈₀ were introduced into *P. ananatis* SC17(0) stepwise using two step technique comprising λRed dependent integration of a PCR-amplified DNA fragment comprising the gene kan flanking to attLₚₕᵢ₈₀ and attRₚₕᵢ₈₀ and a 40 bp sequence homologous to a target chromosome site (Katashkina JI et al. BMC Mol Biol. 2009; 10:34), followed by phage phi80 Int/Xis dependent cleavage of the kanamycin resistant marker (Andreeva IG et al. FEMS Microbiol Lett. 2011; 318(1):55-60). SC17(0) is a λRed resistant derivative of *P. ananatis* AJ13355 (Katashkina JI et al. BMC Mol Biol. 2009; 10:34); an annotated complete genome sequence of *P*. *ananatis* AJ13355 is available as PRJDA162073 or GenBank Accession Numbers AP012032.1 and AP012033.1. DNA fragments each used for the integration into ampH and ampC genes, respectively were formed by PCR using a plasmid pMWattphi [Minaeva NI et al. BMC Biotechnol. 2008; 8:63] as the template and using synthesized oligonucleotides consisting of the nucleotide sequences of SEQ ID NO:163 (primer 13) and SEQ ID NO:164 (primer 14) and synthesized oligonucleotides consisting of the nucleotide sequences of SEQ ID NO:165 (primer 15) and SEQ ID NO:166 (primer 16) as the primers. The resulting chromosomal modification was verified by PCR using synthesized oligonucleotides consisting of the nucleotide sequences of SEQ ID NO:167 (primer 17) and SEQ ID NO:168 (primer 18) and synthesized oligonucleotides consisting of the nucleotide sequences of SEQ ID NO:169 (primer 19) and SEQ ID NO:170 (primer 20) as the primers.

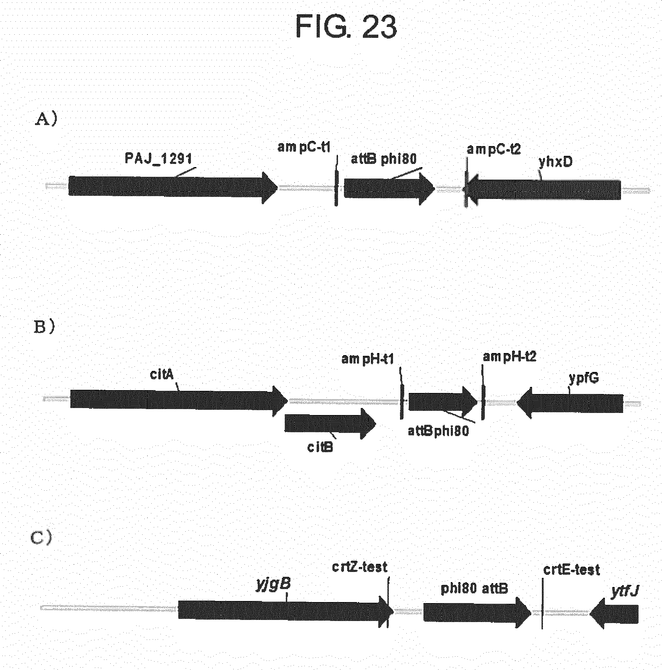

In parallel, a derivative of *P. ananatis* SC17(0) holding an attB site of the phage phi80 in place of an operon crt (positioned on a megaplasmid pEA320 320kb that is a portion of the genome of *P. ananatis* AJ13355) was constructed. In order to obtain this strain, the λRed dependent integration of a PCR-amplified DNA fragment holding attLₚₕᵢ₈₀-kan-attRₚₕᵢ₈₀ flanking to a 40 bp region homologous to a target site in the genome was carried out according to the previously described technique (Katashkina JI et al. BMC Mol Biol. 2009; 10:34). A DNA fragment used for substitution of the operon crt with attLₚₕᵢ₈₀-kan-attRₚₕᵢ₈₀ was amplified by PCR using synthesized oligonucleotides consisting of the nucleotide sequences of SEQ ID NO:171 (primer 21) and SEQ ID NO:172 (primer 22). The plasmid pMWattphi (Minaeva NI et al. BMC Biotechnol. 2008; 8:63) was used as the template in this reaction. The resulting integrant was designated as SC17(0)Δcrt::attLₚₕᵢ₈₀-kan-attRₚₕᵢ₈₀. A chromosomal structure of SC17 (0) Δcrt::attLphi80-kan-attRₚₕᵢ₈₀ was verified by PCR using synthesized oligonucleotides consisting of the nucleotide sequences of SEQ ID NO:173 (primer 23) and SEQ ID NO:174 (primer 24). The kanamycin resistant marker was removed from the constructed strain using the helper plasmid pAH129-cat according to the previously reported technique (Andreeva IG et al. FEMS Microbiol Lett. 2011; 318(1):55-60). The resulting strain SC17(0) Δcrt::attBₚₕᵢ₈₀ was verified by PCR using synthesized oligonucleotides consisting of the nucleotide sequences of SEQ ID NO:173 (primer 23) and SEQ ID NO:174 (primer 24). Maps of the resulting modified genomes ΔampC::attBₚₕᵢ₈₀, ΔampH::attBₚₕᵢ₈₀ and Δcrt::attBₚₕᵢ₈₀ are shown in FIGs. 23A, 7B and 7C, respectively.

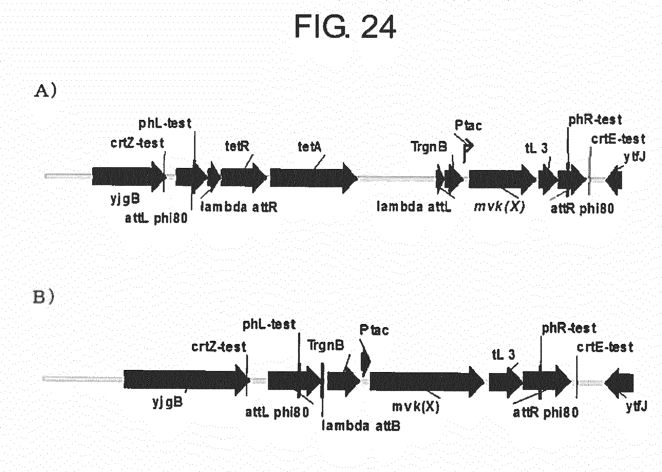

The above plasmid pAH162-Ptac-mvk (*M. paludicola*) was integrated into genome of SC17(0) Δcrt::attBₚₕᵢ₈₀ according to the previously reported protocol (Andreeva IG et al. FEMS Microbiol Lett. 2011; 318(1):55-60). The integration of the plasmid was confirmed by the polymerase chain reaction using synthesized oligonucleotides consisting of the nucleotide sequences of SEQ ID NO:171 (primer 21), SEQ ID NO:173 (primer 23), SEQ ID NO:172 (primer 22) and SEQ ID NO:174 (primer 24). As a result, a strain SC17(0)Δcrt::pAH162-P_{tac}-mvk (*M*. *paludicola*) was obtained. A map of the modified genome Δcrt::pAH162-P_{tac}-mvk (*M*. *paludicola*) is shown in FIG. 24A.

Subsequently, transfer from SC17(0)Δcrt::pAH162-P_{tac}-mvk (*M. paludicola*) toSC17(0) ΔampC::attBₚₕᵢ₈₀ ΔampH::attBₚₕᵢ₈₀ was carried out via the electroporation of genomic DNA (Katashkina JI et al. BMC Mol Biol. 2009; 10: 34). The resulting strain was cured from a vector portion of the integrative plasmid pAH162-Ptac-mvk (*M. paludicola*) using the previously reported helper plasmid pMW-intxis-cat (Katashkina JI et al. BMC Mol Biol. 2009; 10: 34). As a result, the marker-deleted strain SC17(0) ΔampH::attB_{ϕ80} ΔampC::attB_{ϕ80} Δcrt::P_{tac}-mvk (*M. paludicola*) was obtained. A map of the modified genome Δcrt::P_{tac}-mvk (*M*. *paludicola*) is shown in FIG. 24B.

### <5. Construction of strain SWITCH-PphoC>

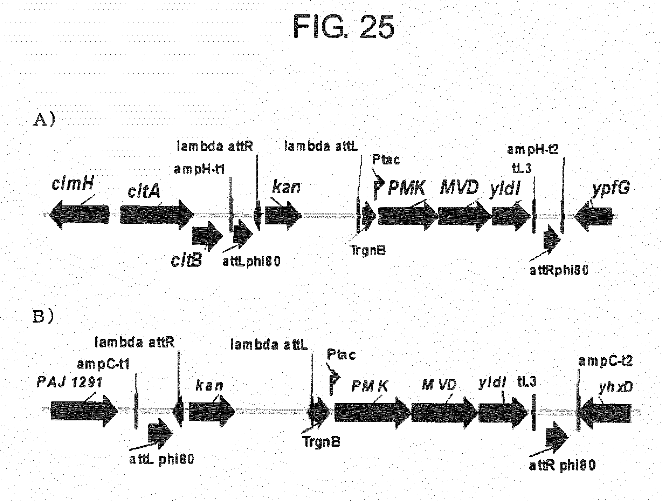

The plasmid pAH162-Km-Ptac-KDyI was integrated into a chromosome of the strain SC17(0) ΔampH::attB_{ϕ80} ΔampC::attB_{ϕ80} Δcrt::P_{tac}-mvk (*M. paludicola*) /pAH123-cat according to the previously reported protocol (Andreeva IG et al. FEMS Microbiol Lett. 2011; 318(1): 55-60). After electrophoresis, cells were seeded on the LB agar containing 50 mg/L of kanamycin. Grown Km^{R} clones were tested by the polymerase chain reactions using synthesized oligonucleotides consisting of the nucleotide sequences of SEQ ID NO:163 (primer 13) and SEQ ID NO:167 (primer 17), and SEQ ID NO:163 (primer 13) and SEQ ID NO:169 (primer 19) as the primers. A strain holding the plasmid pAH162-Km-Ptac-KDyI integrated into ΔampC::attB_{ϕ80} or pC:: attB_{ϕ80}m was selected. Maps of the modified chromosomals ΔampH:: pAH162-Km-Ptac-KDyI and ΔampC:: pAH162-Km-Ptac-KDyI are shown in FIG. 25 (A and B).

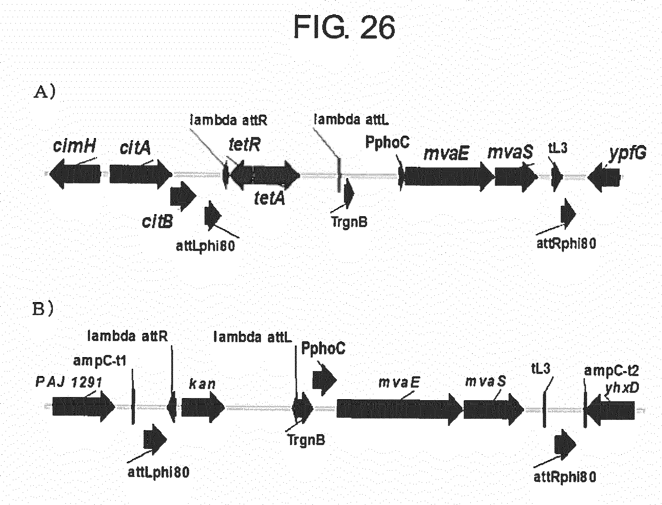

pAH162-PphoC-mvaES was inserted into a chromosome of recipient strains SC17(0) ΔampC::pAH162-Km-P_{tac}-KDyI ΔampH: :attBₚₕᵢ₈₀ Δcrt: :P_{tac}-mvk (*M*. *paludicola*) and SC17(0) ΔampC::attBₚₕᵢ₈₀ ΔampH::pAH162-Km-P_{tac}-KDyI Δcrt::P_{tac}-mvk (*M*. *paludicola*) using the helper plasmid pAH123-cat according to the previously reported protocol (Andreeva IG et al. FEMS Microbiol Lett. 2011; 318(1): 55-60). As a result, two sets of strains designated asSWITCH-PphoC-1 and SWITCH-PphoC-2 were obtained. Maps of the modified chromosomes ΔampH::pAH162-PphoC-mvaES and ΔampC::pAH162-PphoC-mvaES are shown in FIG. 26.

The tetracycline resistant marker and the kanamycin resistant marker were removed from SWITCH-PphoC-1 by the phage phi80 Int/Xis dependent removal according to the previously reported technique (Katashkina JI et al. BMC Mol. Biol. 2009; 10: 34). The resulting strain was designated as strain SWITCH-PphoC-1(S).

### Example 7: Preparation of Pantoea strain in which ppa gene of E. coli MG1655 (b4226) is expressed at high level in strain SWITCH-PphoC-1(S) and production of isoprene by cultivation using the same

A strain in which the expression of the ppa gene (pyrophosphate phosphatase gene) of *E. coli* MG1655 (b4226) was augmented by a strong promoter in *P. ananatis* strain was made by the following procedure.

### (7-1) Construction of pAH162-Ptac-MG-ppa

The ppa gene (b4226) was isolated by PCR using genomic DNA isolated from *E. coli* strain MG1655 as the template and using synthesized oligonucleotides consisting of the nucleotide sequences of SEQ ID NO:175 and SEQ ID NO:176 as the primers. The ppa gene isolated by PCR was introduced into a site cleaved with the restriction enzyme *Sma*I in the vector pSTV28-Ptac-Ttrp (Reference Example 3) using the in-fusion method to construct a plasmid pSTV28-Ptac-MG-ppa-Ttrp. Then, a region comprising the Tac promoter, the ppa gene and the Trp terminator was isolated using this plasmid as the template and using synthesized oligonucleotides consisting of the nucleotide sequences of SEQ ID NO:177 and SEQ ID NO:178 as the primers, and introduced into a site cleaved with the restriction enzymes BamHI and EcoRI in the vector pAH162-Km-attLR using In-Fusion HD Cloning Kit (supplied from Clontech) to construct a plasmid pAH162-Ptac-MG-ppa.

### (7-2) Construction of isoprene-producing strain in which expression of ppa gene derived from E. coli MG1655 is enhanced and construction of control strain

First, a derivative of *P. ananatis* SC17(0) possessing the attB site of phi80 that substituted a ydcI gene (PAJ_1320, Hara Y et al., The complete genome sequence of Pantoea ananatis AJ13355, an organism with great biotechnological potential. Appl. Microbiol. Biotechnol. 2012 Jan;93(1):331-41) was constructed. In order to obtain this strain, a PCR-amplified DNA fragment possessing attLphi80-kan-attRphi80 was obtained at a site flanking to a 40 bp region homologous to a ydcI target site in the genome using synthesized oligonucleotides consisting of the nucleotide sequences of SEQ ID NO:179 and SEQ ID NO:180 as the primers and using the genome of *P. ananatis* SC17(0) as the template. Subsequently, the λRed dependent integration of the DNA fragment was carried out according to previously reported technique (Katashkina JI et al., BMC Mol Biol. 2009; 10: 34). After the electroporation, cells were cultured on the LB agar containing 50 mg/L of kanamycin. The substitution of the ydcI gene with attLphi80-kan-attRphi80 was confirmed using the primers consisting of the nucleotide sequences of SEQ ID NO:181 and SEQ ID NO:182. Then, a strain SC17(0)ΔydcI::Ptac-MG-ppa having the integrated plasmid pAH162-Ptac-MG-ppa using the helper plasmid pAH123 cat was made according to the previously reported technique (Andreeva IG et al., FEMS Microbiol Lett. 2011; 318(1): 55-60). Genomic DNA isolated from this strain was electroporated into the strain SWITCH-PphoC-1(S) according to the previously reported method of chromosome electroporation (Katashkina JI et al., BMC Mol Biol. 2009; 10: 34). That the modified genome ΔydcI::Ptac-MG-ppa had been integrated into the chromosome of the strain SWITCH-PphoC-1(S) was verified by PCR using the primers consisting of the nucleotide sequences of SEQ ID NO:182 and SEQ ID NO:183. The strain SWITCH-PphoC-1(S)ΔydcI::Ptac-MG-ppa that was the isoprene-producing strain in which the ppa gene derived from *E. coli* MG1655 had been introduced was obtained in this way.

Then, genomic DNA was isolated from the *P. ananatis* SC17(0) derivative possessing the attB site of phi80 that substituted the ydcI gene, and electroporated into the strain SWITCH-PphoC-1(S) according to the electroporation method of chromosomes previously reported (Katashkina JI et al., BMC Mol Biol. 2009; 10: 34). The modified genome ΔydcI was verified by PCR using the primers consisting of the nucleotide sequences of SEQ ID NO:181 and SEQ ID NO:182, and then, an isoprene-producing strain SWITCH-PphosC-1(S) ΔydcI for control was chosen.

### (7-3) Confirmation of expression amount of pyrophosphate phosphatase

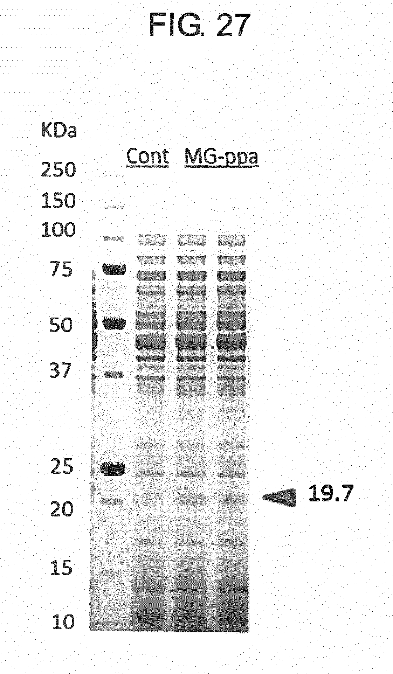

An expression amount of a protein of pyrophosphate phosphatase (PPA) in the strain SWITCH-PphoC-1(S)ΔydcI::Ptac-MG-ppa was confirmed on SDS-PAGE. Microbial cells from this strain were cultured with shaking in 3 mL of LB medium containing 50 mg/L of kanamycin at 30°C overnight. The microbial cells were collected, subsequently washed twice with ice-cooled 50 mM Tris buffer (Tris-HCl, pH 8.0), and disrupted using the multibead shocker (Yasui Kikai, Japan, 4°C, On for 60 seconds and OFF for 60 seconds, 2500 rpm, 5 cycles). A solution of the disrupted cells was centrifuged at 14,000 rpm for 20 minutes to remove cell debris. The resulting supernatant fraction was handled as a soluble protein fraction. The soluble protein fraction was quantified by the BCA method, and 5 µg of the soluble protein was electrophoresed on SDS-PAGE (supplied from Invitrogen, NuPAGE: SDS-PAGE Gel System). Subsequently, CBB staining and decoloration were carried out according to the standard methods. A photograph of a gel showing around the mass of the PPA protein was shown in FIG. 27. As a result, the increase of the expression amount of the protein presumed to be PPA was confirmed in the strain SWITCH-PphoC-1(S)ΔydcI::Ptac-MG-ppa.

### (7-4) Introduction of isoprene synthase-expressing plasmid

Electrocells of the strains SWITCH-PphoC-1(S) ΔydcI::Ptac-MG-ppa and SWITCH-PphoC-1(S) ΔydcI (control) were prepared according to the standard method, pSTV28-Ptac-ispSM (US2014113344A1) was introduced thereto, and the cells were evenly applied onto the LB plate containing 60 mg/L of chloramphenicol and cultured 37°C for 18 hours. Subsequently, transformants that exhibited resistance to chloramphenicol were obtained from the resulting plates. The resulting isoprene-producing bacterial strains were designated as SWITCH-PphoC-1(S)ydcI::MG-PPA/ispSM and SWITCH-PphoC-1(S)ΔydcI/ispSM (Control), respectively.

### (7-5) Measurement of isoprene synthase activity

Microbial cells of the strains SWITCH-PphoC-1(S)ydcI::MG-PPA/ispSM and SWITCH-PphoC-1(S)AydcI/ispSM (Control) were evenly applied onto each LB plate containing chloramphenicol, and cultured at 34°C for 16 to 24 hours. On loopful of the microbial cells from the resulting plate was inoculated to 1 mL of PS medium in a headspace vial (supplied from Perkin Elmer, 22mL CLEAR CRIMP TOP VIAL cat #B0104236), then tightly sealed with a cap for the headspace vial with butyl rubber septum (supplied from Perkin Elmer, CRIMPS cat#B0104240), and cultured in a reciprocal shaking cultivation apparatus (120 rpm) at 30°C for 48 hours.

Compositions of the PS medium are as described in Table 23.

**Table 23. Composition of PS medium**

| | |
|---|---|
| Glucose | 4 g/L |
| MgSO₄ 7H₂O | 1 g/L |
| (NH₄)₂SO₄ | 10 g/L |
| Yeast Extract | 50 mg/L |
| FeSO₄ 7H₂O | 5 mg/L |
| MnSO₄ 5H₂O | 5 mg/L |
| KH₂PO₄ | 10 mM |
| MES | 20 mM pH7.0 |

After termination of the cultivation, a concentration of isoprene in the headspace in the vial was measured by gas chromatography

**Table 24. Amounts of isoprene formed after cultivation for 48 hours.**

| Bacterial strain name | Amount of formed isoprene (mg/L) |
|---|---|
| SWITCH-PphoC-1(S)ΔydcI/ispSM (Control) | 80.2±7.4 |
| SWITCH-PphoC-1(S)ydcI::MG-PPA/ispSM | 128.0±5.9 |

From the result in Table 24, the strain SWITCH-PphoC-1(S)ydcI::MG-PPA/ispSM having the introduced ppa gene of *E*. *coli* MG1655 exhibited the higher activity of forming isoprene than the strain SWITCH-PphoC-1(S)AydcI/ispSM (Control).

### Example 8: Production of polyisoprene

Isoprene is collected with a trap cooled with liquid nitrogen by passing the fermentation exhaust. Collected isoprene is mixed with 35 g of hexane (Sigma-Aldrich) and 10 g of silica gel (Sigma-Aldrich, catalog No. 236772) and 10 g of alumina (Sigma-Aldrich, catalog No. 267740) under a nitrogen atmosphere in 100 mL glass vessel that is sufficiently dried. Resulting mixture is left at room temperature for 5 hours. Then supernatant liquid is skimmed and is added into 50 ml glass vessel that is sufficiently dried.

Meanwhile, in a glove box under a nitrogen atmosphere, 40.0 µmol of Tris[bis(trimethylsilyl)amido]gadolinium, 150.0 µmol of tributylaluminium, 40.0 µmol of Bis(2-(diphenylphosphino)phenyl]amine, 40.0 µmol of triphenylcarbonium tetrakis(pentafluorophenyl)borate (Ph3CBC6F5)4) are provided in a glass container, which was dissolved into 5 mL of toluene (Sigma-Aldrich, catalog No. 245511), to thereby obtain a catalyst solution. After that, the catalyst solution is taken out from the glove box and added to the monomer solution, which is then subjected to polymerization at 50°C for 120 minutes.

After the polymerization, 1 mL of an isopropanol solution containing, by 5 mass %, 2,2'-methylene-bis(4-ethyl-6-t-butylphenol) (NS-5), is added to stop the reaction. Then, a large amount of methanol is further added to isolate the polymer, and the polymer is vacuum dried at 70°C to obtain a polymer.

### Example 9: Production of rubber compound

The rubber compositions formulated as shown in Table 25 are prepared, which are vulcanized at 145°C for 35 minutes.

**Table 25: Rubber compositions of Example 6**

| | Parts by mass |
|---|---|
| Polyisoprene | 100 |
| Stearic Acid | 2 |
| Carbon Black (HAF class) | 50 |
| Antioxidant (*1) | 1 |
| Zinc Oxide | 3 |
| Cure Accelerator (*2) | 0.5 |
| Sulfur | 1.5 |

| | |
|---|---|
| *1 N-(1,3-dimethylbutyl)-N'-p-phenylenediamine *2 N-cyclohexyl-2-benzothiazolesulfenamide | |

### INDUSTRIAL APPLICABILITY

The isoprene synthase-expressing microorganism of the present invention is useful for production of isoprene.

## Claims

1. An isoprene synthase-expressing microorganism that exhibits improved expression of pyrophosphate phosphatase,
wherein an expression unit comprising a polynucleotide encoding the isoprene synthase is introduced into the isoprene synthase-expressing microorganism, and
the expression of pyrophosphate phosphatase is improved by modifying a promoter region of a pyrophosphate phosphatase gene inherent to the isoprene synthase-expressing microorganism, or by transforming the isoprene synthase-expressing microorganism with a pyrophosphate phosphatase expression vector to introduce an expression unit comprising a polynucleotide encoding pyrophosphate phosphatase into the isoprene synthase-expressing microorganism.

2. The isoprene synthase-expressing microorganism according to claim 1, wherein said microorganism is a microorganism transformed with an expression vector for isoprene synthase.

3. The isoprene synthase-expressing microorganism according to claim 1 or 2, wherein pyrophosphate phosphatase is homologous to said microorganism.

4. The isoprene synthase-expressing microorganism according to claim 3, wherein expression of the pyrophosphate phosphatase is improved by modification of a promoter region of a pyrophosphate phosphatase gene inherent to said microorganism.

5. The isoprene synthase-expressing microorganism according to any one of claims 1 to 4, wherein the expression of the pyrophosphate phosphatase is improved by increased copy number of the pyrophosphate phosphatase gene on a chromosome.

6. The isoprene synthase-expressing microorganism according to any one of claims 1 to 5, wherein said microorganism is a microorganism belonging to *Enterobacteriaceae.*

7. The isoprene synthase-expressing microorganism according to any one of claims 1 to 6, wherein said microorganism has an ability to synthesize dimethylallyl diphosphate via a methylerythritol diphosphate pathway,
wherein said microorganism is preferably a bacterium belonging to genus *Escherichia,* more preferably *Escherichia coli.*

8. The isoprene synthase-expressing microorganism according to any one of claims 1 to 7, wherein said microorganism has an ability to synthesize dimethylallyl diphosphate via a mevalonate pathway.

9. The isoprene synthase-expressing microorganism according to any one of claims 1 to 8, wherein said microorganism is a bacterium belonging to genus *Pantoea.*

10. The isoprene synthase-expressing microorganism according to claim 9, wherein said bacterium belonging to genus *Pantoea* is *Pantoea ananatis.*

11. A method of producing an isoprene monomer, comprising producing the isoprene monomer by culturing the isoprene synthase-expressing microorganism according to any one of claims 1 to 10 in culture medium.

12. A method of producing an isoprene polymer, comprising
(I) producing an isoprene monomer by the method according to claim 11; and
(II) polymerizing the isoprene monomer to produce the isoprene polymer.

13. A method of producing a rubber composition, comprising:
(A) preparing an isoprene polymer by the method of claim 12; and
(B) mixing said isoprene polymer with one or more rubber composition components.

14. A method of producing a tire, comprising:
(i) producing a rubber composition by the method of claim 13; and
(ii) applying said rubber composition to manufacture a tire.

## Patentansprüche

1. Isoprensynthase-exprimierender Mikroorganismus, der eine verbesserte Expression von Pyrophosphatphosphatase aufweist,
wobei eine Expressionseinheit, umfassend ein die Isoprensynthase kodierendes Polynucleotid, in den Isoprensynthase-exprimierenden Mikroorganismus eingeführt wird und
die Expression von Pyrophosphatphosphatase durch Modifizieren einer Promotorregion eines dem Isoprensynthase-exprimierenden Mikroorganismus innewohnenden Pyrophosphatphosphatase-Gens oder durch Transformieren des Isoprensynthase-exprimierenden Mikroorganismus mit einem Pyrophosphatphosphatase-Expressionsvektor, um eine Expressionseinheit, die ein Pyrophosphatphosphatase kodierendes Polynucleotid umfasst, in den Isoprensynthase-exprimierenden Mikroorganismus einzuführen, verbessert wird.

2. Isoprensynthase-exprimierender Mikroorganismus gemäß Anspruch 1, wobei es sich bei dem Mikroorganismus um einen mit einem Expressionsvektor für Isoprensynthase transformierten Mikroorganismus handelt

3. Isoprensynthase-exprimierender Mikroorganismus gemäß Anspruch 1 oder 2, wobei die Pyrophosphatphosphatase zu dem Mikroorganismus homolog ist.

4. Isoprensynthase-exprimierender Mikroorganismus gemäß Anspruch 3, wobei die Expression der Pyrophosphatphosphatase durch Modifikation einer Promotorregion eines dem Mikroorganismus innewohnenden Pyrophosphatphosphatase-Gens verbessert ist.

5. Isoprensynthase-exprimierender Mikroorganismus gemäß einem der Ansprüche 1 bis 4, wobei die Expression der Pyrophosphatphosphatase durch eine erhöhte Kopienzahl des Pyrophosphatphosphatase-Gens auf einem Chromosom verbessert ist.

6. Isoprensynthase-exprimierender Mikroorganismus gemäß einem der Ansprüche 1 bis 5, wobei es sich bei dem Mikroorganismus um einen Mikroorganismus handelt, der zu den *Enterobacteriaceae* gehört.

7. Isoprensynthase-exprimierender Mikroorganismus gemäß einem der Ansprüche 1 bis 6, wobei der Mikroorganismus eine Fähigkeit aufweist, Dimethylallyldiphosphat über einen Methylerythritoldiphosphat-Weg zu synthetisieren,
wobei es sich bei dem Mikroorganismus vorzugsweise um ein Bakterium handelt, das zur Gattung *Escherichia* gehört, vorzugsweise um *Escherichia coli.*

8. Isoprensynthase-exprimierender Mikroorganismus gemäß einem der Ansprüche 1 bis 7, wobei der Mikroorganismus eine Fähigkeit aufweist, Dimethylallyldiphosphat über einen Mevalonat-Weg zu synthetisieren.

9. Isoprensynthase-exprimierender Mikroorganismus gemäß einem der Ansprüche 1 bis 8, wobei es sich bei dem Mikroorganismus um ein Bakterium handelt, das zur Gattung *Pantoea* gehört.

10. Isoprensynthase-exprimierender Mikroorganismus gemäß Anspruch 9, wobei es sich bei dem zur Gattung *Pantoea* gehörenden Bakterium um *Pantoea ananatis* handelt.

11. Verfahren zum Herstellen eines Isoprenmonomers, umfassend Herstellen des Isoprenmonomers durch Züchten des Isoprensynthase-exprimierenden Mikroorganismus gemäß einem der Ansprüche 1 bis 10 in Kulturmedium.

12. Verfahren zum Herstellen eines Isoprenpolymers, umfassend
(I) Herstellen eines Isoprenmonomers durch das Verfahren gemäß Anspruch 11 und
(II) Polymerisieren des Isoprenmonomers, um das Isoprenpolymer herzustellen.

13. Verfahren zum Herstellen einer Gummizusammensetzung, umfassend
(A) Herstellen eines Isoprenpolymers durch das Verfahren nach Anspruch 12 und
(B) Mischen des Isoprenpolymers mit einem oder mehreren Gummizusammensetzungs-Bestandteilen.

14. Verfahren zum Herstellen eines Reifens, umfassend
(i) Herstellen einer Gummizusammensetzung durch das Verfahren nach Anspruch 13 und
(ii) Anwenden der Gummizusammensetzung, um einen Reifen herzustellen.

## Revendications

1. Micro-organisme exprimant une isoprène synthase qui présente une expression améliorée de la pyrophosphate phosphatase,
où une unité d'expression comprenant un polynucléotide codant pour l'isoprène synthase est introduite dans le micro-organisme exprimant une isoprène synthase, et
l'expression de pyrophosphate phosphatase est améliorée en modifiant une région promotrice d'un gène de pyrophosphate phosphatase inhérent au micro-organisme exprimant une isoprène synthase, ou en transformant le micro-organisme exprimant une isoprène synthase avec un vecteur d'expression de pyrophosphate phosphatase afin d'introduire une unité d'expression comprenant un polynucléotide codant pour la pyrophosphate phosphatase dans le micro-organisme exprimant une isoprène synthase.

2. Micro-organisme exprimant une isoprène synthase selon la revendication 1, ledit micro-organisme étant un micro-organisme transformé avec un vecteur d'expression pour l'isoprène synthase.

3. Micro-organisme exprimant une isoprène synthase selon la revendication 1 ou 2, la pyrophosphate phosphatase étant homologue audit micro-organisme.

4. Micro-organisme exprimant une isoprène synthase selon la revendication 3, l'expression de la pyrophosphate phosphatase étant améliorée par modification d'une région promotrice d'un gène de pyrophosphate phosphatase inhérent audit micro-organisme.

5. Micro-organisme exprimant une isoprène synthase selon l'une quelconque des revendications 1 à 4, l'expression de pyrophosphate phosphatase étant améliorée par augmentation du nombre d'exemplaires du gène de pyrophosphate phosphatase sur un chromosome.

6. Micro-organisme exprimant une isoprène synthase selon l'une quelconque des revendications 1 à 5, ledit micro-organisme étant un micro-organisme appartenant à *Enterobacteriaceae.*

7. Micro-organisme exprimant une isoprène synthase selon l'une quelconque des revendications 1 à 6, ledit micro-organisme présentant une aptitude à synthétiser le diphosphate de diméthylallyle par une voie utilisant le diphosphate de méthylérythritol,
ledit micro-organisme étant de préférence une bactérie appartenant au genre *Escherichia,* plus préférablement *Escherichia coli.*

8. Micro-organisme exprimant une isoprène synthase selon l'une quelconque des revendications 1 à 7, ledit micro-organisme présentant une aptitude à synthétiser le diphosphate de diméthylallyle par une voie utilisant le mévalonate.

9. Micro-organisme exprimant une isoprène synthase selon l'une quelconque des revendications 1 à 8, ledit micro-organisme étant une bactérie appartenant au genre *Pantoea.*

10. Micro-organisme exprimant une isoprène synthase selon la revendication 9, ladite bactérie appartenant au genre *Pantoea* étant *Pantoea ananatis.*

11. Procédé de production d'un monomère d'isoprène, comprenant la production du monomère d'isoprène par culture du micro-organisme exprimant une isoprène synthase selon l'une quelconque des revendications 1 à 10 dans un milieu de culture.

12. Procédé de production d'un monomère d'isoprène, comprenant
(I) la production d'un monomère d'isoprène par le procédé selon la revendication 11 ; et
(II) la polymérisation du monomère d'isoprène pour produire le polymère d'isoprène.

13. Procédé de production d'une composition de caoutchouc, comprenant :
(A) la préparation d'un polymère d'isoprène par le procédé selon la revendication 12 ; et
(B) le mélange dudit polymère d'isoprène avec un ou plusieurs constituants de composition de caoutchouc.

14. Procédé de production d'un pneumatique, comprenant :
(i) la production d'une composition de caoutchouc par le procédé selon la revendication 13 ; et
(ii) l'emploi de ladite composition de caoutchouc pour fabriquer un pneumatique.
